# EUROPEAN PATENT APPLICATION

(11) **EP 3 906 929 A1**
(43) Date of publication of application: **10.11.2021**
(21) Application number: 20173674.1
(22) Date of filing: 08.05.2020
(51) Int. Cl.: A61K 31/517, A61K 39/275, A61K 48/00, A61K 45/06, A61P 31/14, A61K 39/215

(54) **LETERMOVIR FOR USE IN THE PREVENTION AND THE TREATMENT OF CORONAVIRUS INFECTIONS**

(71) Applicant: AiCuris GmbH & Co. KG, 42117 Wuppertal (DE)
(72) Inventor: BUSCHMANN, Helmut, 52076 Aachen (DE); ANDREES, Lars Christian, 42281 Wuppertal (DE)
(74) Representative: Gaentzsch, Peer Christian

(57) **Abstract**

The present invention relates to the treatment of coronavirus infections and to preparing subjects for such an infection by administering antivirals including parapoxvirus and Letermovir. This treatment is to assist the immune system in combatting the virus and to thereby prevent and to ameliorate symptoms of coronavirus disease.

## Description

### FIELD OF THE INVENTION

The present invention relates to the treatment of coronavirus infections and to preparing subjects for such an infection by administering antivirals including parapoxvirus and Letermovir. This treatment is to assist the immune system in combatting the virus and to thereby prevent and to ameliorate symptoms of coronavirus disease.

### BACKGROUND OF THE INVENTION

Coronaviruses are enveloped, positive-sense single-stranded RNA viruses, which cause diseases in mammals and birds. In humans, they cause respiratory infections, including the common cold, although some viruses such as SARS and MERS coronaviruses can be lethal. Novel coronaviruses emerge periodically in different areas around the world. SARS-CoV (severe acute respiratory syndrome coronavirus) occurred in 2002 and caused 916 deaths during the epidemic, having a mortality rate of about 12%. MERS-CoV (middle east respiratory syndrome coronavirus) emerged in 2012, with 543 dead reported and a mortality rate of about 39%.

Recently, an outbreak of a coronavirus designated SARS-CoV-2 began in Wuhan, China, and has since spread all over the world. The illness caused by SARS-CoV-2, COVID-19 (coronavirus disease 2019) was declared to be a pandemic by the WHO on March 11, 2020. COVID-19 ranges in severity from asymptomatic or mild to severe, and a significant portion of patients with clinically evident infection develop severe disease. The case fatality rate varies by country and is thought to be between 2% and 3%, but the true mortality rate is uncertain, because the total number of cases is unknown. While SARS-CoV-2 apparently has a lower mortality rate than SARS-CoV and MERS-CoV, it appears to be more infectious and has been able spread around the world, with around 3.66 million confirmed cases and 257,000 confirmed deaths by May 6, 2020 and presumably many more unconfirmed ones. The knowledge of COVID-19 is incomplete and evolving.

The recurrent coronavirus epidemics show that these viruses continue to threaten the world as they emerge spontaneously, spread easily and can have severe consequences on human life, including not only health issues but also dramatic social and economic impacts. Therefore, there is a great medical need for prevention and treatment. However, since coronaviruses are known to mutate and recombine often, they present an ongoing challenge for disease control. Neither vaccines nor antiviral drugs for preventing or treating human coronavirus infections are currently available. A vast number of antivirals are being tested around the world for an effect on coronavirus, with promising candidates failing.

The inventors identified agents not expectable to be useful for conditioning subjects for and treating coronavirus infections. Parapoxvirus ovis (PPVO) causes acute dermal infections in goat and sheep, and while it leads to no serious disease in humans, it induces a complex innate and adaptive immune response in humans and treats infections with some specific viruses such as HBV (WO 2019/048640 A1). However, the immune response induced by parapoxvirus is complex and not necessarily useful for treating viral infections in general. Letermovir blocks HCMV replication via a mode of action that involves the viral terminase and was believed to be active solely against HCMV (WO 2014/202737 A1). Therefore, neither parapoxvirus nor Letermovir could have been expected to have utility in conditioning subjects for and treating coronavirus infections.

### SUMMARY OF THE INVENTION

In a first aspect, the present invention relates to Letermovir, or preferably a combination of a parapoxvirus agent and Letermovir, for use in (i) conditioning a subject for a coronavirus infection or (ii) treating a coronavirus infection in a subject. In a second aspect, the invention relates to a composition or a kit comprising Letermovir, or preferably a parapoxvirus agent and Letermovir, as well as a coronavirus agent. In a third aspect, the present invention relates to a pharmaceutical composition or a kit comprising Letermovir, or preferably a parapoxvirus agent and Letermovir, for the medical use according to the invention, wherein the pharmaceutical composition or the kit comprises a Letermovir, or preferably a parapoxvirus agent and Letermovir, and preferably a further medicament.

### LEGENDS TO THE FIGURES

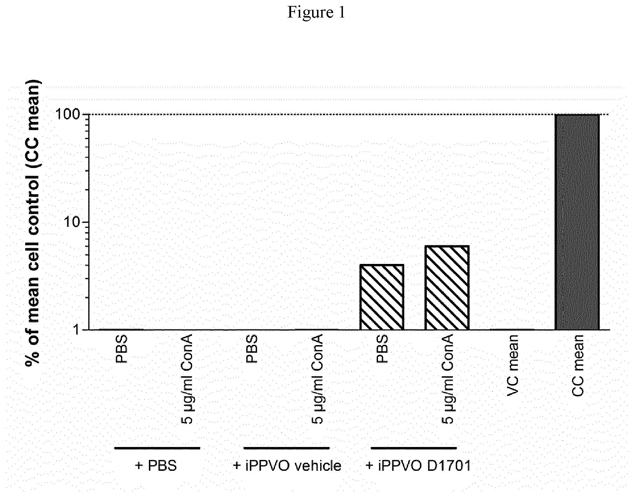

**Figure 1****: Stimulation of inactivated parapoxvirus (iPPVO) induced antiviral activity against SARS-CoV-2 that was enhanced by combinatorial approaches.** Whole-blood cultures were stimulated with iPPVO +/- 5 µg/ml ConA. Where no ConA was added, PBS was added instead. iPPVO vehicle +/- ConA or PBS +/- ConA, respectively, served as a controls. 5 µl/ml ConA was tested as a co-stimulus. Cultures were incubated for 3 days and supernatants were harvested. The supernatant was transferred to Vero-eGFP cells which were 2-4 hours later infected with SARS-CoV-2 to determine the antiviral activity of the respective supernatant samples and incubated for 5 days. eGFP fluorescence as a measure for living cells was analyzed using the ImageJ software. The mean of the cell control to which no virus was added served as positive control (CC mean) and was set to 100 % (see upper dotted line). Cells infected with SARS-CoV2 but not treated gave the highest possible destruction of living cells and served as negative control given as the mean virus control (VC mean). The antiviral activity of the samples were calculated relative to the positive control CC mean and given in %.

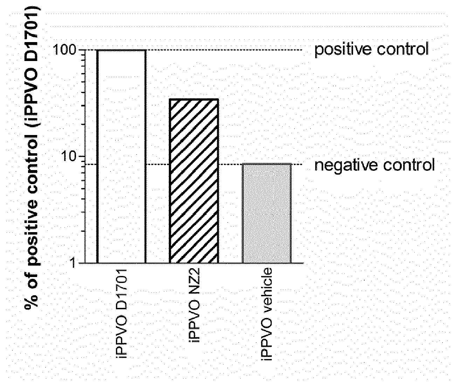

**Figure 2****: iPPVO D1701 and iPPVO NZ2 stimulate antiviral activity against SARS-CoV-2.** Whole-blood cultures were stimulated with iPPVO. iPPVO vehicle served as a negative control. iPPVO D1701 served as positive control. Cultures were incubated for 3 days and supernatants were harvested. The supernatant was transferred to Vero-eGFP cells which were 2-4 hours later infected with SARS-CoV-2 to determine the antiviral activity residing in the respective supernatant samples and incubated for 5 days. eGFP fluorescence as a measure for living cells which have been protected against SARS-CoV-2 due to the antiviral activity of cytokines in the respective supernatant was measured using the ImageJ software. The antiviral activity of the positive control was set to 100 % (see upper dotted line). The antiviral activity of the samples were calculated relative to the positive control and given in %. The lower dotted line marks the value of the negative control.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturers' specifications, instructions etc.), whether supra or infra, is hereby incorporated by reference in its entirety.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. In preferred embodiments, "comprise" can mean "consist of'. As used in this specification and the appended claims, the singular forms "a", "an", and "the" include plural referents, unless the content clearly dictates otherwise.

In a first aspect, the present invention relates to Letermovir or a pharmaceutically acceptable salt, solvate or hydrate thereof, or preferably a parapoxvirus agent and Letermovir or a pharmaceutically acceptable salt, solvate or hydrate thereof, for use in (i) conditioning a subject for a coronavirus infection or (ii) treating a coronavirus infection in a subject.

### Letermovir

The chemical name of Letermovir is (4S)-{8-Fluoro-2-[4-(3-methoxyphenyl)-1-piperazinyl]-3-[2-methoxy-5-(trifluoromethyl)phenyl]-3,4-dihydro-4-quinazolinyl}acetic acid. It has the Formula (I) as depicted below: 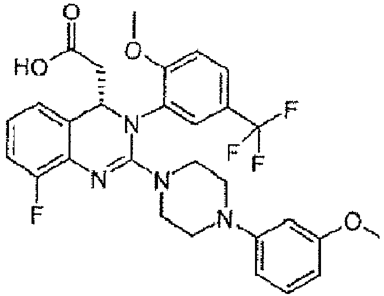 Formula (I); i.e. C₂₉H₂₈F₄N₄O₄.

The preparation of Letermovir is disclosed in US 2007/0191387 A1 and WO 2006/133822 A1. Letermovir may be formulated as a solution for administration (e.g. intravenously), but preferably it is formulated in an amorphous state, which is suitable for use in solid oral dosage forms (see WO 2014/202737 A1).

A pharmaceutically acceptable salt, solvate or hydrate of Letermovir can also be used in the context of the present invention. All references to Letermovir and its uses herein are to be understood such that pharmaceutically acceptable salt, solvate or hydrate of Letermovir can also be used.

### Parapoxvirus agent

The parapoxvirus is, in a preferred embodiment, *Parapoxvirus ovis* (PPVO). This includes any PPVO strain, preferably, any strain of the species PPVO as classified by the International Committee on Taxonomy of Viruses (ICTV). Exemplary strains are NZ2, NZ7, NZ10, D1701, OV/20, OV/7, OV/C2, OV/mi-90, OV-Torino, SAOO, Bo29, orf11, Greek orf strain 155, and Greek orf strain 176. Preferred strains are NZ2 and D1701, especially NZ2.

The parapoxvirus agent is, in a preferred embodiment,
(a) the parapoxvirus itself or a fragment thereof, or
(b) an agent that comprises genetic information encoding for (a).

In a more preferred embodiment, it is
(i) a live parapoxvirus virion,
(ii) an inactivated parapoxvirus virion,
(iii) a fragment of (i) or (ii),
(iv) a nucleic acid encoding for any of (i) to (iii),
(v) a vector comprising the nucleic acid of (iv), or
(vi) a cell comprising the nucleic acid of (iv) or the vector of (v).

The live parapoxvirus virion may or may not be attenuated. Attenuated parapoxvirus virions are known in the art, e.g. virions lacking a virulence gene such as vegf-e and/or gif. The fragment can be recognized by the immune system and preferably stimulates an immune response (i.e. it is an antigen and preferably an immunogen) and can be any fragment, although preferably it is a fragment that is bound by a human pattern recognition receptor (PRR), e.g. TLR9. In one embodiment, the parapoxvirus fragment is a parapoxvirus protein.

Exemplary fragments are (exemplary coding sequence (nucleotide positions of SEQ ID NO: 3 representing strain NZ2) in parenthesis preceding the fragment): (3 to 539) ORF L1, (781 to 449) ORF L2r, (1933 to 1664) ORF L3r, (3269 to 2790) ORF L4r, (2799 to 3851) ORF L5, (2962 to 3753) ORF L6, (3784 to 3122) ORF L7r, (4341 to 4129) ORF L8r, (4904 to 4428) ORF 1ar, (6517 to 4970) ORF 1r, (8042 to 6684) ORF 2r, (9989 to 8070) ORF 3r, (11195 to 10062) ORF 4r, (11493 to 11227) ORF 5r, (11802 to 12038) ORF 6, (12358 to 12080) ORF 7r, (13980 to 12364) ORF 8r, (14826 to 14053) ORF 9ar, (15080 to 15394) ORF 10, (16838 to 15423) ORF 11r, (19021 to 16847) ORF 12r, (19704 to 19156) ORF 13r, (20314 to 19736) ORF 14r, (20401 to 22101) ORF 15, (22125 to 22940) ORF 6, (23003 to 23866) ORF 17, (26908 to 23873) ORF 18r, (26926 to 27213) ORF 19, (27626 to 27216) ORF 20r, (29754 to 27616) ORF 21r, (32217 to 29800) ORF 22r, (33380 to 32418) ORF 23r, (33602 to 33393 ORF 24r, (34466 to 33612) ORF 25r, (34735 to 34502) ORF 26r, (35905 to 34739) ORF 27r, (37194 to 35905) ORF 28r, (37200 to 39248) ORF 29; 41037 to 39229) ORF 30r, (41374 to 42066) ORF 31, (42336 to 41731) ORF 32r, (42407 to 41997) ORF 33r, (42410 to 43765) ORF 34, (43770 to 43958) ORF 35, (43980 to 44534) ORF 36, (45727 to 44537) ORF 37r, (45760 to 46557) ORF 38, (46567 to 47568) ORF 39, (47572 to 48303) ORF 40, (48352 to 48621) ORF 41, (49887 to 48634) ORF 42r, (49917 to 50793) ORF 43, (50719 to 51102) ORF 44, (51059 to 51511) ORF 44a, (51584 to 52591) ORF 45, (52509 to 53066) ORF 46, (53523 to 53023) ORF 47r, (53607 to 57473) ORF 48, (58070 to 57528) ORF 49r, (57700 to 58662) ORF 50, (59674 to 58673) ORF 51r, (62089 to 59678) ORF 52r, (62198 to 62881) ORF 53, (62909 to 63862) ORF 55, (63858 to 64271) ORF 56, (64309 to 66831) ORF 57, (67266 to 66799) ORF 58r, (67803 to 67273) ORF 58ar, (67915 to 68607) ORF 59, (68624 to 70984) ORF 60, (70994 to 72898) ORF 61, (72938 to 73507) ORF 62, (73540 to 74211) ORF 63, (76120 to 74207) ORF 64r, (76749 to 76186) ORF 65r, (77698 to 76799) ORF 66r, (79343 to 77709) ORF 67r, (79816 to 79367) ORF 68r, (80529 to 79858) ORF 69r, (80774 to 80529 ORF 70r, (82815 to 80788) ORF 71r, (83835 to 82834) ORF 72r, (83874 to 85583) ORF 73, (85535 to 84402) ORF 74r, (88096 to 85574) ORF 75r, (87759 to 88667) ORF 76, (88920 to 88642) ORF 77r, (91652 to 88938) ORF 78r, (91667 to 92674) ORF 79, (93466 to 92681) ORF 80r, (93761 to 93486) ORF 81r, (94060 to 93788) ORF 82r, (94238 to 94080) ORF 83r, (94508 to 94242) ORF 84r, (95571 to 94498) ORF 85r, (96187 to 95600) ORF 86r, (96202 to 97665) ORF 87, (97915 to 97643) ORF 88r, (98251 to 99537) ORF 89, (99537 to 99974) ORF 90, (100001 to 101140) ORF 91, (101168 to 104650 ORF 92, (106354 to 104795) ORF 93r, (107947 to 106400) ORF 94r, (108256 to 107990) ORF 95r, (108719 to 108300) ORF 96r, (109679 to 108738) ORF 97r, (109861 to 109682) ORF 98r, (110830 to 10033) ORF 99r, (110208 to 110417) ORF 100, (110469 to 110651) ORF 100a, (110915 to 111397) ORF 101, (111419 to 111913 ORF 102, (111949 to 112485) ORF 103, (112593 to 113450) ORF 104, (113323 to 112967) ORF I05r, (113526 to 114152) ORF 106, (114199- to 115236) ORF 107, (115353 to 115787 ORF 108, (115859 o 116551) ORF 109, (116729 to 117523) ORF 110, (117572 to 117114) ORF 111r, (117423 to 118085) ORF 12, (118968 to 118375) ORF 114r, (118508 to 119119) ORF' 115, (119588 to 120202) ORF 116, (120314 to 21231) ORF 117, (121380 to 123920) ORF 118, (121288 to 122256) ORF 119, (122350 to 123924) ORF 120, (123962 to 125566) ORF 121, (125193 to 124591) ORF 122r, (125689 to 123935 ORF 123r, (123839 to 123297) ORF 123ar, (125652 to 126170) ORF 124, (126121 to 125699) ORF 125r, (126279 to 127769) ORF 126, (127851 to 128408) ORF 127, (128520 to 130076) ORF 128, (130105 to 131700) ORF 129, (131790 to 133283) ORF 130, (133246 to 133920) ORF 131, (133972 to 134370) ORF 132, (134418 to 134693) ORF 133a, (134402 to 134992) ORF R1, (134853 to 134419) ORF R2r, (135628 to 135897) ORF R3, (136780 to 137112) ORF R4, and (137558 to 137022) ORF R5r. The group of fragments includes homologs of the exemplary fragments listed above, i.e. homologous fragments of another parapoxvirus (e.g. species or strains as described above). It also includes functional variants of the exemplary fragments listed above (the function being as defined for the fragments generally above). Thus, the group of fragments includes variants with at least 80%, 85%, 90%, 95%, 96%, 97%, 98% or preferably at least 99% sequence identity to those fragments encoded by the exemplary coding sequences of SEQ ID NO: 3 listed above.

The inactivated parapoxvirus virion can be obtained by any means known in the art suitable for inactivating enveloped viruses, for example by physical inactivation (e.g. exposure to heat, specifically pasteurization, or UV light) or by chemical inactivation (e.g. by exposure to low pH, to a detergent or to an inactivation agent such as ethyleneimine, binary ethyleneimine, formaldehyde, glutaraldehyde, 2,2'-dithiodipyridine, or beta-propiolactone). In a preferred embodiment, the inactivated parapoxvirus virion is an ethyleneimine- or binary ethyleneimine-inactivated virion, preferably a binary ethyleneimine-inactivated virion.

The parapoxvirus agent may comprise genetic information (DNA or RNA) encoding for a heterologous (i.e. not of parapoxvirus) antigen, and/or an immunomodulator, or in may alternatively not comprise genetic information encoding for a heterologous antigen and/or an immunomodulator. In one embodiment, it does not comprise genetic information encoding for a heterologous antigen, but it optionally comprises genetic information encoding for immunomodulator.

In a preferred embodiment, the parapoxvirus agent is a wildtype parapoxvirus. Preferably it is a PPVO virion, more preferably an inactivated PPVO virion (iPPVO).

### Modes of use

Letermovir, or preferably a combination of a parapoxvirus agent and Letermovir, can be used as such or in combination with a further treatment regimen for (i) conditioning a subject for a coronavirus infection or (ii) treating a coronavirus infection in a subject.

In a preferred embodiment, both of the conditioning and the treating, the further treatment regimen can comprise administering a coronavirus agent.

Specifically for the conditioning, the further treatment regimen can comprise administering a further immune stimulatory agent (e.g. an adjuvant, a TLR agonist, a PRR, e.g. a cytosolic PRR such as RIG-I-like receptors including RIG-I, MDA5 and LGP2, or a mitogen, particularly a T cell mitogen or activator such as phytohemagglutinin (PHA), Concanavalin (Con) A, wheat germ agglutinin (WGA), pokeweed mitogen (PWM), a lectin or an anti-CD3 antibody or antigen-binding fragment thereof), or a B cell mitogen or activator such as PWM, lipopolysaccharide (LPS), an anti-CD40 antibody or antigen-binding fragment thereof, or an anti-(B cell antigen receptor) antibody or antigen-binding fragment thereof. The immune stimulatory agent preferably promotes an antiviral cell state. Specifically for the treating, the further treatment regimen can comprise administering one or more further medicaments and/or one or more further therapies (non-medicament/drug treatments) suitable for the treatment of a subject infected by coronavirus or having coronavirus disease. The one or more further medicaments are preferably selected from the group consisting of a further immune stimulatory agent (as exemplified above), an antiviral, an antibiotic, an adjuvant, a glucocorticoid, an antihypertensive drug (e.g. an ACE inhibitor), a hypoglycaemic drug, an anti-shock drug, and a drug suitable for treating one or more symptoms of coronavirus disease and/or to accompany the one or more further therapies (e.g. a pain reliever, a sedative, an anti-fever drug, an antiinflammatory drug such as IL-10, cough medication or an immunosuppressant). The one or more further therapies are preferably selected from the group consisting of oxygen therapy and extra-corporeal organ support (ECOS). Oxygen therapy can be passive (i.e. relying on breathing of the subject) or active (i.e. delivering oxygen to the lungs without relying on breathing of the subject). Passive therapies are non-pressurized oxygen therapy and pressurized oxygen therapy. Active therapies are mechanical ventilation and extra-corporeal membrane oxygenation (ECMO, also a specific form of ECOS). Examples for ECOS include ECMO, continuous renal replacement therapy (CRRT), hemofiltration (HF) and hemoperfusion (HP) e.g. to remove inflammatory cytokines, and left ventricular assistance.

Specific examples of further medicaments for conditioning and treating are: Ivermectin, TMPRSS2 inhibitors (e.g. ammonium chloride, serine protease inhibitor camostat mesylate) optionally together with a CatB/L inhibitor (e.g. E-64d), Favipiravir, Pimodivir, Baloxavir optionally together with Marboxil, IL-6 blockers (e.g. sarilumab, tocilizumab), Sarilumab, Zitivekumab, lopinavir optionally together with ritonavir, chloroquine, hydroxychloroquine, Remdesivir, α-Ketoamides (e.g. cyclopentylmethyl, cyclohexylmethyl or α-Ketoamide 13b), Thalidomide, Maraviroc, Tenofovir optionally together with Disoproxil, Dolutegravir, Nelfinavir, Raltegravir, Tipranavir and Type I interferons (IFN-I, specifically IFN-β1). Preferred examples are Remdesivir and Thalidomide.

Letermovir and the parapoxvirus agent can be administered together or each separately by any route, including but not limited to the intravenous, intramuscular, oral, parenteral, topical, intradermal, or subcutaneous route. In a specific embodiment, it can be administered directly to the respiratory tract, preferably to the lower respiratory tract, more preferably to the lung (pulmonary delivery). As such, it can be administered as an aerosol and/or via inhalation.

The dose of the parapoxvirus agent and Letermovir (in fact of all agents described herein, including a further immune stimulatory agent, a further medicament and a coronavirus agent) is a pharmaceutically effective dose as can be determined by the skilled person. For instance, in case of the parapoxvirus agent being a virion (live or inactivated), it can be in the range of from 10⁶ to 10¹⁰ particles. For Letermovir, for example, it can be in the range of from 5 mg to 1000 mg, for instance from 240 mg to 480 mg, with specific example doses being 240 mg and 480 mg (e.g. in the form of tablets). The dose may be administered once or more than once, for example over a period of < 12 weeks, < 6 weeks, < 4 weeks, < 2 weeks, or < 1 week. For Letermovir, daily doses are preferred.

### Coronavirus

The coronavirus is, in a preferred embodiment, an alpha-coronavirus or a beta-coronavirus. More preferably, it is a respiratory coronavirus, i.e. a coronavirus that causes a respiratory disease. Examples of alpha-coronaviruses are HCoV-229E and CoV-NL63, and examples of beta-coronaviruses are SARS-CoV, SARS-CoV-2, MERS-CoV, HCoV-HKU1, and HCoV-OC43. It is preferred that the coronavirus is a MERS or a SARS coronavirus, wherein preferably the SARS coronavirus is a virus of the species *"severe acute respiratory syndrome-related coronavirus"* as classified by the International Committee on Taxonomy of Viruses (ICTV). In the most preferred embodiment, it is SARS-CoV-2.

The skilled person appreciates that coronaviruses mutate and that new strains of SARS-CoV-2 are isolated daily, with 461 strains having been isolated by April 6, 2020. Thus, in terms of a sequence definition, a preferred virus of the invention has a genome comprising or consisting of a sequence according to SEQ ID NO: 1 (original strain of the 2019 outbreak "Severe acute respiratory syndrome coronavirus 2 isolate Wuhan-Hu-1", NCBI Reference Sequence NC_045512.2, version of March 30, 2020) or a variant thereof with at least 80%, 85%, 90%, 95%, 96%, 97%, 98% or preferably at least 99% sequence identity. Alternatively or in addition, a preferred virus of the invention has a genome comprising or consisting of a sequence according to SEQ ID NO: 2 (a SARS-CoV-2 consensus sequence of 153 genome assemblies) or a variant thereof with at least 80%, 85%, 90%, 95%, 96%, 97%, 98% or preferably at least 99% sequence identity. It is preferred that the variant is classified as a strain of SARS-CoV-2 by the International Committee on Taxonomy of Viruses (ICTV).

### Coronavirus agent

A coronavirus agent generally is an agent that is suitable for treating a coronavirus infection. It can be (a) the coronavirus itself or a fragment thereof which is recognized by and preferably stimulates an immune response (e.g. an antigen and preferably immunogen), (b) an agent that comprises genetic information encoding for (a), or (c) an agent which interferes with the infection of a cell by the coronavirus or with the replication of the coronavirus within a cell (in a therapeutic setting: is capable upon administration of an effective amount thereof to a subject infected with coronavirus to interfere with the infection of a cell by the coronavirus or with the replication of the coronavirus within a cell). The coronavirus agent is preferably selected from the group consisting of
(i) an inactivated coronavirus,
(ii) an antigenic, preferably immunogenic fragment of coronavirus (e.g. the spike protein or a fragment thereof),
(iii) a nucleic acid encoding for (i) or (ii),
(iv) a vector comprising the nucleic acid of (iii),
(v) a cell comprising the nucleic acid of (iii) or the vector of (iv),
(vi) an antibody or an antigen-binding fragment thereof binding to coronavirus or a fragment thereof, preferably the envelope,
(vii) a nucleotide/nucleoside analogue, preferably coronavirus-specific,
(viii) a coronavirus-specific RNAi molecule,
(ix) a small molecule, an antibody or an antigen-binding fragment thereof, binding to a site of protein-protein interaction (PPI) between a coronavirus protein and a subject protein, and
(x) blood plasma of a subject that has recovered from a coronavirus infection, preferably from coronavirus disease.

A coronavirus can be inactivated as described for parapoxvirus above.

The PPI of (ix) is preferably an interaction of a SARS-CoV-2 protein or homologue thereof of another coronavirus and a subject protein (Uniprot protein ID of each subject protein is <name recited below>_HUMAN) selected from
- SARS-CoV2 E and AP3B1, BRD4, BRD2, CWC27, ZCH18 or CTL2;
- SARS-CoV2 M and MPPB, YIF1A, AT1B1, ACADM, ETFA, STOM, VKGC, VATA, PSMD8, REEP5, MPPA, ANO6, PREP, ZNT9, FAKD5, ZNT7, GCP3, COQ8B, SAAL1, REEP6, INT4, ODC, GCP2, SYTM, RTN4, FA8A1, AASS, AKP8L, AAR2 or BZW2
- SARS-CoV2 N and U3IP2, PABP1, CSK22, CSK2B, G3BP1, PABP4, LARP1, FA98A, SNIP1, RENT1, MOV10, G3BP2, DDX21, RBM28 or RL36;
- SARS-CoV2 Spike and ACE2, GOGA7 or ZDHC5;
- SARS-CoV2 nsp1 and DPOLA, PRI1, PRI2, DPOA2, GT251 or PKP2;
- SARS-CoV2 nsp10 and AP2A2, ALR, ERGI1, AP2M1 or GRPE1;
- SARS-CoV2 nsp11 and TBCA;
- SARS-CoV2 nsp12 and SBNO1, BCKD, AKAP8, MYCB2, SLU7, RIPK1, UBP2L, TYSD1, PDZ11, PRC2B, UBAP2, ZN318, CRTC3, UBP54, Z3H7A, LAR4B, RBM41, HTF4, PPIL3 or PKHA5;
- SARS-CoV2 nsp13 and TBKB1, CTRO, HSBP1, PCNT, FR1OP, KAP2, KAPCA, KAP3, RADI, CENPF, TLE1, TLE3, TLE5, GOGA3, GOGA2, GOGB1, GRAP1, CE350, MYOME, CP135, CEP68, CNTRL, RB6I2, GCC2, CLIP4, NIN, CE112, MIPO1, UBP13, GCC1, JKIP1, CK5P2, AKAP9, GORS1, FYCO1, CA050, CP250, TBK1, HOOK1 or NINL;
- SARS-CoV2 nsp14 and AGAL, IMDH2 or SIR5;
- SARS-CoV2 nsp15 NTF2, ARF6 or RNF41;
- SARS-CoV2 nsp2 and S27A2, IF4E2, NCPR, GDS1, WASC4, FKB15 or GGYF2;
- SARS-CoV2 nsp4 and IDE, TIM10, ALG11, P0210, TIM29, DJC11, T10B or TIM9;
- SARS-CoV2 nsp5 and HDAC2;
- SARS-CoV2 nsp5_C145A and GPX1 or TRM1;
- SARS-CoV2 nsp6 and ATP5L, VAS1, SGMR1 or AT133;
- SARS-CoV2 nsp7 and ADAS, CYB5B, ACSL3, NB5R3, RALA, COMT, RAB5C, RAB7A, RAB8A, RAB2A, RAB10, RAB14, RHOA, RAB1A, GBB1, GBG5, LMAN2, MOGS, TOIP1, MARC1, QSOX2, HS2ST, NDUF2, SCPDL, SCRB1, NAT14, DCAKD, F162A, TIM14, SELS, PGES2 or RAB18;
- SARS-CoV2 nsp8 and MPP10, SRP72, ATE1, NSD2, SRP19, SRP54, RT25, DDX10, LARP7, MEPCE, NGDN, EXOS8, SYNM, NOL10, CCD86, SPCS, EXOS5, EXOS3, AATF, HECD1, RT02, RT05, EXOS2 or RT27;
- SARS-CoV2 nsp9 and T2FB, FBN1, FBN2, NU214, NUP62, DCAF7, IF4H, NUP54, MIB1, NEK9, ZN503, NUP88, NUP58, MAT2B or FBLN5;
- SARS-CoV2 orf10 and PPT1, CUL2, MA7D1, THTPA, ZY11B, TIM8B, RBX1, ELOC or ELOB;
- SARS-CoV2 orf3a and HMOX1, TRI59, AR6P6, VPS39, CLCC1, VPS11, SUN2 or ALG5;
- SARS-CoV2 orf3b and STML2;
- SARS-CoV2 orf6 and NUP98, RAEIL or MTCH1;
- SARS-CoV2 orf7a and HEAT3 or MDN1;
- SARS-CoV2 orf8 and PLOD2, TOR1A, STC2, TPA, ITB1, CISD3, CO6A1, PVR, DNMT1, LYOX, PCSK6, INHBE, NPC2, MFGM, OS9, NPTX1, PLGT2, PLGT3, ERO1B, PLD3, FXRD2, CHSS2, PUSL1, EMC1, GGH, ERLEC, I17RA, NGLY1, H6ST2, SDF2, NEUR1, GDF15, TM2D3, ERP44, EDEM3, SIL1, OFUT1, SMOC1, PKHF2, FXL12, UGGG2, CHPF2, ATS1, HYOU1, FKBP7, ADAM9 or FKB10;
- SARS-CoV2 orf9b and NHRF1, CHM2A, CSDE1, TOM70, MARK3, MARK2, DPH5, DCTP1, MARK1, PTBP2 or BAG5; and
- SARS-CoV2 orf9c and UBXN8, GPAA1, WFS1, MRP1, PAR2, SCAP, D19L1, SGMR2, ZNT6, TAPT1, ERMP1, NLRX1, RETR3, PIGO, FACR2, ECSIT, ALG8, TM39B, GHITM, ACAD9, NFIP2, BCS1, CIA30, TMED5, NDUB9 or PIGS.

An antibody or an antigen-binding fragment thereof to a PPI site can be generated by routine methods. For instance, antibodies or antigen-binding fragments thereof to one of the interacting proteins can be generated and then screened for interfering with the interaction.

The small molecule of (ix) is preferably selected from the group consisting of Loratadine, Daunorubicin, Midostaurin, Ponatinib, Silmitasertib, Valproic Acid, Haloperidol, Metformin, Migalastat, S-verapamil, Indomethacin, Ruxolitinib, Mycophenolic acid, Entacapone, Ribavirin, E-52862, Merimepodib, RVX-208, XL413, AC-55541, Apicidin, AZ3451, AZ8838, Bafilomycin A1, CCT 365623, GB110, H-89, JQ1, PB28, PD-144418, RS-PPCC, TMCB, UCPH-101, ZINC1775962367, ZINC4326719, ZINC4511851, ZINC95559591, 4E2RCat, ABBV-744, Camostat, Captopril, CB5083, Chloramphenicol, Chloroquine, PI4K-IIIB inhibitor Compound 10 (Rutaganira, F. U. et al., J. Med. Chem. 59, 1830-1839 (2016)), Cyclophilin inhibitor Compound 2 (Mackman, R. L. et al., J. Med. Chem. 61, 9473-9499 (2018)), CPI-0610, Dabrafenib, DBeQ, dBET6, IHVR-19029, Linezolid, Lisinopril, Minoxidil, ML240, MZ1, Nafamostat, Pevonedistat, PS3061, Rapamycin (Sirolimus), Sanglifehrin A, Sapanisertib (INK128/M1N128), FK-506 (Tacrolimus), Ternatin 4 (DA3), Tigecycline, Tomivosertib (eFT-508), Verdinexor, WDB002, Azacitidine, Selinexor, and Zotatifin (eFT226).

### Coronavirus infection/disease

A coronavirus infection is defined by the entry of coronavirus into at least one cell of a subject and its replication in the at least one cell. Preferably, the infection is an infection of the respiratory tract, including the upper respiratory tract (nose and nasal passages, paranasal sinuses, the pharynx, and the portion of the larynx above the vocal folds (cords)) and the lower respiratory tract (portion of the larynx below the vocal folds, trachea, bronchi, bronchioles and the lungs including the respiratory bronchioles, alveolar ducts, alveolar sacs and alveoli). More preferably, it is an infection of the lower respiratory tract, most preferably of the lungs (including one or more of respiratory bronchioles, alveolar ducts, alveolar sacs and alveoli). The infection can further be characterized immunologically by the presence of at least one coronavirus-antigen-specific immune factor, preferably selected from the group consisting of B cells, follicular helper T cells (T_{FH} cells), activated CD4⁺ T cells and CD8⁺ T cells (particularly also CD38⁺HLA-DR⁺), IgM antibodies, and IgG antibodies. Alternatively or in addition, it can be characterized immunologically by a coronavirus-specific cytokine profile, preferably in the blood or at the site of infection as specified above (most preferably in the lung). These profiles can be determined by the skilled person without undue burden, e.g. by taking a tissue sample, e.g. from the lung, infecting it with the coronavirus and determining the expression of cytokines. Examples of coronavirus-specific cytokine profiles are (a) for SARS-CoV: upregulation of one or more of, preferably all of IFN-I (e.g. IFN-β), IFN-II (IFN-γ), IFN-III (IFN-λ1, IPN-λ2, and IPN-λ3), IL-1beta, IL-6, IL-12, IL-8, MCP-1, MIP-1alpha, RANTES, CXCL1, CXCL2, CXCL5, and CXCL9, and preferably no upregulation of CXCL10; and (b) for SARS-CoV-2: upregulation of one or more of, preferably all of IL-6, MCP-1, CXCL1, CXCL5, and/or CXLC10, and preferably no upregulation of any IFN and/or of any one or all of IL-1beta, IL-12, IL-8, MIP-1alpha, RANTES, CXCL2, and CXCL9.

The preferred subject is human. While to the inventors' best knowledge coronavirus infects all humans equally, there are risk factors for contracting a coronavirus infection:
(i) Recent (within incubation period, i.e. within the past 27, 24, or 19 days, preferably 14 (CDC, NHC) or 10 (WHO) days) presence in or travel from an area with ongoing spread of coronavirus infection. The area is preferably an 'Affected Area' as determined by the WHO. "Recent" can mean within the incubation period of the coronavirus, preferably within the past 27, 24, or preferably 19 days, more preferably 14 days or most preferably 10 days.
(ii) Contact with a person infected with coronavirus, in particular a person having one or more symptoms of coronavirus disease. "Contact" can mean exposed to respiratory droplets of the person (e.g. within range of respiratory droplets in air or in contact with surfaces touched by person or by respiratory droplets).
(iii) Being in an increased-risk-profession, which usually necessitates being in contact with the general public (contact being defined as above) regularly (e.g. at least daily or at least weekly), e.g. being a medical professional, in particular in a hospital, being a care worker (e.g. for children or the elderly) or retail worker (for instance in a supermarket or pharmacy).

A coronavirus infection may or may not cause symptoms of coronavirus disease in a subject. The terms "coronavirus infection" and "coronavirus disease" are distinguished herein by the presence of at least one coronavirus disease symptom. As long as the infection is not accompanied by at least one symptom of coronavirus disease, it (or the subject) is asymptomatic (includes presymptomatic). The term "coronavirus disease" as used herein requires the presence of a coronavirus infection and at least one symptom of coronavirus disease (also referred to herein as "symptomatic infection").

Coronavirus symptoms include dry cough, fever (≥37.8°C), runny and/or blocked nose, fatigue, breathing difficulty, pneumonia, organ (e.g. heart, lung, liver and/or kidney) failure, itchy throat, headache, joint pain, nausea, diarrhoea, shivering, lymphophenia, loss of smell and/or loss of taste. Preferably, the coronavirus disease is characterized by the presence of two or more, three or more, or four or more symptoms, preferably including one or two or more of dry cough, fever (≥37.8°C), breathing difficulty, loss of smell and/or loss of taste.

The coronavirus disease is preferably a respiratory disease (e.g. SARS or MERS), more preferably Covid-19. The coronavirus disease can take a mild (non-severe) or a severe course. A mild course is characterized by the presence of one or more only mild symptoms (i.e. no severe symptoms) of coronavirus disease. Mild symptoms are selected from the group consisting of dry cough, mild fever (≥37.8°C to <40°C), runny and/or blocked nose, fatigue, itchy throat, headache, joint pain, nausea, diarrhoea, shivering, lymphophenia, loss of smell and loss of taste. Severe symptoms are selected from the group consisting of breathing difficulty in particular acute respiratory distress syndrome, pneumonia, organ (e.g. heart, lung, liver and/or kidney) failure and high fever (≥40°C). A severe course is characterized clinically by one or more of (i) the presence of one or more severe symptoms of coronavirus disease, (ii) need for hospitalization, in particular need for admission to intensive care unit (ICU) and/or need for oxygen therapy (as defined above), in particular for a respirator (also referred to as ventilator), and/or (iii) death of the subject. A need for hospitalization is characterized by one or more of: clinical or radiological evidence of pneumonia, acute respiratory distress syndrome, and/or the presence of two or more symptoms including at least fever and persistent cough (with or without sputum), in particular persistent cough (e.g. of at least 3 days). A need for ICU admission is characterized by severe pneumonia and/or one or more severe complications. Severe pneumonia is characterized by tachypnea (age younger than 2 months: 60 or more breaths per minute; age 2 to 11 months: 50 or more breaths per minute, age 1 to 5 years: 40 or more breaths per minute, age > 5 years: 30 or more breaths per minute), respiratory distress and/or inadequate oxygenation (e.g. SpO₂ of 93% or less for adults, and 90% or less for children). Severe complications are complications that are lethal when untreated and include, for example, septic shock, acute respiratory distress syndrome and organ failure. Alternatively or in addition, a severe course can be characterized immunologically e.g. by the presence or development of a cytokine storm, in particular in the lung. A coronavirus cytokine storm is characterized by hyperinflammation, preferably by an increase (vs. infected but asymptomatic subject, preferably as after the incubation period, or average or mean of a plurality of such subjects) of one or more of IL-2, IL-7, TNF-alpha, MIP-1alpha, MCP-1, G-CSF and/or CXCL10, wherein the increase can be characterized as excessive and/or uncontrolled. In particular, the cytokine storm is a Covid-19 cytokine storm. Furthermore, the cytokine storm is preferably in the respiratory tract as defined for the infection above (including preferred parts thereof).

While the coronavirus disease can take a severe course in any subject regardless of age or pre-existing condition, there are nevertheless risk factors for having a severe course of coronavirus disease, including:
(i) age, e.g. at least 40, 50, 60, 70, or 80 years with risk increasing with age,
(ii) male gender,
(iii) smoking, and
(iii) having one or more comorbidities, preferably selected from the group consisting of cardiovascular disease, diabetes, respiratory disease (in particular chronic), hypertension, AIDS, cancer, liver disease, kidney disease and lung disease. A particular comorbidity as risk factor is a respiratory disease.

### Conditioning a subject

The conditioning of a subject is meant to prepare a subject for a coronavirus infection, which is afforded by the immunomodulation resulting in the medical use of the invention. It can also be termed "preparatory treatment", "prophylactic treatment" or, with respect to the disease resulting from the invention, "preventative treatment" or "preventive treatment". It is not meant to necessarily prevent a coronavirus infection. As such, the subject is not yet infected by coronavirus.

Thus, the conditioning can have one or more immunological effects or (preferably and) it can have one or more clinical effects, both once a subject is infected. These immunological effects are preferably selected from the group consisting of
(I-i) increasing the number of one or more of coronavirus-antigen-specific immune factors (as defined above) in the subject or in a part of the subject,
(I-ii) inhibiting (i.e. reducing or stopping) coronavirus replication in the subject or in a part of the subject,
(I-iii) reducing the coronavirus load in the subject or in a part of the subject, and
(I-iv) preventing or ameliorating a coronavirus-specific cytokine profile or a cytokine storm (as defined above) in the subject or in a part of the subject.
Therein, the part of the subject is preferably the respiratory tract (upper or preferably lower respiratory tract, more preferably the lung). (I-ii) is preferred over (I-i), and (I-iii) over (I-ii). Further (I-ii) may involve (I-i), and (I-iii) may involve (I-i) and/or (I-ii). (I-iv) is generally desired.

The clinical effects are preferably selected from the group consisting of
(C-i) reducing contagiousness,
(C-ii) delaying the onset of coronavirus disease,
(C-iii) reducing the degree of one or more prospective symptoms of coronavirus disease, preferably of one or more prospective severe symptoms of coronavirus disease,
(C-iv) preventing one or more symptoms of coronavirus disease, preferably one or more severe symptoms of coronavirus disease,
(C-v) preventing a severe course of coronavirus disease, and
(C-vi) preventing coronavirus disease.
Therein, (C-ii) is preferred over (C-i), (C-iii) over (C-ii), (C-iv) over (C-iii), (C-v) over (C-iv), and (C-vi) over (C-v).

Prospective symptoms are symptoms that are not yet present but can or will arise. Thus, the reduction of the degree of prospective symptoms refers to a prophylactic treatment of symptoms.

Accordingly, Letermovir, or preferably a combination of the parapoxvirus agent and Letermovir, can be for use in one or more of (I-i) to (I-iv) and/or one or more of (C-i) to (C-vi) in a subject not yet infected by coronavirus. In other words, Letermovir, or preferably a combination of the parapoxvirus agent and Letermovir, is capable upon administration of an effective amount thereof to achieve one or more of (I-i) to (I-iv) and/or one or more of (C-i) to (C-vi).

In a preferred embodiment, the subject to be conditioned is characterized by one or more risk factors for contracting a coronavirus infection and/or by one or more risk factors for having a severe course of coronavirus disease.

In another embodiment, the subject to be conditioned is selected by a method according to the corresponding further aspect of the invention described below.

### Treating a subject

The treating of a coronavirus infection (i.e. of a subject that is infected) is not meant necessarily to treat or prevent coronavirus disease, although it is preferred that it does. As such, the treating can be of an asymptomatic infection (i.e. of an infected subject not having symptoms) or of coronavirus disease (i.e. of an infected subject having one or more symptoms).

The treating can have one or more immunological effects or (preferably and) it can have one or more clinical effects. These immunological effects are preferably as defined for the conditioning above, for either an asymptomatic infection or for the disease. The clinical effects for an asymptomatic infection are preferably selected from the group consisting of (C-i) to (C-vi) as defined for the conditioning above. The clinical effects for a symptomatic infection (i.e. on coronavirus disease) are preferably selected from the group consisting of
(C-i), (C-vi), both as defined for the conditioning above,
(C-vii) ameliorating one or more (or ideally all) symptoms of coronavirus disease,
(C-viii) preventing, one or more (or ideally all) further symptoms of coronavirus disease, and
(C-ix) ameliorating, preferably preventing, a severe course of coronavirus disease.
Therein, "further symptoms" are those not yet characterizing the symptomatic infection.

Accordingly, Letermovir, or preferably a combination of the parapoxvirus agent and Letermovir, can be for use in one or more of (I-i) to (I-iv) and/or one or more of (C-i) to (C-ix) in a subject infected by coronavirus. In other words, Letermovir, or preferably a combination of a parapoxvirus agent and Letermovir, is capable upon administration of an effective amount thereof to achieve one or more of (I-i) to (I-iv) and/or one or more of (C-i) to (C-ix). In a preferred embodiment, Letermovir, or preferably a combination of a parapoxvirus agent and Letermovir, is for use in preventing or treating coronavirus disease.

As indicated above, the subject to be treated may be asymptomatic. Alternatively, the subject to be treated may have coronavirus disease. The subject having coronavirus disease may not have any severe symptoms, e.g. it has only one or more mild symptoms. In another embodiment, it may have a severe course of coronavirus disease (excluding (iii) death; preferably (i) one or more severe symptoms). In a preferred embodiment, the subject to be treated (e.g. any of the afore-mentioned subjects except those already having a severe course of coronavirus disease) is characterized by one or more risk factors for having a severe course of coronavirus disease.

In a further preferred embodiment, the subject is characterized by at least one of:
(i) no cytokine storm in the lung, and preferably no cytokine storm at all,
(ii) age up to 75, 70 or preferably 65 years, more preferably 18 to 65 years, and/or
(iii) HScore of 169 or less, preferably 150 or less, 130 or less, or 110 or less,
Preferably, the subject is characterized by (ii) and/or (iii), (ii) and HScore of 169 or less, or (iii) and age 18 to 65 years old.

The HScore generates a probability for the presence of secondary haemophagocytic lymphohistoicytosis (sHLH), a hyperinflammatory syndrome with a cytokine storm profile similar to that of coronavirus disease (Pehta et al., The Lancet Vol 395 March 28, 2020). An HScore of greater than 169 is 93% sensitive and 86% specific for sHLH. The HScore is calculated using the following criteria: temperature (<38.4°C: 0 points, 38.4-39.4°C: 33 points, >39.4°C: 49 points), organomegaly (none: 0 points, hepatomegaly or splenomegaly: 23 points, hepatomegaly and splenomegaly: 38 points), number of cytopenias* (one lineage; 0 points, two lineages: 24 points, three linages: 34 points), triglycerides (<1.5 mmol/l: 0 points, 1.5-4.0 mmol/l: 44 points, >4.0 mmol/l: 64 points), fibrinogen (>2.5 g/L: 0 points, ≤2.5 g/L: 30 points), ferritin (<2000 ng/ml: 0 points, 2000-60000 ng/ml: 35 points, >6000 ng/ml: 50 points), serum aspartate aminotransferase (<30 IU/L: 0 points, ≥30 IU/L: 19 points), haemophagocytosis on bone marrow aspirate (no: 0 points, yes: 35 points), and known immunosuppressiont (no: 0 points, yes: 18 points). *Defined as either haemoglobin concentration of 9.2 g/dL or less (≤5.71 mmol/L), a white blood cell count of 5000 white blood cells per mm³ or less, or platelet count of 110000 platelets per mm³ or less, or all of these criteria combined. †HIV positive or receiving long-term immunosuppressive therapy (e.g. one or more of glucocorticoids, cyclosporine and/or azathioprine).

In another embodiment, the subject to be treated is selected by a method according to the corresponding further aspect of the invention described below.

### Further medical use

In a further medical use aspect, the invention relates to Letermovir, or preferably a combination of a parapoxvirus agent and Letermovir, for use as a medicament for inhibiting virus replication and/or for promoting an antiviral (preferably anticoronaviral) cell state in a subject (i) having a coronavirus infection or (ii) characterized by one or more risk factors for contracting a coronavirus infection and/or by one or more risk factors for having a severe course of coronavirus disease.

In one embodiment, the one or more risk factors for contracting a coronavirus infection are selected from factors (i) and (ii) as defined above.

In another embodiment, the one or more risk factors for having a severe course of coronavirus disease comprise at least (ii) and (iii), or (ii) and (iv) as defined above, or comprise at least a comorbidity selected from respiratory disease (in particular chronic) and lung disease. In addition, the age of the subject is preferably at least 60, at least 70, or more preferably at least 80 years.

The phrase "promoting an antiviral cell state" refers to the promotion of the transcription of cellular antiviral genes coding for host defence proteins, e.g. as promoted by IFN-α and/or β. Preferably it inhibits (i.e. prevents or at least reduces) virus replication in the cell and/or induces apoptosis (to prevent further virus replication in the cell), and more preferably it reduces the amount of infectious virions released by an infected cells compared to a cell in which the antiviral cell state was not promoted by the medicament. Thereby, it stops or at least slows down virus propagation. "Promoting" includes an induction (e.g. for cells not yet in an antiviral state) and a reinforcement (i.e. potentiation) (e.g. for cells already in an antiviral state). Although not limited thereto, the former is preferred for a subject not (yet) having a coronavirus infection, e.g. characterized by one or more risk factors as specified above, and the latter is preferred for a subject having a coronavirus infection.

### Methods of treatment

The invention further relates to the following methods

A method of administering Letermovir, or preferably a combination of a parapoxvirus agent and Letermovir, to a subject (i) having a coronavirus infection or (ii) characterized by one or more risk factors for contracting a coronavirus infection and/or by one or more risk factors for having a severe course of coronavirus disease.

A method of promoting an antiviral (preferably anticoronaviral) cell state in a subject (i) having a coronavirus infection or (ii) characterized by one or more risk factors for contracting a coronavirus infection and/or by one or more risk factors for having a severe course of coronavirus disease, the method comprising administering Letermovir, or preferably a combination of a parapoxvirus agent and Letermovir, to the subject.

A method of (i) conditioning a subject for a coronavirus infection or (ii) treating a coronavirus infection in a subject, the method comprising administering Letermovir, or preferably a combination of a parapoxvirus agent and Letermovir, to the subject.

A method of treating coronoavirus disease in a subject, the method comprising administering Letermovir, or preferably a combination of a parapoxvirus agent and Letermovir, to the subject.

Definitions and embodiments described below, in particular under the header 'Definitions and further embodiments of the invention' apply to the first aspect of the invention.

In a second aspect, the invention relates to a composition or a kit comprising (i) Letermovir, or preferably a parapoxvirus agent and Letermovir, and (ii) a coronavirus agent.

In a preferred embodiment, the composition or kit further comprises one or more pharmaceutically acceptable diluents, carriers, and/or preservatives; and/or it is a pharmaceutical composition or kit. Pharmaceutically acceptable diluents, carriers, and/or preservatives are described below and may in addition thereto include an (e.g. aqueous) medium suitable for structurally maintaining a parapoxvirus, in particular an inactivated parapoxvirus, or lyophilisate of this medium. Specifically, the composition or kit may be for any of the medical uses of the invention described herein.

The composition can be formulated for any route of administration as defined above. Also, it can be formulated for simultaneous or separate administration of Letermovir and the coronavirus agent; of all of parapoxvirus agent, Letermovir and coronavirus agent separately or simultaneously; the parapoxvirus agent and Letermovir simultaneously, and separately the coronavirus agent; the parapoxvirus agent and the coronavirus agent simultaneously, and separately Letermovir; or the coronavirus agent and Letermovir simultaneously, and separately the parapoxvirus agent. Similarly, it can be formulated for simultaneous or separate release of Letermovir and the coronavirus agent; of all of parapoxvirus agent, Letermovir and coronavirus agent separately or simultaneously; the parapoxvirus agent and Letermovir simultaneously, and separately the coronavirus agent; the parapoxvirus agent and the coronavirus agent simultaneously, and separately Letermovir; or the coronavirus agent and Letermovir simultaneously, and separately the parapoxvirus agent.

In a preferred embodiment, the kit comprises a composition of the second aspect.

In another embodiment, the kit comprises a leaflet with instructions for use of Letermovir, or preferably of a parapoxvirus agent and Letermovir, and the coronavirus agent, or of the composition of the second aspect, preferably according to a medical use described herein.

Definitions given and embodiments described with respect to the first aspect apply also to the second aspect, in as far as they are applicable. Also, definitions and embodiments described below, in particular under the header 'Definitions and further embodiments of the invention' apply to the composition or kit of the second aspect.

In a third aspect, the invention relates to a pharmaceutical composition or kit for any of the medical uses of the invention described herein, wherein the pharmaceutical composition or kit comprises (i) Letermovir, or preferably a parapoxvirus agent and Letermovir, and preferably (ii) a further medicament. The pharmaceutical composition usually comprises one or more pharmaceutically acceptable diluents, carriers, and/or preservatives.

In a preferred embodiment, the kit comprises a composition of the third aspect.

In another embodiment, the kit comprises a leaflet with instructions for use of Letermovir, or preferably of the parapoxvirus agent and Letermovir, and the further medicament, or of the composition of the third aspect, preferably according to a medical use described herein.

Definitions given and embodiments described with respect to the first and second aspect (e.g. formulation) apply also to the third aspect, in as far as they are applicable. Also, definitions and embodiments described below, in particular under the header 'Definitions and further embodiments of the invention' apply to the composition or kit of the third aspect.

In a further aspect, the invention relates to a method of selecting a subject for any of the medical uses of the invention described herein comprising the use of a parapoxvirus agent, wherein the method comprises the steps of (i) determining the presence or absence of a cytokine storm in the subject, and (ii) selecting the subject if a cytokine storm is absent.

In one embodiment, step (i) comprises determining the HScore of the subject, and step (ii) comprises selecting the subject if the HScore is 169 or less.

In another embodiment, step (i) comprises detecting in a biological sample of the subject one or more signs of hyperinflammation, and step (ii) comprises selecting the subject if no sign of hyperinflammation is detectable. Hyperinflammation therein is an increased level, e.g. excessive and/or uncontrolled, (vs. infected but asymptomatic subject, preferably as after the incubation period, or average or mean of a plurality of such subjects) of one or more of IL-2, IL-7, TNF-alpha, MIP-1alpha, MCP-1, G-CSF and/or CXCL10; or of one or more of IL-6, ferritin, platelet count and/or erythrocyte sedimentation rate (ESR). The biological sample is preferably selected from the group consisting of blood or a blood-derived sample (e.g. plasma or serum), sputum, saliva, and a sample derived from the lung (e.g. by bronchoscopy including bronchial lavage, bronchial alveolar lavage, bronchial brushing, and bronchial abrasion).

The method of the fourth aspect is preferably an *ex vivo* method.

Definitions given and embodiments described with respect to the first, second and third aspect apply also to the fourth aspect, in as far as they are applicable. Also, definitions and embodiments described below, in particular under the header 'Definitions and further embodiments of the invention' apply to the method of the fourth aspect.

### Definitions and further embodiments of the invention

The specification uses a variety of terms and phrases, which have certain meanings as defined below. Preferred meanings are to be construed as preferred embodiments of the aspects of the invention described herein. As such, they and also further embodiments described in the following can be combined with any embodiment of the aspects of the invention and in particular any preferred embodiment of the aspects of the invention described above.

The term "identity" or "identical" in the context of polynucleotide sequences refers to the number of residues in the two sequences that are identical when aligned for maximum correspondence. Specifically, the percent sequence identity of two sequences is the number of exact matches between two aligned sequences divided by the length of the shorter sequence and multiplied by 100. Alignment tools that can be used to align two sequences are well known to the person skilled in the art and can, for example, be obtained on the World Wide Web, e.g. Needle (EMBOSS) (https://www.ebi.ac.uk/Tools/psa/emboss_needle/), MUSCLE (http://www.ebi.ac.uk/Tools/msa/muscle/), MAFFT (http://www.ebi.ac.uk/Tools/msa/ mafft/) or WATER (http://www.ebi.ac.uk/Tools/psa/ emboss_water/). The alignments between two sequences may be carried out using default parameters settings, e.g. for Needle preferably MATRIX: BLOSUM62, Gap Open: 10.0, Gap Extend: 0.5, for MAFFT preferably: Matrix: Blosum62, Gap Open 1.53, Gap Extend 0.123, for WATER polynucleotides preferably: MATRIX: DNAFULL, Gap Open: 10.0, Gap Extend 0.5 and for WATER polypeptides preferably MATRIX: BLOSUM62, Gap Open: 10.0, Gap Extend: 0.5. Those skilled in the art understand that it may be necessary to introduce gaps in either sequence to produce a satisfactory alignment. The "best sequence alignment" is defined as the alignment that produces the largest number of aligned identical residues while having a minimal number of gaps. Preferably, it is a global alignment, which includes every residue in every sequence in the alignment.

The term "variant" refers generally to a modified version of the polynucleotide, e.g. a mutation, so one or more nucleotides of the polynucleotide may be mutated. Generally, the variant is functional, meaning e.g. with regard to a virus that the virus is capable of infecting a host cell. The functionality is generally that described for the polynucleotide the variant is from. A "mutation" can be a nucleotide substitution, deletion and/or insertion ("and" may apply if there is more than one mutation). Preferably, it is a substitution.

The term "antigen" refers to any substance, e.g. protein or peptide, that is capable of being bound by an antibody, a B cell receptor and/or a T cell receptor. An antigen comprises at least one epitope preferably comprising at least 8 amino acids and more preferably comprising between 8 and 17 amino acids. The epitope can be a T cell and/or a B cell epitope. A T cell epitope is an epitope that can be presented on the surface of an antigen-presenting cell, where it is bound to an MHC molecule. In humans, professional antigen-presenting cells are specialized to present MHC class II peptides, whereas most nucleated somatic cells present MHC class I peptides. T cell epitopes presented by MHC class I molecules are typically peptides between 8 and 11 amino acids in length, whereas MHC class II molecules present longer peptides, e.g. 13-17 amino acids in length. A B cell epitope is an epitope that is recognised as three-dimensional structures on the surface of native antigens by B cells. Epitopes can be predicted with *in silico* tools, e.g. the online B- or T-cell prediction tools of the IEDB Analysis Resource.

The term "immunogen" refers to an antigen that is capable of inducing an immune response.

"Pattern recognition receptor" or "PRR", as used herein, refers to a germline-encoded host sensor which detects a pathogen-associated molecular pattern (PAMP), which is specific for a pathogen, and/or a damage-associated molecular pattern (DAMP), which is specific for components of a host cell that are released during cell damage or death. A PRR can be a membrane-bound PRR (cell-surface PRR) or a cytosolic PRR.

An "adjuvant" is a substance that accelerates, prolongs and/or enhances the quality and/or strength of an immune response to an antigen/immunogen, in comparison to the administration of the antigen alone, thus, reducing the quantity of antigen/immunogen necessary, and/or the frequency of injection necessary in order to generate an adequate immune response to the antigen/immunogen of interest. Examples of adjuvants that may be used in the context of the composition according to the present invention are gel-like precipitates of aluminum hydroxide (alum); AlPO₄; alhydrogel; bacterial products from the outer membrane of Gram-negative bacteria, in particular monophosphoryl lipid A (MPLA), lipopolysaccharides (LPS), muramyl dipeptides and derivatives thereof; Freund's incomplete adjuvant; liposomes, in particular neutral liposomes, liposomes containing the composition and optionally cytokines; non-ionic block copolymers; ISCOMATRIX adjuvant (Drane et al., 2007); unmethylated DNA comprising CpG dinucleotides (CpG motif), in particular CpG ODN with a phosphorothioate (PTO) backbone (CpG PTO ODN) or phosphodiester (PO) backbone (CpG PO ODN); synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:C; polycationic peptides, in particular poly-L-arginine; taxol; fibronectin; flagellin; imidazoquinoline; cytokines with adjuvant activity, in particular GM-CSF, interleukin-(IL-)2, IL-6, IL-7, IL-18, type I and II interferons, in particular IFN-γ, TNF-α; 25-dihydroxyvitamin D3 (calcitriol); and synthetic oligopeptides, in particular MHC-II-presented peptides. Non-ionic block polymers containing polyoxyethylene (POE) and polyoxypropylene (POP), such as POE-POP-POE block copolymers may be used as an adjuvant (Newman *et al.,* 1998). This type of adjuvant is particularly useful for compositions comprising nucleic acids as active ingredient.

The term "recombinant" refers in particular to a virus that is modified to comprise a heterologous polynucleotide sequence in its genome.

The term "immunomodulator" refers to a drug used to regulate or normalize the immune system by inducing, enhancing, suppressing and/or weakening an immune response in a subject ("and" meaning that some parts of the immune system are selectively induced or enhanced and others are selectively suppressed or weakened). Examples are cytokines, non-cytokine agonists or antagonists of cytokine receptors (e.g. antibodies or small compounds), antibodies or small compounds binding (preferably neutralizing) cytokines, and soluble cytokine receptors (e.g. for trapping cytokines). It does not include an antigen.

The term "vector" as used herein includes any vectors known to the skilled person including plasmid vectors, cosmid vectors, phage vectors such as lambda phage, viral vectors such as adenovirus (Ad) vectors), adeno-associated virus (AAV) vectors, alphavirus vectors (e.g., Venezuelan equine encephalitis virus (VEE), sindbis virus (SIN), semliki forest virus (SFV), and VEE-SIN chimeras), herpes virus vectors, measles virus vectors, pox virus vectors (e.g., vaccinia virus, modified vaccinia virus Ankara (MVA), NYVAC (derived from the Copenhagen strain of vaccinia), and avipox vectors: canarypox (ALVAC) and fowlpox (FPV) vectors), and vesicular stomatitis virus vectors, or virus like particles. As used herein, the term "virus-like particle" or "VLP" refers to a non-replicating, empty viral shell. VLPs are generally composed of one or more viral proteins, such as, but not limited to those proteins referred to as capsid, coat, shell, surface and/or envelope proteins. They contain functional viral proteins responsible for cell penetration by the virus, which ensures efficient cell entry. Methods for producing particular VLPs are known in the art.

The term "mitogen" refers to an agent, e.g. a peptide or protein that induces a cell to begin cell division. The term "cell activator" refers to an agent, e.g. a peptide or protein that binds and cross-links cell receptors, e.g. T cell or B cell receptors.

The term "nucleoside analogue" refers to a nucleoside containing a nucleic acid analogue and a sugar. The term "nucleotide analogue" refers to a nucleotide containing a nucleic acid analogue, a sugar and a phosphate group with 1 to 3 phosphates. The term "nucleic acid analogue" refers to a compound that is structurally similar to RNA and DNA in that it has one or more of phosphate backbone, pentose sugar and/or the nucleobase altered such that it has different base pairing and/or base stacking properties. Examples include peptide nucleic acid (PNA), Morpholino and locked nucleic acid (LNA), as well as glycol nucleic acid (GNA) and threose nucleic acid (TNA).

The term "RNAi molecule" refers to a molecule capable of neutralizing mRNA molecules in a target-sequence-specific manner. Examples include dsRNA, miRNA and siRNA.

Particular preferred pharmaceutical forms for the administration of the agents according to the invention are forms suitable for injectable use and include sterile aqueous solutions or dispersions and sterile powders for the extemporaneous preparation of sterile injectable solutions or dispersion. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. Infusion or injection solutions can be accomplished by any number of art-recognized techniques including but not limited to addition of preservatives like anti-bacterial or anti-fungal agents, e.g. parabene, chlorobutanol, phenol, sorbic acid or thiomersal. Further, isotonic agents, such as sugars or salts, in particular sodium chloride may be incorporated in infusion or injection solutions.

Preferred diluents of the present invention are water, physiological acceptable buffers, physiological acceptable buffer salt solutions or salt solutions. Excipients which can be used with the various pharmaceutical forms of the pharmaceutical according to the invention can be chosen from the following non-limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like,
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

The term "pharmaceutically acceptable carrier" refers to any substrate which serves to improve the selectivity, effectiveness, and/or safety of drug administration. Such carriers can be used to control the release of a drug into systemic circulation. This can be accomplished either by slow release of the drug over a long period of time (typically diffusion) or by triggered release at the drug's target by some stimulus, such as changes in pH, application of heat, and activation by light. Carriers can also be used to improve the pharmacokinetic properties, specifically the bioavailability, of many drugs with poor water solubility and/or membrane permeability. A wide variety of drug carrier systems have been developed and studied. Examples include liposomes, polymeric micelles, microspheres, nanoparticles, nanofibers, protein-drug conjugates, erythrocytes, virosomes and dendrimers. Different methods of attaching the drug to the carrier can be used, including adsorption, integration into the bulk structure, encapsulation, and covalent bonding.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

The invention is described by way of the following examples which are to be construed as merely illustrative and not limitative of the scope of the invention.

### EXAMPLE 1

### Aim

Testing the antiviral activity of inactivated parapoxvirus *orf* (iPPVO) virions against coronavirus. PPVO virions stimulate the innate as well as the adaptive arm of the immune system.

### Approach

In whole blood, APCs (antigen-presenting cells) take up the iPPVO virion, which leads to their activation and the release of cytokines. This in turn activates T cells as well as NK cells which also start to proliferate and release cytokines. After 3 days of incubation the cells are spun down and the supernatant containing soluble factors like cytokines are harvested. These supernatants are assayed for their activity on coronavirus.

### Test substances

- iPPVO of strain D1701 (available from Pfizer); 230 IFN units reconstituted in 2 ml water
- iPPVO vehicle (PPVO buffer only, i.e. without virus), negative control
- PBS, negative control
- Concavalin (Con) A, co-stimulus, stock solution 1 mg/ml PBS = 10X (used in well at 5 µg/ml)

### Further materials

- Whole blood of a healthy donor: 9 ml whole blood freshly collected in a Sarstedt Monovette, Li-Heparin
- Medium for whole blood incubation: RPMI (500 ml) supplemented with 1% GlutaMAX™ and 0.25 ml Heparin-Sodium (5000 U)
- Cells of coronavirus assay: VeroE6-EGFP propagated in growth medium prepared by supplementing DMEM with 10% v/v heat-inactivated FCS and 5 mL sodium bicarbonate 7.5%, and cultured in T150 bottle and split 1/4 twice a week. Pen-strep added directly to the T150 bottle at a 1/100 dilution.
- Coronavirus assay medium: prepared by supplementing DMEM with 2% v/v heat-inactivated FCS and 5 mL sodium bicarbonate 7.5%.

### Preparation of samples

Per well in a 96 well round-bottom plate: 120 µl cell medium for whole blood incubation + 20 µl ConA or PBS + 20 µl iPPVO, iPPVO vehicle or PBS + 40 µl whole blood.

The plate was incubated at 37 °C, 5 % CO₂ for 72 hrs and then centrifuged for 5 min at 230 g. The supernatant was aliquoted to new 96 well plates (plate layout maintained). Sample plates were frozen at -80 °C until further use.

### Coronavirus assay

Dilution of the samples (test substance as prepared above): 100 µL assay medium were added to wells of a Greiner Bio One 655090 plate and 15 µL were removed again from wells (except from wells for controls of this assay: virus control (VC) controlling for virus replication and effect on cells, and cell control (CC) for controlling cell viability without virus). 15 µL from wells of sample plate was added to wells of which 15 µL medium was removed.

Preparation of the cell suspension: A confluent culture (monolayer) of VeroE6-EGFP in T150 bottle was washed with DPBS and 10 mL trypsine 0.25%trypsine/EDTA was added. The bottle was incubated for 1 minute at room temperature, and trypsine/EDTA was removed except for 2 mL. The bottle was then incubated for 15 minutes at 37°C. After incubation, the cells were resuspended in 10 mL assay medium, passed through a Cell Strainer (FALCON CAT NO 352350) and counted using coulter (3 samples of 10 µL in 10 mL were counted). 3000 cells were then resuspended in 50 µL assay medium and 50 µL of the cell suspension were seeded to each well of the Greiner plate.

Assay: Plates with cell suspension as prepared above were incubated overnight (37°C / 5%CO₂), after which SARS-CoV-2 (strain BetaCoV/Belgium/GHB-03021/2020, stock titer 4.8 x 10⁷ TCID50/ml) was added to all wells (except the cell control wells) at a final dilution of 1/200,0000 in 200 µl/well (3000 cells/well, resulting in an MOI of 0.016 TCID₅₀/cell). The plates were incubated at 37°C / 5% CO₂ for 5 days, after which GFP signal was measured using whole-well fluorescence, HCI (High Content Imaging) and the ImageJ software. Reduced EGFP expression is a marker for virus-induced cytopathic activity. See also Ivens et al. (Journal of Virological Methods 129, 2005, 56-63).

### Results

The results are shown in Fig. 1. Cells not infected with coronavirus (CC mean) were viable and expressed eGFP. Cells infected with coronavirus (VC mean) died and no eGFP expression was detectable. Results for PB S and iPPVO vehicle alone were similar to VC mean, i.e. PBS and iPPVO vehicle alone had no effect on cell viability, i.e. against coronavirus, as expected. Also 5 µg/ml ConA alone (in PBS) did not retain eGFP expression, and it also did not affect eGFP expression when given together with iPPVO vehicle as shown by the comparison to ConA alone in PBS. iPPVO D1701 alone (in PBS) also had a clear positive effect on cell viability, and eGFP expression was further increased when iPPVO D1701 was used in combination with 5 µg/ml ConA (which by itself showed no effect on eGFP expression at this concentration). Accordingly, iPPVO and ConA act synergistically.

These results show the efficacy of parapoxvirus in promoting an antiviral cell state which is protective and inhibits coronavirus replication and propagation and thereby the infection of further cells. This effect can be improved by adding an immune stimulatory agent such as ConA, which acts synergistically, i.e. the efficacy of iPPVO is enhanced in a combinatorial approach.

### EXAMPLE 2

### Aim

Determining whether the proof-of-principle shown in Example 1 applies to parapoxvirus in general by testing the antiviral activity of a further parapoxvirus strain against coronavirus.

### Approach

See Example 1.

### Test substances

- iPPVO of strain NZ2 (chemically inactivated by binary ethyleneimine, BEI), internal designation AIC649, 1x10⁹ lyophilized virions reconstituted in 1 ml water
- iPPVO of strain D1701, see Example 1
- iPPVO vehicle, negative control, see Example 1

### Further materials, preparation of samples, coronavirus assay

See Example 1. iPPVO and iPPVO vehicle were used alone. iPPVO D1701 shown to increase cell viability in Example 1 was used as a positive control in Example 2.

### Results

The results are shown in Fig. 2. iPPVO D1701, which in Example 1 was shown to be effective, was used as a positive control and as a 100% benchmark. Both PPVO strains D1701 and NZ2 are active against coronavirus as shown by the comparison to the negative control. Accordingly, the promotion of an antiviral cell state which is effective against coronavirus is a hallmark of parapoxvirus in general and not only of the strain tested in Example 1.

### EXAMPLE 3

### Aim

Testing the antiviral activity of Letermovir alone or in combination with iPPVO virions against coronavirus.

### Approach

Letermovir is used to act directly on cells infected or to be infected with coronavirus. For iPPVO, see Example 1.

### Test substances

- iPPVO of strain D1701, see Example 1
- iPPVO vehicle, see Example 1
- Letermovir, starting with concentrations of 100µM and further dilutions
- PBS, negative control
- ConA, see Example 1

### Further materials, preparation of samples, and coronavirus assay

See Examples 1 and 2.

For preparing Letermovir and its dilutions: 50 µL assay medium were added to wells of a Greiner Bio One 655090 plate. 3 µL of a 10 mM stock solution of Letermovir were added to a first column of the plate to get at final start concentration of 100 µM of Letermovir per well in the coronavirus assay plate later on. For diluting a 10 mM stock solution of Letermovir to the final start concentration of 10 µM a 1/20 predilution in medium was prepared (example: 5 µL of the 10 mM Letermovir stock solution were mixed with 100 µL in column 1) and 3 µL of this predilution were transferred to column 2. Further 1/3 dilutions were prepared by transferring by transferring 50 µL from one column of the plate to another.

Letermovir or its dilutions were added at different time points (before or after infecting the VeroE6-EGFP cells with the coronavirus) to the cell plate (see Example 1). When testing Letermovir alone, no supernatants from whole blood assay were added to the VeroE6-EGFP cells.

### EXAMPLE 4

### Aim

Investigating the timing of parapoxvirus and Letermovir addition, specifically determining how the antiviral activity of parapoxvirus shown in Examples 1 and 2 is modulated by adding the supernatants at different time points to the cells of the coronavirus assay.

### Approach

See Examples 1 and 2, but with different time points for adding the supernatants of the sample plate to the cells of the coronavirus assay (before or after the infection of the VeroE6-EGFP cells with SARS-CoV-2).

### Test substances

- iPPVO of strain NZ2, see Example 2
- iPPVO of strain D1701, see Example 1
- iPPVO vehicle, negative control, see Example 1
- ConA, see Example 1
- Letermovir, see Example 3

### Further materials, preparation of samples, coronavirus assay

See Examples 1, 2 and 3. Whole blood was incubated with Letermovir, iPPVO or iPPVO vehicle (alone or in combination with ConA). Supernatants were generated and added at different timepoints before or after the infection of the VeroE6-EGFP cells with the coronavirus (SARS-CoV-2). When both Letermovir and supernatant samples were added to the coronavirus assay plate the volumes were adjusted appropriately.

### EXAMPLE 5

### Aim

Testing further combinatorial approaches, specifically confirming that the strength of the antiviral activity of parapoxvirus and Letermovir according to Examples 1, 2, and 3 can be enhanced in combinatorial approaches using further combination partners, e.g. an immune stimulatory agent or an antiviral as described herein. This includes investigating the timing of drug addition (parapoxvirus to blood samples and combination partner to blood sample or directly to cells of coronavirus assay), i.e. relative to each other, and also the timing of adding the coronavirus relative to parapoxvirus and Letermovir and/or a combination partner. It also includes testing the influence of the concentrations of parapoxvirus, Letermovir and combination partners.

### Approach

See Examples 1, 2, 3 and 4, but with different time points for adding the supernatants (of blood treated with parapoxvirus/Letermovir and/or combination partner, at the same or different time points) of the sample plate to the cells of the coronavirus assay (before or after the infection of the VeroE6-EGFP cells with SARS-CoV-2), and with different time points for adding the combination partner (if not already added to the blood sample) to the cells of the coronavirus assay (before or after the supernatant addition and/or the SARS-CoV-2 addition).

### Test substances

- iPPVO of strain NZ2, see Example 2
- iPPVO of strain D1701, see Examples 1
- iPPVO vehicle, negative control, see Examples 1
- Letermovir, see Example 3
- ConA, see Example 1 (combination partner control)
- Thalidomide (combination partner)
- Remdesivir (combination partner)

### Further materials, preparation of samples, coronavirus assay

See Examples 1, 2 and 3. Whole blood was incubated with Letermovir, or vehicle (alone or in combination with one or more combination partners). Supernatants were added at different timepoints before or after the infection of the VeroE6-EGFP cells with the coronavirus (SARS-CoV-2). One or more combination partners not used for incubation of the blood samples were added at different time points to the VeroE6-EGFP cells (before both the supernatant and the coronavirus, after both or between the two). Different concentrations of the combination partners were used. When both combination partner and supernatant samples were added to the coronavirus assay plate the volumes were adjusted appropriately.

## Claims

1. Letermovir or a pharmaceutically acceptable salt, solvate or hydrate thereof for use in (i) conditioning a subject for a coronavirus infection or (ii) treating a coronavirus infection in a subject, wherein the use optionally comprises administering a parapoxvirus agent.

2. A parapoxvirus agent for use in (i) conditioning a subject for a coronavirus infection or (ii) treating a coronavirus infection in a subject, wherein the use comprises administering Letermovir or a pharmaceutically acceptable salt, solvate or hydrate thereof.

3. A combination of a parapoxvirus agent and Letermovir or a pharmaceutically acceptable salt, solvate or hydrate thereof for use in (i) conditioning a subject for a coronavirus infection or (ii) treating a coronavirus infection in a subject.

4. Letermovir or a pharmaceutically acceptable salt, solvate or hydrate thereof for use according to claim 1, the parapoxvirus agent for use according to claim 2, or the combination for use according to claim 3, wherein the parapoxvirus agent is:
(a) the parapoxvirus itself or a fragment thereof, or
(b) an agent that comprises genetic information encoding for (a),
preferably wherein the parapoxvirus agent is an inactivated PPVO virion.

5. Letermovir or a pharmaceutically acceptable salt, solvate or hydrate thereof for use according to claim 1 or 4, the parapoxvirus agent for use according to claim 2 or 4, or the combination for use according to claim 3 or 4, wherein the coronavirus is a SARS coronavirus, preferably SARS-CoV-2 or a variant thereof with at least 80% sequence identity to SEQ ID NO: 1 or 2.

6. Letermovir or a pharmaceutically acceptable salt, solvate or hydrate thereof for use according to any one of claims 1, 4 or 5, the parapoxvirus agent for use according to any one of claims 2, 4 or 5, or the combination for use according to any one of claims 3-5, wherein the subject to be conditioned is **characterized by** one or more risk factors for contracting a coronavirus infection and/or by one or more risk factors for having a severe course of coronavirus disease.

7. Letermovir or a pharmaceutically acceptable salt, solvate or hydrate thereof for use according to any one of claims 1 or 4-6, the parapoxvirus agent for use according to any one of claims 2 or 4-6, or the combination for use according to any one of claims 3-6, wherein the subject to be treated is asymptomatic.

8. Letermovir or a pharmaceutically acceptable salt, solvate or hydrate thereof for use according to any one of claims 1, or 4-7, the parapoxvirus agent for use according to any one of claims 2 or 4-7, or the combination for use according to any one of claims 3-7, wherein the subject to be treated has coronavirus disease, preferably Covid-19.

9. Letermovir or a pharmaceutically acceptable salt, solvate or hydrate thereof for use according to claim 8, the parapoxvirus agent for use according to claim 8, or the combination for use according to claim 8, wherein the subject has either no severe symptoms, or has a severe course of coronavirus disease.

10. Letermovir or a pharmaceutically acceptable salt, solvate or hydrate thereof for use according to any one of claims 1 or 4-9, the parapoxvirus agent for use according to any one of claims 2 or 4-9, or the combination for use according to any one of claims 3-9, wherein the use comprises the administration of a further treatment regimen for (i) or (ii).

11. Letermovir or a pharmaceutically acceptable salt, solvate or hydrate thereof for use according to claim 10, the parapoxvirus agent for use according to claim 10, or the combination for use according to claim 10, wherein the further treatment regimen for (i) comprises administering a coronavirus agent and/or an immune stimulatory agent, and the further treatment regimen for (ii) comprises administering a coronavirus agent, one or more further medicaments and/or one or more further non-drug treatments.

12. Letermovir or a pharmaceutically acceptable salt, solvate or hydrate thereof for use according to claim 11, the parapoxvirus agent for use according to claim 11, or the combination for use according to claim 11, wherein
- the coronavirus agent is selected from the group consisting of:
(a) the coronavirus itself or a fragment thereof,
(b) an agent that comprises genetic information encoding for (a), and
(c) an agent which is capable upon administration of an effective amount thereof to a subject infected with coronavirus to interfere with the infection of a cell by the coronavirus or with the replication of the coronavirus within a cell;
- the further medicament is selected from the group consisting of an immune stimulatory agent, an antiviral, an antibiotic, an adjuvant, a glucocorticoid, an antihypertensive drug, an hypoglycaemic drug, an anti-shock drug, and a drug suitable for treating one or more symptoms of coronavirus disease; and/or
- the one or more further therapies are preferably selected from the group consisting of oxygen therapy and extra-corporeal organ support (ECOS).

13. Letermovir or a pharmaceutically acceptable salt, solvate or hydrate thereof for use according to any one of claims 1 or 4-12, the parapoxvirus agent for use according to any one of claims 2 or 4-12, or the combination for use according to any one of claims 3-12, wherein Letermovir and/or the parapoxvirus is/are capable upon administration of an effective amount thereof to a subject infected with coronavirus to
(i) increase the number of one or more of coronavirus-antigen-specific immune factors,
(ii) inhibit coronavirus replication,
(iii) reduce the coronavirus load, and/or
(iv) prevent or ameliorate a coronavirus-specific cytokine profile or a cytokine storm.

14. A composition or a kit comprising (a) Letermovir or a pharmaceutically acceptable salt, solvate or hydrate thereof, or preferably a parapoxvirus agent and Letermovir or a pharmaceutically acceptable salt, solvate or hydrate thereof, and (b) a coronavirus agent.

15. A pharmaceutical composition or kit for use in (i) conditioning a subject for a coronavirus infection or (ii) treating a coronavirus infection in a subject, wherein the pharmaceutical composition or kit comprises (a) Letermovir, or preferably a parapoxvirus agent and Letermovir, and (b) a further medicament.


SEQUENCE LISTING

<110>  AiCuris GmbH & Co.KG

<120>  PARAPOXVIRUS FOR CONDITIONING FOR AND TREATMENT OF CORONAVIRUS
       INFECTIONS

<130>  1092-3 EP

<160>  3

<170>  PatentIn version 3.5

<210>  1
<211>  29903
<212>  RNA
<213>  Artificial Sequence

<220>
<223>  SARS-CoV-2

<400>  1
auuaaagguu uauaccuucc cagguaacaa accaaccaac uuucgaucuc uuguagaucu      60

guucucuaaa cgaacuuuaa aaucugugug gcugucacuc ggcugcaugc uuagugcacu     120

cacgcaguau aauuaauaac uaauuacugu cguugacagg acacgaguaa cucgucuauc     180

uucugcaggc ugcuuacggu uucguccgug uugcagccga ucaucagcac aucuagguuu     240

cguccgggug ugaccgaaag guaagaugga gagccuuguc ccugguuuca acgagaaaac     300

acacguccaa cucaguuugc cuguuuuaca gguucgcgac gugcucguac guggcuuugg     360

agacuccgug gaggaggucu uaucagaggc acgucaacau cuuaaagaug gcacuugugg     420

cuuaguagaa guugaaaaag gcguuuugcc ucaacuugaa cagcccuaug uguucaucaa     480

acguucggau gcucgaacug caccucaugg ucauguuaug guugagcugg uagcagaacu     540

cgaaggcauu caguacgguc guagugguga gacacuuggu guccuugucc cucauguggg     600

cgaaauacca guggcuuacc gcaagguucu ucuucguaag aacgguaaua aaggagcugg     660

uggccauagu uacggcgccg aucuaaaguc auuugacuua ggcgacgagc uuggcacuga     720

uccuuaugaa gauuuucaag aaaacuggaa cacuaaacau agcaguggug uuacccguga     780

acucaugcgu gagcuuaacg gaggggcaua cacucgcuau gucgauaaca acuucugugg     840

cccugauggc uacccucuug agugcauuaa agaccuucua gcacgugcug guaaagcuuc     900

augcacuuug uccgaacaac uggacuuuau ugacacuaag agggguguau acugcugccg     960

ugaacaugag caugaaauug cuugguacac ggaacguucu gaaaagagcu augaauugca    1020

gacaccuuuu gaaauuaaau uggcaaagaa auuugacacc uucaaugggg aauguccaaa    1080

uuuuguauuu cccuuaaauu ccauaaucaa gacuauucaa ccaaggguug aaaagaaaaa    1140

gcuugauggc uuuaugggua gaauucgauc ugucuaucca guugcgucac caaaugaaug    1200

caaccaaaug ugccuuucaa cucucaugaa gugugaucau uguggugaaa cuucauggca    1260

gacgggcgau uuuguuaaag ccacuugcga auuuuguggc acugagaauu ugacuaaaga 1320

aggugccacu acuugugguu acuuacccca aaaugcuguu guuaaaauuu auuguccagc 1380

augucacaau ucagaaguag gaccugagca uagucuugcc gaauaccaua augaaucugg 1440

cuugaaaacc auucuucgua aggguggucg cacuauugcc uuuggaggcu guguguucuc 1500

uuauguuggu ugccauaaca agugugccua uuggguucca cgugcuagcg cuaacauagg 1560

uuguaaccau acagguguug uuggagaagg uuccgaaggu cuuaaugaca accuucuuga 1620

aauacuccaa aaagagaaag ucaacaucaa uauuguuggu gacuuuaaac uuaaugaaga 1680

gaucgccauu auuuuggcau cuuuuucugc uuccacaagu gcuuuugugg aaacugugaa 1740

agguuuggau uauaaagcau ucaaacaaau uguugaaucc ugugguaauu uuaaaguuac 1800

aaaaggaaaa gcuaaaaaag gugccuggaa uauuggugaa cagaaaucaa uacugagucc 1860

ucuuuaugca uuugcaucag aggcugcucg uguuguacga ucaauuuucu cccgcacucu 1920

ugaaacugcu caaaauucug ugcguguuuu acagaaggcc gcuauaacaa uacuagaugg 1980

aauuucacag uauucacuga gacucauuga ugcuaugaug uucacaucug auuuggcuac 2040

uaacaaucua guuguaaugg ccuacauuac aggugguguu guucaguuga cuucgcagug 2100

gcuaacuaac aucuuuggca cuguuuauga aaaacucaaa cccguccuug auuggcuuga 2160

agagaaguuu aaggaaggug uagaguuucu uagagacggu ugggaaauug uuaaauuuau 2220

cucaaccugu gcuugugaaa uugucggugg acaaauuguc accugugcaa aggaaauuaa 2280

ggagaguguu cagacauucu uuaagcuugu aaauaaauuu uuggcuuugu gugcugacuc 2340

uaucauuauu gguggagcua aacuuaaagc cuugaauuua ggugaaacau uugucacgca 2400

cucaaaggga uuguacagaa aguguguuaa auccagagaa gaaacuggcc uacucaugcc 2460

ucuaaaagcc ccaaaagaaa uuaucuucuu agagggagaa acacuuccca cagaaguguu 2520

aacagaggaa guugucuuga aaacugguga uuuacaacca uuagaacaac cuacuaguga 2580

agcuguugaa gcuccauugg uugguacacc aguuuguauu aacgggcuua uguugcucga 2640

aaucaaagac acagaaaagu acugugcccu ugcaccuaau augaugguaa caaacaauac 2700

cuucacacuc aaaggcggug caccaacaaa gguuacuuuu ggugaugaca cugugauaga 2760

agugcaaggu uacaagagug ugaauaucac uuuugaacuu gaugaaagga uugauaaagu 2820

acuuaaugag aagugcucug ccuauacagu ugaacucggu acagaaguaa augaguucgc 2880

cuguguugug gcagaugcug ucauaaaaac uuugcaacca guaucugaau uacuuacacc 2940

acugggcauu gauuuagaug aguggaguau ggcuacauac uacuuauuug augagucugg 3000

ugaguuuaaa uuggcuucac auauguauug uucuuucuac ccuccagaug aggaugaaga 3060

agaaggugau ugugaagaag aagaguuuga gccaucaacu caauaugagu augguacuga 3120

agaugauuac caagguaaac cuuuggaauu uggugccacu ucugcugcuc uucaaccuga 3180

agaagagcaa gaagaagauu gguuagauga ugauagucaa caaacuguug gucaacaaga 3240

cggcagugag gacaaucaga caacuacuau ucaaacaauu guugagguuc aaccucaauu 3300

agagauggaa cuuacaccag uuguucagac uauugaagug aauaguuuua gugguuauuu 3360

aaaacuuacu gacaauguau acauuaaaaa ugcagacauu guggaagaag cuaaaaaggu 3420

aaaaccaaca gugguuguua augcagccaa uguuuaccuu aaacauggag gagguguugc 3480

aggagccuua aauaaggcua cuaacaaugc caugcaaguu gaaucugaug auuacauagc 3540

uacuaaugga ccacuuaaag ugggugguag uuguguuuua agcggacaca aucuugcuaa 3600

acacugucuu cauguugucg gcccaaaugu uaacaaaggu gaagacauuc aacuucuuaa 3660

gagugcuuau gaaaauuuua aucagcacga aguucuacuu gcaccauuau uaucagcugg 3720

uauuuuuggu gcugacccua uacauucuuu aagaguuugu guagauacug uucgcacaaa 3780

ugucuacuua gcugucuuug auaaaaaucu cuaugacaaa cuuguuucaa gcuuuuugga 3840

aaugaagagu gaaaagcaag uugaacaaaa gaucgcugag auuccuaaag aggaaguuaa 3900

gccauuuaua acugaaagua aaccuucagu ugaacagaga aaacaagaug auaagaaaau 3960

caaagcuugu guugaagaag uuacaacaac ucuggaagaa acuaaguucc ucacagaaaa 4020

cuuguuacuu uauauugaca uuaauggcaa ucuucaucca gauucugcca cucuuguuag 4080

ugacauugac aucacuuucu uaaagaaaga ugcuccauau auagugggug auguuguuca 4140

agaggguguu uuaacugcug ugguuauacc uacuaaaaag gcugguggca cuacugaaau 4200

gcuagcgaaa gcuuugagaa aagugccaac agacaauuau auaaccacuu acccggguca 4260

ggguuuaaau gguuacacug uagaggaggc aaagacagug cuuaaaaagu guaaaagugc 4320

cuuuuacauu cuaccaucua uuaucucuaa ugagaagcaa gaaauucuug gaacuguuuc 4380

uuggaauuug cgagaaaugc uugcacaugc agaagaaaca cgcaaauuaa ugccugucug 4440

uguggaaacu aaagccauag uuucaacuau acagcguaaa uauaagggua uuaaaauaca 4500

agagggugug guugauuaug gugcuagauu uuacuuuuac accaguaaaa caacuguagc 4560

gucacuuauc aacacacuua acgaucuaaa ugaaacucuu guuacaaugc cacuuggcua 4620

uguaacacau ggcuuaaauu uggaagaagc ugcucgguau augagaucuc ucaaagugcc 4680

agcuacaguu ucuguuucuu caccugaugc uguuacagcg uauaaugguu aucuuacuuc 4740

uucuucuaaa acaccugaag aacauuuuau ugaaaccauc ucacuugcug guuccuauaa 4800

agauuggucc uauucuggac aaucuacaca acuagguaua gaauuucuua agagagguga 4860

uaaaagugua uauuacacua guaauccuac cacauuccac cuagauggug aaguuaucac 4920

cuuugacaau cuuaagacac uucuuucuuu gagagaagug aggacuauua agguguuuac 4980

aacaguagac aacauuaacc uccacacgca aguuguggac augucaauga cauauggaca 5040

acaguuuggu ccaacuuauu uggauggagc ugauguuacu aaaauaaaac cucauaauuc 5100

acaugaaggu aaaacauuuu auguuuuacc uaaugaugac acucuacgug uugaggcuuu 5160

ugaguacuac cacacaacug auccuaguuu ucuggguagg uacaugucag cauuaaauca 5220

cacuaaaaag uggaaauacc cacaaguuaa ugguuuaacu ucuauuaaau gggcagauaa 5280

caacuguuau cuugccacug cauuguuaac acuccaacaa auagaguuga aguuuaaucc 5340

accugcucua caagaugcuu auuacagagc aagggcuggu gaagcugcua acuuuugugc 5400

acuuaucuua gccuacugua auaagacagu aggugaguua ggugauguua gagaaacaau 5460

gaguuacuug uuucaacaug ccaauuuaga uucuugcaaa agagucuuga acguggugug 5520

uaaaacuugu ggacaacagc agacaacccu uaagggugua gaagcuguua uguacauggg 5580

cacacuuucu uaugaacaau uuaagaaagg uguucagaua ccuuguacgu gugguaaaca 5640

agcuacaaaa uaucuaguac aacaggaguc accuuuuguu augaugucag caccaccugc 5700

ucaguaugaa cuuaagcaug guacauuuac uugugcuagu gaguacacug guaauuacca 5760

guguggucac uauaaacaua uaacuucuaa agaaacuuug uauugcauag acggugcuuu 5820

acuuacaaag uccucagaau acaaaggucc uauuacggau guuuucuaca aagaaaacag 5880

uuacacaaca accauaaaac caguuacuua uaaauuggau gguguuguuu guacagaaau 5940

ugacccuaag uuggacaauu auuauaagaa agacaauucu uauuucacag agcaaccaau 6000

ugaucuugua ccaaaccaac cauauccaaa cgcaagcuuc gauaauuuua aguuuguaug 6060

ugauaauauc aaauuugcug augauuuaaa ccaguuaacu gguuauaaga aaccugcuuc 6120

aagagagcuu aaaguuacau uuuucccuga cuuaaauggu gauguggugg cuauugauua 6180

uaaacacuac acacccucuu uuaagaaagg agcuaaauug uuacauaaac cuauuguuug 6240

gcauguuaac aaugcaacua auaaagccac guauaaacca aauaccuggu guauacguug 6300

ucuuuggagc acaaaaccag uugaaacauc aaauucguuu gauguacuga agucagagga 6360

cgcgcaggga auggauaauc uugccugcga agaucuaaaa ccagucucug aagaaguagu 6420

ggaaaauccu accauacaga aagacguucu ugaguguaau gugaaaacua ccgaaguugu 6480

aggagacauu auacuuaaac cagcaaauaa uaguuuaaaa auuacagaag agguuggcca 6540

cacagaucua auggcugcuu auguagacaa uucuagucuu acuauuaaga aaccuaauga 6600

auuaucuaga guauuagguu ugaaaacccu ugcuacucau gguuuagcug cuguuaauag 6660

ugucccuugg gauacuauag cuaauuaugc uaagccuuuu cuuaacaaag uuguuaguac 6720

aacuacuaac auaguuacac gguguuuaaa ccguguuugu acuaauuaua ugccuuauuu 6780

cuuuacuuua uugcuacaau uguguacuuu uacuagaagu acaaauucua gaauuaaagc 6840

aucuaugccg acuacuauag caaagaauac uguuaagagu gucgguaaau uuugucuaga 6900

ggcuucauuu aauuauuuga agucaccuaa uuuuucuaaa cugauaaaua uuauaauuug 6960

guuuuuacua uuaaguguuu gccuagguuc uuuaaucuac ucaaccgcug cuuuaggugu 7020

uuuaaugucu aauuuaggca ugccuucuua cuguacuggu uacagagaag gcuauuugaa 7080

cucuacuaau gucacuauug caaccuacug uacugguucu auaccuugua guguuugucu 7140

uagugguuua gauucuuuag acaccuaucc uucuuuagaa acuauacaaa uuaccauuuc 7200

aucuuuuaaa ugggauuuaa cugcuuuugg cuuaguugca gagugguuuu uggcauauau 7260

ucuuuucacu agguuuuucu auguacuugg auuggcugca aucaugcaau uguuuuucag 7320

cuauuuugca guacauuuua uuaguaauuc uuggcuuaug ugguuaauaa uuaaucuugu 7380

acaaauggcc ccgauuucag cuaugguuag aauguacauc uucuuugcau cauuuuauua 7440

uguauggaaa aguuaugugc auguuguaga cgguuguaau ucaucaacuu guaugaugug 7500

uuacaaacgu aauagagcaa caagagucga auguacaacu auuguuaaug guguuagaag 7560

guccuuuuau gucuaugcua auggagguaa aggcuuuugc aaacuacaca auuggaauug 7620

uguuaauugu gauacauucu gugcugguag uacauuuauu agugaugaag uugcgagaga 7680

cuugucacua caguuuaaaa gaccaauaaa uccuacugac cagucuucuu acaucguuga 7740

uaguguuaca gugaagaaug guuccaucca ucuuuacuuu gauaaagcug gucaaaagac 7800

uuaugaaaga cauucucucu cucauuuugu uaacuuagac aaccugagag cuaauaacac 7860

uaaagguuca uugccuauua auguuauagu uuuugauggu aaaucaaaau gugaagaauc 7920

aucugcaaaa ucagcgucug uuuacuacag ucagcuuaug ugucaaccua uacuguuacu 7980

agaucaggca uuagugucug auguugguga uagugcggaa guugcaguua aaauguuuga 8040

ugcuuacguu aauacguuuu caucaacuuu uaacguacca auggaaaaac ucaaaacacu 8100

aguugcaacu gcagaagcug aacuugcaaa gaaugugucc uuagacaaug ucuuaucuac 8160

uuuuauuuca gcagcucggc aaggguuugu ugauucagau guagaaacua aagauguugu 8220

ugaaugucuu aaauugucac aucaaucuga cauagaaguu acuggcgaua guuguaauaa 8280

cuauaugcuc accuauaaca aaguugaaaa caugacaccc cgugaccuug gugcuuguau 8340

ugacuguagu gcgcgucaua uuaaugcgca gguagcaaaa agucacaaca uugcuuugau 8400

auggaacguu aaagauuuca ugucauuguc ugaacaacua cgaaaacaaa uacguagugc 8460

ugcuaaaaag aauaacuuac cuuuuaaguu gacaugugca acuacuagac aaguuguuaa 8520

uguuguaaca acaaagauag cacuuaaggg ugguaaaauu guuaauaauu gguugaagca 8580

guuaauuaaa guuacacuug uguuccuuuu uguugcugcu auuucuauuu uaauaacacc 8640

uguucauguc augucuaaac auacugacuu uucaagugaa aucauaggau acaaggcuau 8700

ugaugguggu gucacucgug acauagcauc uacagauacu uguuuugcua acaaacaugc 8760

ugauuuugac acaugguuua gccagcgugg ugguaguuau acuaaugaca aagcuugccc 8820

auugauugcu gcagucauaa caagagaagu ggguuuuguc gugccugguu ugccuggcac 8880

gauauuacgc acaacuaaug gugacuuuuu gcauuucuua ccuagaguuu uuagugcagu 8940

ugguaacauc uguuacacac caucaaaacu uauagaguac acugacuuug caacaucagc 9000

uuguguuuug gcugcugaau guacaauuuu uaaagaugcu ucugguaagc caguaccaua 9060

uuguuaugau accaauguac uagaagguuc uguugcuuau gaaaguuuac gcccugacac 9120

acguuaugug cucauggaug gcucuauuau ucaauuuccu aacaccuacc uugaagguuc 9180

uguuagagug guaacaacuu uugauucuga guacuguagg cacggcacuu gugaaagauc 9240

agaagcuggu guuuguguau cuacuagugg uagaugguua cuuaacaaug auuauuacag 9300

aucuuuacca ggaguuuucu gugguguaga ugcuguaaau uuacuuacua auauguuuac 9360

accacuaauu caaccuauug gugcuuugga cauaucagca ucuauaguag cugguggua u 9420

uguagcuauc guaguaacau gccuugccua cuauuuuaug agguuuagaa gagcuuuugg 9480

ugaauacagu cauguaguug ccuuuaauac uuuacuauuc cuuaugucau ucacuguacu 9540

cuguuuaaca ccaguuuacu cauucuuacc ugguguuuau ucuguuauuu acuuguacuu 9600

gacauuuuau cuuacuaaug auguuucuuu uuuagcacau auucagugga ugguuauguu 9660

cacaccuuua guaccuuucu ggauaacaau ugcuuauauc auuuguauuu ccacaaagca 9720

uuucuauugg uucuuuagua auuaccuaaa gagacgugua gucuuuaaug guguuuccuu 9780

uaguacuuuu gaagaagcug cgcugugcac cuuuuuguua aauaaagaaa uguaucuaaa 9840

guugcguagu gaugugcuau uaccucuuac gcaauauaau agauacuuag cucuuuauaa 9900

uaaguacaag uauuuuagug gagcaaugga uacaacuagc uacagagaag cugcuuguug 9960

ucaucucgca aaggcucuca augacuucag uaacucaggu ucugauguuc uuuaccaacc 10020

accacaaacc ucuaucaccu cagcuguuuu gcagaguggu uuuagaaaaa uggcauuccc 10080

aucugguaaa guugaggguu guaugguaca aguaacuugu gguacaacua cacuuaacgg 10140

ucuuuggcuu gaugacguag uuuacugucc aagacaugug aucugcaccu cugaagacau 10200

gcuuaacccu aauuaugaag auuuacucau ucguaagucu aaucauaauu ucuugguaca 10260

ggcugguaau guucaacuca ggguuauugg acauucuaug caaaauugug uacuuaagcu 10320

uaagguugau acagccaauc cuaagacacc uaaguauaag uuuguucgca uucaaccagg 10380

acagacuuuu ucaguguuag cuuguuacaa ugguucacca ucugguguuu accaaugugc 10440

uaugaggccc aauuucacua uuaaggguuc auuccuuaau gguucaugug guaguguugg 10500

uuuuaacaua gauuaugacu gugucucuuu uuguuacaug caccauaugg aauuaccaac 10560

uggaguucau gcuggcacag acuuagaagg uaacuuuuau ggaccuuuug uugacaggca 10620

aacagcacaa gcagcuggua cggacacaac uauuacaguu aauguuuuag cuugguugua 10680

cgcugcuguu auaaauggag acaggugguu ucucaaucga uuuaccacaa cucuuaauga 10740

cuuuaaccuu guggcuauga aguacaauua ugaaccucua acacaagacc auguugacau 10800

acuaggaccu cuuucugcuc aaacuggaau ugccguuuua gauaugugug cuucauuaaa 10860

agaauuacug caaaauggua ugaauggacg uaccauauug gguagugcuu uauuagaaga 10920

ugaauuuaca ccuuuugaug uuguuagaca augcucaggu guuacuuucc aaagugcagu 10980

gaaaagaaca aucaagguaa cacaccacug guuguuacuc acaauuuuga cuucacuuuu 11040

aguuuuaguc cagaguacuc aauggucuuu guucuuuuuu uuguaugaaa augccuuuuu 11100

accuuuugcu augguauuaa uugcuauguc ugcuuuugca augauguuug ucaaacauaa 11160

gcaugcauuu cucuguuugu uuuuguuacc uucucuugcc acuguagcuu auuuuaauau 11220

ggucuauaug ccugcuaguu gggugaugcg uauuaugaca ugguuggaua ugguugauac 11280

uaguuugucu gguuuuaagc uaaaagacug uguuauguau gcaucagcug uaguguuacu 11340

aauccuuaug acagcaagaa cuguguauga ugauggugcu aggagagugu ggacacuuau 11400

gaaugucuug acacucguuu auaaaguuua uuaugguaau gcuuuagauc aagccauuuc 11460

caugugggcu cuuauaaucu cuguuacuuc uaacuacuca gguguaguua caacugucau 11520

guuuuuggcc agagguauug uuuuuaugug uguugaguau ugcccuauuu ucuucauaac 11580

ugguaauaca cuucagugua uaaugcuagu uuauuguuuc uuaggcuauu uuuguacuug 11640

uuacuuuggc cucuuuuguu uacucaaccg cuacuuuaga cugacucuug guguuuauga 11700

uuacuuaguu ucuacacagg aguuuagaua uaugaauuca cagggacuac ucccacccaa 11760

gaauagcaua gaugccuuca aacucaacau uaaauuguug gguguuggug gcaaaccuug 11820

uaucaaagua gccacuguac agucuaaaau gucagaugua aagugcacau caguagucuu 11880

acucucaguu uugcaacaac ucagaguaga aucaucaucu aaauuguggg cucaaugugu 11940

ccaguuacac aaugacauuc ucuuagcuaa agauacuacu gaagccuuug aaaaaauggu 12000

uucacuacuu ucuguuuugc uuuccaugca gggugcugua gacauaaaca agcuuuguga 12060

agaaaugcug gacaacaggg caaccuuaca agcuauagcc ucagaguuua guucccuucc 12120

aucauaugca gcuuuugcua cugcucaaga agcuuaugag caggcuguug cuaaugguga 12180

uucugaaguu guucuuaaaa aguugaagaa gucuuugaau guggcuaaau cugaauuuga 12240

ccgugaugca gccaugcaac guaaguugga aaagauggcu gaucaagcua ugacccaaau 12300

guauaaacag gcuagaucug aggacaagag ggcaaaaguu acuagugcua ugcagacaau 12360

gcuuuucacu augcuuagaa aguuggauaa ugaugcacuc aacaacauua ucaacaaugc 12420

aagagauggu uguguucccu ugaacauaau accucuuaca acagcagcca aacuaauggu 12480

ugucauacca gacuauaaca cauauaaaaa uacgugugau gguacaacau uuacuuaugc 12540

aucagcauug ugggaaaucc aacagguugu agaugcagau aguaaaauug uucaacuuag 12600

ugaaauuagu auggacaauu caccuaauuu agcauggccu cuuauuguaa cagcuuuaag 12660

ggccaauucu gcugucaaau uacagaauaa ugagcuuagu ccuguugcac uacgacagau 12720

gucuugugcu gccgguacua cacaaacugc uugcacugau gacaaugcgu uagcuuacua 12780

caacacaaca aagggaggua gguuuguacu ugcacuguua uccgauuuac aggauuugaa 12840

augggcuaga uucccuaaga gugauggaac ugguacuauc uauacagaac uggaaccacc 12900

uuguagguuu guuacagaca caccuaaagg uccuaaagug aaguauuuau acuuuauuaa 12960

aggauuaaac aaccuaaaua gagguauggu acuugguagu uuagcugcca caguacgucu 13020

acaagcuggu aaugcaacag aagugccugc caauucaacu guauuaucuu ucugugcuuu 13080

ugcuguagau gcugcuaaag cuuacaaaga uuaucuagcu agugggggac aaccaaucac 13140

uaauuguguu aagauguugu guacacacac ugguacuggu caggcaauaa caguuacacc 13200

ggaagccaau auggaucaag aauccuuugg uggugcaucg uguugucugu acugccguug 13260

ccacauagau cauccaaauc cuaaaggauu uugugacuua aaagguaagu auguacaaau 13320

accuacaacu ugugcuaaug acccuguggg uuuuacacuu aaaaacacag ucuguaccgu 13380

cugcgguaug uggaaagguu auggcuguag uugugaucaa cuccgcgaac ccaugcuuca 13440

gucagcugau gcacaaucgu uuuuaaacgg guuugcggug uaagugcagc ccgucuuaca 13500

ccgugcggca caggcacuag uacugauguc guauacaggg cuuuugacau cuacaaugau 13560

aaaguagcug guuuugcuaa auuccuaaaa acuaauuguu gucgcuucca agaaaaggac 13620

gaagaugaca auuuaauuga uucuuacuuu guaguuaaga gacacacuuu cucuaacuac 13680

caacaugaag aaacaauuua uaauuuacuu aaggauuguc cagcuguugc uaaacaugac 13740

uucuuuaagu uuagaauaga cggugacaug guaccacaua uaucacguca acgucuuacu 13800

aaauacacaa uggcagaccu cgucuaugcu uuaaggcauu uugaugaagg uaauugugac 13860

acauuaaaag aaauacuugu cacauacaau uguugugaug augauuauuu caauaaaaag 13920

gacugguaug auuuuguaga aaacccagau auauuacgcg uauacgccaa cuuaggugaa 13980

cguguacgcc aagcuuuguu aaaaacagua caauucugug augccaugcg aaaugcuggu 14040

auuguuggug uacugacauu agauaaucaa gaucucaaug guaacuggua ugauuucggu 14100

gauuucauac aaaccacgcc agguagugga guuccuguug uagauucuua uuauucauug 14160

uuaaugccua uauuaaccuu gaccagggcu uuaacugcag agucacaugu ugacacugac 14220

uuaacaaagc cuuacauuaa gugggauuug uuaaaauaug acuucacgga agagagguua 14280

aaacucuuug accguuauuu uaaauauugg gaucagacau accacccaaa uuguguuaac 14340

uguuuggaug acagaugcau ucugcauugu gcaaacuuua auguuuuauu cucuacagug 14400

uucccaccua caaguuuugg accacuagug agaaaaauau uuguugaugg uguuccauuu 14460

guaguuucaa cuggauacca cuucagagag cuagguguug uacauaauca ggauguaaac 14520

uuacauagcu cuagacuuag uuuuaaggaa uuacuugugu augcugcuga cccugcuaug 14580

cacgcugcuu cugguaaucu auuacuagau aaacgcacua cgugcuuuuc aguagcugca 14640

cuuacuaaca auguugcuuu ucaaacuguc aaacccggua auuuuaacaa agacuucuau 14700

gacuuugcug ugucuaaggg uuucuuuaag gaaggaaguu cuguugaauu aaaacacuuc 14760

uucuuugcuc aggaugguaa ugcugcuauc agcgauuaug acuacuaucg uuauaaucua 14820

ccaacaaugu gugauaucag acaacuacua uuuguaguug aaguuguuga uaaguacuuu 14880

gauuguuacg augguggcug uauuaaugcu aaccaaguca ucgucaacaa ccuagacaaa 14940

ucagcugguu uuccauuuaa uaaauggggu aaggcuagac uuuauuauga uucaaugagu 15000

uaugaggauc aagaugcacu uuucgcauau acaaaacgua augucauccc uacuauaacu 15060

caaaugaauc uuaaguaugc cauuagugca aagaauagag cucgcaccgu agcugguguc 15120

ucuaucugua guacuaugac caauagacag uuucaucaaa aauuauugaa aucaauagcc 15180

gccacuagag gagcuacugu aguaauugga acaagcaaau ucuauggugg uuggcacaac 15240

auguuaaaaa cuguuuauag ugauguagaa aacccucacc uuaugggaug ggauuauccu 15300

aaaugugaua gagccaugcc uaacaugcuu agaauuaugg ccucacuugu ucuugcucgc 15360

aaacauacaa cguguuguag cuugucacac cguuucuaua gauuagcuaa ugagugugcu 15420

caaguauuga gugaaauggu cauguguggc gguucacuau auguuaaacc agguggaacc 15480

ucaucaggag augccacaac ugcuuaugcu aauaguguuu uuaacauuug ucaagcuguc 15540

acggccaaug uuaaugcacu uuuaucuacu gaugguaaca aaauugccga uaaguauguc 15600

cgcaauuuac aacacagacu uuaugagugu cucuauagaa auagagaugu ugacacagac 15660

uuugugaaug aguuuuacgc auauuugcgu aaacauuucu caaugaugau acucucugac 15720

gaugcuguug uguguuucaa uagcacuuau gcaucucaag gucuaguggc uagcauaaag 15780

aacuuuaagu caguucuuua uuaucaaaac aauguuuuua ugucugaagc aaaauguugg 15840

acugagacug accuuacuaa aggaccucau gaauuuugcu cucaacauac aaugcuaguu 15900

aaacagggug augauuaugu guaccuuccu uacccagauc caucaagaau ccuaggggcc 15960

ggcuguuuug uagaugauau cguaaaaaca gaugguacac uuaugauuga acgguucgug 16020

ucuuuagcua uagaugcuua cccacuuacu aaacauccua aucaggagua ugcugauguc 16080

uuucauuugu acuuacaaua cauaagaaag cuacaugaug aguuaacagg acacauguua 16140

gacauguauu cuguuaugcu uacuaaugau aacacuucaa gguauuggga accugaguuu 16200

uaugaggcua uguacacacc gcauacaguc uuacaggcug uuggggcuug uguucuuugc 16260

aauucacaga cuucauuaag augugguggcu ugcauacgua gaccauucuu auguuguaaa 16320

ugcuguuacg accaugucau aucaacauca cauaaauuag ucuugucugu uaauccguau 16380

guuugcaaug cuccagguug ugaugucaca gaugugacuc aacuuuacuu aggagguaug 16440

agcuauuauu guaaaucaca uaaaccaccc auuaguuuuc cauugugugc uaauggacaa 16500

guuuuugguu uauauaaaaa uacauguguu gguagcgaua auguuacuga cuuuaaugca 16560

auugcaacau gugacuggac aaaugcuggu gauuacauuu uagcuaacac cuguacugaa 16620

agacucaagc uuuuugcagc agaaacgcuc aaagcuacug aggagacauu uaaacugucu 16680

uaugguauug cuacuguacg ugaagugcug ucugacagag aauuacaucu uucaugggaa 16740

guugguaaac cuagaccacc acuuaaccga aauuaugucu uuacugguua ucguguaacu 16800

aaaaacagua aaguacaaau aggagaguac accuuugaaa aaggugacua uggugaugcu 16860

guuguuuacc gagguacaac aacuuacaaa uuaaauguug gugauuauuu ugugcugaca 16920

ucacauacag uaaugccauu aagugcaccu acacuagugc cacaagagca cuauguuaga 16980

auuacuggcu uauacccaac acucaauauc ucagaugagu uuucuagcaa uguugcaaau 17040

uaucaaaagg uugguaugca aaaguauucu acacuccagg gaccaccugg uacugguaag 17100

agucauuuug cuauuggccu agcucucuac uacccuucug cucgcauagu guauacagcu 17160

ugcucucaug ccgcuguuga ugcacuaugu gagaaggcau uaaaauauuu gccuauagau 17220

aaauguagua gaauuauacc ugcacgugcu cguguagagu guuuugauaa auucaaagug 17280

aauucaacau uagaacagua ugucuuuugu acuguaaaug cauugccuga gacgacagca 17340

gauauaguug ucuuugauga aauuucaaug gccacaaauu augauuugag uguugucaau 17400

gccagauuac gugcuaagca cuauguguac auuggcgacc cugcucaauu accugcacca 17460

cgcacauugc uaacuaaggg cacacuagaa ccagaauauu ucaauucagu guguagacuu 17520

augaaaacua uagguccaga cauguuccuc ggaacuuguc ggcguugucc ugcugaaauu 17580

guugacacug ugagugcuuu gguuuaugau aauaagcuua aagcacauaa agacaaauca 17640

gcucaaugcu uuaaaauguu uuauaagggu guuaucacgc augauguuuc aucugcaauu 17700

aacaggccac aaauaggcgu gguaagagaa uuccuuacac guaacccugc uuggagaaaa 17760

gcugucuuua uuucaccuua uaauucacag aaugcuguag ccucaaagau uuugggacua 17820

ccaacucaaa cuguugauuc aucacagggc ucagaauaug acuaugucau auucacucaa 17880

accacugaaa cagcucacuc uuguaaugua aacagauuua auguugcuau uaccagagca 17940

aaaguaggca uacuuugcau aaugucugau agagaccuuu augacaaguu gcaauuuaca 18000

agucuugaaa uuccacguag gaauguggca acuuuacaag cugaaaaugu aacaggacuc 18060

uuuaaagauu guaguaaggu aaucacuggg uuacauccua cacaggcacc uacacaccuc 18120

aguguugaca cuaaauucaa aacugaaggu uuauguguug acauaccugg cauaccuaag 18180

gacaugaccu auagaagacu caucucuaug augguuuuua aaaugaauua ucaaguuaau 18240

gguuacccua acauguuuau cacccgcgaa gaagcuauaa gacauguacg ugcauggauu 18300

ggcuucgaug ucgaggggug ucaugcuacu agagaagcug uugguaccaa uuuaccuuua 18360

cagcuagguu uuucuacagg uguuaaccua guugcuguac cuacagguua uguugauaca 18420

ccuaauaaua cagauuuuuc cagaguuagu gcuaaaccac cgccuggaga ucaauuuaaa 18480

caccucauac cacuuaugua caaaggacuu ccuuggaaug uagugcguau aaagauugua 18540

caaauguuaa gugacacacu uaaaaaucuc ucugacagag ucguauuugu cuuaugggca 18600

cauggcuuug aguugacauc uaugaaguau uuugugaaaa uaggaccuga gcgcaccugu 18660

ugucuaugug auagacgugc cacaugcuuu uccacugcuu cagacacuua ugccuguugg 18720

caucauucua uuggauuuga uuacgucuau aauccguuua ugauugaugu ucaacaaugg 18780

gguuuuacag guaaccuaca aagcaaccau gaucuguauu gucaagucca ugguaaugca 18840

cauguagcua guugugaugc aaucaugacu aggugucuag cuguccacga gugcuuuguu 18900

aagcguguug acuggacuau ugaauauccu auaauuggug augaacugaa gauuaaugcg 18960

gcuuguagaa agguucaaca caugguuguu aaagcugcau uauuagcaga caaauuccca 19020

guucuucacg acauugguaa cccuaaagcu auuaagugug uaccucaagc ugauguagaa 19080

uggaaguucu augaugcaca gccuuguagu gacaaagcuu auaaaauaga agaauuauuc 19140

uauucuuaug ccacacauuc ugacaaauuc acagauggug uaugccuauu uuggaauugc 19200

aaugucgaua gauauccugc uaauuccauu guuuguagau uugacacuag agugcuaucu 19260

aaccuuaacu ugccugguug ugaugguggc aguuuguaug uaaauaaaca ugcauuccac 19320

acaccagcuu uugauaaaag ugcuuuuguu aauuuaaaac aauuaccauu uuucuauuac 19380

ucugacaguc caugugaguc ucauggaaaa caaguagugu cagauauaga uuauguacca 19440

cuaaagucug cuacguguau aacacguugc aauuuaggug gugcugucug uagacaucau 19500

gcuaaugagu acagauugua ucucgaugcu uauaacauga ugaucucagc uggcuuuagc 19560

uuguggguuu acaaacaauu ugauacuuau aaccucugga acacuuuuac aagacuucag 19620

aguuuagaaa auguggcuuu uaauguugua aauaagggac acuuugaugg acaacagggu 19680

gaaguaccag uuucuaucau uaauaacacu guuuacacaa aaguugaugg uguugaugua 19740

gaauuguuug aaaauaaaac aacauuaccu guuaauguag cauuugagcu uugggcuaag 19800

cgcaacauua aaccaguacc agaggugaaa auacucaaua auuugggugu ggacauugcu 19860

gcuaauacug ugaucuggga cuacaaaaga gaugcuccag cacauauauc uacuauuggu 19920

guuuguucua ugacugacau agccaagaaa ccaacugaaa cgauuugugc accacucacu 19980

gucuuuuuug augguagagu ugauggucaa guagacuuau uuagaaaugc ccguaauggu 20040

guucuuauua cagaagguag uguuaaaggu uuacaaccau cuguaggucc caaacaagcu 20100

agucuuaaug gagucacauu aauuggagaa gccguaaaaa cacaguucaa uuauuauaag 20160

aaaguugaug guguugucca acaauuaccu gaaacuuacu uuacucagag uagaaauuua 20220

caagaauuua aacccaggag ucaaauggaa auugauuucu uagaauuagc uauggaugaa 20280

uucauugaac gguauaaauu agaaggcuau gccuucgaac auaucguuua uggagauuuu 20340

agucauaguc aguuaggugg uuuacaucua cugauuggac uagcuaaacg uuuuaaggaa 20400

ucaccuuuug aauuagaaga uuuuauuccu auggacagua caguuaaaaa cuauuucaua 20460

acagaugcgc aaacagguuc aucuaagugu guguguucug uuauugauuu auuacuugau 20520

gauuuuguug aaauaauaaa aucccaagau uuaucuguag uuucuaaggu ugucaaagug 20580

acuauugacu auacagaaau uucauuuaug cuuuggugua aagauggcca uguagaaaca 20640

uuuuacccaa aauuacaauc uagucaagcg uggcaaccgg guguugcuau gccuaaucuu 20700

uacaaaaugc aaagaaugcu auuagaaaag ugugaccuuc aaaauuaugg ugauagugca 20760

acauuaccua aaggcauaau gaugaauguc gcaaaauaua cucaacugug ucaauauuua 20820

aacacauuaa cauuagcugu acccuauaau augagaguua uacauuuugg ugcugguucu 20880

gauaaaggag uugcaccagg uacagcuguu uuaagacagu gguugccuac ggguacgcug 20940

cuugucgauu cagaucuuaa ugacuuuguc ucugaugcag auucaacuuu gauuggugau 21000

ugugcaacug uacauacagc uaauaaaugg gaucucauua uuagugauau guacgacccu 21060

aagacuaaaa auguuacaaa agaaaaugac ucuaaagagg guuuuuucac uuacauuugu 21120

ggguuuauac aacaaaagcu agcucuugga gguuccgugg cuauaaagau aacagaacau 21180

ucuuggaaug cugaucuuua uaagcucaug ggacacuucg caugguggac agccuuuguu 21240

acuaauguga augcgucauc aucugaagca uuuuuaauug gauguaauua ucuuggcaaa 21300

ccacgcgaac aaauagaugg uuaugucaug caugcaaauu acauauuuug gaggaauaca 21360

aauccaauuc aguugucuuc cuauucuuua uuugacauga guaaauuucc ccuuaaauua 21420

agggguacug cuguuauguc uuuaaaagaa ggucaaauca augauaugau uuuaucucuu 21480

cuuaguaaag guagacuuau aauuagagaa aacaacagag uuguuauuuc uagugauguu 21540

cuuguuaaca acuaaacgaa caauguuugu uuuucuuguu uuauugccac uagucucuag 21600

ucaguguguu aaucuuacaa ccagaacuca auuacccccu gcauacacua auucuuucac 21660

acgugguguu uauuacccug acaaaguuuu cagauccuca guuuuacauu caacucagga 21720

cuuguucuua ccuuucuuuu ccaauguuac uugguuccau gcuauacaug ucucugggac 21780

caaugguacu aagagguuug auaacccugu ccuaccauuu aaugauggug uuuauuuugc 21840

uuccacugag aagucuaaca uaauaagagg cuggauuuuu gguacuacuu uagauucgaa 21900

gacccagucc cuacuuauug uuaauaacgc uacuaauguu guuauuaaag ucugugaauu 21960

ucaauuuugu aaugauccau uuuugggugu uuauuaccac aaaaacaaca aaaguuggau 22020

ggaaagugag uucagaguuu auucuagugc gaauaauugc acuuuugaau augucucuca 22080

gccuuuucuu auggaccuug aaggaaaaca ggguaauuuc aaaaaucuua gggaauuugu 22140

guuuaagaau auugaugguu auuuuaaaau auauucuaag cacacgccua uuaauuuagu 22200

gcgugaucuc ccucaggguu uuucggcuuu agaaccauug guagauuugc caauagguau 22260

uaacaucacu agguuucaaa cuuuacuugc uuuacauaga aguuauuuga cuccugguga 22320

uucuucuuca gguuggacag cuggugcugc agcuuauuau gugggguauc uucaaccuag 22380

gacuuuucua uuaaaauaua augaaaaugg aaccauuaca gaugcuguag acugugcacu 22440

ugacccucuc ucagaaacaa aguguacguu gaaauccuuc acuguagaaa aaggaaucua 22500

ucaaacuucu aacuuuagag uccaaccaac agaaucuauu guuagauuuc cuaauauuac 22560

aaacuugugc ccuuuuggug aaguuuuuaa cgccaccaga uuugcaucug uuuaugcuug 22620

gaacaggaag agaaucagca acuguguugc ugauuauucu guccuauaua auuccgcauc 22680

auuuuccacu uuuaaguguu auggaguguc uccuacuaaa uuaaaugauc ucugcuuuac 22740

uaaugucuau gcagauucau uuguaauuag aggugaugaa gucagacaaa ucgcuccagg 22800

gcaaacugga aagauugcug auuauaauua uaaauuacca gaugauuuua caggcugcgu 22860

uauagcuugg aauucuaaca aucuugauuc uaagguuggu gguaauuaua auuaccugua 22920

uagauuguuu aggaagucua aucucaaacc uuuugagaga gauauuucaa cugaaaucua 22980

ucaggccggu agcacaccuu guaauggugu ugaagguuuu aauuguuacu uuccuuuaca 23040

aucauauggu uuccaaccca cuaauggugu ugguuaccaa ccauacagag uaguaguacu 23100

uucuuuugaa cuucuacaug caccagcaac uguuugugga ccuaaaaagu cuacuaauuu 23160

gguuaaaaac aaauguguca auuucaacuu caaugguuua acaggcacag guguucuuac 23220

ugagucuaac aaaaaguuuc ugccuuucca acaauuuggc agagacauug cugacacuac 23280

ugaugcuguc cgugauccac agacacuuga gauucuugac auuacaccau guucuuuugg 23340

uggugucagu guuauaacac caggaacaaa uacuucuaac cagguugcug uucuuuauca 23400

ggauguuaac ugcacagaag ucccuguugc uauucaugca gaucaacuua cuccuacuug 23460

gcguguuuau ucuacagguu cuaauguuuu ucaaacacgu gcaggcuguu uaauaggggc 23520

ugaacauguc aacaacucau augaguguga cauacccauu ggugcaggua uaugcgcuag 23580

uuaucagacu cagacuaauu cuccucggcg ggcacguagu guagcuaguc aauccaucau 23640

ugccuacacu augucacuug gugcagaaaa uucaguugcu uacucuaaua acucuauugc 23700

cauacccaca aauuuuacua uuaguguuac cacagaaauu cuaccagugu cuaugaccaa 23760

gacaucagua gauuguacaa uguacauuug uggugauuca acugaaugca gcaaucuuuu 23820

guugcaauau ggcaguuuuu guacacaauu aaaccgugcu uuaacuggaa uagcuguuga 23880

acaagacaaa aacacccaag aaguuuuugc acaagucaaa caaauuuaca aaacaccacc 23940

aauuaaagau uuuggugguu uuaauuuuuc acaaauauua ccagauccau caaaaccaag 24000

caagagguca uuuauugaag aucuacuuuu caacaaagug acacuugcag augcuggcuu 24060

caucaaacaa uauggugauu gccuugguga uauugcugcu agagaccuca uuugugcaca 24120

aaaguuuaac ggccuuacug uuuugccacc uuugcucaca gaugaaauga uugcucaaua 24180

cacuucugca cuguuagcgg guacaaucac uucugguugg accuuuggug caggugcugc 24240

auuacaaaua ccauuugcua ugcaaauggc uuauagguuu aaugguauug gaguuacaca 24300

gaauguucuc uaugagaacc aaaaauugau ugccaaccaa uuuaauagug cuauuggcaa 24360

aauucaagac ucacuuucuu ccacagcaag ugcacuugga aaacuucaag auguggucaa 24420

ccaaaaugca caagcuuuaa acacgcuugu uaaacaacuu agcuccaauu uuggugcaau 24480

uucaaguguu uuaaaugaua uccuuucacg ucuugacaaa guugaggcug aagugcaaau 24540

ugauagguug aucacaggca gacuucaaag uuugcagaca uaugugacuc aacaauuaau 24600

uagagcugca gaaaucagag cuucugcuaa ucuugcugcu acuaaaaugu cagagugugu 24660

acuuggacaa ucaaaaagag uugauuuuug uggaaagggc uaucaucuua uguccuuccc 24720

ucagucagca ccucauggug uagucuucuu gcaugugacu uaugucccug cacaagaaaa 24780

gaacuucaca acugcuccug ccauuuguca ugauggaaaa gcacacuuuc cucgugaagg 24840

ugucuuuguu ucaaauggca cacacugguu uguaacacaa aggaauuuuu augaaccaca 24900

aaucauuacu acagacaaca cauuuguguc ugguaacugu gauguuguaa uaggaauugu 24960

caacaacaca guuuaugauc cuuugcaacc ugaauuagac ucauucaagg aggaguuaga 25020

uaaauauuuu aagaaucaua caucaccaga uguugauuua ggugacaucu cuggcauuaa 25080

ugcuucaguu guaaacauuc aaaaagaaau ugaccgccuc aaugagguug ccaagaauuu 25140

aaaugaaucu cucaucgauc uccaagaacu uggaaaguau gagcaguaua uaaaauggcc 25200

augguacauu uggcuagguu uuauagcugg cuugauugcc auaguaaugg ugacaauuau 25260

gcuuugcugu augaccaguu gcuguaguug ucucaagggc uguuguucuu guggauccug 25320

cugcaaauuu gaugaagacg acucugagcc agugcucaaa ggagucaaau uacauuacac 25380

auaaacgaac uuauggauuu guuuaugaga aucuucacaa uuggaacugu aacuuugaag 25440

caaggugaaa ucaaggaugc uacuccuuca gauuuuguuc gcgcuacugc aacgauaccg 25500

auacaagccu cacucccuuu cggauggcuu auuguuggcg uugcacuucu ugcuguuuuu 25560

cagagcgcuu ccaaaaucau aacccucaaa aagagauggc aacuagcacu cuccaagggu 25620

guucacuuug uuugcaacuu gcuguuguug uuuguaacag uuuacucaca ccuuuugcuc 25680

guugcugcug gccuugaagc cccuuuucuc uaucuuuaug cuuuagucua cuucuugcag 25740

aguauaaacu uuguaagaau aauaaugagg cuuuggcuuu gcuggaaaug ccguuccaaa 25800

aacccauuac uuuaugaugc caacuauuuu cuuugcuggc auacuaauug uuacgacuau 25860

uguauaccuu acaauagugu aacuucuuca auugucauua cuucagguga uggcacaaca 25920

aguccuauuu cugaacauga cuaccagauu ggugguuaua cugaaaaaug ggaaucugga 25980

guaaaagacu guguuguauu acacaguuac uucacuucag acuauuacca gcuguacuca 26040

acucaauuga guacagacac ugguguugaa cauguuaccu ucuucaucua caauaaaauu 26100

guugaugagc cugaagaaca uguccaaauu cacacaaucg acgguucauc cggaguuguu 26160

aauccaguaa uggaaccaau uuaugaugaa ccgacgacga cuacuagcgu gccuuuguaa 26220

gcacaagcug augaguacga acuuauguac ucauucguuu cggaagagac agguacguua 26280

auaguuaaua gcguacuucu uuuucuugcu uucgugguau ucuugcuagu uacacuagcc 26340

auccuuacug cgcuucgauu gugugcguac ugcugcaaua uuguuaacgu gagucuugua 26400

aaaccuucuu uuuacguuua cucucguguu aaaaaucuga auucuucuag aguuccugau 26460

cuucuggucu aaacgaacua aauauuauau uaguuuuucu guuuggaacu uuaauuuuag 26520

ccauggcaga uuccaacggu acuauuaccg uugaagagcu uaaaaagcuc cuugaacaau 26580

ggaaccuagu aauagguuuc cuauuccuua cauggauuug ucuucuacaa uuugccuaug 26640

ccaacaggaa uagguuuuug uauauaauua aguuaauuuu ccucuggcug uuauggccag 26700

uaacuuuagc uuguuuugug cuugcugcug uuuacagaau aaauuggauc accgguggaa 26760

uugcuaucgc aauggcuugu cuuguaggcu ugauguggcu cagcuacuuc auugcuucuu 26820

ucagacuguu ugcgcguacg cguuccaugu ggucauucaa uccagaaacu aacauucuuc 26880

ucaacgugcc acuccauggc acuauucuga ccagaccgcu ucuagaaagu gaacucguaa 26940

ucggagcugu gauccuucgu ggacaucuuc guauugcugg acaccaucua ggacgcugug 27000

acaucaagga ccugccuaaa gaaaucacug uugcuacauc acgaacgcuu ucuuauuaca 27060

aauugggagc uucgcagcgu guagcaggug acucagguuu ugcugcauac agucgcuaca 27120

ggauuggcaa cuauaaauua aacacagacc auuccaguag cagugacaau auugcuuugc 27180

uuguacagua agugacaaca gauguuucau cucguugacu uucagguuac uauagcagag 27240

auauuacuaa uuauuaugag gacuuuuaaa guuuccauuu ggaaucuuga uuacaucaua 27300

aaccucauaa uuaaaaauuu aucuaaguca cuaacugaga auaaauauuc ucaauuagau 27360

gaagagcaac caauggagau ugauuaaacg aacaugaaaa uuauucuuuu cuuggcacug 27420

auaacacucg cuacuuguga gcuuuaucac uaccaagagu guguuagagg uacaacagua 27480

cuuuuaaaag aaccuugcuc uucuggaaca uacgagggca auucaccauu ucauccucua 27540

gcugauaaca aauuugcacu gacuugcuuu agcacucaau uugcuuuugc uuguccugac 27600

ggcguaaaac acgucuauca guuacgugcc agaucaguuu caccuaaacu guucaucaga 27660

caagaggaag uucaagaacu uuacucucca auuuuucuua uuguugcggc aauaguguuu 27720

auaacacuuu gcuucacacu caaaagaaag acagaaugau ugaacuuuca uuaauugacu 27780

ucuauuugug cuuuuuagcc uuucugcuau uccuuguuuu aauuaugcuu auuaucuuuu 27840

gguucucacu ugaacugcaa gaucauaaug aaacuuguca cgccuaaacg aacaugaaau 27900

uucuuguuuu cuuaggaauc aucacaacug uagcugcauu ucaccaagaa uguaguuuac 27960

agucauguac ucaacaucaa ccauauguag uugaugaccc guguccuauu cacuucuauu 28020

cuaaauggua uauuagagua ggagcuagaa aaucagcacc uuuaauugaa uugugcgugg 28080

augaggcugg uucuaaauca cccauucagu acaucgauau cgguaauuau acaguuuccu 28140

guuuaccuuu uacaauuaau ugccaggaac cuaaauuggg uagucuugua gugcguuguu 28200

cguucuauga agacuuuuua gaguaucaug acguucgugu uguuuuagau uucaucuaaa 28260

cgaacaaacu aaaaugucug auaauggacc ccaaaaucag cgaaaugcac cccgcauuac 28320

guuuggugga cccucagauu caacuggcag uaaccagaau ggagaacgca guggggcgcg 28380

aucaaaacaa cgucggcccc aagguuuacc caauaauacu gcgucuuggu ucaccgcucu 28440

cacucaacau ggcaaggaag accuuaaauu cccucgagga caaggcguuc caauuaacac 28500

caauagcagu ccagaugacc aaauuggcua cuaccgaaga gcuaccagac gaauucgugg 28560

uggugacggu aaaaugaaag aucucagucc aagaugguau uucuacuacc uaggaacugg 28620

gccagaagcu ggacuucccu auggugcuaa caaagacggc aucauauggg uugcaacuga 28680

gggagccuug aauacaccaa aagaucacau uggcacccgc aauccugcua acaaugcugc 28740

aaucgugcua caacuuccuc aaggaacaac auugccaaaa ggcuucuacg cagaagggag 28800

cagaggcggc agucaagccu cuucucguuc cucaucacgu agucgcaaca guucaagaaa 28860

uucaacucca ggcagcagua ggggaacuuc uccugcuaga auggcuggca auggcgguga 28920

ugcugcucuu gcuuugcugc ugcuugacag auugaaccag cuugagagca aaaugucugg 28980

uaaaggccaa caacaacaag gccaaacugu cacuaagaaa ucugcugcug aggcuucuaa 29040

gaagccucgg caaaaacgua cugccacuaa agcauacaau guaacacaag cuuucggcag 29100

acguggucca gaacaaaccc aaggaaauuu uggggaccag gaacuaauca gacaaggaac 29160

ugauuacaaa cauuggccgc aaauugcaca auuugccccc agcgcuucag cguucuucgg 29220

aaugucgcgc auuggcaugg aagucacacc uucgggaacg ugguugaccu acacaggugc 29280

caucaaauug gaugacaaag auccaaauuu caaagaucaa gucauuuugc ugaauaagca 29340

uauugacgca uacaaaacau ucccaccaac agagccuaaa aaggacaaaa agaagaaggc 29400

ugaugaaacu caagccuuac cgcagagaca gaagaaacag caaacuguga cucuucuucc 29460

ugcugcagau uuggaugauu ucuccaaaca auugcaacaa uccaugagca gugcugacuc 29520

aacucaggcc uaaacucaug cagaccacac aaggcagaug ggcuauauaa acguuuucgc 29580

uuuuccguuu acgauauaua gucuacucuu gugcagaaug aauucucgua acuacauagc 29640

acaaguagau guaguuaacu uuaaucucac auagcaaucu uuaaucagug uguaacauua 29700

gggaggacuu gaaagagcca ccacauuuuc accgaggcca cgcggaguac gaucgagugu 29760

acagugaaca augcuaggga gagcugccua uauggaagag cccuaaugug uaaaauuaau 29820

uuuaguagug cuauccccau gugauuuuaa uagcuucuua ggagaaugac aaaaaaaaaa 29880

aaaaaaaaaa aaaaaaaaaa aaa 29903

<210> 2
<211> 30005
<212> RNA
<213> Artificial Sequence

<220>
<223> SARS-CoV-2 strain consensus, n can be any nucleotide

<220>
<221> misc_feature
<222> (1)..(132)
<223> n is a, c, g, or u

<220>
<221> misc_feature
<222> (161)..(161)
<223> n is a, c, g, or u

<220>
<221> misc_feature
<222> (168)..(169)
<223> n is a, c, g, or u

<220>
<221> misc_feature
<222> (176)..(177)
<223> n is a, c, g, or u

<220>
<221> misc_feature
<222> (181)..(181)
<223> n is a, c, g, or u

<220>
<221> misc_feature
<222> (197)..(197)
<223> n is a, c, g, or u

<220>
<221> misc_feature
<222> (243)..(243)
<223> n is a, c, g, or u

<220>
<221> misc_feature
<222> (254)..(255)
<223> n is a, c, g, or u

<220>

<221>  misc_feature
<222>  (257)..(259)
<223>  n is a, c, g, or u

<220>
<221>  misc_feature
<222>  (261)..(263)
<223>  n is a, c, g, or u

<220>
<221>  misc_feature
<222>  (265)..(265)
<223>  n is a, c, g, or u

<220>
<221>  misc_feature
<222>  (267)..(267)
<223>  n is a, c, g, or u

<220>
<221>  misc_feature
<222>  (278)..(278)
<223>  n is a, c, g, or u

<220>
<221>  misc_feature
<222>  (298)..(298)
<223>  n is a, c, g, or u

<220>
<221>  misc_feature
<222>  (311)..(311)
<223>  n is a, c, g, or u

<220>
<221>  misc_feature
<222>  (389)..(389)
<223>  n is a, c, g, or u

<220>
<221>  misc_feature
<222>  (411)..(411)
<223>  n is a, c, g, or u

<220>
<221>  misc_feature
<222>  (547)..(547)
<223>  n is a, c, g, or u

<220>
<221>  misc_feature
<222>  (565)..(579)
<223>  n is a, c, g, or u

<220>
<221>  misc_feature
<222>  (671)..(671)
<223>  n is a, c, g, or u

<220>
<221>  misc_feature
<222>  (711)..(711)
<223>  n is a, c, g, or u

<220>
<221>  misc_feature
<222>  (743)..(751)
<223>  n is a, c, g, or u

<220>
<221>  misc_feature
<222>  (890)..(890)
<223>  n is a, c, g, or u

<220>
<221>  misc_feature
<222>  (941)..(941)
<223>  n is a, c, g, or u

<220>
<221>  misc_feature
<222>  (1116)..(1116)
<223>  n is a, c, g, or u

<220>
<221>  misc_feature
<222>  (1120)..(1120)
<223>  n is a, c, g, or u

<220>
<221>  misc_feature
<222>  (1159)..(1159)
<223>  n is a, c, g, or u

<220>
<221>  misc_feature
<222>  (1405)..(1405)
<223>  n is a, c, g, or u

<220>
<221>  misc_feature
<222>  (1442)..(1442)
<223>  n is a, c, g, or u

<220>
<221>  misc_feature
<222>  (1454)..(1454)
<223>  n is a, c, g, or u

<220>
<221>  misc_feature
<222>  (1487)..(1487)
<223>  n is a, c, g, or u

<220>
<221>  misc_feature
<222>  (1526)..(1526)
<223>  n is a, c, g, or u

<220>
<221>  misc_feature
<222>  (1605)..(1605)
<223>  n is a, c, g, or u

<220>
<221>  misc_feature

<222>  (1748)..(1748)
<223>  n is a, c, g, or u

<220>
<221>  misc_feature
<222>  (1791)..(1791)
<223>  n is a, c, g, or u

<220>
<221>  misc_feature
<222>  (1968)..(1968)
<223>  n is a, c, g, or u

<220>
<221>  misc_feature
<222>  (2148)..(2148)
<223>  n is a, c, g, or u

<220>
<221>  misc_feature
<222>  (2238)..(2239)
<223>  n is a, c, g, or u

<220>
<221>  misc_feature
<222>  (2326)..(2326)
<223>  n is a, c, g, or u

<220>
<221>  misc_feature
<222>  (2334)..(2334)
<223>  n is a, c, g, or u

<220>
<221>  misc_feature
<222>  (2503)..(2503)
<223>  n is a, c, g, or u

<220>
<221>  misc_feature
<222>  (2689)..(2689)
<223>  n is a, c, g, or u

<220>
<221>  misc_feature
<222>  (2774)..(2774)
<223>  n is a, c, g, or u

<220>
<221>  misc_feature
<222>  (3028)..(3028)
<223>  n is a, c, g, or u

<220>
<221>  misc_feature
<222>  (3094)..(3094)
<223>  n is a, c, g, or u

<220>
<221>  misc_feature
<222>  (3156)..(3156)
<223>  n is a, c, g, or u

<220>
<221>  misc_feature
<222>  (3234)..(3234)
<223>  n is a, c, g, or u

<220>
<221>  misc_feature
<222>  (3316)..(3316)
<223>  n is a, c, g, or u

<220>
<221>  misc_feature
<222>  (3468)..(3468)
<223>  n is a, c, g, or u

<220>
<221>  misc_feature
<222>  (3575)..(3575)
<223>  n is a, c, g, or u

<220>
<221>  misc_feature
<222>  (3795)..(3795)
<223>  n is a, c, g, or u

<220>
<221>  misc_feature
<222>  (3835)..(3835)
<223>  n is a, c, g, or u

<220>
<221>  misc_feature
<222>  (4049)..(4049)
<223>  n is a, c, g, or u

<220>
<221>  misc_feature
<222>  (4345)..(4345)
<223>  n is a, c, g, or u

<220>
<221>  misc_feature
<222>  (4364)..(4364)
<223>  n is a, c, g, or u

<220>
<221>  misc_feature
<222>  (4459)..(4459)
<223>  n is a, c, g, or u

<220>
<221>  misc_feature
<222>  (4936)..(4938)
<223>  n is a, c, g, or u

<220>
<221>  misc_feature
<222>  (5109)..(5109)
<223>  n is a, c, g, or u

<220>
<221>  misc_feature
<222>  (5119)..(5119)

<223>  n is a, c, g, or u

<220>
<221>  misc_feature
<222>  (5141)..(5141)
<223>  n is a, c, g, or u

<220>
<221>  misc_feature
<222>  (5537)..(5537)
<223>  n is a, c, g, or u

<220>
<221>  misc_feature
<222>  (5629)..(5629)
<223>  n is a, c, g, or u

<220>
<221>  misc_feature
<222>  (5773)..(5773)
<223>  n is a, c, g, or u

<220>
<221>  misc_feature
<222>  (5841)..(5841)
<223>  n is a, c, g, or u

<220>
<221>  misc_feature
<222>  (5902)..(5902)
<223>  n is a, c, g, or u

<220>
<221>  misc_feature
<222>  (6083)..(6083)
<223>  n is a, c, g, or u

<220>
<221>  misc_feature
<222>  (6088)..(6088)
<223>  n is a, c, g, or u

<220>
<221>  misc_feature
<222>  (6092)..(6092)
<223>  n is a, c, g, or u

<220>
<221>  misc_feature
<222>  (6367)..(6367)
<223>  n is a, c, g, or u

<220>
<221>  misc_feature
<222>  (6558)..(6558)
<223>  n is a, c, g, or u

<220>
<221>  misc_feature
<222>  (6693)..(6693)
<223>  n is a, c, g, or u

<220>

<221>  misc_feature
<222>  (6752)..(6752)
<223>  n is a, c, g, or u

<220>
<221>  misc_feature
<222>  (6876)..(6876)
<223>  n is a, c, g, or u

<220>
<221>  misc_feature
<222>  (6953)..(6953)
<223>  n is a, c, g, or u

<220>
<221>  misc_feature
<222>  (7016)..(7016)
<223>  n is a, c, g, or u

<220>
<221>  misc_feature
<222>  (7053)..(7053)
<223>  n is a, c, g, or u

<220>
<221>  misc_feature
<222>  (7073)..(7073)
<223>  n is a, c, g, or u

<220>
<221>  misc_feature
<222>  (7536)..(7536)
<223>  n is a, c, g, or u

<220>
<221>  misc_feature
<222>  (7923)..(7923)
<223>  n is a, c, g, or u

<220>
<221>  misc_feature
<222>  (8058)..(8058)
<223>  n is a, c, g, or u

<220>
<221>  misc_feature
<222>  (8369)..(8369)
<223>  n is a, c, g, or u

<220>
<221>  misc_feature
<222>  (8445)..(8445)
<223>  n is a, c, g, or u

<220>
<221>  misc_feature
<222>  (8825)..(8825)
<223>  n is a, c, g, or u

<220>
<221>  misc_feature
<222>  (8839)..(8839)
<223>  n is a, c, g, or u

<220>
<221>  misc_feature
<222>  (9044)..(9044)
<223>  n is a, c, g, or u

<220>
<221>  misc_feature
<222>  (9091)..(9091)
<223>  n is a, c, g, or u

<220>
<221>  misc_feature
<222>  (9214)..(9214)
<223>  n is a, c, g, or u

<220>
<221>  misc_feature
<222>  (9216)..(9216)
<223>  n is a, c, g, or u

<220>
<221>  misc_feature
<222>  (9237)..(9237)
<223>  n is a, c, g, or u

<220>
<221>  misc_feature
<222>  (9331)..(9331)
<223>  n is a, c, g, or u

<220>
<221>  misc_feature
<222>  (9531)..(9531)
<223>  n is a, c, g, or u

<220>
<221>  misc_feature
<222>  (9534)..(9534)
<223>  n is a, c, g, or u

<220>
<221>  misc_feature
<222>  (9548)..(9548)
<223>  n is a, c, g, or u

<220>
<221>  misc_feature
<222>  (9568)..(9568)
<223>  n is a, c, g, or u

<220>
<221>  misc_feature
<222>  (9591)..(9591)
<223>  n is a, c, g, or u

<220>
<221>  misc_feature
<222>  (9618)..(9618)
<223>  n is a, c, g, or u

<220>
<221>  misc_feature

<222>  (9981)..(9981)
<223>  n is a, c, g, or u

<220>
<221>  misc_feature
<222>  (10093)..(10093)
<223>  n is a, c, g, or u

<220>
<221>  misc_feature
<222>  (10140)..(10140)
<223>  n is a, c, g, or u

<220>
<221>  misc_feature
<222>  (10289)..(10289)
<223>  n is a, c, g, or u

<220>
<221>  misc_feature
<222>  (10564)..(10564)
<223>  n is a, c, g, or u

<220>
<221>  misc_feature
<222>  (11140)..(11140)
<223>  n is a, c, g, or u

<220>
<221>  misc_feature
<222>  (11264)..(11264)
<223>  n is a, c, g, or u

<220>
<221>  misc_feature
<222>  (11290)..(11290)
<223>  n is a, c, g, or u

<220>
<221>  misc_feature
<222>  (11467)..(11467)
<223>  n is a, c, g, or u

<220>
<221>  misc_feature
<222>  (11807)..(11807)
<223>  n is a, c, g, or u

<220>
<221>  misc_feature
<222>  (12013)..(12013)
<223>  n is a, c, g, or u

<220>
<221>  misc_feature
<222>  (12098)..(12098)
<223>  n is a, c, g, or u

<220>
<221>  misc_feature
<222>  (12172)..(12172)
<223>  n is a, c, g, or u

<220>
<221>  misc_feature
<222>  (12217)..(12217)
<223>  n is a, c, g, or u

<220>
<221>  misc_feature
<222>  (12259)..(12259)
<223>  n is a, c, g, or u

<220>
<221>  misc_feature
<222>  (12265)..(12265)
<223>  n is a, c, g, or u

<220>
<221>  misc_feature
<222>  (12270)..(12270)
<223>  n is a, c, g, or u

<220>
<221>  misc_feature
<222>  (12412)..(12412)
<223>  n is a, c, g, or u

<220>
<221>  misc_feature
<222>  (12435)..(12435)
<223>  n is a, c, g, or u

<220>
<221>  misc_feature
<222>  (12521)..(12521)
<223>  n is a, c, g, or u

<220>
<221>  misc_feature
<222>  (12524)..(12524)
<223>  n is a, c, g, or u

<220>
<221>  misc_feature
<222>  (12530)..(12530)
<223>  n is a, c, g, or u

<220>
<221>  misc_feature
<222>  (12548)..(12548)
<223>  n is a, c, g, or u

<220>
<221>  misc_feature
<222>  (12570)..(12571)
<223>  n is a, c, g, or u

<220>
<221>  misc_feature
<222>  (12591)..(12591)
<223>  n is a, c, g, or u

<220>
<221>  misc_feature
<222>  (12629)..(12629)

<223>  n is a, c, g, or u

<220>
<221>  misc_feature
<222>  (12635)..(12635)
<223>  n is a, c, g, or u

<220>
<221>  misc_feature
<222>  (12639)..(12639)
<223>  n is a, c, g, or u

<220>
<221>  misc_feature
<222>  (12657)..(12657)
<223>  n is a, c, g, or u

<220>
<221>  misc_feature
<222>  (12717)..(12717)
<223>  n is a, c, g, or u

<220>
<221>  misc_feature
<222>  (12742)..(12742)
<223>  n is a, c, g, or u

<220>
<221>  misc_feature
<222>  (12830)..(12830)
<223>  n is a, c, g, or u

<220>
<221>  misc_feature
<222>  (12850)..(12850)
<223>  n is a, c, g, or u

<220>
<221>  misc_feature
<222>  (13129)..(13129)
<223>  n is a, c, g, or u

<220>
<221>  misc_feature
<222>  (13282)..(13283)
<223>  n is a, c, g, or u

<220>
<221>  misc_feature
<222>  (14286)..(14286)
<223>  n is a, c, g, or u

<220>
<221>  misc_feature
<222>  (14465)..(14465)
<223>  n is a, c, g, or u

<220>
<221>  misc_feature
<222>  (14714)..(14714)
<223>  n is a, c, g, or u

<220>

<221>  misc_feature
<222>  (14862)..(14862)
<223>  n is a, c, g, or u

<220>
<221>  misc_feature
<222>  (15250)..(15250)
<223>  n is a, c, g, or u

<220>
<221>  misc_feature
<222>  (15381)..(15381)
<223>  n is a, c, g, or u

<220>
<221>  misc_feature
<222>  (15643)..(15643)
<223>  n is a, c, g, or u

<220>
<221>  misc_feature
<222>  (15654)..(15654)
<223>  n is a, c, g, or u

<220>
<221>  misc_feature
<222>  (15664)..(15664)
<223>  n is a, c, g, or u

<220>
<221>  misc_feature
<222>  (15867)..(15867)
<223>  n is a, c, g, or u

<220>
<221>  misc_feature
<222>  (16304)..(16304)
<223>  n is a, c, g, or u

<220>
<221>  misc_feature
<222>  (16524)..(16524)
<223>  n is a, c, g, or u

<220>
<221>  misc_feature
<222>  (16592)..(16592)
<223>  n is a, c, g, or u

<220>
<221>  misc_feature
<222>  (16934)..(16934)
<223>  n is a, c, g, or u

<220>
<221>  misc_feature
<222>  (16944)..(16944)
<223>  n is a, c, g, or u

<220>
<221>  misc_feature
<222>  (16969)..(16969)
<223>  n is a, c, g, or u

<220>
<221>  misc_feature
<222>  (17057)..(17057)
<223>  n is a, c, g, or u

<220>
<221>  misc_feature
<222>  (17304)..(17304)
<223>  n is a, c, g, or u

<220>
<221>  misc_feature
<222>  (17430)..(17430)
<223>  n is a, c, g, or u

<220>
<221>  misc_feature
<222>  (17433)..(17433)
<223>  n is a, c, g, or u

<220>
<221>  misc_feature
<222>  (17467)..(17467)
<223>  n is a, c, g, or u

<220>
<221>  misc_feature
<222>  (17480)..(17480)
<223>  n is a, c, g, or u

<220>
<221>  misc_feature
<222>  (17531)..(17531)
<223>  n is a, c, g, or u

<220>
<221>  misc_feature
<222>  (17804)..(17804)
<223>  n is a, c, g, or u

<220>
<221>  misc_feature
<222>  (17915)..(17915)
<223>  n is a, c, g, or u

<220>
<221>  misc_feature
<222>  (18117)..(18117)
<223>  n is a, c, g, or u

<220>
<221>  misc_feature
<222>  (18625)..(18625)
<223>  n is a, c, g, or u

<220>
<221>  misc_feature
<222>  (18660)..(18660)
<223>  n is a, c, g, or u

<220>
<221>  misc_feature

<222>  (18871)..(18871)
<223>  n is a, c, g, or u

<220>
<221>  misc_feature
<222>  (18934)..(18934)
<223>  n is a, c, g, or u

<220>
<221>  misc_feature
<222>  (19032)..(19032)
<223>  n is a, c, g, or u

<220>
<221>  misc_feature
<222>  (19122)..(19122)
<223>  n is a, c, g, or u

<220>
<221>  misc_feature
<222>  (19232)..(19232)
<223>  n is a, c, g, or u

<220>
<221>  misc_feature
<222>  (19667)..(19667)
<223>  n is a, c, g, or u

<220>
<221>  misc_feature
<222>  (20338)..(20338)
<223>  n is a, c, g, or u

<220>
<221>  misc_feature
<222>  (20355)..(20357)
<223>  n is a, c, g, or u

<220>
<221>  misc_feature
<222>  (20880)..(20880)
<223>  n is a, c, g, or u

<220>
<221>  misc_feature
<222>  (20993)..(20993)
<223>  n is a, c, g, or u

<220>
<221>  misc_feature
<222>  (21037)..(21037)
<223>  n is a, c, g, or u

<220>
<221>  misc_feature
<222>  (21045)..(21045)
<223>  n is a, c, g, or u

<220>
<221>  misc_feature
<222>  (21194)..(21194)
<223>  n is a, c, g, or u

<220>
<221>  misc_feature
<222>  (21204)..(21204)
<223>  n is a, c, g, or u

<220>
<221>  misc_feature
<222>  (21373)..(21373)
<223>  n is a, c, g, or u

<220>
<221>  misc_feature
<222>  (21442)..(21444)
<223>  n is a, c, g, or u

<220>
<221>  misc_feature
<222>  (21446)..(21446)
<223>  n is a, c, g, or u

<220>
<221>  misc_feature
<222>  (21635)..(21635)
<223>  n is a, c, g, or u

<220>
<221>  misc_feature
<222>  (21704)..(21704)
<223>  n is a, c, g, or u

<220>
<221>  misc_feature
<222>  (21767)..(21767)
<223>  n is a, c, g, or u

<220>
<221>  misc_feature
<222>  (21844)..(21844)
<223>  n is a, c, g, or u

<220>
<221>  misc_feature
<222>  (22051)..(22053)
<223>  n is a, c, g, or u

<220>
<221>  misc_feature
<222>  (22093)..(22093)
<223>  n is a, c, g, or u

<220>
<221>  misc_feature
<222>  (22164)..(22164)
<223>  n is a, c, g, or u

<220>
<221>  misc_feature
<222>  (22284)..(22284)
<223>  n is a, c, g, or u

<220>
<221>  misc_feature
<222>  (22363)..(22363)

<223>  n is a, c, g, or u

<220>
<221>  misc_feature
<222>  (22492)..(22492)
<223>  n is a, c, g, or u

<220>
<221>  misc_feature
<222>  (22845)..(22845)
<223>  n is a, c, g, or u

<220>
<221>  misc_feature
<222>  (23044)..(23044)
<223>  n is a, c, g, or u

<220>
<221>  misc_feature
<222>  (23048)..(23048)
<223>  n is a, c, g, or u

<220>
<221>  misc_feature
<222>  (23245)..(23245)
<223>  n is a, c, g, or u

<220>
<221>  misc_feature
<222>  (23463)..(23463)
<223>  n is a, c, g, or u

<220>
<221>  misc_feature
<222>  (24012)..(24012)
<223>  n is a, c, g, or u

<220>
<221>  misc_feature
<222>  (24094)..(24094)
<223>  n is a, c, g, or u

<220>
<221>  misc_feature
<222>  (24385)..(24385)
<223>  n is a, c, g, or u

<220>
<221>  misc_feature
<222>  (24411)..(24411)
<223>  n is a, c, g, or u

<220>
<221>  misc_feature
<222>  (25623)..(25623)
<223>  n is a, c, g, or u

<220>
<221>  misc_feature
<222>  (25647)..(25647)
<223>  n is a, c, g, or u

<220>

<221>  misc_feature
<222>  (25764)..(25764)
<223>  n is a, c, g, or u

<220>
<221>  misc_feature
<222>  (25835)..(25835)
<223>  n is a, c, g, or u

<220>
<221>  misc_feature
<222>  (25858)..(25858)
<223>  n is a, c, g, or u

<220>
<221>  misc_feature
<222>  (26039)..(26039)
<223>  n is a, c, g, or u

<220>
<221>  misc_feature
<222>  (26204)..(26204)
<223>  n is a, c, g, or u

<220>
<221>  misc_feature
<222>  (26386)..(26386)
<223>  n is a, c, g, or u

<220>
<221>  misc_feature
<222>  (26414)..(26414)
<223>  n is a, c, g, or u

<220>
<221>  misc_feature
<222>  (26518)..(26518)
<223>  n is a, c, g, or u

<220>
<221>  misc_feature
<222>  (26789)..(26789)
<223>  n is a, c, g, or u

<220>
<221>  misc_feature
<222>  (27286)..(27286)
<223>  n is a, c, g, or u

<220>
<221>  misc_feature
<222>  (27336)..(27336)
<223>  n is a, c, g, or u

<220>
<221>  misc_feature
<222>  (27444)..(27444)
<223>  n is a, c, g, or u

<220>
<221>  misc_feature
<222>  (27585)..(27585)
<223>  n is a, c, g, or u

<220>
<221>  misc_feature
<222>  (27866)..(27866)
<223>  n is a, c, g, or u

<220>
<221>  misc_feature
<222>  (27985)..(27985)
<223>  n is a, c, g, or u

<220>
<221>  misc_feature
<222>  (28024)..(28024)
<223>  n is a, c, g, or u

<220>
<221>  misc_feature
<222>  (28137)..(28137)
<223>  n is a, c, g, or u

<220>
<221>  misc_feature
<222>  (28204)..(28204)
<223>  n is a, c, g, or u

<220>
<221>  misc_feature
<222>  (28313)..(28313)
<223>  n is a, c, g, or u

<220>
<221>  misc_feature
<222>  (28427)..(28427)
<223>  n is a, c, g, or u

<220>
<221>  misc_feature
<222>  (28438)..(28438)
<223>  n is a, c, g, or u

<220>
<221>  misc_feature
<222>  (28469)..(28469)
<223>  n is a, c, g, or u

<220>
<221>  misc_feature
<222>  (28717)..(28717)
<223>  n is a, c, g, or u

<220>
<221>  misc_feature
<222>  (28852)..(28852)
<223>  n is a, c, g, or u

<220>
<221>  misc_feature
<222>  (28914)..(28914)
<223>  n is a, c, g, or u

<220>
<221>  misc_feature

<222>  (28923)..(28923)
<223>  n is a, c, g, or u

<220>
<221>  misc_feature
<222>  (28938)..(28938)
<223>  n is a, c, g, or u

<220>
<221>  misc_feature
<222>  (28941)..(28943)
<223>  n is a, c, g, or u

<220>
<221>  misc_feature
<222>  (28976)..(28976)
<223>  n is a, c, g, or u

<220>
<221>  misc_feature
<222>  (29155)..(29155)
<223>  n is a, c, g, or u

<220>
<221>  misc_feature
<222>  (29290)..(29290)
<223>  n is a, c, g, or u

<220>
<221>  misc_feature
<222>  (29314)..(29314)
<223>  n is a, c, g, or u

<220>
<221>  misc_feature
<222>  (29361)..(29361)
<223>  n is a, c, g, or u

<220>
<221>  misc_feature
<222>  (29363)..(29363)
<223>  n is a, c, g, or u

<220>
<221>  misc_feature
<222>  (29587)..(29587)
<223>  n is a, c, g, or u

<220>
<221>  misc_feature
<222>  (29613)..(29613)
<223>  n is a, c, g, or u

<220>
<221>  misc_feature
<222>  (29656)..(29656)
<223>  n is a, c, g, or u

<220>
<221>  misc_feature
<222>  (29695)..(29695)
<223>  n is a, c, g, or u

<220>
<221>  misc_feature
<222>  (29796)..(29796)
<223>  n is a, c, g, or u

<220>
<221>  misc_feature
<222>  (29802)..(29802)
<223>  n is a, c, g, or u

<220>
<221>  misc_feature
<222>  (29810)..(29819)
<223>  n is a, c, g, or u

<220>
<221>  misc_feature
<222>  (29834)..(29834)
<223>  n is a, c, g, or u

<220>
<221>  misc_feature
<222>  (29887)..(30005)
<223>  n is a, c, g, or u

<400>  2
nnnnnnnnnn nnnnnnnnnn nnnnnnnnnn nnnnnnnnnn nnnnnnnnnn nnnnnnnnnn      60

nnnnnnnnnn nnnnnnnnnn nnnnnnnnnn nnnnnnnnnn nnnnnnnnnn nnnnnnnnnn     120

nnnnnnnnnn nnuuuaaaau cuguguggcu gucacucggc ngcaugcnna gugcanncac     180

ncaguauaau uaauaanuaa uuacugucgu ugacaggaca cgaguaacuc gucuaucuuc     240

ugnaggcugc uuanngnnnc nnncnunuug cagccganca ucagcacauc uagguuungu     300

ccggguguga ncgaaaggua agauggagag ccuugucccu gguuucaacg agaaaacaca     360

cguccaacuc aguuugccug uuuuacagnu ucgcgacgug cucguacgug ncuuuggaga     420

cuccguggag gaggucuuau cagaggcacg ucaacaucuu aaagauggca cuuguggcuu     480

aguagaaguu gaaaaaggcg uuuugccuca acuugaacag cccuaugugu ucaucaaacg     540

uucggangcu cgaacugcac cucannnnnn nnnnnnnnnu gagcugguag cagaacucga     600

aggcauucag uacggucgua guggugagac acuugguguc cuugucccuc augugggcga     660

aauaccagug ncuuaccgca agguucuucu ucguaagaac gguaauaaag nagcuggugg     720

ccauaguuac ggcgccgauc uannnnnnnn ngacuuaggc gacgagcuug gcacugaucc     780

uuaugaagau uuucaagaaa acuggaacac uaaacauagc agugguguua cccgugaacu     840

caugcgugag cuuaacggag gggcauacac ucgcuauguc gauaacaacn ucuguggccc     900

ugauggcuac ccucuugagu gcauuaaaga ccuucuagca ngugcuggua aagcuucaug     960

cacuuugucc gaacaacugg acuuuauuga cacuaagagg gguguauacu gcugccguga    1020

acaugagcau gaaauugcuu gguacacgga acguucugaa aagagcuaug aauugcagac    1080

accuuuugaa auuaaauugg caaagaaauu ugacancuun aauggggaau guccaaauuu    1140

uguauuuccc uuaaauucna uaaucaagac uauucaacca aggguugaaa agaaaaagcu 1200

ugauggcuuu auggguagaa uucgaucugu cuauccaguu gcgucaccaa augaaugcaa 1260

ccaaaugugc cuuucaacuc ucaugaagug ugaucauugu ggugaaacuu cauggcagac 1320

gggcgauuuu guuaaagcca cuugcgaauu uuguggcacu gagaauuuga cuaaagaagg 1380

ugccacuacu ugugguuacu uaccncaaaa ugcuguuguu aaaauuuauu guccagcaug 1440

unacaauuca gaanuaggac cugagcauag ucuugccgaa uaccaunaug aaucuggcuu 1500

gaaaaccauu cuucguaagg guggungcac uauugccuuu ggaggcugug uguucucuua 1560

uguugguugc cauaacaagu gugccuauug gguuccacgu gcuancgcua acauagguug 1620

uaaccauaca gguguuguug gagaagguuc cgaaggucuu aaugacaacc uucuugaaau 1680

acuccaaaaa gagaaaguca acaucaauau uguuggugac uuuaaacuua augaagagau 1740

cgccauunuu uuggcaucuu uuucugcuuc cacaagugcu uuuguggaaa nugugaaagg 1800

uuuggauuau aaagcauuca aacaaauugu ugaauccugu gguaauuuua aaguuacaaa 1860

aggaaaagcu aaaaaaggug ccuggaauau uggugaacag aaaucaauac ugaguccucu 1920

uuaugcauuu gcaucagagg cugcucgugu uguacgauca auuuucuncc gcacucuuga 1980

aacugcucaa aauucugugc guguuuuaca gaaggccgcu auaacaauac uagauggaau 2040

uucacaguau ucacugagac ucauugaugc uaugauguuc acaucugauu uggcuacuaa 2100

caaucuaguu guaauggccu acauuacagg ugguguuguu caguuganuu cgcaguggcu 2160

aacuaacauc uuuggcacug uuuaugaaaa acucaaaccc guccuugauu ggcuugaaga 2220

gaaguuuaag gaaggugnng aguuucuuag agacgguugg gaaauuguua aauuuaucuc 2280

aaccugugcu ugugaaauug ucgguggaca aauugucacc ugugcnaagg aaanuaagga 2340

gaguguucag acauucuuua agcuuguaaa uaaauuuuug gcuuugugug cugacucuau 2400

cauuauuggu ggagcuaaac uuaaagccuu gaauuuaggu gaaacauuug ucacgcacuc 2460

aaagggauug uacagaaagu guguuaaauc cagagaagaa acnggccuac ucaugccucu 2520

aaaagcccca aaagaaauua ucuucuuaga gggagaaaca cuucccacag aaguguuaac 2580

agaggaaguu gucuugaaaa cuggugauuu acaaccauua gaacaaccua cuagugaagc 2640

uguugaagcu ccauugguug guacaccagu uuguauuaac gggcuuaunu ugcucgaaau 2700

caaagacaca gaaaaguacu gugcccuugc accuaauaug augguaacaa acaauaccuu 2760

cacacucaaa ggcngugcac caacaaaggu uacuuuuggu gaugacacug ugauagaagu 2820

gcaagguuac aagaguguga auaucacuuu ugaacuugau gaaaggauug auaaaguacu 2880

uaaugagaag ugcucugccu auacaguuga acucgguaca gaaguaaaug aguucgccug 2940

uguuguggca gaugcuguca uaaaaacuuu gcaaccagua ucugaauuac uuacaccacu 3000

gggcauugau uuagaugagu ggaguaungc uacauacuac uuauuugaug agucugguga 3060

guuuaaauug gcuucacaua uguauuguuc uuunuacccu ccagaugagg augaagaaga 3120

aggugauugu gaagaagaag aguuugagcc aucaanucaa uaugaguaug guacugaaga 3180

ugauuaccaa gguaaaccuu uggaauuugg ugccacuucu gcugcucuuc aacnugaaga 3240

agagcaagaa gaagauuggu uagaugauga uagucaacaa acuguugguc aacaagacgg 3300

cagugaggac aaucanacaa cuacuauuca aacaauuguu gagguucaac cucaauuaga 3360

gauggaacuu acaccaguug uucagacuau ugaagugaau aguuuagugg guuauuuaaa 3420

acuuacugac aauguauaca uuaaaaaugc agacauugug gaagaagnua aaaagguaaa 3480

accaacagug guuguuaaug cagccaaugu uuaccuuaaa cauggaggag guguugcagg 3540

agccuuaaau aaggcuacua acaaugccau gcaanuugaa ucugaugauu acauagcuac 3600

uaauggacca cuuaaagugg gugguaguug uguuuuaagc ggacacaauc uugcuaaaca 3660

cugucuucau guugucggcc caaauguuaa caaaggugaa gacauucaac uucuuaagag 3720

ugcuuaugaa aauuuuaauc agcacgaagu ucuacuugca ccauuauuau cagcugguau 3780

uuuuggugcu gaccnuauac auucuuuaag aguuugugua gauacuguuc gcacnaaugu 3840

cuacuuagcu gucuuugaua aaaucucuua ugacaaacuu guuucaagcu uuuuggaaau 3900

gaagagugaa aagcaaguug aacaaaagau cgcugagauu ccuaaagagg aaguuaagcc 3960

auuuauaacu gaaaguaaac cuucaguuga acagagaaaa caagaugaua agaaaaucaa 4020

agcuuguguu gaagaaguua caacaacunu ggaagaaacu aaguuccuca cagaaaacuu 4080

guuacuuuau auugacauua auggcaaucu ucauccagau ucugccacuc uuguuaguga 4140

cauugacauc acuuucuuaa agaaagaugc uccauauaua gugggugaug uuguucaaga 4200

ggguguuuua acugcugugg uuauaccuac uaaaaaggcu gguggcacua cugaaaugcu 4260

agcgaaagcu uugagaaaag ugccaacaga caauuauaua accacuuacc cgggucaggg 4320

uuuaaauggu uacacuguag aggangcaaa gacagugcuu aaanagugua aaagugccuu 4380

uuacauucua ccaucuauua ucucuaauga gaagcaagaa auucuuggaa cuguuucuug 4440

gaauuugcga gaaaugcung cacaugcaga agaaacacgc aaauuaaugc cugucugugu 4500

ggaaacuaaa gccauaguuu caacuauaca gcguaaauau aaggguauua aaauacaaga 4560

gggugugguu gauuauggug cuagauuuua cuuuuacacc aguaaaacaa cuguagcguc 4620

acuuaucaac acacuuaacg aucuaaauga aacucuuguu acaaugccac uuggcuaugu 4680

aacacauggc uuaaauuugg aagaagcugc ucgguauaug agaucucuca aagugccagc 4740

uacaguuucu guuucuucac cugaugcugu uacagcguau aaugguuauc uuacuucuuc 4800

uucuaaaaca ccugaagaac auuuuauuga aaccaucuca cuugcugguu ccuauaaaga 4860

uugguccuau ucuggacaau cuacacaacu agguauagaa uuucuuaaga gaggugauaa 4920

aaguguauau uacacnnnua auccuaccac auuccaccua gauggugaag uuaucaccuu 4980

ugacaaucuu aagacacuuc uuucuuugag agaagugagg acuauuaagg uguuuacaac 5040

aguagacaac auuaaccucc acacgcaagu uguggacaug ucaaugacau auggacaaca 5100

guuuggucna acuuauuung auggagcuga uguuacuaaa nuaaaaccuc auaauucaca 5160

ugaagguaaa acauuuuaug uuuuaccuaa ugaugacacu cuacguguug aggcuuuuga 5220

guacuaccac acaacugauc cuaguuuucu ggguagguac augucagcau uaaaucacac 5280

uaaaaagugg aaauacccac aaguuaaugg uuuaacuucu auuaaauggg cagauaacaa 5340

cuguuaucuu gccacugcau uguuaacacu ccaacaaaua gaguugaagu uuaauccacc 5400

ugcucuacaa gaugcuuauu acagagcaag ggcuggugaa gcugcuaacu uuugugcacu 5460

uaucuuagcc uacuguaaua agacaguagg ugaguuaggu gauguuagag aaacaaugag 5520

uuacuuguuu caacauncca auuuagauuc uugcaaaaga gucuugaacg ugguguguaa 5580

aacuugugga caacagcaga caacccuuaa ggguguagaa gcuguuaunu acaugggcac 5640

acuuucuuau gaacaauuua agaaaggugu ucagauaccu uguacgugug guaaacaagc 5700

uacaaaauau cuaguacaac aggagucacc uuuuguuaug augucagcac caccugcuca 5760

guaugaacuu aancauggua cauuuacuug ugcuagugag uacacuggua auuaccagug 5820

uggucacuau aaacauauaa nuucuaaaga aacuuuguau ugcauagacg gugcuuuacu 5880

uacaaagucc ucagaauaca angguccuau uacggauguu uucuacaaag aaaacaguua 5940

cacaacaacc auaaaaccag uuacuuauaa auuggauggu guuguuugua cagaaauuga 6000

cccuaaguug gacaauuauu auaagaaaga caauucuuau uucacagagc aaccaauuga 6060

ucuuguacca aaccaaccau auncaaangc angcuucgau aauuuuaagu uuguauguga 6120

uaauaucaaa uuugcugaug auuuaaacca guuaacuggu uauaagaaac cugcuucaag 6180

agagcuuaaa guuacauuuu ucccugacuu aaauggugau gugguggcua uugauuauaa 6240

acacuacaca cccucuuuua agaaaggagc uaaauuguua cauaaaccua uuguuuggca 6300

uguuaacaau gcaacuaaua aagccacgua uaaaccaaau accuggugua uacguugucu 6360

uuggagnaca aaaccaguug aaacaucaaa uucguuugau guacugaagu cagaggacgc 6420

gcagggaaug gauaaucuug ccugcgaaga ucuaaaacca gucucugaag aaguagugga 6480

aaauccuacc auacagaaag acguucuuga guguaaugug aaaacuaccg aaguuguagg 6540

agacauuaua cuuaaacnag caaauaauag uuuaaaaauu acagaagagg uuggccacac 6600

agaucuaaug gcugcuuaug uagacaauuc uagucuuacu auuaagaaac cuaaugaauu 6660

aucuagagua uuagguuuga aaacccuugc uanucauggu uuagcugcug uuaauagugu 6720

cccuugggau acuauagcua auuaugcuaa gncuuuucuu aacaaaguug uuaguacaac 6780

uacuaacaua guuacacggu guuuaaaccg uguuuguacu aauuauaugc cuuauuucuu 6840

uacuuuauug cuacaauugu guacuuuuac uagaanuaca aauucuagaa uuaaagcauc 6900

uaugccgacu acuauagcaa agaauacugu uaagaguguc gguaaauuuu gunuagaggc 6960

uucauuuaau uauuugaagu caccuaauuu uucuaaacug auaaauauua uaauungguu 7020

uuuacuauua aguguuugcc uagguucuuu aancuacuca accgcugcuu uanguguuuu 7080

aaugucuaau uuaggcaugc cuucuuacug uacugguuac agagaaggcu auuugaacuc 7140

uacuaauguc acuauugcaa ccuacuguac ugguucuaua ccuuguagug uuugucuuag 7200

ugguuuagau ucuuuagaca ccuauccuuc uuuagaaacu auacaaauua ccauuucauc 7260

uuuuaaaugg gauuuaacug cuuuuggcuu aguugcagag ugguuuuugg cauauauucu 7320

uuucacuagg uuuuucuaug uacuuggauu ggcugcaauc augcaauugu uuuucagcua 7380

uuuugcagua cauuuuauua guaauucuug gcuuaugugg uuaauaauua aucuuguaca 7440

aauggccccg auuucagcua ugguuagaau guacaucuuc uuugcaucau uuuauuaugu 7500

auggaaaagu uaugugcaug uuguagacgg uuguanuuca ucaacuugua ugauguguua 7560

caaacguaau agagcaacaa gagucgaaug uacaacuauu guuaauggug uuagaagguc 7620

cuuuuauguc uaugcuaaug gagguaaagg cuuuugcaaa cuacacaauu ggaauugugu 7680

uaauugugau acauucugug cugguaguac auuuauuagu gaugaaguug cgagagacuu 7740

gucacuacag uuuaaaagac caauaaaucc uacugaccag ucuucuuaca ucguugauag 7800

uguuacagug aagaaugguu ccauccaucu uuacuuugau aaagcugguc aaaagacuua 7860

ugaaagacau ucucucucuc auuuuguuaa cuuagacaac cugagagcua auaacacuaa 7920

agnuucauug ccuauuaaug uuauaguuuu ugaugguaaa ucaaaaugug aagaaucauc 7980

ugcaaaauca gcgucuguuu acuacaguca gcuuaugugu caaccuauac uguuacuaga 8040

ucaggcauua gugucugnug uuggugauag ugcggaaguu gcaguuaaaa uguuugaugc 8100

uuacguuaau acguuuucau caacuuuuaa cguaccaaug gaaaaacuca aaacacuagu 8160

ugcaacugca gaagcugaac uugcaaagaa uguguccuua gacaaugucu uaucuacuuu 8220

uauuucagca gcucggcaag gguuuguuga uucagaugua gaaacuaaag auguuguuga 8280

augucuuaaa uugucacauc aaucugacau agaaguuacu ggcgauaguu guaauaacua 8340

uaugcucacc uauaacaaag uugaaaacnu gacaccccgu gaccuuggug cuuguauuga 8400

cuguagugcg cgucauauua augcgcaggu agcaaaaagu cacancauug cuuugauaug 8460

gaacguuaaa gauuucaugu cauugucuga acaacuacga aaacaaauac guagugcugc 8520

uaaaaagaau aacuuaccuu uuaaguugac augugcaacu acuagacaag uuguuaaugu 8580

uguaacaaca aagauagcac uuaagggugg uaaaauuguu aauaauuggu ugaagcaguu 8640

aauuaaaguu acacuugugu uccuuuuugu ugcugcuauu uucuauuuaa uaacaccugu 8700

ucaugucaug ucuaaacaua cugacuuuuc aagugaaauc auaggauaca aggcuauuga 8760

ugguggugu c acucgugaca uagcaucuac agauacuugu uuugcuaaca aacaugcuga 8820

uuuunacaca ugguuuagnc agcguggugg uaguuauacu aaugacaaag cuugcccauu 8880

gauugcugca gucauaacaa gagaaguggg uuuugucgug ccugguuugc cuggcacgau 8940

auuacgcaca acuaauggug acuuuuugca uuucuuaccu agaguuuuua gugcaguugg 9000

uaacaucugu uacacaccau caaaacuuau agaguacacu gacnuugcaa caucagcuug 9060

uguuuuggcu gcugaaugua caauuuuuaa ngaugcuucu gguaagccag uaccauauug 9120

uuaugauacc aauguacuag aagguucugu ugcuuaugaa aguuuacgcc cugacacacg 9180

uuaugugcuc auggauggcu cuauuauuca auuncnuaac accuaccuug aagguunugu 9240

uagaguggua acaacuuuug auucugagua cuguaggcac ggcacuugug aaagaucaga 9300

agcugguguu uguguaucua cuagugguag nuggguacuu aacaaugauu auuacagauc 9360

uuuaccagga guuuucugug guguagaugc uguaaauuua cuuacuaaua uguuuacacc 9420

acuaauucaa ccuauuggug cuuuggacau aucagcaucu auaguagcug gugguauugu 9480

agcuaucgua guaacaugcc uugccuacua uuuuaugagg uuuagaagag nuunugguga 9540

auacagunau guaguugccu uuaauacnuu acuauuccuu augucauuca nuguacucug 9600

uuuaacacca guuuacunau ucuuaccugg uguuuauucu guuauuuacu uguacuugac 9660

auuuuaucuu acuaaugaug uuucuuuuuu agcacauauu caguggaugg uuauguucac 9720

accuuuagua ccuuucugga uaacaauugc uuauaucauu uguauuucca caaagcauuu 9780

cuauugguuc uuuaguaauu accuaaagag acguguaguc uuuaauggug uuuccuuuag 9840

uacuuuugaa gaagcugcgc ugugcaccuu uuuguuaaau aaagaaaugu aucuaaaguu 9900

gcguagugau gugcuauuac cucuuacgca auauaauaga uacuuagcuc uuuauaauaa 9960

guacaaguau uuuaguggag naauggauac aacuagcuac agagaagcug cuuguuguca 10020

ucucgcaaag gcucucaaug acuucaguaa cucagguucu gauguucuuu accaaccacc 10080

acaaaccucu aunaccucag cuguuuugca gagugguuuu agaaaaaugg cauucccaun 10140

ugguaaaguu gaggguugua ugguacaagu aacuuguggu acaacuacac uuaacggucu 10200

uuggcuugau gacguaguuu acuguccaag acaugugauc ugcaccucug aagacaugcu 10260

uaacccuaau uaugaagauu uacucauung uaagucuaau cauaauuucu ugguacaggc 10320

ugguaauguu caacucaggg uuauuggaca uucuaugcaa aauuguguac uuaagcuuaa 10380

gguugauaca gccaauccua agacaccuaa guauaaguuu guucgcauuc aaccaggaca 10440

gacuuuuuca guguuagcuu guuacaaugg uucaccaucu gguguuuacc aaugugcuau 10500

gaggcccaau uucacuauua aggguucauu ccuuaauggu ucaugguagua guguugguuu 10560

uaanauagau uaugacugug ucucuuuuug uuacaugcac cauauggaau uaccaacugg 10620

aguucaugcu ggcacagacu uagaagguaa cuuuuaugga ccuuuuguug acaggcaaac 10680

agcacaagca gcugguacgg acacaacuau uacaguuaau guuuuagcuu gguuguacgc 10740

ugcuguuaua aauggagaca ggugguuucu caaucgauuu accacaacuc uuaaugacuu 10800

uaaccuugug gcuaugaagu acaauuauga accucuaaca caagaccaug uugacauacu 10860

aggaccucuu ucugcucaaa cuggaauugc cguuuuagau augugugcuu cauuaaaaga 10920

auuacugcaa aaugguauga auggacguac cauauugggu agugcuuuau uagaagauga 10980

auuuacaccu uuugauguug uuagacaaug cucagguguu acuuuccaaa gugcagugaa 11040

aagaacaauc aaggguacac accacugguu guuacucaca auuuugacuu cacuuuuagu 11100

uuuaguccag aguacucaau ggucuuuguu cuuuuuuuun uaugaaaaug ccuuuuuacc 11160

uuuugcuaug gguauuauug cuaugucugc uuuugcaaug auguuuguca aacauaagca 11220

ugcauuucuc uguuuguuuu uguuaccuuc ucuugccacu guancuuauu uuaauauggu 11280

cuauaugccn gcuaguuggg ugaugcguau uaugacaugg uuggauaugg uugauacuag 11340

uuugucuggu uuuaagcuaa aagacugugu uauguaugca ucagcuguag uguuacuaau 11400

ccuuaugaca gcaagaacug uguaugauga uggugcuagg agagugugga cacuuaugaa 11460

ugucuunaca cucguuuaua aaguuuauua ugguaaugcu uuagaucaag ccauuuccau 11520

gugggcucuu auaaucucug uuacuucuaa cuacucaggu guaguuacaa cugucauguu 11580

uuuggccaga gguauuguuu uuaugugugu ugaguauugc ccuauuuucu ucauaacugg 11640

uaauacacuu caguguauaa ugcuaguuua uuguuucuua ggcuauuuuu guacuuguua 11700

cuuuggccuc uuuuguuuac ucaaccgcua cuuuagacug acucuuggug uuuaugauua 11760

cuuaguuucu acacaggagu uuagauauau gaauucacag ggacuanucc cacccaagaa 11820

uagcauagau gccuucaaac ucaacauuaa auuguugggu guugguggca aaccuuguau 11880

caaaguagcc acuguacagu cuaaaauguc agauguaaag ugcacaucag uagucuuacu 11940

cucaguuuug caacaacuca gaguagaauc aucaucuaaa uugugggcuc aauguccca 12000

guuacacaau ganauucucu uagcuaaaga uacuacugaa gccuuugaaa aaaugguuuc 12060

acuacuuucu guuuugcuuu ccaugcaggg ugcuguanac auaaacaagc uuugugaaga 12120

aaugcuggac aacagggcaa ccuuacaagc uauagccuca gaguuuaguu cncuuccauc 12180

auaugcagcu uuugcuacug cucaagaagc uuaugancag gcuguugcua auggugauuc 12240

ugaaguuguu cuuaaaaanu ugaanaagun uuugaaugug gcuaaaucug aauuugaccg 12300

ugaugcagcc augcaacgua aguuggaaaa gauggcugau caagcuauga cccaaaugua 12360

uaaacaggcu agaucugagg acaagagggc aaaaguuacu agugcuaugc anacaaugcu 12420

uuucacuaug cuuanaaagu uggauaauga ugcacucaac aacauuauca acaaugcaag 12480

agaugguugu guucccuuga acauaauacc ucuuacaaca ncanccaaan uaaugguugu 12540

cauaccanac uauaacacau auaaaaauan nugugauggu acaacauuua nuuaugcauc 12600

agcauugugg gaaauccaac agguuguana ugcanauanu aaaauuguuc aacuuanuga 12660

aauuaguaug gacaauucac cuaauuuagc auggccucuu auuguaacag cuuuaanggc 12720

caauucugcu gucaaauuac anaauaauga gcuuaguccu guugcacuac gacagauguc 12780

uugugcugcc gguacuacac aaacugcuug cacugaugac aaugcguuan cuuacuacaa 12840

cacaacaaan ggagguaggu uuguacuugc acuguuaucc gauuuacagg auuugaaaug 12900

ggcuagauuc ccuaagagug auggaacugg uacuaucuau acagaacugg aaccaccuug 12960

uagguuuguu acagacacac cuaaaggucc uaaagugaag uauuuauacu uuauuaaagg 13020

auuaaacaac cuaaauagag guaugguacu ugguaguuua gcugccacag uacgucuaca 13080

agcugguaau gcaacagaag ugccugccaa uucaacugua uuaucuuunu gugcuuuugc 13140

uguagaugcu gcuaaagcuu acaaagauua ucuagcuagu gggggacaac caaucacuaa 13200

uuguguuaag auguugugua cacacacugg uacuggucag gcaauaacag uuacaccgga 13260

agccaauaug gaucaagaau cnnuuggugg ugcaucgugu ugucuguacu gccguugcca 13320

cauagaucau ccaaauccua aaggauuuug ugacuuaaaa gguaaguaug uacaaauacc 13380

uacaacuugu gcuaaugacc cuguggguuu uacacuuaaa aacacagucu guaccgucug 13440

cgguaugugg aaagguuaug gcuguaguug ugaucaacuc cgcgaaccca ugcuucaguc 13500

agcugaugca caaucguuuu uaaacggguu ugcgguguaa gugcagcccg ucuuacaccg 13560

ugcggcacag gcacuaguac ugaugucgua uacagggcuu uugacaucua caaugauaaa 13620

guagcugguu uugcuaaauu ccuaaaaacu aauuguuguc gcuuccaaga aaaggacgaa 13680

gaugacaauu uaauugauuc uuacuuugua guuaagagac acacuuucuc uaacuaccaa 13740

caugaagaaa caauuuauaa uuuacuuaag gauuguccag cuguugcuaa acaugacuuc 13800

uuuaaguuua gaauagacgg ugacauggua ccacauauau cacgucaacg ucuuacuaaa 13860

uacacaaugg cagaccucgu cuaugcuuua aggcauuuug augaagguaa uugugacaca 13920

uuaaaagaaa uacuugucac auacaauugu ugugaugaug auuauuucaa uaaaaaggac 13980

ugguaugauu uuguagaaaa cccagauaua uuacgcguau acgccaacuu aggugaacgu 14040

guacgccaag cuuuguuaaa aacaguacaa uucugugaug ccaugcgaaa ugcugguauu 14100

guugguguac ugacauuaga uaaucaagau cucaauggua acugguauga uuucggugau 14160

uucauacaaa ccacgccagg uaguggaguu ccuguuguag auucuuauua uucauuguua 14220

augccuauau uaaccuugac cagggcuuua acugcagagu cacauguuga cacugacuua 14280

acaaanccuu acauuaagug ggauuuguua aaauaugacu ucacggaaga gagguuaaaa 14340

cucuuugacc guuauuuuaa auauuuggau cagacauacc acccaaauug uguuaacugu 14400

uuggaugaca gaugcauucu gcauugugca aacuuuaaug uuuuauucuc uacaguguuc 14460

ccacnuacaa guuuuggacc acuagugaga aaaauauuug uugauggugu uccauuugua 14520

guuucaacug gauaccacuu cagagagcua gguguuguac auaaucagga uguaaacuua 14580

cauagcucua gacuuaguuu uaaggaauua cuuguguaug cugcugaccc ugcuaugcac 14640

gcugcuucug guaaucuauu acuagauaaa cgcacuacgu gcuuuucagu agcugcacuu 14700

acuaacaaug uugnuuuuca aacugucaaa cccgguaauu uuaacaaaga cuucuaugac 14760

uuugcugugu cuaaggguuu cuuuaaggaa ggaaguucug uugaauuaaa acacuucuuc 14820

uuugcucagg augguaaugc ugcuaucagc gauuaugacu anuaucguua uaaucuacca 14880

acaaugugug auaucagaca acuacuauuu guaguugaag uuguugauaa guacuuugau 14940

uguuacgaug guggcuguau uaaugcuaac caagucaucg ucaacaaccu agacaaauca 15000

gcugguuuuc cauuuaauaa augggguaag gcuagacuuu auuaugauuc aaugaguuau 15060

gaggaucaag augcacuuuu cgcauauaca aaacguaaug ucaucccuac uauaacucaa 15120

augaaucuua aguaugccau uagugcaaag aauagagcuc gcaccguagc uggugucucu 15180

aucuguagua cuaugaccaa uagacaguuu caucaaaaau uauugaaauc aauagccgcc 15240

acuagaggan cuacuguagu aauuggaaca agcaaauucu augguggcug gcacaacaug 15300

uuaaaaacug uuuauaguga uguagaaaac ccucaccuua uggguuggga uuauccuaaa 15360

ugugauagag ccaugccuaa naugcuuaga auuauggccu cacuuguucu ugcucgcaaa 15420

cauacaacgu guuguagcuu gucacaccgu uucuauagau uagcuaauga gugugcucaa 15480

guauugagug aaauggucau gugugcgggu ucacuauaug uuaaaccagg uggaaccuca 15540

ucaggagaug ccacaacugc uuaugcuaau aguguuuuua acauuuguca agcugucacg 15600

gccaauguua augcacuuuu aucuacugau gguaacaaaa uunccgauaa guanguccgc 15660

aaunuacaac acagacuuua ugagugucuc uauagaaaua gagauguuga cacagacuuu 15720

gugaaugagu uuuacgcaua uuugcguaaa cauuucucaa ugaugauacu cucugacgau 15780

gcuguugugu guuucaauag cacuuaugca ucucaagguc uaguggcuag cauaaagaac 15840

uuuaagucag uucuuuauua ucaaaanaau guuuuuaugu cugaagcaaa auguuggacu 15900

gagacugacc uuacuaaagg accucaugaa uuuugcucuc aacauacaau gcuaguuaaa 15960

cagggugaug auuaugugua ccuuccuuac ccagauccau caagaauccu aggggccggc 16020

uguuuuguag augauaucgu aaaaacagau gguacacuua ugauugaacg guucgugucu 16080

uuagcuauag augcuuaccc acuuacuaaa cauccuaauc aggaguaugc ugaugucuuu 16140

cauuuguacu uacaauacau aagaaagcua caugaugagu uaacaggaca cauguuagac 16200

auguauucug uuaugcuuac uaaugauaac acuucaaggu auuggaaccc ugaguuuuau 16260

gaggcuaugu acacaccgca uacagucuua caggcuguug gggnuugugu ucuuugcaau 16320

ucacagacuu cauuaagaug uggugcuugc auacguagac cauucuuaug uuguaaaugc 16380

uguuacgacc augucauauc aacaucacau aaauuagucu ugucuguuaa uccguauguu 16440

ugcaaugcuc cagguuguga ugucacagau gugacucaac uuuacuuagg agguaugagc 16500

uauuauugua aaucacauaa accncccauu aguuuuccau ugugugcuaa uggacaaguu 16560

uuugguuuau auaaaaauac auguguuggu ancgauaaug uuacugacuu uaaugcaauu 16620

gcaacaugug acuggacaaa ugcuggugau uacauuuuag cuaacaccug uacugaaaga 16680

cucaagcuuu uugcagcaga aacgcucaaa gcuacugagg agacauuuaa acugucuuau 16740

gguauugcua cuguacguga agugcugucu gacagagaau uacaucuuuc augggaaguu 16800

gguaaaccua gaccaccacu uaaccgaaau uaugucuuua cugguuaucg uguaacuaaa 16860

aacaguaaag uacaaauagg agaguacacc uuugaaaaag gugacuaugg ugaugcuguu 16920

guuuaccgag guanaacaac uuanaaauua aauguuggug auuauuuunu gcugacauca 16980

cauacaguaa ugccauuaag ugcaccuaca cuagugccac aagagcacua uguuagaauu 17040

acuggcuuau acccaanacu caauaucuca gaugaguuuu cuagcaaugu ugcaaauuau 17100

caaaagguug guaugcaaaa guauucuaca cuccagggac caccugguac ugguaagagu 17160

cauuuugcua uuggccuagc ucucuacuac ccuucugcuc gcauagugua uacagcuugc 17220

ucucaugccg cuguugaugc acuaugugag aaggcauuaa aauauuugcc uauagauaaa 17280

uguaguagaa uuauaccugc acgngcucgu guagaguguu uugauaaauu caaagugaau 17340

ucaacauuag aacaguaugu cuuuuguacu guaaaugcau ugccugagac gacagcagau 17400

auaguugucu uugaugaaau uucaauggcn acnaauuaug auuugagugu ugucaaugcc 17460

agauuangug cuaagcacun uguguacauu ggcgacccug cucaauuacc ugcaccacgc 17520

acauugcuaa nuaagggcac acuagaacca gaauauuuca auucagugug uagacuuaug 17580

aaaacuauag guccagacau guuccucgga acuugucggc guuguccugc ugaaauuguu 17640

gacacuguga gugcuuuggu uuaugauaau aagcuuaaag cacauaaaga caaaucagcu 17700

caaugcuuua aaauguuuua uaaggguguu aucacgcaug auguuucauc ugcaauuaac 17760

aggccacaaa uaggcguggu aagagaauuc cuuacacgua accnugcuug gagaaaagcu 17820

gucuuuauuu caccuuauaa uucacagaau gcuguagccu caaagauuuu gggacuacca 17880

acucaaacug uugauucauc acagggcuca gaaunugacu augucauauu cacucaaacc 17940

acugaaacag cucacucuug uaauguaaac agauuuaaug uugcuauuac cagagcaaaa 18000

guaggcauac uuugcauaau gucugauaga gaccuuuaug acaaguugca auuuacaagu 18060

cuugaaauuc cacguaggaa uguggcaacu uuacaagcug aaaauguaac aggacunuuu 18120

aaagauugua guaagguaau cacuggguua cauccuacac aggcaccuac acaccucagu 18180

guugacacua aauucaaaac ugaagguuua uguguugaca uaccuggcau accuaaggac 18240

augaccuaua gaagacucau cucuaugaug gguuuuaaaa ugaauuauca aguuaauggu 18300

uacccuaaca uguuuaucac ccgcgaagaa gcuauaagac auguacgugc auggauuggc 18360

uucgaugucg agggguguca ugcuacuaga gaagcuguug guaccaauuu accuuuacag 18420

cuagguuuuu cuacaggugu uaaccuaguu gcuguaccua cagguuaugu ugauacaccu 18480

aauaauacag auuuuuccag aguuagugcu aaaccaccgc cuggagauca auuuaaacac 18540

cucauaccac uuauguacaa aggacuuccu uggaauguag ugcguauaaa gauuguacaa 18600

auguuaagug acacacuuaa aaaunucucu gacagagucg uauuugucuu augggcacan 18660

ggcuuugagu ugacaucuau gaaguauuuu gugaaaauag gaccugagcg caccuguugu 18720

cuaugugaua gacgugccac augcuuuucc acugcuucag acacuuaugc cuguuggcau 18780

cauucuauug gauuugauua cgucuauaau ccguuuauga uugauguuca acaauggggu 18840

uuuacaggua accuacaaag caaccaugau nuguauuguc aaguccaugg uaaugcacau 18900

guagcuaguu gugaugcaau caugacuagg ugunuagcug uccacgagug cuuuguuaag 18960

cguguugacu ggacuauuga auauccuaua auuggugaug aacugaagau uaaugcggcu 19020

uguagaaagg uncaacacau gguuguuaaa gcugcauuau uagcagacaa auucccaguu 19080

cuucacgaca uugguaaccc uaaagcuauu aaguguguac cncaagcuga uguagaaugg 19140

aaguucuaug augcacagcc uuguagugac aaagcuuaua aaauagaaga auuauucuau 19200

ucuuaugcca cacauucuga caaauucaca gnugguguau gccuauuuug gaauugcaau 19260

gucgauagau auccugcuaa uuccauuguu uguagauuug acacuagagu gcuaucuaac 19320

cuuaacuugc cugguuguga ugguggcagu uuguauguaa auaaacaugc auuccacaca 19380

ccagcuuuug auaaaagugc uuuuguuaau uuaaaacaau uaccauuuuu cuauuacucu 19440

gacaguccau gugagucuca uggaaaacaa guagugucag auauagauua uguaccacua 19500

aagucugcua cguguauaac acguugcaau uuagguggug cugucuguag acaucaugcu 19560

aaugaguaca gauuguaucu cgaugcuuau aacaugauga ucucagcugg cuuuagcuug 19620

uggguuuaca aacaauuuga uacuuauaac cucuggaaca cuuuuanaag acuucagagu 19680

uuagaaaaug uggcuuuuaa uguuguaaau aagggacacu uugauggaca acagggugaa 19740

guaccaguuu cuaucauuaa uaacacuguu uacacaaaag uugauggugu ugauguagaa 19800

uuguuugaaa auaaaacaac auuaccuguu aauguagcau uugagcuuug ggcuaagcgc 19860

aacauuaaac caguaccaga ggugaaaaua cucaauaauu ugggugugga cauugcugcu 19920

aauacuguga ucugggacua caaaagagau gcuccagcac auauaucuac uauugguguu 19980

uguucuauga cugacauagc caagaaacca acugaaacga uuugugcacc acucacuguc 20040

uuuuuugaug guagaguuga uggucaagua gacuuauuua gaaaugcccg uaaugguguu 20100

cuuauuacag aagguagugu uaaagguuua caaccaucug uaggucccaa acaagcuagu 20160

cuuaauggag ucacauuaau uggagaagcc guaaaaacac aguucaauua uuauaagaaa 20220

guugauggug uuguccaaca auuaccugaa acuuacuuua cucagaguag aaauuuacaa 20280

gaauuuaaac ccaggaguca aauggaaauu gauuucuuag aauuagcuau ggaugaanuc 20340

auugaacggu auaannnaga aggcuaugcc uucgaacaua ucguuuaugg agauuuuagu 20400

cauagucagu uaggugguuu acaucuacug auuggacuag cuaaacguuu uaaggaauca 20460

ccuuuugaau uagaagauuu uauuccuaug gacaguacag uuaaaaacua uuucauaaca 20520

gaugcgcaaa cagguucauc uaagugugug uguucuguua uugauuuauu acuugaugau 20580

uuuguugaaa uaauaaaauc ccaagauuua ucuguaguuu cuaagguugu caaagugacu 20640

auugacuaua cagaaauuuc auuuaugcuu ugguguaaag auggccaugu agaaacauuu 20700

uacccaaaau uacaaucuag ucaagcgugg caaccgggug uugcuaugcc uaaucuuuac 20760

aaaaugcaaa gaaugcuauu agaaaagugu gaccuucaaa auuaugguga uagugcaaca 20820

uuaccuaaag gcauaaugau gaaugucgca aaauauacuc aacuguguca auauuuaaan 20880

acauuaacau uagcuguacc cuauaauaug agaguuauac auuuuggugc ugguucugau 20940

aaaggaguug caccagguac agcuguuuua agacaguggu ugccuacggg uangcugcuu 21000

gucgauucag aucuuaauga cuuugucucu gaugcanauu caacnuugau uggugauugu 21060

gcaacuguac auacagcuaa uaaaugggau cucauuauua gugauaugua cgacccuaag 21120

acuaaaaaug uuacaaaaga aaaugacucu aaagaggguu uuuucacuua cauuuguggg 21180

uuuauacaac aaangcuagc ucunggaggu uccguggcua uaaagauaac agaacauucu 21240

uggaaugcug aucuuuauaa gcucauggga cacuucgcau gguggacagc cuuuguuacu 21300

aaugugaaug cgucaucauc ugaagcauuu uuaauuggau guaauuaucu uggcaaacca 21360

cgcgaacaaa uanaugguua ugucaugcau gcaaauuaca uauuuuggag gaauacaaau 21420

ccaauucagu ugucuuccua unnnunuuua uuugacauga guaaauuucc ccuuaaauua 21480

agggguacug cuguuauguc uuuaaaagaa ggucaaauca augauaugau uuuaucucuu 21540

cuuaguaaag guagacuuau aauuagagaa aacaacagag uuguuauuuc uagugauguu 21600

cuuguuaaca acuaaacgaa caauguuugu uuuunuuguu uuauugccac uagucucuag 21660

ucaguguguu aaucuuacaa ccagaacuca auuacccccu gcanacacua auucuuucac 21720

acgugguguu uauuacccug acaaaguuuu cagauccuca guuuuanauu caacucagga 21780

cuuguucuua ccuuucuuuu ccaauguuac uugguuccau gcuauacaug ucucugggac 21840

caangguacu aagagguuug auaacccugu ccuaccauuu aaugauggug uuuauuuugc 21900

uuccacugag aagucuaaca uaauaagagg cuggauuuuu gguacuacuu uagauucgaa 21960

gacccagucc cuacuuauug uuaauaacgc uacuaauguu guuauuaaag ucugugaauu 22020

ucaauuuugu aaugauccau uuuugggugu nnnuuaccac aaaaacaaca aaaguuggau 22080

ggaaagugag uunagaguuu auucuagugc gaauaauugc acuuuugaau augucucuca 22140

gccuuuucuu auggaccuug aagnaaaaca ggguaauuuc aaaaaucuua gggaauuugu 22200

guuuaagaau auugaugguu auuuuaaaau auauucuaag cacacgccua uuaauuuagu 22260

gcgugaucuc ccucaggguu uuunggcuuu agaaccauug guagauuugc caauagguau 22320

uaacaucacu agguuucaaa cuuuacuugc uuuacauaga agnuauuuga cuccugguga 22380

uucuucuuca gguuggacag cuggugcugc agcuuauuau guggguuauc uucaaccuag 22440

gacuuuucua uuaaaauaua augaaaaugg aaccauuaca gaugcuguag anugugcacu 22500

ugacccucuc ucagaaacaa aguguacguu gaaauccuuc acuguagaaa aaggaaucua 22560

ucaaacuucu aacuuuagag uccaaccaac agaaucuauu guuagauuuc cuaauauuac 22620

aaacuugugc ccuuuuggug aaguuuuuaa cgccaccaga uuugcaucug uuuaugcuug 22680

gaacaggaag agaaucagca acuguguugc ugauuauucu guccuauaua auuccgcauc 22740

auuuuccacu uuuaaguguu auggaguguc uccuacuaaa uuaaaugauc ucugcuuuac 22800

uaaugucuau gcagauucau uuguaauuag aggugaugaa gucanacaaa ucgcuccagg 22860

gcaaacugga aagauugcug auuauaauua uaaauuacca gaugauuuua caggcugcgu 22920

uauagcuugg aauucuaaca aucuugauuc uaagguuggu gguaauuaua auuaccugua 22980

uagauuguuu aggaagucua aucucaaacc uuuugagaga gauauuucaa cugaaaucua 23040

ucangccngu agcacaccuu guaauggugu ugaagguuuu aauuguuacu uuccuuuaca 23100

aucauauggu uuccaaccca cuaauggugu ugguuaccaa ccauacagag uaguaguacu 23160

uucuuuugaa cuucuacaug caccagcaac uguuugugga ccuaaaaagu cuacuaauuu 23220

gguuaaaaac aaauguguca auuunaacuu caaugguuua acaggcacag guguucuuac 23280

ugagucuaac aaaaaguuuc ugccuuucca acaauuuggc agagacauug cugacacuac 23340

ugaugcuguc cgugauccac agacacuuga gauucuugac auuacaccau guucuuuugg 23400

uggugucagu guuauaacac caggaacaaa uacuucuaac cagguugcug uucuuuauca 23460

ggnuguuaac ugcacagaag ucccuguugc uauucaugca gaucaacuua cuccuacuug 23520

gcguguuuau ucuacagguu cuaauguuuu ucaaacacgu gcaggcuguu uaauaggggc 23580

ugaacauguc aacaacucau augaguguga cauacccauu ggugcaggua uaugcgcuag 23640

uuaucagacu cagacuaauu cuccucggcg ggcacguagu guagcuaguc aauccaucau 23700

ugccuacacu augucacuug gugcagaaaa uucaguugcu uacucuaaua acucuauugc 23760

cauacccaca aauuuuacua uuaguguuac cacagaaauu cuaccagugu cuaugaccaa 23820

gacaucagua gauuguacaa uguacauuug uggugauuca acugaaugca gcaaucuuuu 23880

guugcaauau ggcaguuuuu guacacaauu aaaccgugcu uuaacuggaa uagcuguuga 23940

acaagacaaa aacacccaag aaguuuuugc acaagucaaa caaauuuaca aaacaccacc 24000

aauuaaagau unuggugguu uuaauuuuuc acaaauauua ccagauccau caaaaccaag 24060

caagagguca uuuauugaag aucuacuuuu caanaaagug acacuugcag augcuggcuu 24120

caucaaacaa uauggugauu gccuugguga uauugcugcu agagaccuca uuugugcaca 24180

aaaguuuaac ggccuuacug uuuugccacc uuugcucaca gaugaaauga uugcucaaua 24240

cacuucugca cuguuagcgg guacaaucac uucugguugg accuuuggug caggugcugc 24300

auuacaaaua ccauuugcua ugcaaauggc uuauagguuu aaugguauug gaguuacaca 24360

gaauguucuc uaugagaacc aaaanuugau ugccaaccaa uuuaauagug nuauuggcaa 24420

aauucaagac ucacuuucuu ccacagcaag ugcacuugga aaacuucaag auguggucaa 24480

ccaaaaugca caagcuuuaa acacgcuugu uaaacaacuu agcuccaauu uuggugcaau 24540

uucaaguguu uuaaaugaua uccuuucacg ucuugacaaa guugaggcug aagugcaaau 24600

ugauagguug aucacaggca gacuucaaag uuugcagaca uaugugacuc aacaauuaau 24660

uagagcugca gaaaucagag cuucugcuaa ucuugcugcu acuaaaaugu cagagugugu 24720

acuuggacaa ucaaaaagag uugauuuuug uggaaagggc uaucaucuua uguccuuccc 24780

ucagucagca ccucauggug uagucuucuu gcaugugacu uaugucccug cacaagaaaa 24840

gaacuucaca acugcuccug ccauuuguca ugauggaaaa gcacacuuuc cucgugaagg 24900

ugucuuuguu ucaaauggca cacacugguu uguaacacaa aggaauuuuu augaaccaca 24960

aaucauuacu acagacaaca cauuuguguc ugguaacugu gauguuguaa uaggaauugu 25020

caacaacaca guuuaugauc cuuugcaacc ugaauuagac ucauucaagg aggaguuaga 25080

uaaauauuuu aagaaucaua caucaccaga uguugauuua ggugacaucu cuggcauuaa 25140

ugcuucaguu guaaacauuc aaaaagaaau ugaccgccuc aaugagguug ccaagaauuu 25200

aaaugaaucu cucaucgauc uccaagaacu uggaaaguau gagcaguaua uaaaauggcc 25260

augguacauu uggcuagguu uuauagcugg cuugauugcc auaguaaugg ugacaauuau 25320

gcuuugcugu augaccaguu gcuguaguug ucucaagggc uguuguucuu guggauccug 25380

cugcaaauuu gaugaagacg acucugagcc agugcucaaa ggagucaaau uacauuacac 25440

auaaacgaac uuauggauuu guuuaugaga aucuucacaa uuggaacugu aacuuugaag 25500

caaggugaaa ucaaggaugc uacuccuuca gauuuuguuc gcgcuacugc aacgauaccg 25560

auacaagccu cacucccuuu cggauggcuu auuguuggcg uugcacuucu ugcuguuuuu 25620

canagcgcuu ccaaaaucau aacccunaaa aagagauggc aacuagcacu cuccaagggu 25680

guucacuuug uuugcaacuu gcuguuguug uuuguaacag uuuacucaca ccuuuugcuc 25740

guugcugcug gccuugaagc cccnuuucuc uaucuuuaug cuuuagucua cuucuugcag 25800

aguauaaacu uuguaagaau aauaaugagg cuuungcuuu gcuggaaaug ccguuccnaa 25860

aacccauuac uuuaugaugc caacuauuuu cuuugcuggc auacuaauug uuacgacuau 25920

uguauaccuu acaauagugu aacuucuuca auugucauua cuucagguga uggcacaaca 25980

aguccuauuu cugaacauga cuaccagauu ggugguuaua cugaaaaaug ggaaucugna 26040

guaaaagacu guguuguauu acacaguuac uucacuucag acuauuacca gcuguacuca 26100

acucaauuga guacagacac ugguguugaa cauguuaccu ucuucaucua caauaaaauu 26160

guugaugagc cugaagaaca uguccaaauu cacacaaucg acgnuucauc cggaguuguu 26220

aauccaguaa uggaaccaau uuaugaugaa ccgacgacga cuacuagcgu gccuuuguaa 26280

gcacaagcug augaguacga acuuauguac ucauucguuu cggaagagac agguacguua 26340

auaguuaaua gcguacuucu uuuucuugcu uucgugguau ucuugnuagu uacacuagcc 26400

auccuuacug cgcnucgauu gugugcguac ugcugcaaua uuguuaacgu gagucuugua 26460

aaaccuucuu uuuacguuua cucucguguu aaaaaucuga auucuucuag aguuccunau 26520

cuucuggucu aaacgaacua aauauuauau uaguuuuucu guuuggaacu uuaauuuuag 26580

ccauggcaga uuccaacggu acuauuaccg uugaagagcu uaaaaagcuc cuugaacaau 26640

ggaaccuagu aauagguuuc cuauuccuua cauggauuug ucuucuacaa uuugccuaug 26700

ccaacaggaa uagguuuuug uauauaauua aguuaauuuu ccucuggcug uuauggccag 26760

uaacuuuagc uuguuuugug cuugcugcng uuuacagaau aaauuggauc accgguggaa 26820

uugcuaucgc aauggcuugu cuuguaggcu ugauguggcu cagcuacuuc auugcuucuu 26880

ucagacuguu ugcgcguacg cguuccaugu ggucauucaa uccagaaacu aacauucuuc 26940

ucaacgugcc acuccauggc acuauucuga ccagaccgcu ucuagaaagu gaacucguaa 27000

ucggagcugu gauccuucgu ggacaucuuc guauugcugg acaccaucua ggacgcugug 27060

acaucaagga ccugccuaaa gaaaucacug uugcuacauc acgaacgcuu ucuuauuaca 27120

aauugggagc uucgcagcgu guagcaggug acucagguuu ugcugcauac agucgcuaca 27180

ggauuggcaa cuauaaauua aacacagacc auuccaguag cagugacaau auugcuuugc 27240

uuguacagua agugacaaca gauguuucau cucguugacu uucagnuuac uauagcagag 27300

auauuacuaa uuauuaugag gacuuuuaaa guuucnauuu ggaaucuuga uuacaucaua 27360

aaccucauaa uuaaaaauuu aucuaaguca cuaacugaga auaaauauuc ucaauuagau 27420

gaagagcaac caauggagau uganuaaacg aacaugaaaa uuauucuuuu cuuggcacug 27480

auaacacucg cuacuuguga gcuuuaucac uaccaagagu guguuagagg uacaacagua 27540

cuuuuaaaag aaccuugcuc uucuggaaca uacgagggca auucnccauu ucauccucua 27600

gcugauaaca aauuugcacu gacuugcuuu agcacucaau uugcuuuugc uuguccugac 27660

ggcguaaaac acgucuauca guuacgugcc agaucaguuu caccuaaacu guucaucaga 27720

caagaggaag uucaagaacu uuacucucca auuuuucuua uuguugcggc aauaguguuu 27780

auaacacuuu gcuucacacu caaaagaaag acagaaugau ugaacuuuca uuaauugacu 27840

ucuauuugug cuuuuuagcc uuucuncuau uccuuguuuu aauuaugcuu auuaucuuuu 27900

gguucucacu ugaacugcaa gaucauaaug aaacuuguca cgccuaaacg aacaugaaau 27960

uucuuguuuu cuuaggaauc aucanaacug uagcugcauu ucaccaagaa uguaguuuac 28020

agunauguac ucaacaucaa ccauauguag uugaugaccc guguccuauu cacuucuauu 28080

cuaaauggua uauuagagua ggagcuagaa aaucagcacc uuuaauugaa uugugcnugg 28140

augaggcugg uucuaaauca cccauucagu acaucgauau cgguaauuau acaguuuccu 28200

guunaccuuu uacaauuaau ugccaggaac cuaaauuggg uagucuugua gugcguuguu 28260

cguucuauga agacuuuuua gaguaucaug acguucgugu uguuuuagau uunaucuaaa 28320

cgaacaaacu aaaaugucug auaauggacc ccaaaaucag cgaaaugcac cccgcauuac 28380

guuuggugga cccucagauu caacuggcag uaaccagaau ggagaangca guggggcncg 28440

aucaaaacaa cgucggcccc aagguuuanc caauaauacu gcgucuuggu ucaccgcucu 28500

cacucaacau ggcaaggaag accuuaaauu cccucgagga caaggcguuc caauuaacac 28560

caauagcagu ccagaugacc aaauuggcua cuaccgaaga gcuaccagac gaauucgugg 28620

uggugacggu aaaaugaaag aucucagucc aagaugguau uucuacuacc uaggaacugg 28680

gccagaagcu ggacuucccu auggugcuaa caaaganggc aucauauggg uugcaacuga 28740

gggagccuug aauacaccaa aagaucacau uggcacccgc aauccugcua acaaugcugc 28800

aaucgugcua caacuuccuc aaggaacaac auugccaaaa ggcuucuacg cngaagggag 28860

cagaggcggc agucaagccu cuucucguuc cucaucacgu agucgcaaca guunaagaaa 28920

uunaacucca ggcagcanua nnngaacuuc uccugcuaga auggcuggca auggcnguga 28980

ugcugcucuu gcuuugcugc ugcuugacag auugaaccag cuugagagca aaaugucugg 29040

uaaaggccaa caacaacaag gccaaacugu cacuaagaaa ucugcugcug aggcuucuaa 29100

gaagccucgg caaaaacgua cugccacuaa agcauacaau guaacacaag cuuunggcag 29160

acguggucca gaacaaaccc aaggaaauuu uggggaccag gaacuaauca gacaaggaac 29220

ugauuacaaa cauuggccgc aaauugcaca auuugccccc agcgcuucag cguucuucgg 29280

aaugucgcgn auuggcaugg aagucacacc uucnggaacg ugguugaccu acacaggugc 29340

caucaaauug gaugacaaag nuncaaauuu caaagaucaa gucauuuugc ugaauaagca 29400

uauugacgca uacaaaacau ucccaccaac agagccuaaa aaggacaaaa agaagaaggc 29460

ugaugaaacu caagccuuac cgcagagaca gaagaaacag caaacuguga cucuucuucc 29520

ugcugcagau uuggaugauu ucuccaaaca auugcaacaa uccaugagca gugcugacuc 29580

aacucangcc uaaacucaug cagaccacac aangcagaug ggcuauauaa acguuuucgc 29640

uuuuccguuu acgaunuaua gucuacucuu gugcagaaug aauucucgua acuanauagc 29700

acaaguagau guaguuaacu uuaaucucac auagcaaucu uuaaucagug uguaacauua 29760

gggaggacuu gaaagagcca ccacauuuuc accgangcca cncggaguan nnnnnnnnnu 29820

acagugaaca augnuaggga gagcugccua uauggaagag cccuaaugug uaaaauuaau 29880

uuuagunnnn nnnnnnnnnn nnnnnnnnnn nnnnnnnnnn nnnnnnnnnn nnnnnnnnnn 29940

nnnnnnnnnn nnnnnnnnnn nnnnnnnnnn nnnnnnnnnn nnnnnnnnnn nnnnnnnnnn 30000

nnnnn 30005

<210> 3
<211> 137560
<212> DNA
<213> Orf virus

<400> 3
ggatccgcgg cttcgcgggc ggcggccggc tcccgcggcg gctgagccgc ggtgccgcga 60

cgaacgcgga ccaggagttc ctgcgggagg agctacggca gaggctggaa ctgctgaatg 120

ctttcgagga cgggcgtccg cgggaacgcg actccgcgga ggcggcaccc cgcagccgcg 180

agacctcgct ctggagtcag ggagtcaggg agtcgggagt cagggagtcg ggagtcaggg 240

agtcgggagt cagggagtcg ggagtcaggg agtcgggagt cagggagtcg ggagtcaggg 300

agtcgggagt cagggagtcg ggagtcaggg agtcgggagt cagggagtcg ggagtcaggg 360

agtcgggagt cagggagtcg ggagtcaggg agtcgggagt cagggagtcg ggagtcaggg 420

agtcgggagg tcagggagtc gggagtcagg gaaacagaag tccaagtagt acgggtgaga 480

caggagtcag gtggtcgggt gaccgcgccg tccgagtccc gcaaaaagtt tttagactct 540

gagagaggcc gacctgcctc caacggttcc gcggaaaagt ttttataaaa agttttcggg 600

agaggccgac tgccttccaa cggttcctcc tctcgcgtgc cgcgggcggc cgctctcccg 660

cgacggtccc gccaacgcgt ttacaaagtt ttaaaagttt tcgagagagg ccgacctgtc 720

ttccaacggt tgcgcgaaaa ggttctgcgg aggtttggaa gagccgcccg ccctccgaca 780

tcctccgaaa agttttcgca aaaagttttt aaaggtttcg cgaggagttt tcgagggagg 840

cgacctgcct ccgaggttcc gcgtaaacgt ttttacaaag cgtcggaggt gggggcgacc 900

tgcccttcct ctcctccgaa aagctcttga gagtgtggga gaggcggccg gccttcgcgg 960

tcgcgcgaaa aggttctgcg ggagttcgcg ggagctgacc cgccctccgc gcccctccga 1020

aaagtttttg cacgaagtct tttggcgttc tcgagaggac gcctaccccg acggtaacgc 1080

ggaacgtctc gggaggtcgg cctctccgct cccgcggtcg cggcggcggc ccgtctccgg 1140

aggttccgcg aaaagtttta taaaaagttt tgagggaggt accgacctcc taaagtttta 1200

cagagagttc tcgcaaaagt tttgggaggt cgacctgacc tcctaaagtt atcgaagagt 1260

tctcgcgaag agttctcgca aagagtttga gaggccgacc tgcctccaac ggttccgcgg 1320

aaaagtttta taagagtttt gagagaggtc gacctacctt ccaacggttc cgcggaaaag 1380

ttttataaga gttttgagag aggtcgacct accttccaac ggttccgcgg aaaagtttta 1440

taagagtttt gagagaggtc gacctactct ccgaggttcc gcggaaagtt ttataaaaag 1500

tttttacaaa gtttttgagg gaggtcgaca cctcggatcc tccgcctcgc gtgccgcggg 1560

cggcctccaa gagttttacg gaacgttctg gagaggagag gccgacctcc caaagatttt 1620

gcggaacgtt ttggagagga ggactggcct cgaaaagatt ttacaaagag tttggaggga 1680

ggactggcct ccaacggttc cgcgaaaaag ttttgcaaga gtttggagag aggtcgacca 1740

ccctccgagg ttccgcgaaa gtgtttgcgg agagtccgag agaggcagac actgcctgcg 1800

aaaagttttc gcggacggtt gagcgggtca ccgacctccg acagtttaca aacgttttac 1860

ggagagttag agaggcagca gaccgacctc cgaacagttt agttttacaa agttttggag 1920

ggtgaactgt catcgggaaa gttttacgaa gagttttggg agaggccgac ggttagccga 1980

gcgagcgcgc gagcgagttt acgttctctc gctcgtgtgc gcgttaactc gcactggttc 2040

cctctctaac tcggagtggg cgagcgagtg gttgactcgt cctccgctct cactctgagt 2100

ggtgataact gttaaccatt aactcgtcct ccactctctc actctctcac tctgagtgag 2160

gattgacttg ttaactcgtc ctccactctc tcgctctctc gctctgagtg agtgaggatt 2220

aactgttcaa ctcgtcaact cgtcctctgc ctctcatcca agactgagtg ggtagttgac 2280

tgctcttgtt ctcttactcc gaggggtgat tgagttagca acagctactc gttctcttgt 2340

tctctcacac cgagtgagcg agtgagcaac gttacttgtt ctctcacacc gagtgagcga 2400

gtgagcaacg gttaactcgt tctctcgttc tctcgttcta ttattccgag gagtggcgag 2460

tgaggtaacg gttactgtta cttgttctct gttcccttcc tcggtgagcg gtgcgtcaac 2520

ttgttctcgt gagtgagtac ggtcacttgt tctctcgttc tctacctcga ggagtgagtc 2580

aacttgttct cgtgagtgag ttgaccgtaa ccgttccctt acctcaagtg agtggcggtc 2640

gcttgttctc gtgagtgggt taaccgcgtt cccttaccgg agtgagcggg cgggcgataa 2700

aaataatcaa ttgactgatt cgctcgtgag cgagcgaagg gcggcggaca agggcgcggg 2760

atgctggtct aatctactaa ggccgattac aaaaacggat gggagaccgg gagggagagg 2820

gtcacagctc cgagcggtgc atccgcgcca gctggcggcg ccactcggcc gcgggccgcg 2880

gccgcccagg ccgcgttgta gccgcccgcc accgcgacgc aggtcagctc gaactccggc 2940

ccgagcgcgc gcacgtcgta gatgtgcacg gtcgcgacgt tcagcagcag cgcgcccttg 3000

cgcagcgagg cgaaggtcgc gtgcatgccg gcggcgagcg ggtacaccgg cgagagcgtc 3060

ccgccgccgt gcgcgaccac cgccatgtgc cgcccgtcgc cgccgacggt caggcaggtc 3120

accgaggcgg agccgttcgc gcggacgggg cccgcctcca cgagggcggc cggcagcggc 3180

ggcgccgcgg ggcggaagag cccgccgagg aggaagccca gcgccaccag cgcgagcgcg 3240

ccgagcagcc tgcgcggcga ggggtgcatg cttgttggct gcggtgttgg cgtctggcgg 3300

gtggaaggcg ggtgtggggt gtccgcggct gcggcgggag ggtctcgact gctaggcggt 3360

cctttttcac tttgctccgt ggcgcctggt ccggggcaag ggctgcggcg ggcgcccggg 3420

cgggcgggcc gctaccccgc cgcgcggccc gcggccgcca gcgccgcggc cagccgccgc 3480

caccgcgggc ccgccgcgcg cagcagcccc gccgccgagc gccccgcgcc gccgcgggcg 3540

cgcgccgagg ccgcggcccc gcgccgcgcc agcagcagcg gcagccgcgc gtccagcggg 3600

ccgccgcggc gcagcgccgc gcgcagcagc gccgccagcg gcagccgccc cgccgcgtcc 3660

gggcccgccg cgcgcgcgcc cgccgccagc agcgcgcgca ccagcgccgg cgagggcgcc 3720

gcgcgcggac caggtgctcc acgagcaggg tggtgagcaa ggattctcga gaagtaggag 3780

tcatgtgtga cgacaaggag agacgttata ttaggcgcgt cctacttcac tttgaagatg 3840

gtgtaaagtg ttaaaacttg aacaccgttc actccaccac tgccgttacc gtgtcctgcc 3900

ccaaaagcaa ccacagtgct ttttccacca cctgttccaa atccgttcca aaagctccca 3960

tccattgttg ttagaacttt cagatgtttc tctaggttgt ttagttccac tgcaagtttc 4020

gaccattatc gttactggac atgctgttgg taatgagttt aataaccaat cataaaaata 4080

gttataattt gttataaagc taataaagta gcaaacactt taatgttata ttttgcctaa 4140

ccctccgtta acaccaccat taacaccacc acttaagctt ttactaccac cactaccacc 4200

tccaacacac attcttttct ctaaaggtcc ccaaattcca cctcctgaac ttggacgttt 4260

tacagcacct ccgggtgtac ttgcgtaccc tttagaagtt ccactgtgac tgtagatatg 4320

atactgtcct tctccaggca tgattaaagt gtgttgtaat tagtgttatc tacgcaactg 4380

tgcgagactc tcgaataaaa agaagctaca ttttacaatt ttgattagct gatgtaccac 4440

gctgtatcgc ggccaccaca agcacccgat ccagtagaac caaatccaga gtcgccgcgg 4500

tcagtgttgt ccaagcagtt aacctcttga actgctgggc acgatatgcg ttcgcatatt 4560

agctgagcta tcctgtctcc cttcttaacc tcaaagtcac tgtttccaaa gttaaacagc 4620

accactccga cgttgcctcg gtagtcttcg tcgatcacgc cagcgcccac gtcgataaag 4680

tgtttgactg caaggccaga acgtggtgct atgcgtccgt agcaaccaga agggggcttt 4740

atcagaaggt cagtaaatac tacgcgactg caatgcgaag ggatgacaca gtcgtatgca 4800

ctacataggt ctaatcctgc ggcaccagga gatcctctgg ctggtatagt ggcgttttgg 4860

ctgaggcgaa caacctgaag agtttccgtg tggcagaact ccatggctag ggtggcgagc 4920

ggccgatcga ctacggggtg tacaatttac actttctcca gaaaaatcag gggcgggtca 4980

gcatggcgcg gcgcaagtcc agcagcgagt cgtacgacag gaagcacagg atggaggtca 5040

cgatctccgg cggcagggcg cacgggcaca tgaggccggc gatctgctcg gccagcgaga 5100

cgcgcagccg catcatgcag atcttgccga agagcgccgt cccgtagatg gggaactcgg 5160

ccgcgcgctc caaaaaggcg ttctgcacga agagcgcctt cgcgtcgtcc gacgcgcgca 5220

gcacgtccaa cagcgtcgcg tccgtgtggc agcgcaccgc gcgcatgctt gcgatctcct 5280

gctcgcacgc gcggatcacg gtcgcgtagt ccgccagcgc gcgctccgcc agcatgcgcg 5340

cgccctcgcc gcgcagcgcc agctcctgca cgcacagcag cgcggcctcc gagcgtcgga 5400

acacgtggcc ccattgctcg gatgtgatca gcgcgcgcgc gagcagctcc gtcggcggcc 5460

ggcgcgcgag cacggccgcc gtcgcgcgca cgttgttgcg gcgcagcatc tccgaaaccg 5520

cgcataggcc cgaggccgac atgtgctcga gctccgcgcc catgcgcacc agccggcagc 5580

aggcgccgtg gctgaacacc gccgcgcggt gcagcgcggt ctgcaggttg ttgttgcgca 5640

ggttcaggtc cagcccgcgc tcgagcacga agtccacgac gccgcgctcg cagctcccgt 5700

aggtcgccat gtagtgcagc atggtgttcc cacacgcgtc tacggcggcc gggtccacgc 5760

ctaggcccgt gagcgtgcgc accatgccct cggagatctt ggccgtgcgc gcgaggtggt 5820

gcagcgttgt gcgcccgtac gcgtccacga cgcacgcgtc cgcgcccgcg cgcagcatca 5880

tgtccacgag cgcggcggag acgccgccgg agcacagcag cgccgccagc ggcgtcaagc 5940

cgttgcagtc gcaggcgttt gggttcgcgc cgcgctcgag cagcagccgc agcacgtcct 6000

cgcggatcca ctggttcttg gcgtacacgt gcagcggcgt tacgccgtag gtgttgccct 6060

cgttcacgcg cgcgcccgcg tccagcagca gccgcgcgac ctcgagctcg gcgccgtcgg 6120

ggccgcagaa agccaggaag gaggagagca cgctgtcgca gacaacgacg ctggcgtcgc 6180

agaccacgtc cgcgcccgcc tccagcatga gcgcgaccac ctccggccgc acgccgtcgt 6240

actgcacgta ggcgtgcagc ggcgtgcggc cgcaggagtc cttggctttc acgtccgcac 6300

cggcctccag cagcacgcgc acgatctccg cgcactgctc gtgccgcgcg aagtgcacgc 6360

agaggtgcag cggcgtgcgc ccgtgctcgc cgcggaagtt cacgtctgcg tcggtggcta 6420

cgagcgcgcg gaccgtttcg aggtccacct gcccggactc caggtagcgg aagagcaggt 6480

ccgcgtgcgg gaccacgacg gactcccgcg agagcatggc ggcgtttaca aatattgaaa 6540

tcttttttca ctcatcttta tggcgctgaa cgcgcaataa gggtgagagt aaaaaacttc 6600

tacaaaaagc gtacaaaagg tacaaaaggt aaaaaaggcg gggcggggac gggctggggt 6660

gctgcgagct gaattggcct ctacacaggg acgccctcgc cggagccggt gagccggtag 6720

ccggcgccgg cgatcatggt caagcgctgc acgagctcgt tgcgcttgac gccggcctct 6780

gaaacgcaca ccatgtggtg gatgtaccgc tcgatgcact cgcagcgcgg gagagtggag 6840

tcaagatcgg atgcgagttg cagaatgtca tcccagagct cggagaactt gctgtacagt 6900

tctcggaggt ctctctccat tcgagccgta agagagtcag gatgcggtgt tccttcggga 6960

gtctgagcga acaccgcgaa caggctggtt atgccgtgtt ctagaataga gtggttccgc 7020

gttaatgccg cagacaaggg tcgtcgtccg cgcaacgact ggcggcagag cgctgtttgt 7080

gccgcaccgc ccattcctct ggcgatcgca tccaccgacg cagtgatcat ctgcgcgccg 7140

acgtcattgt agcgcgcgtt aaactcagta atcatgatta cgagattgca gatttcatag 7200

tagcactttt ccaagtcgac gcgcagtttc acgatctggt tgacaatctt gcacgccttt 7260

cgccgcgtct ccgccacgtt ggcgactcgg acttgcgctt cctggtcgat ggacggcgga 7320

aacacttcaa acccaaggtc gcacagttca gcggtgggga ctagcgtcac gatgatgtac 7380

tccgcatcgc cacccacttg cggcaggaag aacaccgacc gcgcggcggg aacgaccaga 7440

acgtcgcctt cctgcatgtt ggtttttaga aacttagtgt tgttcacgga gatgccggcc 7500

atgccctcgt ttttgacaca tattatggtg acgtacgcgg cgaccgtggg ggccatgtgg 7560

tggcgcatgt accactcgtc gtgcttgagt ttcagaccgt gagattcgcc aacctcgaag 7620

tgcatgttgg cgtctctgac gtagcgcgag aactcgctgc gacagattcg cgcgggcgcc 7680

cggtggaacg tcgactcgaa gagactgatg gctgtccatt cgcccacatg agtgaccacc 7740

gaagaagtgt tttcgatccg agtctcgaac accgagtcca cgagcaccgg acagttggtt 7800

ccgggcaccg tcagcaccaa gggccgcgcc tccacggggg cgacggacga agccacggag 7860

tcggtgtccc cgtacccgta gtcgtcgtcg gagtcgccgc ctccgtcggc cccgtcgcgc 7920

ggcctccgca gcggcatgca gccggcggtg ggaacgcact ggtttcggcc acggccgaag 7980

cggccaaaca gtctcgccag ggctgacatc cttggacggc cacaccaaaa ccaaaaaaac 8040

atattttatc agttatttgt cgattttcac cggctcaccg agggcaggac ctcctggatc 8100

ccggacaccc ccgccaggca gcgggccgcg cgctcgcgca cccaaaagcg gtcgtagccg 8160

tgccggagca cgaaggccgc cgtggcgtgg cagtccacgc gctcgatgaa gccgtggacg 8220

gcgcggcgcg cgtagctcgc cgcgaaggcg cggaccaccg ccgagcagcg ccccgagggc 8280

gagtcgtccg tctccagcgc cagcggcatg ctcgcgatgc gcgacatcag gttggaggtc 8340

tgcgggatgt tgagctcgcg cgtggcggtc atctgcgcct cgagcccggc cttgagcacc 8400

tcgtcgcagc ggccccactc cagcgcgcag accacgcgga tctcgtaccc cttgagccgc 8460

agcgcggtct cgatgtccac ggaggtgagc accgcgctga agcgcaggcg ctcttcgtcc 8520

gcggggtcga agagcacggg gatcttaacc tccgcgctgc gcgtgacctc gcagagcgcg 8580

atcgcgagca gcccgcgcgt gagcttgctc accatgcgcg gcttgcccac ggggtacagc 8640

tggctcgcga cctcgcgcag cgggtacgcc agtcggaagc agcgcgcgtc cgcgggcacc 8700

gggctcgcgc ccgtctcctc gaggaagagc gcggcctcaa ccatgttcag cgcggagaag 8760

tgcaccgggc aggcggcgca gccgcgcgcg gcgttcgcga gcaccatctc gcgcagcccg 8820

cggaaggccg ccatgtcgca ggaggggaag atgcgcgcga gcgcggcctg gtgcgcgagc 8880

gccgcgtccg agagcgcctg cgcgccgcgg cggcgctcct cggcggcggc cgcgctctcg 8940

tccgcggaga ccacgtcttc gggcacgtcc acgcagacgc cgccccagaa ctcgcagtac 9000

tcggagaaga gcgtcgcggg cgcaaagcgc gcgaggtcca cgaaggcgac gcggttgccg 9060

agcctggaga gcagcgtgtt ctccgagatg cgcgtccagc ccttgccggc gagctccatg 9120

acctgccgcg tgtcgaagag ggagctgtag aagccgtaca cggtgatgtt ttccttgcac 9180

gtcgtcagcc acatgaggaa gtcgcgcacc accagcttcg cgcagtctcc ggagaacacg 9240

gggccggcgt tcgtcgcgat ggagttcagg cgcacggtgc cgtcactgcc gaagcggtac 9300

acgaaccagg cggccacgct gttgccagag ggcgtgtgaa cgtgtggctg cgcccaggag 9360

tcggcgctcg cggcggtgcg cacgtcgtgc gagagcacct cggtgtcggg gcgcgagtag 9420

gtgctggggt ctttgatcca gatggcgtag ctgcccacgc agcacacgtt catgaggtcg 9480

agcagcgtct gccggcgcag cggcgtgccg agccggcgca cggcgtcgtg cgagaccatg 9540

cgcaggtcgt agaggcccac gtccgagagc cactggttga gctcgtccat ggacagggcg 9600

tcgcgggggg gcgggctgtc ttcgaaggcg gcgcggagct cgggctccgt ctccgcgcgc 9660

tgccgcagga tgtccaggaa ggggctggag gagtcgggga tgtagcagtc ggggtcgtgc 9720

ctggacacta tagcgaaccg ctgcgtcgcg ggcggcgggg ctagcgcgtc ggcgcgtgcg 9780

tcgatgaagg tgcacgatat acgcacggac ttgagcgagg ggaggacgac tgcggcggcg 9840

cgcgcgccct ccgcgtcgaa gatcatcgtc tttccgtccc tcgcctttgc gagcgcgtat 9900

tctccaggca cgaggtccgt cggcggcggc tcgtcccagg cctgccggtc agggacgccg 9960

ccgcacacct ttccccagaa ccccagcatc ctccaaaata cctataagga cggccaatag 10020

cggggcttgc gggcgttcgg accttccgcg ctttaatttt aatttattgg cttgcagaac 10080

tccgagcgcc agtcccgctc gaagaccgcg gacaggtcct tgacgatgtc gcccttctcg 10140

gcgttcacgc tcacgaaggc gtggtagcgg tagtgcgtgc cgtcgaggtt ggcgaccgtg 10200

aggtgcgcga aggtgtcgtc cacgatgagc agcttagtgt tgttcgcggc gtcgtcccgg 10260

ccgggtacca cgaacttgcg cacggacatg tccacgctgc cgacgccaaa gtcgtcgagg 10320

ctgcgcgcgg ccgagaccga aagcgggtcc gcgttcttcc actcggtaat gatcacgcgc 10380

acgcgcacgc cgcggttgat ggccgcgcgc agcagcgcgt caatgatctg cggccagtac 10440

tccacggcgc tggcgtgctt gatcaccggc accatcgaga gcagcgagag gtcgatgctg 10500

ttcttggcgt tctcgatgcg gtgcagcacg aggtcctcgt cgagcgtgcg gtagaagcct 10560

aggaagcgct ccggcgagtc cgagaagaat acgccgcccc cggagtggtc gaggtggaag 10620

ttcgtggccg tgggcgtgac gatggcgcag cagagccgcg tgaacggcac cttcggctcc 10680

acgatcatgg agtagaaggt gttgtagcgg ttcatgaggt cccaggccag gtgcttgttg 10740

gtggagtaga gcccgaggtt cttgatggtg gacacggacc cgcccgtgag cgaggcgctt 10800

cccacgtacc agtgcccggc gtccgagagc cagaagctgc cgagaaggtt gccgacgccc 10860

tccttggtgg acaccttgac cttgtagtag ttgacgcccg cctcgcgcag ctcgtccgcg 10920

tccttgtcct tgctctgcac gtccacgagc agcgtgacgt ctacgccctc cttggcgagc 10980

gtgcagagct tgtccttgac gtcgacgccc tccttggtgg agctcaggtt gcagcagaag 11040

ctgcagatgt acaagaactt cttcgcggac tcggcgatag cggtgaagca gtcgagggtg 11100

ctcatgttgc cctgcgccaa agacgccacc tctgcgggca gcgtctccac gacgcggcag 11160

tcggcgccca gggggatgga ggagaacggc cacatttatt tatctcacaa aaataatagg 11220

gcttcaggga aagtctttta gcaggcgggc gagttcttcg agttcgctta ggagttcttc 11280

catttcttcg gaagtcagca actggagctc ggacttgatt tgaatatctt cgaggaaacc 11340

gtctagcatg ttcgccatgt cttccgggga gcactgcgcc acatcttcgg ggacaggatc 11400

gggtgtgggc attaggtctc cgcttacttg aacgtcgtcc atcatcctgt cgatgaggtc 11460

ttcgacttct agacggggtc cgtagatcag catatttggt gatggaggta gtttaaggtg 11520

cgagagttag tgttatacga ccgccaacgt gtgtttatcg cgcgtacatt ttcaataatt 11580

aacaaactcc ccttcctgcg cctgctcgag aagcagctcg tccagctcct cctgtcggcg 11640

cgcggccacg cgtctttccg cgaagagtac catcagctcc agccccacgc cgcacagacc 11700

caggacgccg aacaccaccg ccgccgagat cgacagaccc agcagcaccg acatcctcac 11760

gcgggcatcc ggctatttaa tcgttctgga aacgtattaa tatgggcgtc gtcatgtgcg 11820

ggtgtctgtt tgtgtgggcg ggctggatcg cgcgccgcgt gcgcggcctc tgcgcggcgc 11880

tgcgccagag ggtgtcgcgc gacaagggct acgtggccgt catccagacc tgcgacgacg 11940

actacttcac agaggaggag ttcgacgacg gcaagcaggt ggtcgcgctc ctgcgcgacg 12000

tctcgcgcgt ggttgccgcg cccgcgggcg tgacggaata agttaggata aggagtcgag 12060

gggagaaaaa cagcggtcac actataaact cgcgcgaggc cgattttgac gtgctcatgt 12120

ctggaagctc cgctttctgc agcgcggagc ggcacacgaa gcacacttcc gtgttggtgg 12180

gagttatgca gtggacgtgg tagccgtgcc cgcacaccat gacttggaac ggacacgcgc 12240

cgggacaggc cgcgtttatg catccttccg gcgagcgctt gttgcagatg tagcacacgt 12300

ctgagcacgc cagcgtgcag gacacggcca gcttccactg cttaacctta acgggcatgg 12360

ctagttgaac acgaccatgg gcgagtcgcg agcctcgagt cgggggttca gggcaaaccg 12420

tttcacgccg tcaacggttc ttctctttgc aattttctct ctgcacaggc tcgtcagcgt 12480

catctcggcc aggcgcgcgt cgttgcctag gtgccgcgcg gcgtcctcga ccgtcacgcc 12540

cgtcttgccg gcctcgtcca tgagcacaat gcataccagg tgcgcgctag agcatatgac 12600

ctcctgctcg cgcccgccgg cagcggggat ggttagctcc gcgcgcccga aggccgccag 12660

cggcgccacg tcgtaggcag tgtctgctcg ggcgagcgcc gactccacgg caccgcggag 12720

cgactccggc ggcgtcagcg cggccagcgg caccggcgtg ggcacggtgt acacgttcac 12780

gggcatgagc accatctccg ggtcgtggtg gccgctctct tcgccgtcgt gctccatggg 12840

ctgcggcggc ggcagcagcg ggagcagcag ccgtccggac atgagccggc gcacaaggtc 12900

gttgagcgcg gacgaggcca tcggcgggta cagctccatg gccagcttca gcgataggtg 12960

cttctcgagg ttgacgccgg tgtagacgct cttcacgatg cgcgcgaagg ccacgcgcgc 13020

gaaggccgcc agctcctcgc gcggcaggcg ctcgatgtag gagagcagca tgtcggtgtc 13080

gcacggcggc gccgcgacca ccgcgccgta gagcgccttg cccgagagct tttccagcgc 13140

ccttgcgtgc aggccgtggg tcttgagcac gtccacgtag ttcacgtaca ggcagagcgc 13200

gcgatcgagg ttgctctccg cgacgtgcgt ctcgatgcac tccacgatga gcgggcccat 13260

gcggtccttg atgaggtcta tgagcccgcc gtaggcgact cgcgcgctca tgaggcacgt 13320

gcggcagtac gccatcaggc cctcgaggtc cgcggctatc acgtcctcga ccacgttcgc 13380

cacgacgcgc tgccagagcc gcaccttgct cacgttctgg tgccgcacca tgtccacgag 13440

ctcgtcgtac gagccgccgg gctcgtgcgc gcgatcgacg atgcacctcg ccatggtgcg 13500

gctctggcgc atgagctcgt tcgtgaagcg cacgcacgcg tcctcggaga agagcgcgct 13560

caggcaggag tagcagcggt ccgcgacgag gtgcgggaag cggcactcca cgacgccgcg 13620

gccgatccgc agcacgcact cgccgtacat ctcgtccatg gcctcgcgca gacagtcgtc 13680

cagcacgtcc gcgttgtgcg cccactggat cacgcagagg tagggctcga tgttctcgcg 13740

cgcgttttcc acctcctgca ccatgtactc gagcacggtc atgtcctcgt ggatgtcggt 13800

gcccagcatg cgcccgggcg gcagccagct cttgcgcgcg atcgcctctc gcaggcacgc 13860

caccgccgtg aaggtgttga cgcggagctt ggtcagcagc cgccgcagtc gggagatgtg 13920

tgccacggag aggtccatct ccatggcctg ggcgatgagg cgcgtgagtt cctcctccat 13980

ggcggcggct ccgcgggcag atatacgcga acaacggtaa gccgtgctat ttcatttttg 14040

gacaaaaagc tagtcgtcga cgcgcatgtt gtcgaggttc cggcacagcg agagcacgtc 14100

gtcgcgcgcg cgcctccggc gcagttgatt gttcgcgcgc cgcgcgtccg cgagcgcctg 14160

tctgtacatc gcggagtccg cgtacccgtg cagcggcgag cgccgagcgc cgggcctcgg 14220

gctcgcgcgg cgcgggagcg gcgttggcgc gcgcctcgag cgccgcgcga agtgcgcctg 14280

catggccagc aggcaaccga acggcaccat gtatcggtcc atgaggcact ggctggccgc 14340

ggacggctcg cgcgggtgca tcacgccgcc gcccacgtcc tccatgacgt cgcgcagcac 14400

gcagcgcagc atggtctcca tgctgcgctg cgtgaaccgc accggggagg cgtcgacgta 14460

gaagccgtcg gccacgaagt agagcgcgtc cagcccgccg agtttctcgc cgagaccgac 14520

gaagagctcg tccacgtgcc agtccaccac cgaggccttg aagagcacca cgtgccggat 14580

gtcgtgcgag cgcgcgagct cccaggtgtc ctcgccgatg ttgctggcgt cgatgcggcc 14640

tgtcatgcgc acgctcacgc acggcgtcat cccgttcttg tagcagaact ggcgcgcgag 14700

ctcctcctgg cgtacgatgt cgaccatgct ctccatgaag gaggtggaga gcagcatcgc 14760

gccgcgcgcg gcgcgggtcg cgttttcgtc cacctccact tccatcccgc cgccgatcct 14820

aatcatctat cgtatttaaa ttttcggcgg agcagacacg cggctgctcg ctgcgcgatc 14880

gcttcagccg cggcggcgtc acgcacgcgt tgcggcggcc ggcacgcacg gacgaccgcc 14940

ggggctgttc gctgagcgag cgccgcggcc gcgtgacgcg acagtcgcag gtgggttgcc 15000

gggagtcgct cgcgcgcctt cttcgcattt cgccggaacg ccgcgtttat gtaggggatt 15060

atattttcaa cgtaactaaa tggacggggg cgtgcacaaa cggcctttca tcgtgaacgt 15120

ggatggcatg ggcaaggtgc tcgtgctccg gtacttgcgg atgtgcgagg tgcccgaggc 15180

caagtgcgag gggtcgcgcg cgtcctgcgt gctcaagatg gaccctcccc gctcacccag 15240

ctgcgagcgc aggccgtctc tcccgccgtc ccccccatgc cccatgcgca cgcctcccgg 15300

gtcgccgctc caggctccct tgatgcgcac gcagatgctc caggggctgt tcgacgctgc 15360

caaaaacaac ggcgagcaga tgtgccgccg ccagtaacct aggctgcgca gtacgaaagt 15420

tagtgcgtga tcacgttttt tgcaatgtcg atcacgccgt gcgtgcccgt cttgcgctcg 15480

cgctccacca cgctagtcac gggccgcgcg tccgagacta gcgaccccag catcgagcgc 15540

acggcgccct ccgcggcggg gtggcgcgtc agcagcagga acatcacgat gtgcgcggag 15600

acgccgcgcc ggctcagatc gtgcacggcg tcgccgtcca tgagcacggt gttcgagaag 15660

tacgtgaaca gagtgttgtc tcgcaccagg aaggccgagt tcgagacgct ctcgaagtcc 15720

acgatctcgt cgtcctgcac gcccatgtcc aacagcgtct gcacgagcgc gggctcgtcc 15780

aggaacacca ctgcgcgcgc gaacccgcag tccagcgcgc gcgcgtccgc ctccaacacg 15840

cgcgaggcgc cgccctccgg cggcaggaag gcgcagggca gcggcgtgcg tccgtccgcc 15900

ggcgcctccc cgagctcctc gagcgcgaag gccagcagcg tctccatgcg cgcgcgcgcc 15960

ttgtcgaagt tgtccgcgag gtcgcggatg cggtctgtct gcgagaacat cttcagcatc 16020

gccatgagct gcacgaaggg gtgcagcacg tatatgttgt ccacgagcag cgtgggcagc 16080

gcgcgcagcg tggcctgccg cacgttgaag ctgtccagga tgtgcccgcc ctcctcgtcc 16140

tgcagcacca cgtagttctt caggtagggc acgcgcagca gcacggtctg ccgccccgtg 16200

acgaagtata tcaggaaggc gaggttgatc aggaacgggc gcgcgttcgt ctgcaccatg 16260

tcgatgtcgc cgtactctat ctcggggttc agcaggtgca gggcgtacga gccgtagcac 16320

acgcaccgct tgttgtgtcg gcgcaggtgc tccttcacga gccgcttgac cacctccacc 16380

aggtccgagt gcttgtgccg cgccatcggc gcggcctcct cgggcggcgg cagcactgcg 16440

tacgagttga gcgcgcggct ggcgagcgcg cgcgcgcggg gcgcgtccac gcgccgcacc 16500

gccgcgttga ttgcaggcgt cggcgtcgtg agagagccca gcgtgcgcgt gaactcgctc 16560

acgatcacgc tctgcagctc cagcaccgtc aggatctggc ccagcttctc caggcgccgc 16620

tgcctcgaga agtactcctc gatgcgcgcg gcgatctcct tctcggagcc gcctagcttc 16680

ttgaagaagc gacgacgact ctttacaaca agagagagaa aaagcttcct atcgaagttg 16740

aggacgcggg tcatgttgcg gcgctgcgcg cgcaggagca cgcagcgctc catggagggg 16800

cgcgagccga ggtactcttc gatcacgggt ggagccatga cagctatttt ctgaacccgc 16860

gattattgta cagcgcaagc cgcgcgcaga cctgctggca cagcagcgtc gtgtttcgca 16920

tgcacacgcg cgaggactcg atcgtgcgcg cgtccggtgc ccaggcgcgc agctccatca 16980

gttcctgctc gacgaagtcc acgggctcca cgaagcgctc tgcgcagagt ccgtccgtga 17040

acgcgttgac gatctgccgc acgagcacta ccacgtccac ctgctccacg aggcgcacgc 17100

ccatggcgac gtgcacgaag aggcagcgga agagcgcgtc catggccatc tggtggtccg 17160

agcagggccc gaccgcggtc ttgcagccca gcgcgaagcg cccgatgccg cggtactgca 17220

ccatctccga gggcgagaag gagagccgct ccatcttgag cacgggcggc gggcccgccg 17280

gcagtccgcg cgcgaggtcc agcaccggcg tccacccggg cgtgaacatg tccgggatca 17340

ggaagagccc gtagctggcc atgcgcgcga tgtcgaaggc gtggtccacg accttgttca 17400

cggcgctgtc cgcgcggttt acgcgcagcg cctgcaggat cacgtttccg gaagcgtgcc 17460

gcgtgatcgc gaggtccgcg gtcgcgtacc cgcgcaggcc cggcaccgcg tacgcggtca 17520

gacacacggc caggcgcgcg ctgtgctccg aggagaagat ctcctgctcg tagccctcct 17580

cgggctcctc gcactcgcga gggcgccgca cgtcctcgac cgagcgcagc cgcatctctc 17640

cctccgacac cagacagcca agcgactccc tcaccgccgg cgcgagcacc tccgtggcgc 17700

agagcgcgtc gtgcacgcgc ttgagtgtgt tcggcttcag cgcgtagccg aagagcagcc 17760

gcgtcatccg cgagcccgag aacgcgaagc ggcgcacgta ctcctccgcg agctcggggc 17820

ggtcgttgat ccacgaggta gagaagacgt ggtcggaggc gaaggggtct gcaccaaccg 17880

cgagcagtgt ggacagaggt acggtgtcga ggaagtccac gacgtcgggg aagttctggc 17940

gcacgcaggc ctcggcgacg cgtctggtgt gcacgcacat gtcggtgacg ggcacccggt 18000

ggccggactc cacgacggac acgcagacgt cctcggtgac ggcgtccacg ggcatggtcc 18060

gcagcagctt gccgagcacg tcgccgaacc cgccctcgag cgccttgcgc cacacgaacc 18120

ccgcgtcgaa cttcccgggg aagtccgcga tcaccgaaag ctccgcgtgc gagaggttgt 18180

ccacgttgag gtaggtggcg gcgtccacga agatgggccc gaaggcgccg gtgtcggaga 18240

cgcggtctct gaggtagtcc ctggcgtagt ggaggtactc ccgcacctgg ccggcgcgga 18300

tgcgctcgag cgcgaaggcc ttcatggtct cggagcagag cacggagtgc cgcagcccgt 18360

ccagtgtgcg ccgcacgtcg tagacgccgc gcatctgcgc gagcatctcg acggcgtccg 18420

ccggcgtcgc cgccgcgagc cccgggttca ctggaggtat cctgtgttct gcgagcatgc 18480

gcttgaggaa acagaggtcc agcggccgcg tggtgtacag cgcggaggcc atctcggggc 18540

gcgtctcgac gatgtcctcg atcatctcgt ccgtgaatgc cgcgttgatg ttgtgcacgc 18600

tgcgcgcgtt cacgtgcagg aggatgtcgc ccacgttgtc ggggaatcgc tcctcgatga 18660

gccggacgtc gtcctccgtg atgttcatgt agggaataca gcggcagagc agcgcgtagt 18720

ccgcgaactg cgcgatgtag ggcgtgtgga actcgatgtg tctggcgaag agcgcgccgc 18780

agcgccgccg cgagagctct tcgagcaggt cctcgggcgt gacgtgctgc gggcggaaga 18840

ggtgcaggtg cgtggggtgc tcggcggcca cgcgcgcgta cagccgccgc gggaggtgtc 18900

tggggtgcac gccggcgagc acgagatcca tggcctctga ggtagacagt gcggcgaacg 18960

cgcgctcggt gccgcccgcg gcgacagcgg cgccgacaaa tctcttgagc agctgcagca 19020

tcgcgtgttt gggctttcgc ggaaggcgct tattttaatg ttattggcgg tggccggtgc 19080

gagataaaaa ttagaactga tgccgcagtt gttgatgata tgatgattgc gctggccggc 19140

gcgagataaa aattagaagc tgatgccgca gttgttgatg aggatggtga gtgcgctgga 19200

gcaagcggtg tggcgcgcta gcttcttgct ggccccgtcg gccacggcaa cgacctttcc 19260

ggatatcgtg atggtgcagg tgaagcgcgg acagtgatcc tctccgccag cacgcgtctc 19320

gcagaactcc agaggtctgt gtgtcatcat gcagaactcg ttgaccgcgc tgaccgggtt 19380

aagacttttg aggcgtatca cggcagactg agtcatgatg tcgatgtcgc cgccgaaaag 19440

cgtatcgcac ccagcctcgg tctccatggg ctcggtgtcg gagttttcgt cctcctcggt 19500

gggcgcggag ggcgcgcact ctacgaacca gcggggcggg tttccgtcct cacagcaaac 19560

ctcgtccgag tccagcaggc ggtacagctg gcggtttgcc tcgtgtttgg atatgccgag 19620

ctccttcgcg atttgcttgg ccggcagctt gtcgtcggat tttctgagaa gctcgaggat 19680

cagagacgcg cactcgcagg ccattgtggc gatttacggg gcgtgcgttt ttttaggatt 19740

ttggcttgcc tttcttttcg cagaacttgg gaggattgaa actcttttgg caatttttgc 19800

aggcgtactt gatcaagggc ggctcgtccg ccgagcgcgt ctggatcatc atcggcatgg 19860

tgttcttgct ctggcacgag gggcagggca ggttgaactt ctcgtcgagc acgttgaagt 19920

acccgctgta gtcgtggtcc ggcacctcct cgatgtcgta gggcacgcgc gcggccacgc 19980

acttgaccgc gaagagcagg taccgcagcg cgtcgtgctc cgcgccgctg gtcgcgcgga 20040

tctgcgcgca caggtccgcg tagtcctcgt tcgcgtccac ctccaggctg cgcttgttct 20100

tgtacgagag ccggttcttg gcgtccttcg agtactcgat gccgatgttg tgcgcggggt 20160

caaagttggt ctcgtcggtg ttcgaggtct tggtgttcac gatgttcttc agcgcgaagc 20220

gctgcgcgca gtccagcgcc catcgcgcga tgcgcgcggc ctccgccgcg tcggtgtgct 20280

tcgccgcgag gtcgcgcagc cggtcttcgt ccatcgcccg attttaggtt gggtatatta 20340

tctcaattcc gctcttccgc gggccgcggg cgcgcgcccg cggcaaatta ggcgttacaa 20400

atggacttcg tgcggcggaa gtacatgata cacgccatcg accgcaacct cgacttcatg 20460

aaggccgagg tccagcagaa ggtctccatc ttctccctcg ggcacgtgct cgcgctccac 20520

tacctggtca ccgcctttcc gcaggcggtc atcaccaagg acgtgctcgc gagcacaaac 20580

ttcttcgtgt tcgtgcacat gtcgcagcgg cacgaggtct tcgacgccgt gctcaaggcg 20640

gccttcgacg cgcctcagct ctttgtgcgg gcgctctcgc ggcacttcga ggccttcgtt 20700

gccgccatcc gggcctaccg cgcgacctgc gcggagctgc tggccgacgc gcgcttcatg 20760

gaggtggctg cgcgcgcggc cgagctcgcg gaggtcattg gcgtgaacca cgacatcgcc 20820

gcgaacccgc tcttcgcgga cggcgagccc gtgcgcgacg cggagctcat tttcgcaaag 20880

accttccgca agaccgagtt ccgcgccgtc aagcgcctcg ccgtgctgcg gctgctggtc 20940

tgggccttcc tcgtgaagaa ggaccttggc ggcgagtacg cggacaacga ccgccaggac 21000

ctgtttacgc tgctgcagaa ggccgcgggg cccgtgcgcc acagcgcgct cacagagagc 21060

atccgcgagt acctcttccc cggagacagg cccagccact gggtctggct gaacgcgcgc 21120

gtggccgacg acgcagaggt gtaccgcgac cggcccgcgc gcacgctcta cgagcgcgtg 21180

ctcagctacg cgtactcaga ggtcaagcag gggcgcgtga acgccaacac gctcaagctc 21240

gtgtaccggc tcgaggacga ccccgacatc aagggtctgc tgctgcagct catctacgac 21300

gtgcccgcgg acatcgtcgg cgtcgtggac tccgcgaacg aggagtggcg gagctacttc 21360

gtgagtctgt accgcgagaa cttcgtcgac ggacgcacct tcacctcgga cgcgcgcttc 21420

cgcgacgacc tcttccgcgt ggtcgccgcc gtcgatcccg acttcttcga gcccgagcgc 21480

atccgcgagg ccttcagcgc agacgcgcgg ctgcgagagc gcttcacgga catggacctc 21540

aacaacgcct tcatgtcgca cctcatctac gactccgtgg accccgacgt cgccgccgcc 21600

gagcgcgggc tcgcactgcg cgtgcacaac gaggactccg actacttcat ccgggagtac 21660

aacacctacc tcttcctcag cgagaaggac ccgctggtgc tggaccgcgg ggcgctcacg 21720

cggctctcgg acgtccccgc cgagcgcttc cgcgacctct tcagcgacag tgtgctgcgg 21780

tacttcctgg acgcgaagct gggcacgctc gggctggtgc tcgaggacta ccgcgaggac 21840

gtggtcgccg ccatgcttcg gcacctgcgc cgcgtcgagg acgtgtcttc cttcgtgacg 21900

tacgccgcgc gcaagaaccc cgcctgcgtt cccggcgtcg tgcgcgcggt cgtgagcaac 21960

ttcaaccccg cggtggtcgc ggccatgcgc cccttcctgc gcgagcacat gacgcgcgtg 22020

gacgcgctgc tggacggaat gccgcacctc tcggaggccg accgtcggta catccgccgc 22080

gtggtgctgc agggccgcgc ctgattcgcc gtcaataaat cgcgatggtg gacagcggca 22140

cgcacgacgt ggactccgcc gcgcaggagc gcacgcccaa ccagcagacc ttcttcacca 22200

aggggctcag tccgctgatg cgccacacct acatctacaa caactacgcc tacggctgga 22260

ttcccgagac cgcgctctgg agcagccgtc tgggcgacta ccgcgtcacg gacttctacc 22320

cgatctcgct gggcatgctc aagaagttcg agttcatgtt ctcgctgctg gcggaccccg 22380

gcggcgcctg ccccgcgtac gagcccaagc tcaacaccga gttcctgaac cgcggctcct 22440

tctcgggacg gtacgtgaac cccttccacc gcttcgcggc gctgcccgag cgcgagtaca 22500

tatccttcct gctgctgagc tcggtgccca tcttcaacat cctcttctgg tttaagggcg 22560

agaccttcga cactgccaag cacagcctgc tcggcgccgt gtacaccacg cccgagcggc 22620

acatcgagct cgcgcggtac ctgcggcgca cgggcgacta caagccgctg ttcagccgcc 22680

tgggcaacga cgacacctac tcgaagccct tctctgggtt cacgcgcatc agcaacccca 22740

cgcccatcgg gcggctgccg ccctcggact tcgagacgct ggccaacctg agcaccattc 22800

tctactacac gcgctacgac ccggtgctct gtttcctggt cttctacgtg ccggggctct 22860

ccgcgaccac gaagatcacg cccggcgtgg agttcctcat ggagaagctc tcgctcgcgc 22920

ccgagaacgt ggtgctgctg tagcctcaaa cataaaatat aggcgccttt gatcgcactg 22980

cttcagttca gacagagcta agatggcttc ctacatcagc ggcgctagcg ccagcgcgaa 23040

caccgcccag ggcggcgatt ctcagtaccc acagtactat tatcacacac gcacctccca 23100

aggcgacatc cgcgacgaaa gcgaaggttg cttccacacc acggacgacg agcacttgga 23160

tctgtccgac gactacctcg gcgatggcgc accacactgc ggacacagcc acaaccacag 23220

tcgcagagat ggagatcggc accgccagcg cgcaccgcgg ctctatgagg acccggtgcc 23280

cgcgaacatc atggtgccca cgctcagtct agagcagctg ctggaggaaa cctcggtcgc 23340

ggggggcctt ctcggcggca ggacagagag ggacgtggaa cagctcctgg aggagttctc 23400

cgcactctgt cccggggacc agatcaccgc gctgcgctgc atggcggcct ccttttaccg 23460

cgacgcgctg ttcgcgccgt acgcctgcat gcacctcatc gccagtcgga tgcgcgtgca 23520

ctacgcgcgc gaggtcgtgc acgtggccga ggacctcgcg gacgcgatgt ctgcgaacag 23580

cggcgtctgc ttccggcggt accgaaagcg cgtgctagag gacatgctcg cggaggagat 23640

gggcgtgtac aattacctcg cgcgcgccaa cgcggacatc tgcgaggaca acctgctatc 23700

ggccgtggag acgctgctgc ggcgcttccg tcggatgggc tgctaccgct ctctgtgcat 23760

gctcaagatc ctcgcgctgc agcacgagga cctggccggc ttcatccgcc gcagcataag 23820

aaaaacctgc aacttggcac acgcgcgcac gcacacggtt tacgtgtagt taccctgtaa 23880

agacgggctt gctcccgaac aagcgctcga agaagagcgt gcacatagcc ttattgtcca 23940

gcaagttgac tatctctgta cacagcctct tgaagtacac ctcgtacatg atccgctcgt 24000

ttttatccag tctgaaggtc ttgtcgacca cgcgctcgta ggacttcacg ttcgcgatcc 24060

ggcgccgcca ggggccctcc tcgcacacgt acgcgaagaa gtagcgctcg ccgatctcga 24120

tggcctccgc gttcgccgcg ttgtaccgcg tcactagcgc cacgttgggg ttgtcggggg 24180

acttgaagtt cttgtggtgc gttcggctca gcaggaacca gtccagcggc atgctgcgcg 24240

cctcgaactc gaaggtgagc tcgtcctcca gcgagcgcag gatctccacg cccacgttcc 24300

cggagccctc ctccgccagc gcgcggcaga gcatgtcctt gtacttgcgg atcatgagct 24360

tgtggaaggg cgccacgtcg cggcgcgtct cgctggtgcc cttgctcacg cgctcgctgc 24420

cgccgccgtc gctcaccgca aacttgatcg tggtgtactt cttcttggac tgcatgatca 24480

ggttgcagta caccgcttcg aactccacct tgaagttcgc gaagagcacg tgctcgttga 24540

tcacgcgctc cagacagcgc cccacgcgcc gcgagaacgc gatgtcggag gcgcccacct 24600

ccaggaacac ggagtcggtg tcgccgtaca cgctgcggaa gcccacgcgc tccgtgcgct 24660

cgccggccac cgccgcgtcg atctctagct ccgcggcgcg gcccgcgaag gcctcgtcgc 24720

gcagcagcgg gttgtccggc gccgccgcca gcgacagccg cgtgccgcac accgacgcgc 24780

cgtctagcgt gcgctccagg tacgcgatca tggtgcgccc gatggccgtg cagctcttgg 24840

ccgaggcgta cgagaagagc gcgctgttgc ggaagcccat gagcccgtac acggagttgg 24900

ccgtgatctt gtacgtgtac tgcatcgagt tgtagatctc gcggtccacc gcggtctccg 24960

cggccttcat cagcttcttg tacttggcgc gcgcgtccag gaaggagcgc agcagcatcg 25020

ggatgatgcc cttggcctcg cggtcgaaga tggccacctc ggcgacgagc tccggcgagc 25080

gcggctcgca gggcaccgcg atgtaccgcg gcgccgggaa catccgccgg acgtccacgg 25140

ccgcgacctc cgcgtcgagc cggttgtccg agacgaccac gccgacaagc gtctccggcg 25200

acaggttcgc gtagatgcac acgttcgggt acaggctgtt gtagtcgaag atgagcacgt 25260

gcttgttgtg catcttctgc ttgggcgcca tcacgcggcc gccctcgtag aagaacttgg 25320

acttcgtgtc cgcgcgcacc atcaccgtgc ggttctccag cagcagcttc atcagcgggc 25380

ccttgatgca ggtgctcgcg cggtactcga agaccacgct ctgcggcagc aggtacgtgg 25440

acgcggcggc cgcgatcttg gtctccacgc cgtagtgcga ccagaggtag aggcagaggc 25500

aggcgtcgtg caggcagtac cgcgccatgt ccagacacac gtccagcgag tagttcgcgt 25560

acatgtccgc gaggctgacg tcgtccttgc cgaaggccag cgtgacgcgg tcgccggcgg 25620

cgcgcgccgc ggggtccgcg aggtccacgg tgaagccgtc ctcgtcgacg cgtttgtgca 25680

gcacgcggca cacgcgctcg tccacggtca cgtagttgcc ggtgctgagc acgcgagcga 25740

acacggccgc gttcccgtcg gcgtcggtgc tgcggtcgcc gcgaaagcgg accgcgtccg 25800

gccgcgcgtc ctccacgacc gcggtgcagt ggaaggcgtt cttggatatg gcgtccagct 25860

tgtaggagtc cagcttctcg gtgcgctgga tgaaggcgta caggtcgaag tagatggtcc 25920

cgttgttgtt gttgatgtgg aaggtggtgc tcgagacgcc gccgacgccc ttgtggctgg 25980

acttcgtgcg ctcgtacacg cagaagttga ctgtctcggt cccgtccggc agccggaagc 26040

ggatgtgctc gcccgtgagc agcgacagcc gcgagtccag gtaccgcagg tcgaagttgt 26100

ggccgttgaa ggtgaccacg aagtccagcg gcatctcgag caggcgcttg gccacgcgca 26160

gcagcgtcac ctcgggacac agcgtgacct ccgcgtcgaa cttcacgtcc gccgggtcca 26220

ggcagaccgg gatctcgcgc cgtgccgcct cctcgaggtc cgcgtcggag agcatatcgg 26280

agttcgtaag cgtgaatcgc cgctccgcgc cgtccttgtc caccacgcag aagctgatgt 26340

gcgagacggc gttcttgaag acggaaggaa actttttctc gaagtggcac tctatgtcga 26400

ggaagagccc cgagcgcgtc acgttgaagc gcgggatctt ctccgcgaag cacgcgccgg 26460

ggtcgtcgca gtggaagcag ttgctcccca ggtcgcgcag cagcgcgggg tccacgcggt 26520

agcacccgtc agggtcgatg tcgtgcgcca cgaagaacca ggacacgttc agaaagtcgg 26580

acatgaagac ctctggcggc gcgagcttgc gctcgctggc caccagacac agctctatct 26640

ccgagcgctg gcgctccgga atcttcgccg agcgcgctac gatctcgtcg atgctgacca 26700

cggacatggg cccgagcgcg cgcgtccacg ccagcggctg ggcgatgtcg gccaccgcgt 26760

ccgcgcgcac cacgtagtaa aagtgctgca cgaagcgcag gtacacgacg gcgttgtcgg 26820

cgcggcgcgc cttgaggaag aggaaccggc tgtcattgcc gcggttctcg aaccagttca 26880

aacatttcag ctccatttca aagagcataa taacatttca tttaaatgga gcctcgcttc 26940

tggggccgcg ccatgtgggc ggtgatcttc atcgtgctgc ggcgcttcga ggagcaccgc 27000

gacctcgagc gctgcaagcg gcagctgtac gtgatctgct ccacgctgcc ctgcatcgcg 27060

tgccgacgac acgccaccgc cgccatcgag aaaaacaacg tcctctccag cgaggacccc 27120

aactacgtgc tcttcttctt catcaagctc ttcaacaacc tcgccttcga cgacagatac 27180

aagatcgacc ccgcgaaggt gcgcccgctc gtctagagca tgccctcgta cgcgcgcgag 27240

ttgtccgagt acacggtcac cgcgatgccc tcgcgcggcg tcacgtgcac gtgcatggag 27300

tccgtgatca cgaagcccac gcccgtgacc ggctcctcgc ccgcgtcgtc cgtgaagagc 27360

agcccgtggt tgaagaggta gaactcgttc tctgcgagcg aaagccgccc gcggtcgcag 27420

aggtagtaga agatgtcgta gtcgcgcacg gccagcgtga acgtggactc gctcaccacg 27480

atgtacttgt ccagctcctc cagcacggac gcggccgcgc ggcgcgcggt ggcgcggcac 27540

tgcgtcgggc actcgcacgt gtctgcagag tacaggatgc gcgttcgccc cgaggcggtc 27600

tcgagaaaaa cgttaatcgc ctccatcgcc cagaagcgac tcgaggatcg cgagcaccgt 27660

gcgcagcacg agcccgatgg tgcagaaggg aacctctccc gactccgagc actcgcggat 27720

ctccgtctcc acgcggtcgt gcacttttat ggagggagcc gtcgttccag tggcctccat 27780

cgcgacggac accaccttgg ccacgaactc gcggatcttg ctcatgcgcc ggagcacggt 27840

cacgcggaag aagacggccg cgagcaggta ctcggccacg cagctcacgg cgatgagcgc 27900

ctgcgcgtgc ctggtgttga gcacgtgcgc gtcgggcacg aagtccgaga tcttcaggtg 27960

cgagagccgc acgagcgcgt tggtgtcctt gacgccgtcg caggaaatgt tcgcgaagat 28020

gagcttctcg tagtcgagcg cctcgaccac gcgctgcgcg tccatgtgcc gctcgcccga 28080

gagcgcgcgg ctcaaagggc cccagcacac cgagcccgcg aagcaggggt ccaccacgcc 28140

gtgcatcgcc agcagcgtca cgtcggccac cgccgcgagg atggccgcgt cgtcgagctc 28200

gttcgcgggc gtcgcgcccg tcagggacgc gctgcgcagc gcggacccgc ccggcgccgc 28260

gtgccgcgcg cagaactcgc acccgcagcc cggcggcagc ggcggcttcg agagcagact 28320

tatgagcccg cccacgtgcc cgtgctcggt gatcagaagc gccaggatgc tgtcggtgct 28380

gccctctatg gcggctgtgg ccgcgctaga aggctctccc gtgacctgcc atccgcagac 28440

aaccttgagc atcttgcgct tgagctcgtt gggcgcctcc gaggccagca tcgtgcgcgg 28500

gaacgtcgcg ttgaggcgga agtcctgcag cagcttttcg agcgtcgcgg tcttggcgtg 28560

ccgccctttg cgccactcct cccccaggtg ccacatgagc tcctcggccg tgtccagcgt 28620

cggcgagacg cgggccttgg gcacgcgcgc cgcgctgcgc atcagcggct ccgaggcgcg 28680

gaagctgccg cgcgcgtgca ggcgcatgga cgcagacgag gaccgcatcg agggtctctg 28740

gtggaagctc gtcatcgtga agcgccgcgt gagcggacct acctcgtcgc gcgactcgtc 28800

gaagtccggg tcagggtccg tcgtgtcggt ctcggcgctg tgcgtgctgg gcgcgctgct 28860

gcgggcgctg cgcgtgctgc gcgtgctgcg ggtgctctgc gtgctgaagc tgcgcgagca 28920

ggagtccgcc gtgtccgctt cctcgtagtg gaagtccacg tgctcctccg gcagccggcg 28980

cgagcgcgac ttggagatgc cgaccatctt gtccgcgccg accgggcgca cgcagagcgc 29040

catcgcgccc tcgcgcaccg cctgcgcgaa ctcgcggctg gcaaccatgc tggggttgag 29100

gaacttgatg atgttgaagt atggccactc gcagacgagc cgcgcgcagg tcgcgacgtc 29160

gtcgacgctt ctgagggccc tgttcagcgg ggggatgttg ctgccgtcgg ccagtgcgac 29220

gaggtcctcg ggggagatct cgagcttggg aatcatctcg ttcacgagcg cggggtcgat 29280

ggcgtggcag acggtctcta cctcggtgcg cgagagcttg agctgcgcgc aggccgccca 29340

cggcgcgcac gtgagcgcga acaccgcgga gactcggccc ttgccgacca tcgccacaac 29400

ctcgggctcg tagaccaggc cggccttgag cagagcctcg agcacctcta tgtcgtcggc 29460

ggcgagcagc gcgcgcctgt ggaagcacac cgcgggcatc atcttcttgc agatgccgcg 29520

ggtgctcttg agggccgtga taaggtcggg gagcctgacg tgctgcggct ggaagaacat 29580

gacgttggcg gggctcgcgg ccaccgcctc gtcgtacacc gacatcggca ggtcgccgtg 29640

gagcccgcac ggcagcaggc tcagcagctg cgcgcgcgag agcttctcgg cgcagaagtt 29700

cttcttggcc agggcggccg ccgcgtcgcg ggccttcttg gggtataaca acatggcggg 29760

ctttaaacac gaaacaaaaa tccgggttgt aacatttcaa ttttgcatgt tctgggcctc 29820

ctcgcagagt ttctccaggc cgccggccac gatggcgtcg acgaagaggt cggcctcggt 29880

gaagcggtgg ttgccgcgca ccgccaccag cgcgttctcg gtgaccacca ccgacagctg 29940

ctgcgcagcc gtgcgcaggt cgaagtgtcg gcacgcgagc ccgtggccca gagtctggtc 30000

cagcgccgcg agcacctcct ccaggctctc ctcgcggtgg ttgtagaacc acatcagcac 30060

gaagtaggcc acgtaggtgt agaggtagtg cgcgaccgcg cgggcgcgca ccagcggctg 30120

gttgcagcgc gcgaaggcca ttccgctggc gatgaggtcg tcgtccagcg tggcgtagtc 30180

gccccagttg agcgcgctga actgcacgct gtagaccgcg cgcacgaacc cggactcgag 30240

gatgtcgatc tgcgacggcg cgccccaggt caccagccgg tacacgatgc cgcgctgcat 30300

cagccgcatg aggtcgccgc cgacctcgcg caggcggtgc gtcagcgagg cgaaggccag 30360

cttccgctgc gtgtactgca ggcccacggc cacgcacccg tccgactcca ccagcacgag 30420

cttgtggtcc gtgcagccgt cgctgcgcag gccgccctcg cgcgccatca tgtcggtgac 30480

gaacatgtac ttgcgcgcgc gcggcccgac caggatgcgc ttcttgcctt ccatgcgcag 30540

caccacgtcc tctagcagcc gcgtgctgtc cacgcgctcc acctgcgggt ggatggtggg 30600

ctggtagttg tacagcagcc gcggggacca gttgtaggcg aacgcgaaga ggtgcgtgtc 30660

caggagagag atggggaaga cgccgccttc ggtggcgcgc cggaggatgg cgagcttggt 30720

ctccgcgggc agcaggctcc cggtcaccgc gccgaagaac atggggtggt tgcggaactt 30780

gagcgcgtac gcgctcagca cctcctctcg cgagaatatc gagtccgcgg ggttggagct 30840

cgcgggcagg agcacgtcct ccacgctcag cacctcgtcg atgaagggca gcaccatgtc 30900

cacgttggag gcggtgaacc actccatgtc cacggcgttg cgctccacga tcacccggat 30960

cgtctcctcg gatatgttct cggcgaaggc gctctgcagc tcgatcaggt tctcggtggc 31020

gtcgatgctg agcccgaggc gcatgccctg ttgcagcacg tcgttgatgg ggttcacgta 31080

catggccacc gccatgcacc cggccggctg cacgcgccag aggttggagt acgcggacac 31140

gtccgtgatg cgcagcgtct ccagcacgtt gtacatcgcc gcgcgcattt ccagcgtgtc 31200

cacgtacacc tccgcgtgct cgaagatgat gtcgagctcg aacgcggaca gcggcagact 31260

cacgtagatc tggcaggcgt caaagagttc ctgcgcgtac tcagagaagc acgcgtacgc 31320

gatcacgccc gcgcggttga cgatgtagtc cgccttgaac atcttcgtga ggtaggcgta 31380

cagcgaccgg caggccacgt gcggccttag ctcgtcgagc acggcgtcca gcacctcgcg 31440

gtgctgcagc acggaggggt gcaggaactg cgtgaagtcc accgcggtct catccatgaa 31500

gtcggggatg gtctcgacca ccaagcggtg gttccgcacc gcgcggaagc cggtgttcat 31560

cggcgcgatg ctgtgctggt cgtggacctc cagcaggatc tcgtagccga tctctcggca 31620

gctcagcgcc gcgcgcgcct ccgtcacgct cgcgttccgg aagaactgcc gcaggtgctc 31680

gtccgtgcgg cagagcgtga cggattgggg gatgcgggaa aggtcgcaaa gagggctgtg 31740

gacggagatg cgccgcagcg cgtggtctac gaactcaaaa ccgcgcctcg acatcgtgaa 31800

gtcgcggagg gcgtacatgt tgtaggaaca gaggcggaag aggcagtcta tgtctagcat 31860

gttggaaacg cagtacgcgt gtctgtggag gtgcgggagc atggcgggca cgacctcggt 31920

cgtgttctgg agcatggtgc atacgaggtc gggcgctgtg aggtcggggg tgacgatgag 31980

gatgcaggag ctggagaact tgctgagctc ggaggccagc cggtgaaggt tgtagtcgtg 32040

gcgctggctg aagttgctgt ttatcgtgcc cgtgaagccc atgagcgccg ccagcgtcag 32100

gtcctcgcgc tcgatggccc agatgtctag gccggaggct atcatgcagc gcaccagcgc 32160

ggcggcgcgg tcgctcgtgg ggtagctcat ggtgctgtcg gtcgtgcgaa tcagcatggg 32220

ataatgcttc atttttacgg tcgggggttg cggactgtgg ggcgcacagg gcctgcgggc 32280

ggctcgtgcc ggtccgcggc gttcgccgaa cgcaggaacg ggcccatgcg cgcccaggcc 32340

atccacagcc ccgccgtcag cgccagcagc cagacgaata ccacgatcat cttttatgta 32400

gctggaactc gcgctcactc cccgccgcac ggcgacgggg agagcccaga gccgagctcc 32460

atgcgcgtgc tctgcacggt gagcgactcc acgagcttgg acacgttcat gcgcgtgttg 32520

tcgggcacca ggtgcgcgag gcgcgcgtac acgtcatcgt gcatgcgctt gctgcagcgg 32580

tccaggctcg cggagagcgc ggagactagc gcggtgtgct tcgcgtacac gaagtcgccg 32640

agacacacgg tctcgagccc gagcacgtcg gcgctctccg cgtccttgag cgccacgaat 32700

atcttctgct cctcgcgcgt catcgagcgc atgaggtagt cgtgcagccg cgagcgcgag 32760

atgagcccct gagagatctg cgggctgcgc atgaagcgcc ggcgcatcgc gcacagcagc 32820

tcctcgtcga cgacgtacat gctgtccttg atggagctct tctcgtcgat cacgagcaga 32880

ccgtcgttgg cgaccacgtt gatgaaatcg tccacgtgcc gcgcgtctat gtcgtagcgc 32940

gtgccgccgc actcgatgtg cgagggcggc gacttgagcc ggttcgcgag cgccttcacg 33000

tcggagacgt cgatgtacag cgaggactcg cgcgggacgc agccgaggat gcgcgtctcg 33060

agcggcgtga ggatgagcac gcgctcggcg ccgtcgacga gcttgccgtc gtcgggggaa 33120

aagaagttgt tctccacgat gctcgagacg aggctggcga gcacgccgtc gcggtaggcg 33180

ccgagcgcaa actcctgcac aaagggcgcg tgcagcaagt ccacgggaat gcgcatctcc 33240

acgcggcgcg cgaccgactt cttcttctgc aggtgccgcc ggtccaccat ctcgtccacg 33300

atgtccgata tgcgggagca gaggtacgcc ttgaggacgt tggcgtttac cttgttgaag 33360

atgagccgtt cgtcctccat ttaagctgct cagacgagct ttaaatagtg gaaacacagc 33420

agcacgccga tcgccgccgc tatcaggccg attagaaaaa cggtggtcca ggggacgccc 33480

ttgggcctat cgcacgccgg cttttcggtc attacggtgc gcacgatgtt taggaactcc 33540

tcgaagtcct cgtccgagtt ggagaggaag gagccgaaga cgccggtgta cagtttgtcc 33600

atttactact agatattaaa cggcgcttcc aactcctcgt cctcgaagcc cgcgccaggc 33660

tcgacgacgc ccaggccgcg cacgtcctgc tcctcgttga acgtggtctg agtctcgctc 33720

atgcgcacac acgtctgctc gccctcgaga ccgagcacgg tcagcgagca ctcgcgcggc 33780

atggtgatct tcttaaccgc gaaggtgact ttgccctcgc cgcccgagcg gtagaacacc 33840

accggcgcca ggatgagcgt cgccatctgc gcgtcgcggg ttgcgaggtt ttctatctct 33900

cgcgtcagcg gacatatctg cggctgggtg tcgtcgtcgc tggtgaactc caggagagcg 33960

ccggtcaggc ggttgagata cagacatccg gacttaaagg tgttgtcgat ggccgtttcg 34020

gtgttgaagt tgcgcagcga cggcggaacg cgagcgccgg ttttgatgtt gtcgtatatg 34080

ttctccagca gctggtagag cagcggactg gcgctcacgg gcttgaggcg accgaagtac 34140

ccgctgtcgt tctgccgctg catgtcggtc ttcttgctcg ggtagatctt aaactcgccc 34200

ttcacgacga tgagcggcga gacgagcttg gatgctagac tttcgacgag acacacgttg 34260

atgttggagc agctcgggta ctgcgacgga gttagagtca cggcctcgat gaccttggtt 34320

tggctcgacg agagcgactt agcgaagttg atcgcgtcgg tgcacgacat cgcctggttc 34380

tcgccgaacc gcctggcgga cgctgcatcc tcctgctgag gagcgcggtt agacgcgacg 34440

gtggttttgg atacagcgcg tttcattatt gcggcgattt taaagtacgt gtatactttc 34500

agttttgtcg ccgagcgttc agcgcctgca tgcagaggaa gtacaggatg atggtgcacg 34560

ggatcgtggt cagcagcgat acgaagtcca tcactgtgag gacgcgcagc gccccgcgcg 34620

agcggatgcc cagcgagggc gcgccgcggc gcgcgatggt ggccccgttc gtcaccacta 34680

ccagcagcat taggatggtc gcgcccacgg cgacgcccag gtcccgcgac tccatttata 34740

gtacagtata gagcgaccgc gtcacgaact ctcggctggc caacacgcgt ccgtcgggcg 34800

ggtgtccgcc ggccttcccg cggaactccg ggacctcgaa gctggacttc gtcacgcggt 34860

acgtgtactt gccgcgccag accaggtttt ccttctggaa gacgccgtcc atggtcacgc 34920

ccgccatgaa ggcgtccttg acgatgacca gcaccgcgtc tagcttgcgc ccgttgatgt 34980

gcgtgacgaa gtccgtgccg ctgcggctcg cgcagcggat gtccacgccc gagggcaggt 35040

ccaccacgaa cacgaagcgc ttcggcgcgt agagcaccag gtccgaggac ggcgacgccg 35100

aaggcgccga ggggaactgc cggtggtcaa aagggtgcac cacgcccacg atggacgtga 35160

cgcggtcgtc cgggaactgc gtcgcggcgc cgccgccgcg gtgccgcgtg accgtgcttc 35220

tgcccacgtc gtcgcagacc acgtgcagct ccgacacgat cggcagcagc gtggccagca 35280

tgcggtcggt ctctgtgcgc gtcgcgcagc ggtacgcgat cccgcagtgc gcgtcctgcg 35340

tgcgcccgaa gaagagcacc agcacgctcg cgtcctggtc gaagggacac acggccatca 35400

cgcccaccgg cggcggcccg tggcctgcgt acgcggagga gaactcctgc acctcgacca 35460

cggcgtcctc gcgcgcctcg ccgggcacca tcgccgccgc cggccgcagc gcccgcacgg 35520

tctgcttaac cgcacgcgcg gcggaggccg cgctcggcgc gactacgcgc acggccgcgt 35580

gcgcgcccgg cggcggcgcg gttccggcca tccagcccac cggcgagaag aacacgtcgc 35640

agacgtgcac gcccgcggcc tgcagcgcgc gcgcgagcgc gcgcacggcc tcccactcct 35700

cgcgaaaggc gctcgcgacc gcgagcgcct tcagcaccgt gtccacggag ttgacgggct 35760

tctggaagag gttctcgttg ttgtagatga actcggggag ctccacgggc actgtgaaca 35820

gcctaatctc gtgcgcgccg ctgggcgtga gccgcgtcgc gggcttgcgc acgccggcgc 35880

cgatctgctt gaagaagtgg ttcatggcgc cgccggcttc tcgggctccg gcgggagcag 35940

actatttatt cgggaggtta tcctttccga aagcacctgc acggacttcc gcgtccagcg 36000

ctccatcttc atgtactcct tcatgccgtc gctgagcacc tcgacggcct ccagcttggg 36060

cgctgtcggg tcgaagagga tgctcttgag cagcgtcatc ttcttgtccg cgaggaagcg 36120

gaagtaagtg tagatgcagc gcagcgcgcg gaagttctcc gggtgcttga tggtgcacag 36180

gatcatgaag atgcaggtga acatgccgca ctcggactcc atgagctggt tgacctcgag 36240

gttgatgcag ccgcgccgcg ccttgaagtt gtccacgaag aagcgcatga gcacgtccac 36300

gtcgcagttg cggttgtcca ggtccgcggt ctcggcgttc acgttgaagc cgtccgagaa 36360

ggagtagaag tagaagtact tgcaggggtg gaactccgag gggctgttgc cgccggagtc 36420

gtagaaggac acgagccgcg agacggtgtc gaagatgcag cacttccagt ggaacatgta 36480

gcagaagccg aacatcacgt agcgccgccc ggcgcgctcg atcttgtcct tgagcgtgag 36540

gctgaccatg ttgcagcgga agcggtccgc cttttcgtgg atggccgcgc cgttgaggaa 36600

gttcaggttg aactggccca ggtacgcgac ctcggtgccg aacgcgaagg gcgccaccag 36660

actctggatg ctcacgttgc tcatccaggc gctgcggtcg ggctttatgg cgatgggcac 36720

taccttggtg ttcacgcccg tgctgacgcc cgcgcgcgcg aggtcgtcca cgttcagcgg 36780

catctgcgag aagtccacgg cctcggacac cttctcgcgc aacgagggct tgaagaagaa 36840

gcccagcggg accttccact ccagcgcgat cgcctcgcgg aagccgtagc gacccttgag 36900

gctggccagc aacgcggtct tctgcgcgac ctcgtccttc tcggtgtccg gcggcgcggc 36960

gtcgatgagc ccgcgcttcg cgaagtccag cagcgccgcc agcgggatgc acgagacgcg 37020

accggccgtc gcggattcgt cgaagcgccg caccacgtac ccgttgcagt tggtcttgaa 37080

gttggacacg tccaggtgcg cgctgagccc caccaccgag tagatgtggc acagaaggtt 37140

ggtgaacccc agctccggga ttttgctcac cactaaatcc gtgtacttgt ccatttatca 37200

tggagaatca tctgccggac atgctgatgt ttcccaactg cgtttctgtg tttccctttg 37260

agtactcgct ggaggacgtg ttccgcctcc ccgaggagcg acggcgcgcg ttcgccatgg 37320

ccgtgttccc gctctccaag caccgctgga gggcgcgcgg gctccagcgc gacgagcgaa 37380

gcgtgtggct cagcgtcgag gaggaccgcg ggcgcgcgct ggacgagcgg aactgctcct 37440

ggctctcgga cgtggccgcg cgcatggtcg acgacgaggg ccgcgcggtc acgcccgagg 37500

cgtacgcctt catgcgcgcc gcgcccggcg cgcgcgtcgc cgagctcgcc gcggacgcgg 37560

gcgtgctagc gggccttgtc gccggcggca acgcgctgcg cgtcttctcc tcggagtcca 37620

cgcaggcgcg cgagggctgg aaggcgcgca gcgtgggcgt gctcggcaac gcggcgccgc 37680

tggcgcccgt gccgctggca tcgctgcgtc cggaagtgca gcgcgagctc ttcgccgcct 37740

ggatcggccg ccgccccgtg gtgctcacgg gcggcacggg cgtggggaag acctcgcagg 37800

ttcccaagct gctgatgtgg ttcaactacc tcttcggcgg cttcgagcgc ctggacgccg 37860

tccgcgagtt cgcggagcgc ccgctcgtgc tctcgctgcc gcgcgtcacg ctggtgcgcg 37920

cgcacaccgc gacctacctc gcctcgctgg gcttcggctc ggccgacggc tccccggtct 37980

cgccgcggta cggcgccatc ccggacgccg agcggaacac ggccccgcgc gcctacgggc 38040

tcgtggtggc cactcaccgg ctcacactga ctgccatccg ccgctacgac acggtcgtag 38100

tggacgagat ccacgagcac gaccagatgg gcgacatcgt ggtcgcggtc gcgcggaaac 38160

tgggctcgaa catgcgatcg ctggtgctta tgacggccac gctcgaggac gaccgcgcgc 38220

gcctggagga gttcctggac cggcccgcct ttgtgcacat agagggcgac acgctcttcc 38280

ccatccgcga ggtctacgtg aagaacacgc aacagccgcc gctctcgcgc aagtacgcgg 38340

aggcggagct gcagaacgtg gcgcaggcgc tgggcacctt cgtccccgag cagggaaagt 38400

gcggcatcct cttcgtagcc acggtggcgc agtgcgcgct cttcgcggag accatcgagg 38460

ccaagcaccc cgggctgctg gtgcgcgtgg tgcacggaaa ggtgccctcc gtggccgcgg 38520

tgctcgagga ggtatacgcc gcggaccggc ccgcggtgct ggtttccacg ccgtacctgg 38580

agtccagcgt gaccgtgcgc accgccacgc acgtctacga cactgggcgc gtgtacgtgc 38640

ccgagccctt cggcggccgc gagaccttcg tctccaagtc catgtacacg cagcgcaagg 38700

gccgcgtggg ccgcgtggcg cccggcacct acgtgcgctt cttcgacacg cggctcgcgc 38760

tgccgctgaa gcgcatcgac tccgagttcc tgcacccgta cgtgctttac gcgcgcatct 38820

tcgggctaac gctgcccgac gacctgctcg tgcagcccag cgacctcgcg ctgctgcgcc 38880

gcaccgagga gtacgtcgac ggcttcggca tcagcctctc gcgctggacg cagctgctgg 38940

accggcacta catgcacatg gtcgagtacg cgaaggtgta cgtgcgcggc gggcgcctcg 39000

ccgccgcgct ggacgccttc gagcgcaccg gcgtgatgac gcacgaggcc accgaggcca 39060

tccgcgccgt ggacatgctc gcggccgtcc taaacgtgcg caagtccaag gaccgctacc 39120

gcgcggagtg caaggtgctc ttcgggccct tcgcgggcaa aaagttcgtg gtcgccgggc 39180

ggcgtccgcc cggctcgcac gtgctcatgg tcacagaccg cgtcttcatc gaggccgagc 39240

ccccattctg aggaccacct tcttggagac gcccgagaag tcgtcggcga cgccgcggcg 39300

cgccaccaca aggcagtacg aggtcacgtg cgggcagcgc gcgatgcagc ggaaggcttc 39360

ctcctgcgac agcgagaagg cgaacacgta gaaggtgtgc ggggacttca gcggcgtgtg 39420

gtccatcgag tagatgacac cgagcttctt catgcgccac ataagcgcgt tgatgtggtc 39480

ggcgcgcagc gcgcggccct tgagcacgcc gcagacgaag ctcgagcagg ccacgacgtc 39540

gtagcgcgtg ttcctgccga agaccaggtg cggcgcgccg gcggcgcgcc gcgcggccgc 39600

gcgattctcc acgatgtcct ctatggagcg ctcgctcgca aagaagtcca ggaacatgta 39660

ctggtaggcc acggccgggc gcgacttgct gaacttcatg aaggcgtccg agtccatgat 39720

ggcgtccatg tcctcagcgg cgagccggtg ctgcagccgg atgccctcga aggtgtggaa 39780

gagccgcgcg tccgcgtgca tggacagcgc gagagtgacg aagttgagaa ggtccgcgtc 39840

gccaaagcgc acgagcacgt taccgggcgt gcgcgtcttg cgcatgagcc gcgcgggcgc 39900

gccgtcgttg tggctgcggc ggcgcatttt gtcgccgggg gactcgggcg gcaggtcgat 39960

catgaccagc cggtgccgct gcgcgtcctc ggcgttgaag atcgaggacg tgaagcccgg 40020

gtacagcacc acgcagtcgc gctccgagat ggcgtgcagc acgtcgcgct tgagcccggc 40080

caccagccgc tccgcgttct cgacgaagta gttctcgtag tccaggatgt cgtgcgccat 40140

ccaggggaag ttcaggtacg cgttcatggc gtagtcctcg gcgtcgaagc agatgcgcgt 40200

gtctggcgtc gccgcgatcg gaaggtcctt gatgccgcgg agcagcccgt cgtagtcgga 40260

ctcgtccacg aaggagagca ccacaaagag gtcctcgccc acggtttcgt agtcgaagag 40320

gtggtaaagc tctcttagcg ccagcacggc gagcgcgttg tccagcgagg cgtgcacgcg 40380

cgccaggatg ctgtagaagg gcgtggccat catcacggcc ttgccgccct cgcaggcaac 40440

ggcgcgcggg aaaatgacct ccggcgtgcg cggcagccgc ccgaacgtcg cgttcagcag 40500

cgcgaccgtg gccgcgtcgc tctggcgcag gaacactacc accgaggggc ccgagatgct 40560

gagcatgcgc tcgcgcatgc gcgctggcag gtccggcgtg gtcacgaggt ccgcgaagcg 40620

gccgccgttg tagaggtcgc cgccgccgag gaaggtgagc acgtcgaagc agtgcagcac 40680

ctcgttgcgg aagtagtact cgttctcgag ctccttggcg tacgcgcgta tgtccacgtt 40740

ctcgaagttt gttcgcagac cgccgccgtc gaagaaccag gacgcgagct cgcggacggc 40800

gtccgcgggc ctgttgcggc ggctcttgca ccagaagctc atgtagttgc gcgaggtgga 40860

ggcgttcgcc aggaagaagc ggtggtcgaa ggagatgagc acatgctcga gcaggtgcgc 40920

gagccccagg accgcgccca cgtcgcgccc aaaaccgaag tttgatatcc ccaggtagac 40980

gtcccgtttc atagacggcc tcaggaacac cctgacgccg ttttccaaca ctatcattct 41040

ccggtattta cttacccaaa agtagtatgg ggagaagtgt ttgaacgtcc cctcgccttt 41100

ttaaatcaaa agtagacttc tcgcgcccgt gcgccaccgt cacgcgcgcg cggcgcgagt 41160

ccataccggc gatcaccgcg ctgctctgcg gtgcgtccgg ccgcgggaag agcacggtct 41220

cggagatccc gtccagctgc gcgtcggtgc gctgccgcca cgcgtgcgcg tccgcgagct 41280

cgcgcacggc cagctgcatc ttgttcgtcg gcaggaacgt gaacacgtac gccgccgcca 41340

ggaaggctgc gaagagcacg aactcaaccg cccatgacat ttagggagct gattttgttc 41400

cacgcggcga cgcacgtcgt gacgggcgac cccgaggcgc cgcggcgcgc ggcctcgctg 41460

tgccgcggct tcggcgtgga cttccgcgcg attcacgcgg agttcgcgcg gcggtacccg 41520

cgcaccgcgg ccgccgtgga gcgcgcgcag ccgctgcccg aagtcgatgc cgcctttccg 41580

ccggacgcgc gccggcaagt cgtgcggctg cgcctcgagg ctgcggcgct ggtcgtcaag 41640

gagtcgcgtg cgctatcggc ctccatgcgc ggcgtggcgg tggtcgacgg ctgctgcgtg 41700

cgcgtgtgcc gcgccaacga cgagctgcta gagttcctcg cgcggcgcta cgaccccgcg 41760

gtctaccgct acgcggaggt gccctcgccg agcgtgcgcc cgggctcgaa agtcttcgcg 41820

tgcgcgggcc gcagcgtcac ctttgcggcc gcgcaccgga gccgcatcac ggccaaccgc 41880

ccgctgcgcg tggtcgtgac cgaggcctgt gtggacggcg tgctcgcgcg cggcgccgcg 41940

gaggtcttcg accgcggctc cggcgtgctg ccccgcgcgc tgcgcgagat cttctaccgc 42000

ctcgacgagg acggctgtcc cacgggccag acgccaggct tcgcggacag tatggcgtcg 42060

cgcagctgat ctatgtccac ctttttctcg tcgatctgcg ccacgaccac gaaactgcga 42120

atgtccacag cggccatggt cttggccacc gggtcgtact tgaggagcag cacgtactcg 42180

ttgccgaagt gctcggtgac ctcggtgatg agccggtaca cgcccatgcc gagcacgttc 42240

accgcaccgt ccttggcgaa gagcgagagg atgttcacgc acttcagctc catctcgccc 42300

tcgaggcgcg cgagcatgcg ccgggtgacc tcgcatactg aacaaagagg cttacctagt 42360

aagataagcg ttagcttagc cgcggtcggt gacgcgtcgg aggccattta tggggatcaa 42420

aaacttaaag gcgttgctgc tcagccacgg cgcgctgacc ccgcacgagc cgggcggcga 42480

cgagcgcttc cctgccgtgt tcgtggacgg cttcagcgtc atgatgacca tggcgtactc 42540

gtgcgcggac gaagacgagt tccgcgcggc cgtcgaggag cgcgtgcagc actggatgag 42600

cgtgtccgag agcgggcgga tcgtggtctt cctcgaccgc ggcgagattc cgatcaagca 42660

gccgctgcgc gaccagcgcc gcaaagccac gcgcgaccgc gccgcgcgcc accgcgagtt 42720

catcgccgcc gcggaggcag acgcggcggc agaggccgtt ggcgcccgcg aggacaaaca 42780

ggaggacgag cacgcggagt tcgccgagga gatccgcgct gagaagcagc taaagctgca 42840

gcgcatccgc ttccagctca gcatcgccaa ccacgaggtc gttaagtcgc tgatagagtc 42900

cacgctcgcg cgcgctggcg atgccgtgga gatcgtcttc tgcgacggcg tcgacgcgga 42960

gatggtcatg tgcgcgcgcg gacgcgccga ggccgagcgt cgcgggcgct ggccgctgct 43020

cgtgaccacg gaccaggacg cgcttttgtt cacgtccacc gatcgcgacg agaagatagt 43080

gagcaccgtc tccgcctgct acgcgttcag gcccaccgag acgaccgagt acctgtgcaa 43140

acttgcggcg ctggccaacg gctgcgactt cttccccggg ctcggcggca tatgcgtgag 43200

tgtggagtcg ctgcgccgcg ccacgctttt cccggaattc tccgtgcgca acgccgccgt 43260

gagtctgtgc acgcggccca tgcggctgtc cacgcaggac gcgctggagc cagaggccgc 43320

cgccgaggtc gtggaattca tcaggcggta cgccgccggc gacgagcgca tctaccgcga 43380

ggtgccgccc ggcgcgtgct gcggacgcgc gtttgtgcgc ggagcgctcg cggccgagtg 43440

ggccgacgcg ctgccggcgg ccacgggtct gagcgtggtc gcggacatga tcgcgtgtct 43500

gcccgcgcgg cgggaccccg cgcccgagga ggtagagcgg ctgctggcgc tggaggcgcg 43560

cgcgcgaggc gcgcgcgtca cggatgcgat gctcgcgcag actgcgcagc tgctgggtta 43620

cggcgcgagt gcgggcgccg acggcgcctc cgccttcgcg gtctcgggcg ccaagggcct 43680

gatgtgtcgc ctgcgcggca cggccatgtt cttcaacgcg gagtacgtgg aaattgaaag 43740

cgaacccaga ctgttaaagc tgcggtagca tggtgttccc gatcgtgtgc tcaacgtgcg 43800

gccgcgacct gtcgcacgag cggtttctgc tcatcgtgcg acagcggccg ctaaaggttg 43860

ttttgcggac ggtgcgcaac gtctgctgcc gtataaagtt gtctacacaa atagagccgc 43920

accggaacct gacggtgctg cccatgctcg acataagctg atttttcttt tccgctcgta 43980

tgcgcgagtt cggactcgcg gcgcgcatgg cccgcgccat cgaggacgtg tgtccgcgcg 44040

gcgcggtgat attcgtatcc agcgccgcgt ccatgaccga ctgcctgaac ccgtcggtgt 44100

tcaagcacgc ggcgatatac gcgggcgcgg tggaccgcgc gccgctgccg ccgccctcgc 44160

cggtcccggc ggaggccgtg acggagccct gtgcgataga cgccatagcg ccttacggcg 44220

cgcgcgtggt cctgctctcg gagctgctgc ggagctgcgt ggccgttcag gcctaccgcc 44280

tggcagtccc cggcgccctc gcgctcatga acctcgcggc cgacgcggcc ttcgagctcg 44340

tgggcacgcc ctacggcttt aacagcgacc gaacgtactg cttcaagctc gttgccgact 44400

gctttgctag cgtgggcgtg acaacgaaga ccaggcgcat catgggtcgc gacgtcgtgc 44460

tcagccagga cttcctggag agcggcatgt ggaccaaggt gctggactcc gccgcggagc 44520

cgccgtggct ggtctagaac agcggcggcg cgcgggtccc gagcacgggc cgcgccacct 44580

gcagccgctg ctgcagcgcg cggcactgcg cctcggcgtc ggccgtctcg gcggggtcga 44640

cgggcgtcgg agttgcggag gtggtcctga acggctgcgt gttcaccgag acgcggatgc 44700

gctccttgca ggagcgctgc tcgatgcagt tggccagcat cttcatcacg tgcaggtact 44760

ccagcaacac gaacttttcg agggtgatgc cgtcgaaggg cgacgacccc accacgccca 44820

gcgggctgga caccgcgccg tcgagcacct cgccgcggga ctccttgcgc gcgcgctcga 44880

gcaggtcctc tgtccgagcc accacactgc cgaagtcggc ggccgcgggg gcgggaacag 44940

gcgcagcagc gctgtccgcg tccgccggca tctcctcgat cttgagaccg gccgcgaact 45000

ccgaggccgc gtgcacgggc gaggcgccgc gccgcaccat gaagtcgcac agacgcgata 45060

gcgcggagga gcgcaccggc atgtcgagca ggcgctcggc ctccatctcg gcgaccgagt 45120

cggcgcacgc gtccggcgcg cccgcccgca cgagctcgtc gcagcacccc gcctccttca 45180

tgagcgcggg catgagcttg tactgcgcca tgttcaccag cccgtacttg agctcgagca 45240

ggtccgcgag ctcggaggcc atgggtcggt ttttggtgta gatgacgcgc tccacggcct 45300

ccgccatgtc cacggcctgc atgagctcgc cgacgagcac gctggccacg agcgtggcca 45360

gcgtgacgcg cacggtgggc acgcagaccg cgaagaagga ggtggagtgg gtgaagcgca 45420

tgagcgcgcc gtgcagacgc gcgaggtccg cgctgttgcc cgcgtgcacg aagcgccggc 45480

gcagccgcgc cagcgcctcc acaaggtcct ctcgcgtggt cacgcgcacg ttcgcgatgc 45540

acaggtcgtg gatcgcgttg gcgatctgcg cgcggcgctg cggcgagctg ccgggcagca 45600

gccgcgcctt ggcctcgacg tcgacggtgc tcgagagaca gccgcaggcg gcgccgcgga 45660

cgacgaactt caacaacgac tcgaacacgc gcgcgcccgc gcggggcgct tgcttggacg 45720

actccattta ctttaaataa tttacgagat caaaataaaa tgactctgcg catcaaactc 45780

gagaagctca agcagatcgt aacttacttc tcggagttca gcgaggaggt ctcggtgaac 45840

gtggacgtcg gcgatggcct catgtacata ttcgcggcgc tgggcgggtc cgtgaacatc 45900

tggaccatcg tgccgctcag cgcgagcgtg gtatacgacg gcgatgtcag ccgcgtgttc 45960

aacctgcccg tgctcaaggt gaaggcctgt ctgtgcagct tccaccccga ctcggtggtg 46020

agcctggagc ccgacctcga ggacaacgtg gtgcggctct cgagccacca cgtggtcagc 46080

gtggactgcg acaacgagcc cgtggcgcac cgcacgaaca ccgccatctg cctgggcatt 46140

aaccagcgca agtcctacgt gttcaacttc cggcgctacg aggagaagtg ctgcggccgc 46200

accatcgtca acctggacct gctgctgggg ttcatcaagt gcatccacca gtaccagtac 46260

atcacggtct gcttccgcga caagaagatg gtgctgcaca cgcccgggaa ggtggacaac 46320

ttcttccgcg agtactccat gaccgagtgg gcgcccgacc tcgagcgctt ctcgttcaag 46380

atccccatct cctccgtgaa caaactccgc ggcttcaaga agcgcgtggt catgttcgag 46440

tcgcgcgtgg tcatggacgc cgacgacaac atcatcggca tgctcttcac cgaccgcgtg 46500

ggcatgtacc gcgtgaacgt gttcatgtcc tttcaggacc ggtctctttc atgcgactaa 46560

atactcatgg gcgggtcggt gagcctgccc tcgcgggacc tgccgccgcc ggtgcgcacg 46620

ccggagatga acatcgtgcc cgagcgcgac ctcgcggaca cgatggcgcg cctctccacc 46680

gcagacccgc cgcagccgct gggcgtcggc gacgacgcgc gcatggccgt gctgaagacg 46740

accttccccg agttcgcgat atcgcggccc gcgacgggca tgctcgccgc gcagcgaatc 46800

aggtacgacg gcgacccgcg cgtctgctgc ggcgggttcg ggatctcgca ttactgggag 46860

aagggggcgc gccgatcgaa cgtcgcgttc gagggcgcgg cgctgcgcac ctgcgacccc 46920

acgcgcttcg acgcgggcgc gtgcgacgcg ctgctcttcc gcgagtgcgc cgccggcggc 46980

gtcgacgcgg acttctgcgc gcactggatc aacgcggccg tgacgcggcg cacggaccga 47040

cagtcgcgcg cgcggctgaa cgacatgttc gtgcgcgatt gccaaaacga cgccgcccgg 47100

cctcactgcg tggcctggat ccgcgcgatg cgaagcgcgc gcgcgacggc ggacgacggt 47160

ctaatagacg ccgtgctctc ggtgcagagt cccgagttca agggcaagca catgcgctgc 47220

agctacccct cgccggccac tctcgccatg gccgcgaacg tggacgagcc gcgcgagtgc 47280

tgggaccccg agtgcgtggc cgggaacgtg gacttcatgc taagcgataa ctacacgaac 47340

ctgggcttgt gtcggctctc gcgctgctcc atcggcgtca cacacctgcg gattgacgcg 47400

cgttcgcggc tgcgcatgcg gtgcgccggc gcgcttgccg ggctcacgaa ggcgcccgtg 47460

aaccagactg tcgtcgtcgg cgacaacctc gcgcgcgcct tcgagccgcg cgtggaaacg 47520

ctcagcgtgt tggcgctgtg cgtggtgtat ctgctaattg tctggctcta aatgggggcc 47580

gccgccagca ttcagaccac cgtgaccacc gtcagcgagc gcatccgcaa cgagctcgag 47640

cagagcgcga gcgctagcgc gaccgccgac tgcgacgtca ccatcgggag tctgattatc 47700

cgcaagaacc taggatgcag cgtttccgtc cggaacatgt gctcggccaa cgccggcgcg 47760

cagctggacg ccgtcatgaa ggccgtgagc agcaccttca acgacctctc gtcggaccag 47820

aaggcctacg tgcccgggct gctcacggcc gcgctcaaca tccagaccac ggtgaacacc 47880

gccgtcaagg acttcgagac gtacatgaag cagacctgca cggcggacgc ggtcgttcac 47940

aacaaaatca agatccaaaa catcgtcatg gaagagtgcg cctctctgcc agggagtccg 48000

gccacgcacc tggatttcgt gaacaccggc acggccgtgg gcaactgcgg cgtgaaggcc 48060

gtgatggacg tgctcgcgaa ggccagcacc accgtgcgca acgaccagga ggccggcaag 48120

ggctaccaga ccatcatcat cgcgatcgtg gtcgccatcc tggcggccat cttcgcctgg 48180

tacgcgcggc acatgctatt catgtccacc tccgacaaaa tcaagctcga gctcgccaag 48240

aagcccgtgg tgcactggac cacctacctg gacaccttct ttacggaatt tccgccgtcc 48300

gtctagatac gcgcaacatt gaaacattat atccacctct caaacggcgg tatggtccga 48360

cgcgtcctcc tcgagcgcgt ggacggcatc gtcgagcact cgcgcgcaga ccgacgctac 48420

ttggaggcca ttcagcgaca cctcgagggg tctacgcccg ggctgcggca gatgtggcgc 48480

ttcctctacg acctgctgct gacggtgttc gtcgtcatgt acatcgtctt ccgcctaatc 48540

gtgcgcaacc ccggcatctg cgccatcctc gcgctcgcgg ccgcggtgta ctacctgttt 48600

ttgtgtctct ttagcatgga ctgatggcga tcacagacag accatcgccc gcgcgcgcgt 48660

gaccagctcc ggcgccgcga agacgtcctg caccgggaag tcgtcgatct cgaacacgga 48720

gccgtccgcg gaccagatca cgcgcacgtt gtcgctcacc gagacctcgg tcagcgtcac 48780

gcccagcaca accgcgtcgt tggtgctcac cagcaccagc gcgccgggct ccgcgcgccg 48840

gtgcagcggc ggccccgaga ctgagcgccg ctgcacgcgg aacatgtccg cgaactgctt 48900

cgagagcaag tccaggtggt tgcggatgat ccactcgaag aagtacgcgc aaccgccgcc 48960

gccgcacagg aagcgcgaac ccgcgggcat cagcagccgc acaacgtcca tgtagcaggc 49020

ctgcggcagg ctcgcgcggt acagccgcgt cttcggcgag agcaccacca ggctggaggt 49080

gctcatctgg aagaccagct ggctaacgga gacggtgagc gtgcacgcgg gcacggaaac 49140

cacgtccagg cagatgtcgt ccagaaagat gctccgctgg tagaggtggt acaggatggc 49200

cacgatctga aaggccgtgg cgtcgctgat ggcgcagggg cggtcggcgc agcgcatctg 49260

cgcgcaggac cagcccccga aggactcgaa gcagacggtg agcatgcccg tgctcggaca 49320

gtgtggcgag cgccgacaca ccggaaagcc cacggccttg cggcagcgca ccatggtcga 49380

gagctctatc cagcagcctg cctcctcctc gcccatgccc atggctaccg gcgtgaaggc 49440

cgtgacgtcg tcgcagatgc gccgctccag aaaccccacg cccgaggagg ggtgcgcggc 49500

cggcggcgag gtgatgcgcg ccgggacgcg gctcggagcg ggctcgggag gcgagctgcg 49560

ctcgacccgg gcagtcgccg ccggccgcga tgccctgcgc gcgggcgcgc gctcgcgcaa 49620

cttgtttgac ttgctggcct cgtcgctagc gtcatcgaag cggtcgttcc tgtcgccgcg 49680

gacgtccgcc tcgtcgcccg tcggctgcgc ggcgggcgac gtgccgtccc gcgtacggcc 49740

cgcgttcggc gcgaatgtca cgcgccggtg cacgtacggc tccgtagagc ccgtgggggc 49800

gccgcgcccg cgcccgcggc ggaaggcctg ccgggacgcg ccgaagcggg cgaactcccc 49860

cttcgcccgg cccctttttt cttccatgat atttatcaca aaaaaaactt ctctaaatga 49920

ccaatctgct ttcgttggtc gacccggagg acctggcctt ctgcgccggg ttcccgtcct 49980

tcgacgagac catgctcgtg atcgcggggg cgcgagtgcg cttcccacgc tcgctgctct 50040

cgctcttcaa cgtggtgccg cgcaccatga cgcgctacga aaccgagctc gtgggcaccg 50100

agatggtggt gggcgccgtg ttcaccaccg cgtacaacgt ccgccgcaac ctaggcctcg 50160

gcgaggagcc cgtgaccatg cgcgacatcg agaagtactt cctggactcc gagaacgagg 50220

tgctcacgct catcgtgcac aacaccgact tttccgccat gagcggcgtg cgccggcgcg 50280

gcggccggcg catcgccaac cccgtcatct tccgcagcgg gtccacgccg ctgctcatcg 50340

tgatggagtc gcgcaagaag accaacatct accgcgagcg caccgcggag caggccaacg 50400

cctcctacag ggaggtcggc tcctcgctcg cgctggtcac tcggtacgcg ggtctgcagc 50460

tggttgacgt gcacacgccc agctccgtgc taacggtctc cgccgtctac ggcttcaccg 50520

aggacaaggg gctcaagaag ctgggctccg acaaggagct cgcggactac cagtccacgc 50580

cgctcaccga ccccatccgg ctcagcgact tctccaatat attcgacggc gtcaagaaga 50640

gcatccagct cacgaacgtg cccgtgccct ccaccggcgc cgaggccgcg ccgtaggctt 50700

tcatgcgcga taaatcggat ggcggcgccg acgacgcccg cggtgcacct cacgccggtg 50760

ttcgtggagc ctacgatcgc gcactcgctg ctgcgcgcag agtcctacct cgcgatcgcg 50820

gtccttgagc tcgtgctcgc gctcgcgctc gcgctcgtct tcttccgcga cgagctaggc 50880

tcgctattcc gctgcgcgcc gcgagcgcct tcgccgctgg acgcgtacct gcaggcgagc 50940

ctcgtctgcg acggcgacgc gctgctgatc gagctgcccg agggccgggt gccggcgctc 51000

gcgctggacg ggcggcccgt cgcgttcccg gggtgcgaga gccttttgta ccgcataaat 51060

ggaccacgaa aagtacgtct tgtcgatgtt cttggaggaa gataactcct tcttctcgtt 51120

cgtcgccgcg ctgtccgatg acgaggcgct cggcgccgtg cagtccgctg ccgccctcct 51180

ggacttcctg ctctccgtgg tggtccgcgg caaggagaag ctcgccgccg cggggcacca 51240

ctacgactcc atcgcggacg gacgcgcgcg cgccgcgttc gagttccgag acctgcgcga 51300

gctggcgcag ctcttcgacc ggcggccctg cggcgtccag gaccgcgtgc gtgtgcgcga 51360

cgggcccgcg cgcgccttcg tggacgcggc actggggctc atgcgcgagc gaggcttcga 51420

cggcacgcag gccgcggagc gcgcgcgcta catcgcgccg aacgatctgc ccgcgctggg 51480

ggcaatatcg gccacgctct cgccgggtct ataacgtaaa aaatattagt aaaattctga 51540

aggtccgtgt gtttcgcggg cggccaacaa accagtcgct taaatggagg gggtggaaat 51600

ggacaagccg ctcctctact tcgacgagat cgcgggcgcg cgcgactacg acgcggcctt 51660

cgcggagaag cacgagccgc ccaagatccc cggccgcgga cagatgaagc tgctggtctg 51720

cgagctcgtg tttctcaacc ggctgcacct gcacggcatg ctcgacggca gcgtcatcgt 51780

gtacgtgggc tccgcgcccg gacggcacat ctgctgcctg cactcgcact tccaggagct 51840

cggcgtctcg cttaagtggg tgctcattga cgggcgcaag cacgacccct gtctctcggg 51900

gctgcggaac gtgaccacgg tgacgcgatt cgcggacgag gcctacctcc gcgagctgcg 51960

cggcgagctg cggcgcgcca agatcgtgct catttcggac atccgctcca accgcgtgga 52020

cacagagccc accaccgcgg acctgctgcg cgactacgcg ctccagaaca ccatggtgag 52080

cgtgctcaag cccgtggcct ccagcctgaa gtggcgctgc cccttcccgg actcctggga 52140

gaaggacttc tacgtgccct gcggcaagga gatgctgcag ccgttcgcgc cgccgttctc 52200

cgcggagatg cggctgctca ccgtgtactc ggagacgcgc ccgaagctgc gtctgatcac 52260

gctcagcgac gcggtcaact atgaaaagag gatgttctac ctcaatagcg tggtccgcca 52320

gcgcgtaatt ctgaactttg actatcccaa ccaggagtac gacttctttc acatgttctg 52380

tctgctctcg tcggtggtgt gctcgtgcga atttaaatcg cccaaagaga aggtgctgag 52440

cctgcagaac cgcttcttcc gcttcctgcg catcccgccc tccatcacgc tcgggctgcg 52500

ccggcacgat gaaccgccac aacacgcggt acctggccaa gatcctctgc ctaaaggccg 52560

cggtaagaag cgaccccttc gcggtggtaa gtagggacac cgtgcgcatg tacgacatcg 52620

aggtcgagta cggcgacctc gtgacggtgg tcaccgtcac gcacaaactc gagaccagcc 52680

gcaccgtctt ccaggtcttc aacgagacct ctgtcgcgta ctcgccgctg ccggacgact 52740

acggcgagcc catcgtgctc accacgtaca tgcagcgcga gcacaccaag ttcccgctct 52800

ccatgctcta catcgacgtg gtcgcctcgg acatgttccc cacgtttaag cgccccaccg 52860

aggaggaggc cgcggtggtc gcggccatgc agcgcgtggg cgggcgccgc gagcccgtgc 52920

tcaagctccc gcgcatgctg gacaccgagc tcgtgtgcaa gatactgcac ctgcccgagc 52980

acccgctgcg cgtggtgcgc ttcctgcgcc gaaacatgtt cacgggcgtg gaggtcgccg 53040

accgctcggt gtccgtggtc ctcgactgac gaagggcagc acggtcagcg aggccgccgc 53100

caccaagcac agcggcagcc acgcgcgcgg gtccgccacg ggcacgaaga cgtgctggtt 53160

caggtacttc gcctggaagc gctccgcggt ggagtccacc ttggacccgc aggcgttggt 53220

gaggcgcacg accgcgtccg cgacgcgcac gtccccgagc gatatcacgc agtcagagac 53280

gttgcacccg gcgatgtttt tcttcagcgc gcgcggcagc agcgcgtccg cgcgcttgca 53340

gggcgcgtac cagcagtagt agggcaggcg cgtgtcgcgg ccggtgtcga ccacggcctg 53400

gctgggcttg aggcacgcgc agcgctcgtc gtccgggtgc gcgtcgcaga aggcgtaaat 53460

ctcctcgtcg ggcgcgtccg gcccgggcgc ggtcggcggc gccgcgcgac ggcggaagaa 53520

catctctgaa aaaatacttc gaccagaaaa cgaccaccga tcttatttca aagataaaaa 53580

tactattaat acgcactcgg agaatcatgt cggtggtggc gcgcgtgtcg tacagcctgt 53640

actcgcagag cgagataagc gccacggacg tggtcatcag ccagttgaag aacgacgagg 53700

acctgggcac ggtgaaggac ccgcgcctgg gcgcctcgga cgggtccata tgccgcacct 53760

gcgggctcac ggagatggag tgtttcgggc actggggcaa ggtgcgcatc tacgagtcct 53820

acatcgtgcg ccccgagtac atccccgagg tggtgcggct gctcaaccac ctctgcgtgc 53880

gctgcgggct gctgcgctcg cgcgacccgt acacgacgga cttggccgcg ctcagcgtgc 53940

acgagatgcg caagatgaag gaccggatga tgtccaagaa gaaggcctgc tggaacagca 54000

agtgtctgca gccgtaccag aagatcgtct tctccaagaa gaagatctgc ttcgtgaaca 54060

aggtggacga gatacccgtc cccaacgcgc tcatctacca gaagctgacc tccatccacc 54120

gcaagttctg gccgctgctg gaggtgttcc aggaccccgc gaacctgttc tacaaggagt 54180

acatgcccgt cccgccgctg ctcatccggc cggcgatcag cttctggata gacaacatcc 54240

ccaaggagac caacgagctc acctacctgc tgggcatgat cgtgaagtac tgctccatga 54300

acgccgagga gcaggtcatc cagcgcgccg tgatcgagta cgacaacatc aagatcatct 54360

cctcgaactc gagcagcatc aacctctcct acatcatcgc gggcaagagc aacatgctgc 54420

gcagcttcgt ggtcgcgcgg cgcaaggacc agaccgcgcg ctcggtcatc gggcccgact 54480

ccgcgctctc ggtgtgcgag gtcggcatcc ccgactacat ccggaacacg ctcacgcaga 54540

aggtgttcgt gaactacctc accagcaagc gcgtgcgcgc gctgttcgag gaccgcgcgg 54600

tcaagttcta cttcaacaag cggctgcgcc agctcacgcg catcaaggag ggcaagttca 54660

tcaaggacaa gatccacctg ctgcccggcg actgggtgga gatccccatg tccgagggca 54720

cgaacgtgat attcggccgc cagccctcgc tgcaccgaca caacgtcata tcctcgaccg 54780

cgcgcgcctc gcccggctac accatcaaga tcccgcccgg gatcgcgaac tcgcagaacg 54840

cggacttcga cggcgacgag gagtgggccg tgctcgagca gaaccccaag tccgtgatcg 54900

agcagagcgt gctcatgtac ccggtgacta tcttcaagca cgacgcgcac ggcgcgccgg 54960

tgtacgggtc catccaggac gagatcgtgg ccgcgttctc gctgttccgg caccagaacc 55020

tctcgctgga cgaggtgctg aacctgctcg ggcgctacgg gcgagacttc gcgccggagc 55080

ctggccagaa gaccttctcg ggcgccgacg tcttccgatt catgataggc gcggacataa 55140

acttcaaggg cgtgctcgag aacgggcgcg tggtggcgcc gaacgtcgac agcgacctcg 55200

tggtggccat gcgcgcaacc tcgctagcgg ggctgatcgc ggactacgcc acgaacgtgg 55260

agggcgtgcg cttcgtggac atggcctcct acgtgtacaa gcggtacctg gccatctacg 55320

gcttcggcgt gaccttccgc gacctgcgcc cggacccgag tttggttcgc cggctgcacg 55380

cgctgaacac cgagaagata gagcagatca aggacgcgta ctcgcggtac ctgcaggacg 55440

tcgcggacgg gaagctggtg ccgatggcgc ccgcggacga ggccgacgcg ctggactcgc 55500

tgctggccaa cctgaccaac ctcaacgtgc gcgagatcaa cgagtacatg cgcgagacgc 55560

tggagcgcaa ccccgataac agcctgctaa agatggcgcg cgccgggtac aaggtcaacc 55620

ccacagagct catgtacctg ctgggcacct acgggcagca gcgcgtgaac ggcgccgtcg 55680

ccgagaccaa gatatacggg cgcgtgctcc cgtacgcgtt ccccgactcc gcggacccgg 55740

aggcgcgcgg ctacatcatc aactcgctca tgaacggtct ctccggctcg cagttctact 55800

tcgcgatgct ggtggcgcgc tcgcagtcca cggacatcgt ctgcgagacc tcgcgcacgg 55860

gcacgctcgc gcgcaaggtc atcaagaaga tggaggacac ggtcgtggac gggtacggac 55920

agatcgtgag cggctcggta ctgctcaagt acgcggccaa ctacgcgaag atcccggggt 55980

ccaccaccaa gcccgtggag ctgctcttcc cgcacgagag catgacctgg ttcctggaga 56040

taagcgcgct ctggacgaag atccggcacg ggttcgtgcg catgcaccgg cagcgcctgg 56100

ccaccaagat cctggcgccg ttcaacttcc tggtcttcgt gaaaccggcg ccctcggagg 56160

cggaggcgct ctccgcgcgg gacctgtacc acatgatcca gcgcgtgatg aacgacgtgc 56220

gcgagaagta cttcttctcg ctggcgaacg tggacttcat ggagtacgtc ttcctcacgc 56280

acctgaaccc ctcgcgcgtg cgcatcacgc gcgcgaccgc cgagctcatc ttccgcaagc 56340

tgtaccagaa gctgaacgcg ctgctcggcg gcggcacgcc cgtgggcatc atgtccgcgc 56400

aggtgctctg cgagaagttc acgcagcagg cgctctcgag cttccacacc accgagaaga 56460

gcggcgccgc gaaggtgaag ctgggcttca acgagttcag caacctcatc agcatgagcc 56520

gcaaccacac cgagatagtg gcgctgaccg cgccgagcgc ggacaagctg atgccgctga 56580

aggtaaactt cgagttcgtg tgtctgggcg agctcgtgcc cgagatcgag acccggccct 56640

cgggacggcc ctccgtgcac cgcgtggaca tcacggtgca ccgcctgcgc atcaagcgcg 56700

cgcacctgac cgaggtcctg gtggacacca tcatcgagcg cttcgtgtcc ttcaacgtgc 56760

tcgtgaagga gtggggcagc gacatgaccg tggagggcga ccgcgtcacg tacacgctgc 56820

tgctgcgctt cgtggagccg gagcagctca acttccacaa gttcatgctg gtgctgcccg 56880

gcgccgcgaa caagggcaag gtgagcaggt tcaagatccc gatcaccgag accacggtct 56940

acgacgactt cgacgccgcg cgcaaggcct accgcatgaa catcgagctc atgagtctga 57000

aggagctggg gatattcgac ctcgaggacg tgaacgtggt ccccggcatg tggaacacct 57060

tcgacatatt cggcatcgag gccgcgcgcg ggcacctctg cgagagcatg ctggacacct 57120

acggcacggg cttcgactac ctgtttccct cctgcgacct gctcgcgagc ctgctctgct 57180

ccgggtacga gcccgagtcc gtaaacaagt tcaagttctg gaacgcgagc gcgctgaaga 57240

aggccacctt cggcgacggc cgcgcgctgc tgaacgcggc gctgcacaac cgcaccgacg 57300

cggtcgcgga caacagcagc tgccacttct tcagcaagac gccctgcgtg ggcacgggct 57360

actacaagta cttcgtgaac gtggagatgt tcatgcgcat ggagcgcgag atccaggcgc 57420

gcgtggcggc gcgcaagatg gaggagatcg aggaggccgc cgaggaggag ttctaggcgc 57480

gacggcgcct tactttgcga ccgtgtcacg acgacacgac acggttagga cggcgagtcg 57540

cagacgaaca tttttatgag ctggtagcgg aagttggcgt tttccaggaa ggcgccgcgg 57600

aggtcccgga tctcgtagta ggttttgagg aagtacacga agcgcgcggg ctgcgtcata 57660

gtcgggttct ccgcaagccg cttgtgcatc acgtacccca tggcggcggc gccgctgcgg 57720

ttgacgccgg ccacgcagtg cacgagcgtg ggcttctgct cggcctcgag gcgcgccagc 57780

agcttcacga gcgcgggcat gatggaagcg atgttcgtcg tgtcgtcgtc tctcagcgga 57840

atgtggtacg ccgttatccc cgcgggcgtc gagtacttgg acatggtcat gttaaccaga 57900

cacttgaagt cgacgccgga gtccccccgc agcacggcgc gcgcgtcctc ggcgctgccc 57960

aagtacacgt ggtccgtgag ccgcgtcatg cccgagggca gggccagcgg cggccccgcg 58020

cgcgtgcacc gcagcaggag cctggcgtac cactcgctct tatcgcccat atttatttat 58080

atgatacaaa tggcagacgt cacaacactg acggccaacg gtctgaccct ggagttcgcg 58140

cgcgagcgcg ctctgcgcag tctgcgcgcc gcgcgcacct ccacgctggt gttcttcacg 58200

ctcacgctcg cggcctcgct gttcgtgctc tggctgcagc taaccgagtt tcccgtcttc 58260

gaggagctcg gcaagtacgc gcgcatcaag agcgcggtgc ggtcctggcg cccgctggtg 58320

gaggctaaga cagagatcga gtccgacctc ggccggcaga agaccgccga ccggcccgag 58380

ctcttcgagt tcaggtgcgt ggacttcggc aagttctacc tgccggtgag gtacagcccc 58440

acgaccttcc tgccgcaagc cgtgcgccgc ggcgcgggcg atggctggat ggtgcacaag 58500

gcggcggccg tggacctcgc cgcgcagcag ttctgcgagt ccgtgctgcg gcaccgcgcc 58560

aacaacgtca tcacatgcgg gtcagagatg atgcggctgg tgggctacag cggctacttc 58620

gaggacgacc actggtgcgc cgcgacgtcc ggcgtgctga cgtgaacgat cacacgatgg 58680

ccgtgaccag cagcccggcg atgaaccaca gcagccgcga gttcggcagc agcagcacga 58740

gcaccagcag gtacgccagg atgaagatgt cgaccacgtc cacgtcgaag agccccatga 58800

aggagaagag cggcgtggtg aggaagtaga tggcgccggg ccagaagcgc gccagccacg 58860

tggcgagcag cgaccacagg gagggcgcgc cgctgagccg cgtcttcacc tgtatgtagt 58920

actcggggta gaccacctgc tcggcgccgg agagcaccac gcgcgccaga gagagccgct 58980

tctccagcgt gaacacctcg gtgagcaggc cgctgcgcag ccctccctcc ttgatgatcg 59040

cgtcgtagag cttcttcatg ccgccgacgc tgatgatgta ggcgtctagc gagacgtcgt 59100

acccgccggg gtagaccatg agctcggggt cgccggtgcc ggggacgttg gtggccagcg 59160

cgccggtcat gtaggtctcc ttgagctgcg tcatgtacca gccgttcgcc ttcatcgcct 59220

cgatgagcgg cttcaccatc tcgggcttgc ggaaggtcat gtcgttgtcg accaccagga 59280

tgaagtcatc gtcggagtac ttggtgggga cagtgccggc cgatatgctc tcccagaggt 59340

tgaggtggtg cgctgcgcgg cgctgcatct ccttcggaca cgtggacttg cacatgtccg 59400

tgaagaagtg cgggtagtct ttggagtcca cgtctttcca ttccaccgcc ttgagcacgt 59460

ggtcgccctt ggggtgcggc gcgggaggag atggcttggg tgccggcgcg ggggccggtg 59520

cgggggctgg ggcaggagag ggagcaggcg cgggttgagg cttgggcggg tcgtcggcga 59580

ggcccaccag gtacggcagc gtggggaaca cctccttggt cccgcggcct tcggcaaccc 59640

cgattatgta ggccgtgatt tcgggtggat ccatttagtt attaaaatta atcatataca 59700

actcttttat ggcggctatg gattcggcta tccagtcctt gaccgagccc acgatgcccg 59760

ccaggaacag gaagaaggcg aactccaggt ccacgcggtt cagagagtcg ctgaagtaca 59820

cgaagacgtc gctgtccggg aagaagctgc gccggaacat gttgtacccg ttgaccttgt 59880

gcgcgacgtg ctccgcgctc agcagcgtct cgtcgaaggg gtacgggtcg ctgaagcgga 59940

acacgtacat ggccgggttt gcgtagtagt acttcatggt gtttgtgacg aagaggctcg 60000

ccagcgagat gatgattttt ttcttctcga tctcgatctt gatgtggtcc tcgaagcgct 60060

tcatgttgta ggcgttggtg tcgtgcacgc ggatgagcac gcgcgagtcc gacatgatgt 60120

cctggaactc cgcgcgcgcg tcggggctct cggcgggcgt ctccgcgggc cgcgccacct 60180

ccgcgcacac cgtcggccta gcgcgcggcg gcgtgcgcat gggccgcgcc cccacgcgct 60240

gcgaagcgaa aaactccacg gcgcgagcct cgcccgcgtc cgcgtacgac tccaccaggt 60300

agttgcggct gcgcgtggtg cggccgatgg tgttcagccg gtgcagctcc gcgaccagcc 60360

ggcggtagtg cgcctccagc tcctcgggca tgatggaggt gtacacctcg gtgagcagca 60420

tcacggtgtc gaagtcctcc ttgccgcaga cgcgcgtctt cacgaggaag tggtgcacag 60480

ccgtcgcgat agagagccgc agcgtggact cggtgacctc gacgctggcg tccttggtct 60540

tcttcgcgct ccgcgaggcc atgaacgaga cgaggaagtc cgcgctgctg ttgagcacga 60600

tgaccagcgc gacgatgaag ttgaggttca gcgtcttcgc ggactggaac agctcggtgg 60660

ccgacgcgtg cacgtcgagc aggttcgcgg agagccgcag gaagaacacg ccgcgcttga 60720

tctcggccgc gaagcgacgt tcgtactcct gccggcgcgc gttgatcgcg atgaggaagt 60780

tcaggatgag ccggttgatg ttgtacttca cggcccaggt ctgcgtcttc atgatggtgt 60840

cgaaggacat cacgatgttg aagatgaagc gctggctgtg cgagaagtag ctgtagggct 60900

cgctgaggaa gatggacttg ttggtcgcgg gcaccaccac gcccgcgcgc gcgccggacg 60960

cgtcggtgtt caggtccggg atgttcatgc cgcagatgcg gcagtaggcc atgccgtcct 61020

caaagtacac gaactcctcc acgaactcgt tgatcttggc gaagtagtcc acgtccacgc 61080

gcatcgcgac cgcgagccgg atctggtgct cgcagggcgg cgactcgaag cgcaccccct 61140

cgccccagcc cggcggctcg cgcacgacca gcgcggtgcg cgaggccggg cggaacttgg 61200

cgtcgcgcgc gttgagcagc gccgggaaga ggtcgcagag gtgccggctc gagaggaaca 61260

cgtacttgta cagcagccgg cgcgcgtccg cggccatggc gtccacgaag gcgcggcccc 61320

actccgcgac cgcgggctgc tcctccgcaa agttgttcgg gtagaccttg tccgtggccg 61380

cgaggaacac cttcttcacg tcgaggaagt cgcggatcac gatggggacg cgcgcgccgt 61440

cgagctcgta catgaacacg tagcgcaggt tgagcttgcg ccgcgagacc gggatgccga 61500

tgtgccgaca caggtacgcg aactcgaggt acttcttcga gaagcggatg cggtccaggt 61560

tcttggagac gtactgcagc atgttgcgca tgttgaaggg gatctcgcgc acggcgggct 61620

ccgcggcgtc gtcgaaggcg gtgcgcagat cgctggtgcg ctgtacgacc acggcttcgc 61680

cggtggcgtc gtcgtgcacc agcacgttaa cgcgccgctg ccggatgacc atgtcgaagg 61740

tgttgaagaa catctcgtac atgctgtgcc gagtgtcgtc cgcgatgcgc tcgcccaccg 61800

agaggctcgc ggtggcgtcg tcacgcacct gcttctcgaa cttgtacccg atgtaggaga 61860

atatcgagat cagcgtggcg tcgtcggcgt cggggttctg ctccatggtc gcgaagagca 61920

ggcggatgtc gtcctccgtg atcgcgtcca cgttgtacag gttgaccacg aagatggact 61980

tgttctcggc gatgaagtcc gtgtaggact tggtggccgt gttcgggtcg cgcatgtacg 62040

cgcggatctt cggcacgatg ctcgcgagga tggactccct ggaatccatt taaggacggc 62100

aagggcgcgc gagaccgtct caaaactgaa atcgtataaa ctcttaaaaa atcggtattg 62160

aaagtacgca ccaccaaata aagcgtcgag gtcgggcatg tcttcgtggc gactcaaaat 62220

gagcaagtgt tcaggttcca gcagcgtcca gactctcgag gatctgcgta atcgtcttcg 62280

ctccgaggcc ttgggcaacg attgccaaga gccccgcgac gacctcttcc ccagcggcga 62340

ggagtgtctg gacatcgacg ggccctgccc ttgcgatgag gcggagcagg agatcgacca 62400

ggagcagttg cccgtgcccg aaaccgtgcc cgaaccgccg gccaagactc ctaagcgccg 62460

accagtgaag aaggataagg cagataaggc agataaggac aagtcgacca gaggcgcaaa 62520

gaaaccgtgc ccttcggacg acaaggatga cgagctcaag agcaacgacg tcgacaacaa 62580

cgaagagtcc ggcgacacag acggcggcgc gagcgcccga agccccagcg acatcgacaa 62640

cgtggacgaa atggacgact ccgacctcat ggtggcgttc tccaccatcc tcgcagactt 62700

caaggacctt acccaacgag tgaaagctct ttcgtccgtg ctcacggacg tgcaggcggc 62760

cggcatacgc aggagcttct cgacgctcgg caaggctctg acggaggcgg cccacatcgc 62820

caacaccgga tctaagccag tcactgcgcc tcgcaagaag aaggccgccg cctgcaagaa 62880

gtaggcgcac taaatagcga ggctcggtat gcgggcgctg cacctgtcag acggcaaact 62940

tttttttgac aaggagctga cgcagccggt ccccgacgac aaccccgcgt acgctgtcct 63000

tgcgaagatc cggatcccac cgcacctctc ggatgtggtc gtgtacgagc aggacctcga 63060

gtctgcgcag cagggcctca tcttcgtcgg gcgcgacgcc aagggccgaa agcagtactt 63120

ctacgggcgc ggacacgtgg agcggcgcac ggccgtccgc aacgccgtgt tcgtgcgcgt 63180

gcaccgcgtc atgaacaaga taaacgcctt catcgacgac cacctcgcct ccggcagcga 63240

ggccgaggcg cagatggccg ccttcctgct catggagacg agcttcttca tccgcgtcgg 63300

caagacgcgc tacgagcgcg agagcggcac cgtgggcatg ctcacgctgc gcaacaagca 63360

cctcgccgag gccgagggcg gtgaggagat ccgcgtcgcc ttcgtgggca aggaccgagt 63420

cgcgcacgag tttgccgtgc gcgagggcca gcggctcttc gcggcgctgc gtcggctctg 63480

ggacccgggc gcgcccgaca ggctgctgtt cgaccggctg agcgagcgcc gcgtgtacac 63540

cttcatgcga cgcttcggca tccgcgtcaa ggacctgcgc acctacggcg tgaactacac 63600

cttcctgtac aacttctggt ccaacgtgcg ctcgctggag ccgcgtccct ccgtgaagtc 63660

gctcatctgc acctccgtgc ggcagaccgc cgagacggtg gggcacacgc cctcgatctc 63720

gcgcagcgcc tacatggcca ccgcggtgct cgagctcgtc agggacggcg cgttcctgga 63780

cagagtcgcc gccaccgaca cgctcgacga cttcgtggac atcgtcgtgg actatgtaaa 63840

taactctgag caggtaaatg gatgaggcgc tgcgcgtggc ggcgcgcgtc gtggacgggc 63900

tccggccgct ggacgtggcc gtgtgtctcg cgcagctgcg cggagccgcg cccgagcgcc 63960

gcttcccggc gctcgacgag tgctccggcg aggccttcct ggactttgag ttcgccggcg 64020

gggacgtggc gtcgcggtac ctctccgcgc acacgcgcga gctccgtgcg gcggagcggc 64080

gcgagcacat ggccgcgatc gcgcgctgcg tcaccgaggc cgacctggcg ctcgcagacc 64140

gcccccgggg caaggcgcgc gcggcgctgc gcgtgtgccg caaccgcgag aaagtcgcgc 64200

gcttggcgag gctgctgcgc gacgccgaga gcagcggcgc ggacttcgcc ttcatacgcg 64260

cggccgtggc gtagcaaaac gtaaaaacaa cacattccct aaatcgccat ggacgcgcca 64320

agtctcgact gcatgctcgc cgcactcgcg gcgaaggcgg cctcggtgga ccgaggcgct 64380

cccgaggacg aggtgcacca cgaagtggag ctcgtgctcg tggacccgcc gctgtccacc 64440

ctggccgcca cgctgcgcct ggcctcggag acggagtcct tcatcctctt cacggtgacc 64500

gcgctcgcca aggaggaggg caagctgcgc gcgcgcgtgc ccatgtcgcg cgtcgtcggc 64560

ctggacgtga agaacgtgca gctggtcaac gccatcgaca gcatcgtctg ggagcgcaag 64620

gcgctcgtgg aggagaccgc gctgcaggaa ggctgtctgc tgcgccactc caccgagcgg 64680

cggcacctct tcgtggacta caagaagtac ctctcggcca tccgcgtgga gctggtaaac 64740

cgcgtgcgcg tgcgctccaa agaagtcgtc gcggacttca agttcaagta ctttctgggg 64800

tccggcgcgc aggccaagag ctcgctgctg cacgcactca accaccccaa ggtgcggccc 64860

tcgcccacgc tggagttcga ggtcgtcccc gcgggcgagg ccgtggacga ggccgccgtg 64920

ctcgcggagc tgcgcgccgt ggcgaaggcg ctcttcatgg cgcccaccga cgccgtcttc 64980

ctggcgccgc cggccgagat gccggtgcgc acgctcatgc tgcagaagca ggagatcccc 65040

gcgctagacc tcgacggcct tttcgcggtc tccaagacgg acggcgtctc cgcgagcgtg 65100

tgcgtggacg aggacggcgt cttctgcgcg ttctcgcacc tcgcgtacac catccggtac 65160

ccgctcgcgc gcgaagtgca gggccggtac cggctctggt gcgaggccgt gcggcccgtg 65220

ggcgagcgcg tgtggtccat gttcgtgctg gtcgtggagg agcctgcggg cgatgaccgc 65280

gtcgcggccg tggccggcgc cgtggaggcg ctgcgcggcg tgtgtgcacg cgtcgagttc 65340

aaacctaagc gcgtggacgg gcccttctcg gcgacctccg agctggtgga gcacatcaag 65400

agcgcgctgc agacggagcc agagggcgtg gtgctcttct acgcgcgcgg agagaagtcc 65460

aagcgcgacc tcaaggtcaa gcgcgacaac acggtggacc agaccacgaa cgtgatgttc 65520

cggtacatgt ccagcgagcc catcgtcttc ggcgagggct ccaccttcct ggagttcaag 65580

cggtacagca acgaccgcgg gttccccaag gagtacggcg cggggcgcat cttcctgcgc 65640

gaggacgtgg tctaccacaa caacatctac tgcatcgagt tcacgaagac gcacctggag 65700

gtgggcctcc gcagcgtggt cgtgcccgtg aagttcatcg gcgagttctc gcaggagggg 65760

tacctgctgc ggccgcgcct ggccaaaacg gagtgctact tccgcaaccc ctcattctac 65820

gggaaccagc actcggtggt gctcgagcac actcgcgacc agctgctctc ggtgggggac 65880

gtgttcgacg agagccgcat ggccgccgtc gggcagacgc tggccaacga cgccttccgc 65940

ctgaacccgg acacgcccta cttcaccaac cgacgcacgc gcgggccgct gggcgtgctc 66000

tccaactacg tgaagacgct catgatatcg ctgtactgct cgaagacctt cctgaacaac 66060

gccgagcgac gcaaggtgct ggccgtggac ttcggcaacg gcgcggacct ggagaagtac 66120

ttcttcggcg agatcgcgtc catggtggcc acggacccgg acgcgcgcgc gatcgagcgc 66180

gccatggagc gctacaaccg cctcaacgcg gggctgaagt cgcgctacta caagtttaac 66240

tacatccagg agaccatccg atccgagacc tacgtggaga gcatccgcca ggtcatgtac 66300

ttcgggcgct tcaacatcgt ggactggcag atggccatcc actactcctt ccacccgcgg 66360

cacttcgcca cggtgatgcg caacctgcgc gagctcaccg cgcccggctg caaggtgctc 66420

atcaccacca tggacgggga cttcctgtcg acgctctccg agaagaccag cttcgtgatc 66480

aaccgcaacc tgcaggagag cgaaaacttc atgtcgatcg agcgcgtggc cgatgaccag 66540

gtcatggtct acgcgccctc gaccatggcg cagcccatga cggagtacat cgtgcgccgc 66600

gcggacatcg tcaagctctt cgcggacaac ggcttcgacc tcgtggacca cgcgaacttc 66660

gagaccgtga tccggcgcag ccgccgcttc gtcgagggcg tctcgcggct ggagacgcgg 66720

ccctccacca agaacttctt cgagctcaac cgcaacgcgc tcacggagat ggacagcacc 66780

gacgtggccg cgctgctaaa gatctacgtg ctgtacgtct tcagcaagcg gtaggcagaa 66840

ccagggcgtc gattccgcgc ccgcgccggc gcggaaggcg ttgaacagct ccgccagcca 66900

ggctgcggtc tcgcgcgcgt cgatcgggcc gccgtcgtcc ggcggcggct cgcgcgccgc 66960

gcgcaacacc agcgtctccg cgggcggcag aggctccaga gcctcgaaga ccgcgcggct 67020

cgggaacagc gcgcgcatca tgcgcgcgcg gtggccgaac accgccttga ccgcgcgcag 67080

tgccgagcgg ttgtccagcc gcagcgctcg gtcaaaacga tgcacgcgcg cgggcgcgcc 67140

gcggtggtcg cgctccacga gcacgtgccg ccacgccagc gccgcgccga cgcggtccag 67200

gctgggcgcg agcgccacca ggcttttcag cgcatgtaaa tctccgcgca tggccgacgg 67260

ctccatttac tactgcggag gaacgcacgt ggtcgcggcc gcgccgggcg ccgcgcttgt 67320

ggtgctggac gcgcccggtg cggcggcggc ggccgcgccc gcggggcagc gcgtcttctt 67380

cgccgagtac ggcctcgaga agcgggccgg cggcccgatc acggcgcggc tgcgccgctc 67440

cgggttccgc ggcgccgcga acgcctgggc ctccgtggcg gacttcgagg ccggcggccg 67500

tccctccgcg tggacgctgc gcgcggagga ggcttcgcgc gtgccgctgc cgacggacgc 67560

ggcgctggtc ctggcctggg gcgcgcgcga ggagccgctg cgggcgtgcg tgctggcgcg 67620

cgcggcagac gcggaggcgc cggtgggcgc cgcgctcaaa gaagccgcct tcgacgcgcg 67680

ggcgccggcg gccgcgctgt tcgcggcgct gggcgcgccc gcgctcgcgc ccccgctgcg 67740

ggcgcggcta gtggcgccgc cgggcgcgcc gccgcggacg cggctctgcg agaacccggc 67800

catgctgcgc gcgttcgcgg tgggctggtt cggcgcgcag ctgggcgagg cctccgaaaa 67860

tgaaaaggta tttgccgcct ttgataaggc gaggtcgtgt ttggacgacc gctgatggcg 67920

acgcccgcga acgcgcccgc gctgctcgtc gcggcgctgc gacaccgccc gtaccgcgtg 67980

gagtaccacc cggactggga gccggtcatc gagacgctgg tggacgagta cgacgcggtc 68040

gcgccctggc tgctgcgcga cgcgacgagc cccgagcccg agcgcttctt cgcgcagctg 68100

gcgaagccgc tggcggacaa gcgagtgtgc gtgtgcggca tcgacccgta cccgcgcggc 68160

ggcaccggcg tgcccttcca gtccccggac ttcagcaaga agaccatccg cgcgatcgcg 68220

agctcggtcg cgcgcacgac cggcacgcag ggctacgcga actacgacct ggacgcggtt 68280

ccgggcgtgc tgccctggaa ctactacctc tcctgccgcg agggcgagac caagagccac 68340

gcgatgtact gggagcgcat ctcgcggctg ctgctgcagc acgtggccaa gcacgtgagc 68400

gtgctctact gcatggggcg cacggacttc cagaacgtgc gcgcgcgcct ggacgtgccg 68460

gtgacgctgg tggtgggctt ccaccccgcg gcgcgcgacg ggcagttcgc gcgcgagcgg 68520

gccttcgagg tcatcaacgc cttattggag ctcaacggga agtctcaagt ggactgggcg 68580

cgaggatttt ctttttatag tgaaaattaa tccgtggtcc taaatggcgg cgcccatatg 68640

cgataactct cacgtgttcc tcctcaagcg cctgggcgtg ccgtcttcct gccggcgctc 68700

ggaggacccg cgcttcgtgg agatcctgac tcccttcgag ctctcaaact acatcgagcg 68760

gcacccggga tgctgcctct tcgagacgct gcgcgacgag gaggactgct ccgtcgtgcg 68820

cgtcttcgcg gacgtggaca tggacagcgt gctcgaggag gaggacttcg tcgcggcgct 68880

ggaggacctc atcgtggagc tcgcggcctt cttcgaccgc ttcgcgagcg gctcctgcgg 68940

caccgtgccc ggcgaggtca agcgcgccat gctcgcgaac ttctcggtca cgcgatccac 69000

ggccgagcac aagaccagct tccacctgat cttcacggag acgtacacca cgctggacac 69060

gctggtggcg gcgaagcgcc cgctgctgga cctgtgccgg cgctcggaca acgtgctgct 69120

gcgcgcgctg gacacggccg tgtaccgccg cggcgcgacg ctgcgcgtgg tgggcacgcg 69180

caagacgccg gagtcgagcg cggtccaccg catgcagtcg cccgacgacg acatcaagga 69240

ctacctgttc acgttcgtgg agctctcgga cgcgagcgtg tacttcgagc tcgcggagcg 69300

cgagcagcac acgctgagca ccgtctgctg ggagacctcc tacatcccct tcggcgacgc 69360

gatgcggcgc gtgtgccagg cggtggtcaa cgacatcgtg aacctccgcg acatcaccga 69420

ggacaacttc ctcgacacgc cgctggtcat cgactacgcg acgcgctgcg cgctgtgcaa 69480

gaagcccaag cacaagcacg cgcaccacat caccatgggc aacggctgcc tgcgcctggt 69540

caagggcggg aacgcgcaca gctgcaaggt caagatcatc cagctcgagg gcaaccggct 69600

cttcacggcc gcgcagatca tcatcgcgtc cgaggtcgtg aagctcaccg agcgcaacga 69660

ctacatcgtg tggctgaaca actcctggcg cttcagcgcg gaggagtcgc tcatcaccaa 69720

gctcatcctg gacgtgcggc actcgctgcc cgcggactac gccaacgaca tgctgtgtcc 69780

gcgcaagcgc aaggtcgtgg agaccaacat ccgcgacatg ctcgtggaca tctccgagac 69840

ggacacgcag tacgacaagc tgcccttcac gaacggcgtg ctggacctgg ccacgggcga 69900

gttcctcacc ggcgaccgcg cgaaggcctg cgtgtgcacg gtctccaccg ggtacgcctt 69960

ctcgcgcgag gagttcgcgg ccgcggcgga ctcggaggcc atgcgccggc tggtcggcgt 70020

catcgacgac atccagccgg acacgcccga gaacgccgat aaccgcgcgc tgtacgagcg 70080

cgccatgtcc agcgcgctct gcggcgccac gaagacggtc atcgtcttct tctacggcga 70140

caccatgacc ggcaagtcca cgagcaagcg tctgctcatg tccgcgctcg gcggactctt 70200

catcgagacc gggcagaccg tgctcacgga cgtgctcgac aagggcccga accccttcgt 70260

ggccaacatg cacctgcggc gcgcggtctt ctgcagcgag ctcccggact tcgcctgcaa 70320

caacgcgcgc aagctgcgct ccgacaactt caagaagctg accgagccct gcatcgtggg 70380

ccggccctgc ttctccaaca agatccacaa ccgcaaccac gccaccttca tcatcgacac 70440

caactaccgc ccggtcttcg accgcgtgga caacgcgctc atgcgccgcg tggcgctggt 70500

gcgcttccgc acgcacttct cctcgtcggc cactcgcgcg gccgccgcgc acaacgtcga 70560

gtacagcgcg gtcaaggaga tggacgagag cctggacacc aagatccagc gcaactactt 70620

ccgctacgcc ttcctgcgcc tgctcgtgca gtggttcggc aagtaccacg tcccgcaggt 70680

ctcgctggcg cccacgcccg acgcggtccc cgacttcgcc ttccaccgcc gcgtggccga 70740

gctggtggtg gccagcaacg acgcgcaccg ccgcgcgatg gagtcgctgt ccaagctggg 70800

gtacgtgctc gtgggcggca acgtggccat gcccgcggac gccttccggc agcggctggc 70860

cgcgcacttc aacgcgcgcg tgcacggcgg cgacatagac gccttcatgt tcaagcacaa 70920

gaaggtcgtc aacgtaacgg aggagtacgt ggagtacgta ttcatcgaag atgtcgagaa 70980

taaatagacg ggtatgaact cggacgtgat aaagctgttc gtcgggcacg acgagtccgt 71040

gcccggcatc ctgccgcacc agctcgcgac cgtggacttc ctgatacgcc gcgttctaga 71100

cgacaacgtc agcgtgcttc tcttccacat catgggctct gggaagaccg tcatcgcgct 71160

gctgttcgcg atggtggcct cgcgcaccaa gaaggtgtac atcctggtgc ccaacgtgaa 71220

cgtcatgaac atattcaact acagcatggt catggtcgct aacctgttca acgcgccctt 71280

cgtggccgag aacatattcg tgtactcgac gactagtttt tattcgctaa actgcaacga 71340

cggcgtcata aactacaacg gcctcggcaa gtacgagaac tcggtcttcg tggtcgacga 71400

ggcgcacaac atcttcggga acaacaccgg cgagctcatg atggtgatca agaacaagac 71460

gcgcgtgccc ttcctgctgc tctcggcctc gccgatcacg aacacgccgc tcacgctcag 71520

cagcatcatc agcctcatgt ccgagaagga cgtggacgtc ggcgacatcg tggtgcaggg 71580

caagaaggtg ttccagatcc tgctgaacga gcacggcgtg cgcgtgatcc gcgaggtgct 71640

caaggggcgc atctcctact acgagatgcc ggacacggac atgcccgagg tgctctacca 71700

cgggcgccgc ttcctggaca cgcgcgtggt ctactgccgc atgtcgcgcc ggcaggagga 71760

cgactacctc accgtgcgcc ggctctgcaa caacgagatg ttcgagaaga acatgaacaa 71820

cgtgtccatg gcggtgctgg gcccgctgaa cctggtgaac aacctggacg tgctcttcca 71880

ggcgcaggac aaggacctgt acccgaacct gcgcatcagc aacggcgtgc tctacgggaa 71940

cgagctcacc aagctggaca tcagctgcaa gttcaagttc ttcatctcga aggtgggcgc 72000

catgcgcggg aagcacttca tctacttctc caactcgacc tacggcagcc tggtcatccg 72060

caacgtgatg ctcagcaacg ggtactcgga gttcggcggc tcgcagagca acaatccgca 72120

caccacgccc gacgggcgcg ccaagacctt cgcgatcgtg accagcaaga tgaaggcctc 72180

gctggaggag ctgctcgagg tgtacaactc cgcggagaac aacgacggtg gcaagctcat 72240

gttcctcttc tcctcgaaca tcatgtccga gtcctacacg ctcaaggagg tgcggcacat 72300

ctggttcatg accatccccg acaccttctc gcagttcaac cagatcctgg gccgcgccgt 72360

gcgcaagttc tcctacgcgg acgtggccgc gcccgtgaac gtgtacctca tggcggcggt 72420

gtactcggac ttcgacgagg acatcgtctc gctggaggac tacagcgtgg aggacatcaa 72480

cgcgctgccc ttcgacgtga agaagctctt ctacctcaag ttcaaggcca aggaaaccaa 72540

ccgcgtgtac gccatcctgc aggagctctc ggacgcgtac tccgcgcgcc cgcacccgca 72600

gctcgtggac gtggtgctgg gggagatcgt gcgccagttc ttcgcacggc actgccgcgt 72660

gcccgccgag gacgccgcgc tcgtggccgc cgtcgaggcc gttctcggca cgcgcgaggc 72720

agcggccgag tacatccgcg cgatagtgga cggacacttc ttcgtgacca acaagacctt 72780

cgggaagtgc ctgctcttcc ggcacgagcg cgacatcgtg accgtgccct tcgagctcga 72840

gcacgacccc ttcgcgtggg cgatcaactt ccgcaaggag gtcagtgtgg tgaatatata 72900

acggcaaaca taaatagaaa gactgtcctt ttgcgcgatg tcgaccttcc ggcagacggt 72960

gtacctggcg gtgacgctgc agccgcacga gctcacgctc gacttccgcg gcaacgtcgc 73020

ggaggcggtc atgcgcgagt acctctacaa ggagaagggc gggctcatgg ccaccgacat 73080

cgaggtctgc ctcggaaacg agatgccgct ggggcgcatc gtgaacaacg cggttgtggt 73140

ctcggtgccc tgcaacgtga ccttcaagta ctaccgcgtc ggcgacaccg tgagcggcac 73200

gctcaacgtc gaggacgaga ccaacgtctt cgtggactgc ggagacctca tctgccagct 73260

cggcaagagc tcgggcggcg tgaccttcaa cgagtccaag tactgcctcg tgcgcaacgg 73320

agtcgtctac gagcacggca gccgggtctc ggctgtgctg cgcgaggcgc gctccggacg 73380

cgagtccgcg ttcgtgttct ccgcagtgct gctggacggc gtccccgccg aggagaagga 73440

cgagaagaag gacgagggcg agaaggccgc ggaggaggag acgcccgcga gccccgccgc 73500

caaaaactag cattattggg ccgcgcgaac cttcgataaa tgcgcacgta cacgtcgctg 73560

ctctcgaagc tgctcaagag caaccggcgg ctcgggagca cgcgcgtctt ccgcgacccg 73620

ctgcagcaca tcagcgcgac cgcctttgtg caccggcgca tcgaccggca ccggcgcgtc 73680

tccatctgcg ccgtgctcac caccaccgac gggctcgtgg tcgcgtgccg gcgccggtac 73740

tcctttttgt cctccgagct cgcggagacg cgctcgcccg cgcggcgcgt gctgctcgca 73800

accaagcacg cggacgctct cgcgcgcctc ggcgccgcgc gcccgcgcga cgacgtcatg 73860

tttccgggcg gcgccccgct gtccggggag tcgccgctgg cgtgcgtgct gcgcgaggtc 73920

gaggaggaga ccgggctgcg cggcgaccag gtcagcgtgg acgagcggct gttcgtgcac 73980

gccttcatcg acgacctggt ctcgggccgc gacttcgacg cgatcatctt cacgggcgca 74040

gtcgcgcttt cgagcgcgga ggtggcgaag cagttccggc ccaacgacga ggtcaagggg 74100

ctggtcttcc tgcaccccga ggacgcggag ggcgtgggcg tgatggcgcg gctggcggcg 74160

ttcgcgcgct gcgcggcgcg cctgcgctgc tggggcgcgg ccgtcacgcg atagaggcgg 74220

ggtccaccac gtacacgagg cgcccgccgc tcacgcgcac ggtgggcggg tcgcccagcg 74280

cggtcaggaa gttcccgtcg tcgtcgaaga ggcgcccgcc gcgctcgagg aagcccttgc 74340

gcaccgtgac cagcgccgtg gaggtggagt accacacgct ctgcccgtcc gcgagccgcg 74400

ccgcgcgcgc gggcccgcgc gcgtccgccg ggcgcgccac cagcgcggac cagccggagt 74460

cgtcctccag cggcgcgaag tccgtgaagg cctcgcgcac ccactccagc gagcagcgct 74520

tgagcacgcg gaagagctgc gtgaactgcc gggacttgtc gcggatgagg gccagcaggt 74580

cctcgtccac ggtggcggcg ccggagtcct ggcgcgcgac cacgaagtgc acgttcacgt 74640

agcggcggtc gggcggcgtc atctcgtggc tgttcaggcg caccgcgcgg cccacgatct 74700

ggcgcagcga ggcctcgttc caggtcatgt ccaggatgaa gatgtcgttg atggagagga 74760

agctgaggcc ctcggagccg ctcagcgaga acacacagac cttgatcttc tcgccgtcgg 74820

tgttgtcgca ggcgttgaag gcgtccacga gcttggcgcg cgtgtcgcgc gtgcgcgagg 74880

agaactccac gctggagacg ccgaaggcgc ggaagtagag cagcagcatc tcgatgccgg 74940

tcacgttgac gaagggctcg aagaccagac acttgcccgg cgaggccagg atgcgcaggc 75000

agacctcggt gtacttgcag ctgcgctcgc gcagctccgc gagcagcgag acgtccgcgg 75060

aggtcatgcg gtcgccgctg acgggcgcgc cgctgcggaa gagccgcatg gccgcctccg 75120

agaagacgcg gtccttgacg gcgcgcgcga agtccaggaa gagcgcggcc acggcctcgt 75180

cgtactcctg cttggagagc acggacttgt cgggcgcgtc ctcgaaggcg aaggtggccg 75240

cgatgcgccg gtacacgcgg aagaccgcgg cgccggactt gcgctccatg gcggccgcgc 75300

ggcggtaggc ctcggtctgc ttcgcggtca tgtccacgta catcatgcgc acgcgcttgc 75360

gcgcgaaggc ggcggagccg tcgacgtcgt cgaagatgga ggcctcgttg gtgactaagt 75420

acgagcacag gccgccgagc ttgtccacga ggtcctcggg gttcgcgagc gcgccgccgt 75480

tgaagagcgg cgtctgcccg accacgccgg ggcgcagcag gttcacggcc atggagaact 75540

ccttgacgct gttcaccacc ggcgtggccg tgaggcagag cagcttcccg cggcccatgg 75600

ggatgttctt cgcgaggtag ttgtacaccg tgcgcgcggg ccgctggcgc ccgtcctcct 75660

tggtcagcga catcgagatg aagttgtgga actcgtcgat gaccacgcag acgcggctgc 75720

tcgacgaggc ggtcttcatc agcgtgaaga agcggtggtg gaagcgcggg tcgtcgtagt 75780

tgatgaaggt gcacccgggc acggcctcgg gcgcgaagcg catcatcgtc gaggtccagg 75840

gctgctccac gagcgccttc ttcacgagca cgaccaccgt ccagtccgtg aagacgtcgc 75900

gcaggtgctt gagcacgtac accgcggtca cggtcttgcc cacgcccgtc tcgtggaaga 75960

gcagcagcga gtgcatgctg tccaggccca ggaacacgcg cgccacgaag agctggtagt 76020

ccttgaggcg cacggactcc tccacgccct gcatctcgga gggcatgtgc gcggtgcgcc 76080

gcagcgcgta gtcgatgtag gccgcgtgcg cgctggtcat ggcgacggtc ggcgctcctt 76140

ttacggggtc tgtcgtctat ctattgtcgg cgcgggtctg atttaggggc agtagttaca 76200

aaaacgtttc cgctgctcgg cgcggcgttt ggaggagcgg ttgcggccgc ggcggcgcag 76260

gcgcgcgcgg cgcgtcttcg tggtgcggtg gccgaaccag cgccggtgca tgaccgggtg 76320

cgagaccgcg gccgcgcgat ccgcgctcat gcaggttgcg taggtgcggc acatgctgcg 76380

cagcacgcgc cgcgtgcgcc gctccacggc gtcgagccgc ctcgcgacga tgggaaagag 76440

ccggcgccag ccgcgcacgg cgaagagcgg gcgctcgcag accggcgcgg cgagcgcgtg 76500

gtaggcgccc agcagccgcg ggtccagcga gcgcacgtag gtctccacga agccgttgcc 76560

gaagacgatg gcctgtgcgc atagcgggtt cgtcatctcc ctcttggagg cgatggcgtc 76620

gcccacgaag gcgcgcacgc cgcagtgccg cagcaccagg cgccgccgcg ggaagtgcag 76680

gtgcgggccg agcgccgcgc gggcggcggg gatgtgcagc cgcggagaaa aacgcgcgcg 76740

tccctccatg gcatctaagc gctccgtctg ttttcagtta tatcgccgcg ggcggctact 76800

gcagcagcag cttgagcttg cgctggctct cgttctcgat gctcttggac tcggaggtca 76860

tgctctcgta gagcagcgag tgcgtgacgt agagcgcctc gtacacgcgg ctggcgaagg 76920

ccacgaagcg gtccacgaac tcgctctcta cggggtcctt gagcacgcgg aagggcacgg 76980

ccagcgcgtc gcgccaggcg gcggccttgg tgcgctcgcg cacgtgcgtc acgaaggcgg 77040

cgatggccgc gcgccgcggc tcgctggcga ccatgacggc gctgtccttg agccagctgt 77100

cgctcacgca cttgaagagg cgcacggtgc cgaagaggct gcagtacacg cgcagcgcgt 77160

gcaccacgtc ggtgccgaag agcgtgggca gcttgagcac cacgaagcgc tcctgcgtga 77220

tctcgagcag cgggcgcatc accgcgaagg tgatcgcgtg gtagtcagcc acgtagaggt 77280

tgttctcggt gaggtggttg ttggagcgga tggcgccgcg ctccttgcgg tagagccggt 77340

tgcgcgcgct gaggtcgagc acgaccgcgt cggccctgcc gcgcgctctg gagcgcacgc 77400

tggtgatgcc gtgcgcctcg agcaccttct cgacgtcgcg ctcgtcgatc atgaggtcgt 77460

gcgtgtacag actcagcatc tccgtgggca tgcggttgat gtcgttcacg cgcgagcact 77520

gcaggaagta gttggtcccg taggccaggc tgggcaggtg cccgacgccg agctgcaggt 77580

ccagcgcggg cgtcgagtcg aaggtgggca gcgtcacgct gagcccctcg cggatgctgc 77640

ggcgcacggc ctccaccgcg tccatggccg atttattgga cgcacagtct gttttcattt 77700

cgcggctact gcgcagtcac cttctcggcc acgatccccg cgtcgtagct gagccggtac 77760

acctcgttgc acaccacgac catctgccgc ggcacgtaca tgagcgggtt gtgcgcctcc 77820

atgtgcgcgg tggtcacgcg caccgccagc ttgtccttgc ccctggagac gttggagttc 77880

agcgcggtgg gcgagaagaa ggtgctgggc gtaaagttga actgcagcgt gcgcacgccg 77940

ggcgtcttgc cgaggatctc gccgaagacg cgcgagactg cgctgttctc cgagtacagc 78000

acctcgttgc cgaagcgcac gtccatgcgc gcgatgacgt cgatcttgtt cttgaagtcc 78060

acgcccttga ggaaggggtc ggccacgaag aggtccttgg cgcgcgcctc cggcgagcgg 78120

ttgtcgccgt tgtacacgtt gcgctggcag gtccacacgc ccacgggcac ggaggcgtcg 78180

ccgatgttca cggagtggat ggcggtcgtg aagcggatgc gggaggtcgc gcggctgtag 78240

gcccccgtga tggcggagaa cttcttggac atgttgtaca cgacggagtt cttcctggtg 78300

gcgaacacta ggatgttcgt gtgcaggaac acgcgcatgc ccacgggcac gtcgtcgatg 78360

cgcacgaaga cgtcggtgtc ctggatggac acgacgcccg acgggggcac ctcgactatc 78420

tccgcggtct cggggaagcc ctcggggtag cagttcgaga cgatcaccat gtcctccagc 78480

aggcgctcca cgaaggccat cacgaagtcg ccctcggact gctggaagcc ggggtacgat 78540

atgaagcggt tgttggcgtc gctgagcacg ggcttcatgt acacggacag ggaggtgcac 78600

gcgtgcacgt ccgtgatcac cgcggtggtg tggttgatct gctccacgcg ccgccgcggc 78660

atctcgatga aggccgggcg cgggcacagg ttcttgacca tgtagccgat gaagctcagc 78720

tccatggagt aggggaactc cttggcgagc ttggcggcgt cgaaggtctc gtcgtagacc 78780

atgacgcagg cgacggggtt cagcgtgacc gtgaccgtga ccttgctgtc gctgagcttg 78840

agcgtgctga aggtcttgtc cgcgtcaaag ggcgtcttga tgtaggcgtg cacgcaggcg 78900

gcctccttga tgacgtcgtt gggcgagctc ccggtggaga ggtcgttgag ctcgcgcgag 78960

aagcccgaga gctccatcac gcgctcgttg tccaggcagg agtcgaacag ctcctcgccg 79020

gaggtctccc agatggtgtc cgcggcggag ttcacggcca cgtggcggat gagcttgtac 79080

gcgatgtagg gcacgtagca catcttgccc acgcccttta tctcgggcag gtccacgctc 79140

agcacgaagt tgttcatggc cgagatgtac ttgtcgcgga tctcgaaggt cacggtgacc 79200

gcgtcgctgg tggtgtccac cacgccctgc gtggtgatgt actgcggcat gtacaccgtg 79260

ggcgcgcggt ggtccgtggc gaacacgctg gcgcgccgca cggcgtcgtc gccgcccacc 79320

aggctcacca cggagttatt catttattcc ctgggaaaac cagttaaata aggctcttca 79380

gagccatgcg caccgtccgg ccgtcgggct ccaggtagca gcgcccgtag acgccctccg 79440

tggcgcgcgt ctcgttgatg agcgcgcgca cgcggtcggg gtccgcgtac atctccagcg 79500

gcagcagctc gatcttgggc tcctcgcgca gcgcgacgag gtgccggatg gagcccgcga 79560

aggagtcgcg gcacaaccgc gagcagaact cgcccacggc gccgccgtcg agcgtctcca 79620

cggccagcgc ggccgtgccc acgcgctgac ggcagaacca gcacgtgccg tccgcggcgc 79680

gcagcgccag ccgctccgcg gacaccgtgt tgaagtactt cggcagcacg tactcgatgc 79740

ggcacgccgc cggcggcgcg caggccgacg cccgcggcgc ggatatgtcc acccgcgaga 79800

gcgcgatgcg cttcatgggc ggcggtggct gctatttatg tcgcccgcgg cttttcaaag 79860

gtcgagcgag cacgccgcga agcgcgcggg cgagaacacg tactcgtggc cgaactccgg 79920

gatctgcgcg gcgcgcttgc gcgcgcgcat gtgcgcgagg aagttctccc aggtgagctg 79980

gttgctgttg ttcttcgcgt agttcttcac ggtctgcgga cgcaggttgc gcgtcacgcc 80040

cgtgacctcg aagatcttgt ccaggaagaa ggagtagttg atggttttgg tgggcgtgat 80100

ctcctggcag aagaagacca gctgcttgaa tatctcgatg acctcgttga tcttctcggt 80160

gctgaggtcc agcttctcgt tttgacctgg gttgatgatc tcgaagacca gcttgtagtc 80220

cttcttgttg atcatttcgc tgtccttgag gaagctggag acgtagttgg cgtccacgtc 80280

ctcgggccgg atctggtgcc ggtccatcat cgcgcgcagg tcgcggatga cctcctccga 80340

gcactgcttg gagagcagcc gccggagcac gttccgcagg tggatgagct tgttcgacac 80400

gtggaagttg gacctcttct gcacgcggat gcccatggga aacacggtct cgcagaacag 80460

gcagaactcg tagtccgcgt cggacacgag cccgttgcgg cggcagccgc cgcacatgcg 80520

caggttcatg cttgctccag ccccagcacg cgcaggatct cgcggtccag cactttagtg 80580

tccagcgtgc gggttctaca gaactggagg aagcccgcga gcgcgcgcgc gcgccctggc 80640

tgcgagagca gcagcatgcg cgcgttctcg gggtcctcgt tgatgacgcg cgtgaggttc 80700

agcgagcacc gcgtgcagcg ccgcggcggg tcgagctcga ccgagtacgc cgcgaaccag 80760

acgttgtcgc ccatgtatta tttattaaca cagaacgtcg cacatgttgc gcgaggacat 80820

gtacgggtcg tactcctgcc cgtagatgag gatggtgcag taccgcgaga tcatgagcat 80880

ggcctcctcc atggtgagca ggtcgtcctc gaacatggcc ttgtgctgca tgtgctggct 80940

ctgcttggcg gccatcgcgg ccgcgccgtc gccgcgcagc cactcgttca gacactggcc 81000

gtcgccgccg gcgccgctct cctgcgcgaa ggtgttgcgc atggcgcgca tgagcctggc 81060

ctccctggcc gagcggctga gcacggtcat ggggtcgtag agccaggggc ctgcgtccgt 81120

gaacaggatg gtgcagtacc cgttggcgaa ggcgtccgcg ccgctgcagt tgtcgatgcc 81180

gtcgccgacc ctgtagcaca ccgcggagac cagccggtac atgatgccgt tgagcatcat 81240

gtcctgcgac acctcgatgg gaatgtcgct gatcacgggc cgcatgttcg tgaagcagtc 81300

gcccgtgctg gccatgcccc cgcgccggtt caccaggaac accagcacgc cgttcgtgat 81360

cacgggcgcg cggtcgcgct cgtagaggta gccgctcgcc gcgcacacgg cggacgccac 81420

gtccgtgcgc gagaccgcgc ccatgttctg ggcgggcatg tacagcactc gcccggcctc 81480

cgcggtgcac gagaagggct gctccccgcc cacgtggatg ggcgccgtcg atgtcgtgat 81540

catcttgctg gagtccacca ccaggtaggg caccgtgtgc atggccatgt cgcccatgcc 81600

cccgatcccc gttccgaacg acggccgcga cacgctcacg agcgtcggct tgaacgagac 81660

tatggagaag atggaggcca agatctgctc ctcgtcggtc atgatggagg cgcacgaggg 81720

gtggatgatc ttcattaggg cgttgtcgat ggactcgtcg ctctcgcagt agaagacgcc 81780

catgcggagg ttcaggatgc accgccgcag gttggtgtgc agcaccgcgc gctggatctc 81840

catggacacg gagtcgccga cgccgggcat cacgatgggc gtttcctcgg tgagcttgtt 81900

caccagcagc tggtagttgt tgggtcgcac gcggctgttg tggtggagct gcgccaggag 81960

cgagaggctg tccccgttga cgaacgcgga ctcgatggcc ggcagcttta cgccgaacag 82020

cgccatggcg atggggtgca cgaagcccac ggagtcggac gacttgaacg agaagagcag 82080

gtcgcccgag gagctcatgt ctttgaagtg cacggactgg aactgcgtgg cggagagcaa 82140

gttctggtag ctggacatgc tctgcaggtc gtcgatctcc ttcatctgct tgtttacgcg 82200

ggtcgagttc ctcccgtaga tgatcaccag cgggtgcgtc tggctcacgg atagccccga 82260

gtccgtcatg gcggcgcgca cgctgttcag cagatcgaac agctccgacc ggtctctgtt 82320

ctggatcccg acctttgcca tcacagacat gagctcctgg atggtcatgt tcttgtggtc 82380

tcggcgcgtg gaccgcaggt agtcggcgat catctcgccc tccttacgga tcttcatctg 82440

ccagtcgtgc atggaggtca tgcggtccac aggcatgagc acgctgtcgg aggacgactg 82500

cgcggcgctg ccggactggc gcgagccagg gcgcgcggac gacgggcgcg cggagctgcc 82560

gcggctggag gaggacctgg acctccgcga ggatcttcgc tgcgaagagc tgcgcacggg 82620

ccgctgggcg cgcgccccgg cggaaaccat gtcctcgcgg tttatgctga ggagcgagct 82680

gcagaccgcg cacgacagcg actgctttgg aatgtggatg tggtcgcact ccagggacat 82740

gcccgcattg tcgtagcccg ggaccaagtc gaactttgca ttaaaaaaat ctgatgcgca 82800

cgcgggcgat tccatttata ccggaagttt ttatgaggtg ccggtattat ccacgcgatc 82860

tcgcagtgtg ctgggagact ctagcgtagc cacggacccc gtgagcagac gacgcaagtc 82920

gttgatggcc gactgcgtga cggacttcgc cgtctcgatg tcgcgcgtaa ggctgagcga 82980

ctcggcgttg aggtcgcgca cgctgtccgc gatgtcggcc agctcctttt tgatgaaatc 83040

cttatcatta tcggcgttga tgactttgtc cggcactcta gactctagaa ccggtgacgc 83100

ggcgggcgcc ttgatcgtcg ggcagctgga cgccgggtac tggggcggcg gcaatgattg 83160

ctgtaggaag atgggcttag cggcaggcgc cggttttgtc ggcaagggcg gcgccggcgg 83220

gcactgtcgt gtagacggcg ggcacgccgg cgcggggcac gccgccggtg gcggacatgt 83280

cggcgcagtt gcgggtggac acgcgggcgc gggcgccggc gcgggacacg tcgcggcagg 83340

agccgggcac gcgggagccg gagctggagc cgggcacggc gcggcaggag ccgggcacgt 83400

cgcggcaggc gcagggcacg cgggagccgg agccgggcac gccggcgcag gaggacatgc 83460

cggcgcagcg gcaggacaga ccaccgctgt cgcggagcac gcggcaaccg gcgtggagca 83520

cgcggcgggc attacgttca gaggcgtcga ctggaccttg gcaccgcggg gcagtagcga 83580

tggcgctagg ggcgactgtc cggtggttgg acgctgcatg caggcagtcg cagatttcag 83640

acgggactgg taatacctgc ccgcttcctt caccgtgtac ttgtcgacgg agtctacctc 83700

ctcatctgga ggacatggct gctccggtgc gggaacgact gtttgaggac acttggtgaa 83760

cagactggag ctggtctccg atagcaccag tttagacttg gccaagtcgg aggcaaacct 83820

tcttctgaga tccatttaag ccttcaaaat tgaacgtgta cgccgaccgc taaatggaag 83880

aatcggtggc cgtcgagtac gcggacgagg acgaagatga gattgaggag tacgaggagg 83940

aggacgatga cgaggaggaa gagtctgccg agggcgccgc cgcctcctcg gtcagcgacg 84000

tagcgctctc tgccgccgag aagctggtgg cctcggaggt cccggacgac gcggctgccg 84060

cggacaccaa cgtgcgtcaa cgcgtcaccg cgcgcgtgga ggagcttaag gcgcgctaca 84120

cacggcggat gagtctattt gagctcaccg gaattgtagc agagagtttc aatcttctgt 84180

gtcgcgggcg gctgccgctc gtggcggacg ccgcagaccc ggcgctcgac aacgagctaa 84240

aagtggtggt tcgggagctc gaggagggcg tctgccccat cgtcatcgag aaaaacggcg 84300

agttcctctc gccgggcgac ttcgaccccg agtgcctgcg ctaccacctg acgtacatga 84360

ccgacctctg gaagtcccag gggcgcatgt agccgcggct acgccgactc ggcggcctcc 84420

gcgatttttt cttttatcat gtccagcagc tcgcgcacca cgatggggcg gccgcagtac 84480

gtgatgccgt tttcggatat cacgctctgt gcgatgtcca ccagcgagcc ctcgcgctcc 84540

cagtactcgc gcgcgagcac ctccttgtac aacgcgcggt ggttggccac gtaccgcacc 84600

agcgtctgga tgttcttcac gcccacgctc ttgaggtcct gcggcgagaa cttctcgcgc 84660

agcgacacga agacgtcccg gacgagcttg ccgatctcca cgttggtctt gaactcgttg 84720

tacagcacca cgtagagctt gcacacgacc gtagcgaact tcgcgggctt gagatccttg 84780

ttctggaaga ccagcatgct gctcatcacc ttcttcatga aggccaggta cttcgcgcgg 84840

tcgccctcga cgctcacgct cccgacctcg aggtcggaca cgcagcggat tccgtgctcc 84900

gcgctctccg cggagacgcg cagcagctcc tggtactcct tgagcttctg cttgtccgtc 84960

atcagcgagt tgtcgaatac cgccaccagc ttgagcacgt agttctcgtc cgagaagacc 85020

ttgttcagac acttcaccag gaagctgtag tggctctgca ggatcttcat gaccgcgttg 85080

gcgccgctgg ctccgcggac gtgcgatatc atctccatga tcttcttaga gtcgtcgatg 85140

atctcctcgg tgtcgttgcg catgttgcgg tacattgcgt tcagcgagac cagcgtctgc 85200

gcggccagga gcacgtcgcg gaacacgcgc gcgaactcgc gcttctcctc cgcgtcggtg 85260

atgctgttgt acacggactt cgccaccgcg ttcgacttca ggaaccagaa ggagagcgcc 85320

tggtagttga agtgcttcat cagcgccagc acgtccgcct cgctcatttc cggcgcaatg 85380

gggcacaccg agctctcgag cacgggcacc atgctgacga gcgtgtccac gtccgtgtcg 85440

aagtccaggc agtccacgca gagcccggtg ccgcggctca ggtgatcgcg gctgatgtcg 85500

tagaagcgct cgtagcaggt gcggaggcgg tccatgtcgg ctgcgtttta gagagacaca 85560

cactcttgaa ttatggctgc gggtagaact cctgcagcag cgccggcgca cgcgcggagt 85620

ccggctccac tcccagcttc agcgcgcagt tcacggacca ggtcttcatg aagcggtcgg 85680

gcgcgtccgt gaccacgtgc cggaagagct tcgcgaagtg gcggctcacg gcgttgggca 85740

cggtcgcgtt gcgcacgaag gccgtgaagc gcgaggtcag cttcggcgcg aagcgcttgc 85800

cgtccacgaa gaagcccgag gtggtgagcg agagcccgtt ctcctcgcgc accacgcgtc 85860

gcgcggcctt gtgcggaaac atgctcgcga ggcgcccgct cgcgtcctgg tctaggtgga 85920

tggcgtccgt ggccgcgtcc ttgcggatgc gcaccacgtc gtgcacgatc tcctggatga 85980

ggatgcgcgt ggccgcggtc tccgccagcc gcatggggaa gtagaccatg tccccggaga 86040

tgagcacgtt cccgctagcg tttacgtagc tcactatctc ggacacggtg cgcagacgca 86100

cgatcgcgcc ttcgcagcag tgcaccacgt agtacccagc ggtggcgcgc aggcgcttgt 86160

tgtccgcctc gaagtccgcc tccaacccct cgttgaagta cttgtcgaat atgatgggca 86220

ggaaggatag ttttgactcg gtgaccacct ttccgaagtt gaggatgtac gggttcagcg 86280

cgctgcggtc gacctcttcg tcgtacacgc aggacttgaa ggtgtcggtg tgcgcctggc 86340

tgcgcaggaa gcagcacgga atgcagatgc gctgcaggcg gtggaagatg gagaggaagc 86400

ccacgctgtt gtagcgcccg tcggggtcca tgcacgaaaa catgacgccg tttccgttta 86460

cgaagacctc gcgcgtctcg gacttgaaga agttgttgct gaccttggcc atgttcgcgt 86520

ccagcgactg cacgatcacg ggcttgcggt tcttggtctt ggtgttctgg cagatgcgcg 86580

accagtacac ggtctccacc ttggtgaagt ccgaggactg cttcacgttg ttgaacatca 86640

cgctgatggc cacgatcaag aacgtgaagt acttctcgat gttcgggatg tagttcttta 86700

ccttcacgga cacgtgcgac ttcgcgagga tgatcgagat gcgcttgtcc gtggagagca 86760

ggatgttgtt ggtcgccgtc tccacgaaga tgaagctcgt ttccatgtcc agcttcatct 86820

tagacgtgat ggtcgtgttc agcgacacct tgtaggtgat gtcgcccttg acgcggtcca 86880

tctttacgtc catgctctcg atgagcttcg tgaacagact cacgtcgttc accgtgagcg 86940

tcttcccgtc gctcgagatg accaggtcgc cttccgggcc ccacaccgag aggttcagcg 87000

gctcgtccac cagcaggaag cgagtccccg tcatcgacac gaagaagtcg tccgtcttcg 87060

agagcaggat gtcgaagtcg cccacctccg ctcgcttgtc cggcgactgc tgcgcgatcg 87120

cgcggagccc ggactcgcgc aggttcgtgc ggaagatgtt gttgaacttg gtctccacgt 87180

tcatgtttag gtcgaggttc gcgaactcgc ggatgagccg ctcctcgaac ttgaggatgg 87240

agtcgttggg ctcctcgaag gagccgaact ccggcgcgga ggtgtccgcc gcgcgcgcca 87300

cccagaccac caggaagttg cacgcgtccg cgtacgcgtt gtagaggatg ccgtccgtgc 87360

ggatgagcgt tttcttttgc gtgggcgaga acgggttgaa gatggtgttg tccacgtagc 87420

tgtactccag gttgttcttg tgcgagtaca cgatgatctc gtcctgcagg cccagcaggc 87480

tccccaagta ccccttgagc tgccgcacgc gcatggtcag caagatgtgt ctgcgcacgt 87540

gctcggggtc cttctggatg tactgcttcg cgaagaagta gatcggcgag gcctcgtcca 87600

cggagtcgta cagcgatagg tacagcacgc gctcgatctc ctggtggcgc cccaccagca 87660

ccaccagctg cggcgcgacg gtgtagagca tgttcgcgcg ggcgtattta tagccggcgt 87720

taaactgaaa taaaatacgc gggtcgcgag gcagcgccat gttccagccg gtgcccgaca 87780

tggccgccga ggccgacatc gacctcggcg acgtcagcgt ggacgcgacg cgcgcgggcg 87840

cgcgcgagaa gaccgtcttc ttcgcgcgca acaagcgcat gtacccgcac cgcagcaagg 87900

acgaggagcg caagctgtcg ctgggcttct tcttgcagcg gctggacttc ctcacgtcgc 87960

gcgaggtcaa cctgcagttc cggtcgctgg acgcgctgcg caccgagaac gtcatgaaga 88020

agaacaacgt gctcgtggcg ccgtacatcc tcatcgcgac gctcgcgggg cgcggtttcc 88080

gcatgacgga gaccatggtc gagctctact tccccgagct gtaccgcgag accagcaagc 88140

gcttccgctt ctgcgcgcag ataaaggtca tccaggactt cctggggttc gcccacgaca 88200

gctaccacac ttacgacttc gagacgtact tcgcgttcgt ggcgctggtg ctgcgcggcg 88260

cggactctgc ggccgaggcc ttcgacgtcc gcgccgagag cgggcttgtg cgcagcctca 88320

ccgagatcac gtaccggctc tacgtgatgc agctgcgctc cgacgccgcg cagtggagcg 88380

tgagcaccgg cgccgtagtc tcgcaggcgg tgaacaccgt gctgtcggtc gtcggcgacc 88440

tcgctgcgcg cgcggaggcc gagcggctca cgcccgtgtg cgacctcgcg cgcgagaacc 88500

cgctctcgct cgaggacctg cgcaagtacg gcccgcggct gcgctcgctg ctcacgacca 88560

tggcgcgcgc gcgatccttc aagacgaacc ggcgggacaa ggacgcgctg tcccggttct 88620

gccgactgac ggcgggccct agcccgtctg cgtgccgcgc gtcgccatag gcgtcggcgc 88680

gcgctcgccg ccggaacact cggggtcgct gaacatgtag atgagcgcga cgcctagcag 88740

caggtacatg atcatgctga tcacggtttt gaacacgacg gcggcgaacg tgttggaccg 88800

cagtcggtgc tcgcagaagt gcatgaacag gtgccgcatg aggtcgatgg ccccgttggc 88860

cacctggaaa agggcgaggc cgccgatgga cttgatcacc gtcacgtagc acggccgcat 88920

tccgacgacg ctatttactc actgtcaaaa gaaacggcgc catccgaccg gaggttgagg 88980

ttgcgcttca tgttgttcca gtacatctca ccgatgctcg agtagtacgc cgtcagccgc 89040

gatatttttt ctcgcaccag ctcgtaggcc ttctgcatct ccgcaacgcc gatctccgcg 89100

tcgcccacgt accggccgct gcggcgcacg atcagcagca gcgccttcag gttctccagc 89160

gcgatcatgt ccatgtacag cgacttcgag agctgcacga agaggttgta ccgctccagg 89220

atgctgttct tcacctcgtc cgcgatcggg actccgaaga tgcgctccgt ggtgtacacg 89280

gactgcgtga gctgcttgaa gagcgcggag atgcagcagg tcgcgcgctt gacggcgtcg 89340

agctgcttct cggagcgcgc gctcgcgatg ctcagcgcgc tgttcacgac gttgctcgtg 89400

tcgcgcacgt agcgcgtctt cagcgcggcg ttgatggcat ccgcgatctc gttgctgctc 89460

acgctcgagt cgtccgagct gcccgagacc tcgtccagca gccccgagat cgtgatgtcg 89520

ggcgagccgc cgacggtcac cagacggtcg agcaggttgc agggcatgga catgaggatg 89580

ccctcgctcg agaggcagcc ctcgtcgatc atgctctgca ggttgcgctt gaaggccgtg 89640

ttttcgggca tgaacccgtc cacgctcatg agctcgtcga ccgtgctcgc ggaaaagatg 89700

cccctcacgt tgatgcggtc cagcatgccc atgtcctgcg agcacagcac caccgactgg 89760

tcggccgtct cggcggcgtc cttggcgccg ccgtagatga tgcgcggaaa ccgccagctc 89820

gccggaaaag agaaggaggg aaaccggcac tgcgcgctcg ggcctcggta gccctgcgcg 89880

tcgcgcacgt tggtggccgt gaccatgaac tgcagcaggt cgtgcgcgga cgccatgatc 89940

ttctccacct cctccttgct gcagcagacc ttgcccaggc tgcgcgcgat gttcgttttg 90000

ctcactgagg gcgagaccgt gacggcggtg tgtcggcggc tgccgagcgt gtacgcgctc 90060

acgctaacgc ggtaccccat ggcgccgaag agcagcttca cgaagtccag gtagctctcc 90120

ttattgatgt agtgcggcgc gcccttgtct tccatcctca gcccggcgta ggccatgagc 90180

acttccttca tcgccgtctc ggggtccgag ttgcacacca gccgcagcat ctggaagaac 90240

tgcatgaagg cgcgctgcga gagcccgatg tggtggttgg gctgcgtcga ccggcgcggg 90300

aactccctgg gcgtcatggc gttgatgccc gagagcgtct ccatcacgag cgcgcccacg 90360

gtcttctggc ccatgacgcg cgggtaaaag cacacgcgga ggggctcctt gccggccgcg 90420

agcgcgtccg agagcagcga gcagtacgtg acgttgtcgt ggtcgaagag cgcgaaggtg 90480

tagcagacgg agctcatgaa gagcgagtcg gcggtgctca tggacttgaa ctccgtgtac 90540

gcgattccgt cccagaacag gctctttccg ggcgcgatca gcggggacgc gcggtcggcg 90600

cgcatcagca tggagagcag cgtcacgtag taacggatgt tggcggaaat gtctacgaac 90660

tgcatgccgg gcgaggccac gcgcagggtc gcgcccgagg tagtgagcac ctccaggctg 90720

tccatgagcg tcacgctggg gtgcagctgc gcaaggcgcg ccagctggct ctggtagaag 90780

atggacacgg cgaggctggc cacgctgccg cgtgccatgc gcaggttttg cccgttgaag 90840

gtgagctggc gcagcgagaa cacggagtcg aagtactgga agaaggtgag caggtacttg 90900

agcggcatgg tcgtcagctc ggtatccacc tgcggcgtct gtgcgagcac gattccgttc 90960

ttggccgcgg cgtcggggat gtcgtacatg gcgtccattc tggcgcggga ggcgtcggtg 91020

agcagcgcgc gcacgttgag cagcatgagc aggtcccgcg ctagcatggt cccgtcgacc 91080

agccgggcgc gaaagccgat ctcggcgggg ccggcgatgt tggggtagat caggttcagc 91140

aggtacgtgt tgtcgaagct cagcgagggg aaggagatgg gagacttcgc cgggaggccg 91200

gtggggtagc gcacgtagcc gccgcagatg cgcgcgtgcg cctcaaagct ggtcacgcga 91260

gtcttcagca ggttgcgggt gaagggcggc acgtccttga aggactgcgt gcagatcacg 91320

gggttggcgg tgtcggtcag cttgaggttg gtgggcttaa gctccgcgaa gttggggccc 91380

agcagcacgg ggacgaggtg cgagttggcg gcgctgtcga gcaggaagtt gatgccgaac 91440

tgcttcacgg cgacctcggt ttcctcgtcg ctggcgagct tctccgcgtc ctcgaggaag 91500

agcgcgtcca gcgggtgcac gtacgtgcgg ttgacgtcgt agctgggctt gaagtccgaa 91560

cacagcgtgg ggagcacggt ggagacgagc tggaacatgt attccgcgcc ctccacatgg 91620

tgcaaggcca tgtgcacgtt tggggccgtc atttatttag tattaaatga cggccgtacc 91680

ggtaaccgat attcctggag actacggccc gacgtccttt tcggaggaca actacccgct 91740

gaacaagcac tacgagctca ccaaaggcca gctctcgatc ctgcgcacgg tcaacgacaa 91800

gctgctcgcg cgcaccgtgc agcactcgga cggggagagc gatgagagcg aaagcgagga 91860

ggacgacatc tccagtccgc tgccgccgga cgaggaggag ccggactcgt gcgtggcccg 91920

ggtcatgccg cgggacgcgg acctggcggc gccaaaaaag gccgacggct acatcattgc 91980

cgccgagcag cagcgccagc agcgcataaa cattctggta tccgatcgag aggccgtcgt 92040

ggagcgggag ccggttcaga cgtcgttcgc gcgcgtctcg gctatcccga tccacgggga 92100

cggcgcgcgc cgcaccaccg cctccttctc cgcgaccacg ccgtcgctgg gcgccgtgtt 92160

cgacgacgcc aagcgcgtgc ggctgctgga ggaggaggtc aaggagctcc gcagaaagtg 92220

cgcgacctct caggataacg gaaacctgga gaacttcacc aaggtgctgt tcggcaaggc 92280

gccgcgcgcg agcgagctga acaagcgcgt ggtcatcgtg aactacgcca cgctgaacaa 92340

cgtgacgctg tccatggagg acctcgagaa gtgctccgac gaggaagtgg accgcatgta 92400

ctcggtcatc cggcgctaca acgagacgcg gaagaagaag atcctggtca cgaacgtggt 92460

catcatcggg atcaccgtgc tcgagcacgt gctggtgaag cttggcttct cggaggtgcg 92520

cgggctcagc gccgacctct cgtcggagct catcgacgtg gagatcggcg aggactgcga 92580

gcacatcgcg gagcgcctgg ggttcgggaa cagcccggtg ctaaacgtgg cgctcttcgt 92640

ggtaaagctg ttcgtgcgga agctgaacct gatctgatca acacatgccg ccgtcgaggt 92700

ccatggcgtt catgaggttg gaggcgcggc ggcgcgcgcc ggtggaagcg gtggaggcgc 92760

tcgaggtcgt ggagcaggga gtgttgctgg aggaggcgcg gcggcgggag ctagaagcgg 92820

aactcgaggt tccgctggtg gtgctgcggc gactcgtgcc gctcgtgccg ctcctgctag 92880

tgccagtgcc agtgccgctg cggcgtgaag taccggtgcc ggacctgccg ctggagcttt 92940

tcttgcggcc gccgttaacg ctgtcgatgc cgagcaggtc ctcgcacacc tcgccgacgg 93000

ttccctgcac gtccaacttg ccgttcttga caaccccgta cacgatcttg ccgcagttgg 93060

acacagcctg gatggtggtc tcgtcgctgt caaaggcgtt cattccgccg cacccgccgt 93120

cgttgtttct tcgagaaggc gcgccgctgc ggcgactcct ggtgctgccg ctggaccgag 93180

ttccggagga cctggagccc gtggaccggc tgccggtcga cctggtgccg gtagtgcgct 93240

ttctggacga agaggaggag gcgcttccgc ggcgggtgga cgaactagcc tccagcgcac 93300

cggcgccgcc cacacaatcc acgtcggcgg cggcgcctcc gcgaatgacc tgctcgttgt 93360

tgagctgcgt caggagagat cgcagctgcg gcgcgatctt ctgcaaggtg ctcacgtagt 93420

cgtcgtagct gctctgcggg cgctgcgcca ttttttcgga cgccatttat tacgcggaat 93480

atctacgacg acgcagcact gaatcggttt ctcgcgacgg gagattccgc ggtcggcgcc 93540

ggtgcggggt tgtcaccggg cgacgaggta accagcgcgt ggaaggcgcg cacctggtcg 93600

tccgtcatct tgtcctcgaa cgaggacgcg cccgggggga gcaggtcctt gttgcgcgga 93660

acggcgggcg ccgagacgca cgaccggcgg tacatcatga tgacgatgta gcacacgatc 93720

gagatgacga tcacggtcag cagcgcgtcg aggagcccca tttattacct gtatatgccc 93780

gcgtttaccg ggcggtgagc tcaatgtcgg tgttgtttag ccgggcgtac gggacgctgc 93840

cggagcactt cctgtacatg ctgaacacga acagcccgag cagcagcacg gcgcccacta 93900

tgaagcaggt tacgcacagc gcgcgccaca cgtagtcggt gacgttgttg gtgttcttgc 93960

tgaaatccac gaaggcgaag acgcaggcgg ccgtcagcag cagcacgccg catatcagca 94020

ctccggagta gtaagagctc aaggtctcga atatgtccat ttatctgagg agaaatttaa 94080

attactgaat ggacgaagtg gaatagaaac cacgagaaca cgacggactg cagcacgaag 94140

atggtgctca gcttcgtctt catgggcatg cagaagttcg cggccagcgc catacagaag 94200

atgaacacga gcaccgccgg gtcgtagtcg gacaccattt acactacgct aaaaggcata 94260

tctcggcgcg cgacgtccac gagcaccagc acgcggacgc ccgcgggcgc gccggcggcg 94320

accgcggcga gctgcccggc cgtggggttc accagcagca gtgcgcgcgc ggttcgcggg 94380

acggggtctt cgtaggccat ggtcggcgtg gacccgggac gcagcggccg cccctgtctg 94440

tcgaagaggc cctcgggaaa cgaggtgccc ggaacggcca cgacgacggt gtcgctatct 94500

agaaacattt atggtcttgg tttccacgga tcgcctcgag tagaccgcca cgaagtagaa 94560

gatgacgccc gccgcgagcg ccgccaccag gaagggcggc acggcgggca ggttcgcgga 94620

cgcgttgtcg cgcacgccgg ggtccgggtc tgcgtagccc gcgcccacgg ccttgccgca 94680

gtcggcgatc atgtgcgcgc gcgagttctg catgaccagg ctgtccacgt cgatgcggca 94740

gcccacgtag cggcaccgcg agcgctgctc gtcctggctg aagaagagcc acttgcggtc 94800

gcgcgactgg tccgtgcact cgtgcgcgcg acagacgcgc gggcccaggt acttcccgag 94860

cgtggtgccc gcgacacacg cgcactccgg cgcggcgcgg tgcgcgtcgc agtagcgccg 94920

cagcgcggag tcgccgaagg cgaaggaggc gggccgcgcc acgcgcacga actccgagca 94980

gaagcgcgcg tccatgtgct tggcgcagag cgccgcgtag gtgtccagcg ccgcgtagcg 95040

gcccgtgcgc agccaggcca tgcactcggg cgcgtcaggc tccaccgcgc agcggctggc 95100

cataacgccg tcgcagtgcg cggtcttgta cccgttcgcg aacacggacg ggcacccggg 95160

ctccggattt gtgcagcagc gcgccatggc ggcgtccgtg ggcggcgccg aggcgccgat 95220

ctcgaacgcg cacatggtgc cctggcgcag gtacggcttc gcgatctcgg gaacgtagtc 95280

tgcgcgcagc agcgagcccg ggcggaagaa gagcgagtcg cagggcgggc ctcgcacgag 95340

ccgcgcgcgg ctcgccagct ctggcgagag gaagcgcccg cactgcccgg ggtccatggt 95400

cggcagcaga cagaaccgcg gccgtacggt cttcagcttc gggtcggaga aggtttctga 95460

ttcttccgcg aaggcgaagg tgtccgtggc gctcgtgtgt gtgacgcgca gcgcgtactc 95520

gcctggcgtc ggcgtgtcga gcacctccac cttggatacg gtgtccccca tttgaagacg 95580

ctatttacgc cgctgcctac tcggcgaaga ataggtcctc cgacttggcg cccgcgtaca 95640

ccgggcaggc gggcgcggcg gagcgagtgc gcacgatacc gcggccagtg aggcggaagg 95700

cgtagatggc gaacagcagg ccgagcacga tgtacatgaa agtggtggcg cccacagacc 95760

cggtcacgtg cgtcacgatg atggtgacga tggacatgat cgtgcacacg atggccatgc 95820

cggtgttgtt ggccgcgtag gggtgcatga tctgcatggc cgcacagtat ccgatgacca 95880

ggcacggcag cgggaggata agtgaggcaa tacctatcat tactagagcg agcacggggg 95940

tggacgtcaa ggccaataca aaaatcacaa tacctgttag tatgcggata tcctcgtact 96000

ggaggacgct gtaaggcgcg atattccctc cgggcactgg cctggggtta gccgggacta 96060

ggggggagtc ggcagtgccg gggtccttgg ggagaaaggc attctgctcc tccgggctga 96120

agagctcggc gtcctgaacg ccgccggcgg tgaactcgtc gttatagtaa ctaaagtagc 96180

tttccattta tatgttgaaa aatgtttgga ggcgtacagg tggacgacaa actctacgcg 96240

tacctaaaaa aactcgccgg acgcgggcgg ccgctgtgtc tgttccgcga caacggcgag 96300

ttcgtcgaag tcttcgcggg gtccgcgttc cgctttgtgc tgcccgtggg cctcttcgcg 96360

gacctgcgcg tgcgcacgcg cggcgtggcc ttcccgaaac tgcgcgactc cgcgcgcatg 96420

cgcggcgtgc gggtggacgc gcacacgctg ccctcgctgt accccaacca gcgcatcgtg 96480

gtggacgagg tgctcgcggc ccgcgaccag ttgctggccg cgggccgcgc cgtgtacgtg 96540

acgctgcatc tggcttgcgg cttcgggaag acgctgaccg cgtgccacct catcgccacg 96600

cacggccgcc gcgcggtggt gtgcgtgccc aaccgcatgc tggtgccgca gtggcgcgcg 96660

gccgtggcgg agctgcgggt gcccttcgcg gtctcctgtg acggcgcggc ctcgctgctg 96720

cgctcgggcg agctcgaccg cgccatggtg gccatcgtgg tcagccggca cttcgccaac 96780

gacgacttct gccgcgcggt gagccggcag tttgacgtgc tcgtgctcga cgagtcgcac 96840

acatacaacc tcatgaacaa caccgcggtc tcgcgcttct tgaccaagta cccgccgccc 96900

atgtgcttct tcctgaccgc gacgccgcgc acggccaacc gcatctactg caaccgcgtg 96960

gtgaacgtgt ccgtggtcag ccgcctcacc aaggtagtgc gcgtggtgga cgccttcttc 97020

gagccgtaca ccacgcccaa gatccgcacg ctcgagcgca gcctcgaggg accccagaac 97080

aagtaccacg tcttcaccga gaagatcctc ggcgaggacg tgcaccgcaa caagctcatc 97140

gtggacaccg tggtcgcggc catggccgcg ggcgaggcgc ggcgcgtgct cgtgctcacc 97200

aagctgcgcg aacacatggt cgggctgcac gccgcgctct gcgagcgcct cggtgcggag 97260

acggtctttc tcggcgacgc caagaacagg aagacgcccg aggtcacgcg cgcactgcgc 97320

gacaaggacc gcttcgtgct cgtgtccacg gtcttcttct cgggcacggg cctggacctg 97380

cccaacctgg acgcgctcgc ggtggccgcg gccgtgctca accgcatggt catggagcag 97440

atgatcggac gcgtgtgtcg cgagtcgcac gccaacacgc gcacgctgtt cgtgttcccg 97500

gactcctccg tgcgcgcgat ccgcgacacc gtgtctgcgt ttgcgcagcg gctcgtggcg 97560

ctggcggtgg acgggctggg cttcgtccgc gagcgcgccg ccgccggcgc gaagaacgag 97620

ccggcgctgt acagcgccat cagcgggcga gatctcgcag cggtgtaagc gcggacccgc 97680

acgccgcgca cgagagcgtg ctggagcagg cgagtcccag cgacagtgtg gacagcctgt 97740

ccacgtcctt gatgctcacc agccgcgagt tgcacgacga gcacacgggg tcgctactat 97800

catcgaccac cgtggtgacg cggcggcgtc tgcgcttttt gtttccagcg gcgacatcga 97860

ccacgcctcc cttagagccc cccttcgccc ccgccttagc tttcaccgcg ctcatctttt 97920

atttatcata aaaacacgtc tgcgtacgcg ttcgcgcaca cgtcccgcaa atccgcgcgc 97980

gcgccgcagc gcgtgaagcg cgcggcgtcc gcctccgcga tccgcgcgca cggcagcggc 98040

gcgcccttct cgtccgccat cacgcgcgca gagatcccgg tggcccccag cgcgtacgac 98100

accaccacgt cgccgacgca gcggtacacg ttgccggagc cggcgaggcg gtcgaacgcg 98160

gcgccctcct ggcgcagctt gtcgaatatg cgaggaacga ggatgttaaa aatgagaacg 98220

aaatagcaga tcagcaaaaa cagcgagatc atgacctccg agagcgattt atataccttg 98280

aaagagctaa tacgacttcg ggactcgctg cacctcgcca ccggcgccgc cgtcgagcgc 98340

tacaacgcgc tcgtggagtg ggccgcgcgc acgtactgga cggtcgcggt gctgccctcc 98400

gcgccgtgcg cctccatcga gaagtactac tgcgtgtgca aacccgactg cgcgctcgag 98460

cccggcgagt actccgtgag ccggctgcac ttcggactca cgcacgcctg ggtgcgcggc 98520

gccgccttca actcggccag cggcgccgag gtcgagccgc cagaggaggt gcgtagggcc 98580

tgcgaggcgc tcgacgccgc cttcgcggac ctcaccttcg tgcgcttctc ggtcttcggc 98640

cgcgagtgga cggtcgacga cgccgtcaca gaccactcct cgcgcgacga ggtgctcgcc 98700

gcgtgcgccg cctccggcgt gcgcgtcgcg cgcacgctgc gtgtgcgcgt gcgggcggga 98760

gagtccttcg cgcgcgcaga cttcgacgcg gtgcacgccg cgctgcgcgc ggagggcgac 98820

gtcgctcgcg gcaccgcggt ctgtctcgcg ctgcgcgggt catcgcgccg ctggatagcg 98880

gaccgcgcgc ctcgatgctt catgcgcgtg cgccgcgtgg agctcgagcc cgtggacgcg 98940

cggcaccact gcccggtgct gatctccgcg cgcggcgacc gggtgctctg ccgcggcgtg 99000

gggcacctcg cggacgcgcg cgcgcgcgag ggcgtcttcg tggccgtgcg caggtacccg 99060

gagtgtctgg tgctctgcga cgaggcggcc gccggcgcgg cggagtgctc gcgcgaggag 99120

gcgctgcggc tgctggtgcg ccgcttcggg cgcgacttcg ccgtcagcga ggagggctac 99180

gtcttccgcg tgcaggacat ggacttgcgc ggcgtgtccg cgcgactggg gctcgcgccc 99240

tgcgcgagcc tggaggagct gcgccgagcg gtggagcgcg accgcgcgct gatgaggcgg 99300

ctgcgcgcgg agggcgccgt gcgcctcgcg tgcgagtgcg tgggataccc gcgccagaac 99360

gcggtggagc tcataaataa tatgcgcttt caaataacgg aagaaggcgc ggtggcgaac 99420

tttgagctgg cgaacgcgag ctgtctcggc aacccgaccg cggagtccat cttcgcgagc 99480

ttcgcgcagt tcgtgccggt cttcaacgtg ctctcggcga tcgcgcgcgc gcagccatga 99540

tcgtggcggc cttcgacctt ggcacgcgca accccgcgcg caccgtgctg gaggtgctcg 99600

acggcacggt gcgcgtggtg gacgtggcca agctggactg gagccgcgac tgggagaagc 99660

gcgtgcaccg cgacgtgacc gccttccccg cgaacgtggt gctcgtggag cgccagtgca 99720

agatgtcgcc tttttctaag ttcatatact tcatacgcgg gctgctctac gacgggcggc 99780

gccgcacgcg cgtgctcgcg gtgccgccgg ccatgaccgg cagcacctac cggcagcgca 99840

agcgccgctc ggtgcgcacc ttcctcgcgc tcgcggagag cttcggcatc ctggacgccg 99900

tgcccgcgcg gaagaagctc gacgacgtcg cggacagctt caacatggcc atcaattacg 99960

tgctccgaac aaactgaaat acgactggaa cgaataagtc atgctggcgc tgttcgagtt 100020

cctgcggtcc gtggaggact gctaccggcg caccatcttc aacttccaca tcgcgcacag 100080

cgccgaggcg ggcgatgtct acggcgtgct gcgcgaccgc attttggcgg ccacgcgctt 100140

cgaggaggta gcgccgccag ggctcgcgga cgcgctggcc aaggtggtct actgcgacat 100200

aagcaccacc aagcacctgg tcaaccacgc ggccttcgcg gcgcgcgcgc ggccggcgcg 100260

gcgcggaggc agcctcgcgc agttcttcga cgtgcacgtg ggcgaggacg cggagagccg 100320

ccgcaccgcg gagatcttcg accgcgagcg ctcctcgctg gtctcgtacg tgaagaccac 100380

ggccaagcgc tgcaagatcg actacggcga gatcaagcgc accatccacg gcgggcggca 100440

gacctacttc tcggggcggc gctcggacga cttcctgagc accaccgtgc gcgcggaccc 100500

gagcaagccc tggatcaagt ccatctccaa gcagctgcgc gtggacatcc tgcaccacgc 100560

gatctgcacg cgcggcaaga gctccatcct gcagaccatc gagatcgtgc tcacgaaccg 100620

cacctgcgtg aagatattca aggactcgac catgcacata atcctctcca aggacgaccg 100680

cgagcgcggg ctcgcggacc tcgcggacaa gctcttcggg acctacgcga ccaccttccg 100740

cgtcatcgcg gccatcaccg gcaacgcctg cttcgcggcg gtggcggacg cggccgcgcg 100800

cgtggtcgcg ctcccggacg cggacgcgaa gctggcggcg gtgcgcgggc tcgcggagtg 100860

ctacggcgtg cgcaacttca aaatcggcat gttcaacctc accttcacgg gcgccatcga 100920

gcacacggtc ttcccctcgc tgatccccgc ggagagcaag atcaagttct tcaagggcaa 100980

gaagcttaac atcgtcgcgg tgcgctccac cgaggagggc cgcgagtgcg tggagcaggc 101040

gcaggcgctg ctcgcggcca tgcgcgagcg ctccgcgcgg ctcgcggccg cggacgtggc 101100

caccgcgagc gtggacttcc tcaaggagct gctggggcca tagtgaaata atactgattt 101160

cttaaatatg gagcaggcgc tcggatacaa gtttttgttg cccgacccca aggacgacgt 101220

ctactaccgc ccgctccact tccagtatga gtcatacgcc aacttcatca agcaccggct 101280

taaggacatc ctcacggtgc ggcgcacgct gctcaccttc aagaacggca ccgagtccat 101340

cgtgctcgag atcgacgacg tgaagatctc ggcgccggag ttctcgccca tcgtggccag 101400

catcaagggc cacagctacg aggcgctggt caccttcacg gtgaacatct accggcacgt 101460

gatgaccaag gacggcctca ccgttaccaa gatcaacagc tacgagggca ccgactcgca 101520

cctcgtcaag ctcccgctgc tcatcggcta cgggaacaag aacgcgctgg acccctccaa 101580

gttcgtggtc ccgaacgcca tcggcggcgt cttcatcaac aagcagtcca tcgagaagct 101640

cggcatcaac atgatcgaga agatcaccac ctggcccaag ttccgcgccg tgaaggccaa 101700

ctccttcacg ctctccttct cctcgatctc gcccgtgcac gtgatgcccg cgcggtaccg 101760

acactacaag atcctgctcg acgtgaacca gcccgacaac ttcgtgatct cctccgcgaa 101820

gaccttcatc accgtgaacg tgatcgtgat ggtgcagttc ctcgcggacg tcacgctcga 101880

gttcgtggcg cgcaacctct gcttcgacat gccgcccgag gccgcgcacc tggccaccgc 101940

gctcgtggag agcgcgaaga ccgtgcccgc gggcgcggac gtggccgagt acgtgaacgc 102000

gctcatcgcg gccgagcacg cgaagcagaa gtcgacgctg tccaaggagg agttccgcta 102060

cgagatgctc agcaacttcc tcccgcacat gcaggacagc gccaaccagc tcaagggcct 102120

gtacctgctc tcgctggtgc gcaagatggt cttctgcgtg ttcttcccga accggtaccc 102180

ggaccgcgac tcgctggtct gccaccgcgt gtacacctac gggcgctact tcgaggcgct 102240

ggccatggac gagctcgaga cctacatcgg gaacatccgc aacgacatcc tcgcgaacca 102300

caagaaccgc ggcacctgca ccgtgaacat ccacgtgctg accacgcccg gcttcaacca 102360

cgccttcgcg gcgctgctca gcggcaagtt ccgcaagtcc gacggcagct tccgcacgca 102420

cccgcactac tcctggatgc agagcatctc catcccgcgc agcgtgggct tctaccccga 102480

gcaggtcaag atctcgaaga tgttcaaggt gcgcatgtac caccccagcc agtacggctt 102540

cttctgcgcc tcggacgtgc ccgagcgcgg gccgcaggtc gggctcatct cgcagctctc 102600

cgtgctcgcc tccatctcga acatccgcac cgcggacttc gtcgagctca ccaagcgcgt 102660

ctgcgactac gtgcgctcct accccgcgcg cgacatcagc tacttcgaga ccgggttcgc 102720

ggtcaccgtc gagaacgcgc tcgtggcctc gctgaacccc gcgatcgtgg acgcgttcgt 102780

gctcgacctg cgccggcgca agcggctcgg cttcttcggg aaccgcgaga tcggcgtcgc 102840

gctcgtgcgc gaccgcatga acgaggtgcg catcaacttc ggcgcgggcc ggctcatccg 102900

cccgctgctc gtggtcgaga acggcgtgct cgtcatggac gcggaggcgg agcggctcga 102960

gcgcgacctc gccgcgatga ccttctcgga cgtgctgcgc gagttcccgc acgtgatcga 103020

gatcgtggac gtggagcagt tcagcttcag caacgtctgc gactccgtgc agcgcttccg 103080

cacgctgccg cccgaggagc gcgcgctctt cgacttctgc gacttcccgg ccgagttccg 103140

cgacgggtac gtggcctcct cgctcgtggg catcaaccac aactccgcgc cgcgcgccat 103200

cctcggctgc gcgcaggcca agcaggccat ctcctgcctg agcgcggacc tgcgcaacaa 103260

ggtcgacaac ggcatccacc tcatgttcgc ggagcggccc atcgtggtca gcaaggcgct 103320

ggagacctcc aagatcgcgg acaactgctt cgggcaccac gtcaccatcg cgctcatgtc 103380

cttccgcggc atgaaccagg aggacggcat catcctgaag cggcagttcg cggagcgcgg 103440

cgggctcgac atcctcacct gcaagaagta ccaggtcgag atcccgctcg agaacttcaa 103500

caaccgcgag cgcgtgcgct ccgcggcgta ctccaagatc gacgtcaacg gcgtggtgcg 103560

cctgaacgcc ttcctcgagc agggcgacgc catcgcgcgg aacgtgtcct cgcgcacgct 103620

cgacgacgac ttcgtcgccg acaaccagat cagcttcgac atcgcggagc ggtactcgga 103680

catctacgcc gcgcgcgtgg agcgcgtgca ggccgacctc accgacaagg tcaaggtgcg 103740

cgcgctgacc gtgcgcgagc gccgcgccat cctcggggac aagttcacca cgcgcaccag 103800

ccagaagggc acggtcgcgt acgtggccga cgagactgag ctgccctacg acgagaacgg 103860

gatcgcgccg gacgtgatca tcaactcgac ctccatcttc tcgcggaaga cgctctccat 103920

gctcatggag gtcatcctca ccacggccta cgggcacaag cccttcgccg aggacggctc 103980

caaccgcccg atctgcttcc ccagcaccaa cgagaccgac ttcgagacct acatcgagtt 104040

cgcgcggcgc tgctacgcgc tctcgcaccc cgaggccgcc gcggacgacc ccgagttcga 104100

gcaccgcgtc ttctgcgagc gcgtgctctt cgaccccgag accgacgagc ccttcgcggc 104160

gcgcgtcttc ttcgggccgc tgtactacct gcgtctgcgg cacctcacgc tggacaaggc 104220

cacggtgcgc tgccgcgggc gcaagaccaa gctcatccgg caggccaacg agggccgccg 104280

ccgcggcggc ggcatcaaga tcggcgagat ggagcgcgac tgcatgatct cgcacggcgc 104340

ggccttcacc gtcgccgaga tcctgcgcga ctccgaggag gacgcgcagg aggtgctcgt 104400

ctgcgagaac tgcggcgaca tcgcggcgcg gctcaacggc acgcacgtct gcatccgctg 104460

ctccaagatg agcctctcgc cggtgctcac gcgcatggac tccacgcacg tgagcaaggt 104520

cttcaccacg cagatgaacg cgcgcggcat aaagatccgc gtggagttcg agaagcagga 104580

cccctgcttc tacgggactc cgaaacggtt cagcctcgcg cccgacgagt cgctgttctc 104640

gccggaggac tgaacccgcc gtcgcgaccg cgtcgcgacg actagcttat cgttcgactg 104700

atgcgaaacg cgcggcggcg ccgcgactta gcttatctcg actgatgcga acgcgcgacc 104760

tctcgcgact ttctagcttc tcagactgat gctaccatat cgcggcgtgc tggccccacc 104820

accagggctt ctcgccgtgg ctgacgcggg gctggctgcg acgcgcgctg cagtagctgc 104880

gcgcgcccca gtcgccgcgc acgtgcgccg ggggcaggct cccgtccagc gcgtgccgcg 104940

tcacctcggc gccgggccgg cggcacgtgt gcacgtccgt cttgttggag acgagcaccg 105000

cgtactgccg catggtctct atgtgatgct ccaagtgctt gcccgccatc cggttggact 105060

cgcagcacgt ttttgcttcg gctaaggttt tttctagagg ggatagtagc ttatccacgc 105120

gctcgggcag gacgcacgcg gagccgtcga accctacttt gaacggggtc accttgatgt 105180

tcccgtcgta gcggtcccac agcatcctga ggtaggttgt accgtcgggg tctgggtctg 105240

tccacactct aagcttttcg ctacagcggc cgtcgtacgt aagacggtct ctacgctcgt 105300

agtagtttct gcttatgttg ttggggtctc catgctcgta gtagtataaa tcgtacgcgc 105360

ctggcttttt taagtcgttt tcgtcgttgc tgacgtgtat cacgtcggga taataggata 105420

tcctaactgc actacaatct atagtatttg gtctagtaag ctgttcgaga tcaccttgtt 105480

catcatgatc tactgatttg tacacggcac cgtcgtgttc cgacggacgt atgaatatgt 105540

ccatggtaaa cgatgtaccc actttggaaa acgtatccca tgcagtaaag catagtccgt 105600

ccattataaa ctcaggaaca ctcataacaa atcgaaatct gtgaagtttt tcgaacacca 105660

cttttacatg gtctttgtca cgaacatcat tgccgtttac ttcagacatg aattgaagga 105720

acgctaaaga gtttcttgtt tcttcatgaa tctttccatt atacgtccat ccagtttcta 105780

gaattctata tatgcttttt gcatcgaccc cgtaccacca gtacatggga actccgaaat 105840

atatagctgg gtttgagtac caatgggcaa gagtgcccat tgcgtttaaa aagtcttgac 105900

aaaaaaatgc agtttttctg tcgataactt gacttggact acgttcgtgg acatcgtaca 105960

tgtccataat tggttcattg gtaacggtta catgacccgt cattatcttt ttaacaatca 106020

taagatacag tttgcctaaa gtcgaaatat gtataacgtt aatttttaca tgttctccta 106080

acgtaattgc gtttttactt agccattcgt cgtctacaaa aatcttacga tacataggat 106140

ttctctctac gtatcttcta aagtatagat ttaccggtct accggcgaca ttagcgccat 106200

ctatagcagg agcaagctgt atgtatcgtc gtataatgtc tcgtataagc tttctgtctt 106260

ctctgggaat acacgacacg gaacttagag actggtgcca gtgtctttca accaaagact 106320

tgaacctagc aaccaacgcg ttgtcactct ccatttataa ttaaataatt atcccaactt 106380

cgtatgttaa tccttattac cagatagcac cgctccttcc tctccaccac gtactatcta 106440

aaggatacct gtaagggtaa tgtctggata acgggcgtgt gagccaagac gtgttatggt 106500

gtcttcccca ccaacggtcc acttctctaa ctaccggagt gctagacgtt gtcgatccca 106560

ctactgttgt ttctccatta cctgtaatct ttgaagcgca acaagtgtta gtctttgcca 106620

aatcttctaa cggcttaatt aggtcgttaa gtctgtcaat atccatgcac tgaggtgtat 106680

cgttaccagt ttccccaact ttgggaggaa cttcctgttt agtgtccaaa taacccataa 106740

acctgtctct aagcattctt ctgtacgtgc ttttgtcatt gtctatgtct cccaaaaacc 106800

tgtgatttac gcatccgttg tacgtaagtc tgtcccttcg ctctcgctcg ttattcccta 106860

cttcttctat tgtactgtaa tgttgatagt ccaagtaata gccactgttt tcatgatttc 106920

ttgtaaatat aatcggtgtt ttattattga catcgtgttg cctactgtac gtatcttcca 106980

tggatctagg aacttgtcta gaaaattgag gactagaaat acgccttcca aatcctggat 107040

gataaaccaa acgcaatgca ctacagtcga catcgtcact gtctctagtt atcccatcaa 107100

caactccttc tttgtgatgc tccactgttt taaagttaac tcctctgtat atgttgagtg 107160

ttaaaaaaat atccacattg aacgcagttc catattttgt tattttatcc cacgacgtat 107220

aacacaaacc atccataatg aattccggaa ccgatactac aaaattagta tcgaacttat 107280

caaacgaaat gtttactcgc ggttgtgctt cgtatttttt gtcatacaca tctgtcatgt 107340

attgtacaaa atctatagct cctctagcat cgggcatgct aacgtctgga aaatcatttt 107400

ttaactgttc tagtacgaac tgttgatttt ggtcatctct ccaccagtac attggcaatc 107460

ctatcacaag agacttattt ttataataat ttgctacaga agctatatgc cacatgaaat 107520

tatagcaaaa ataatccatc tgtatgttta aaaccggttt actagtctgc tgagagtagc 107580

tatccatgat agtgtttccc tcgccaatac ttcctgacat tattctgtaa acaaccatta 107640

acaaaaatct tcctaccgtt gttatgttgt caaaagttct tatgttttga gcactttcga 107700

gtaaccatat gttattttca aatatacttt tgtaaactgg atttctgtcc acataactct 107760

ttaaaaatag atttactgga aggccgcttg ggttatctcc ctgtataggc ggcgcttgct 107820

ttatgtattc gcgtaacaaa tctcttacaa cttttctagt tcttctaggt atacatgacc 107880

tattattcaa aggctttgtc cagttagcgt ttataaacgt tgtaaagtca ctgactagct 107940

tctccattta taattaaata attacagacg gcaacacagc ggttatctaa tatctgctgt 108000

atcctgtctg tacatctatt tttctgttga gatcaagaag agctttacgt agactctcca 108060

agtgtctttc tagtctgtct aaccggttac ctgtttctct gcagcaatca gttatagttt 108120

tgtaactgtc taacaagctt acgaggcgct cttccacact ttctttagtt ggagctccag 108180

ccgcgtacac tccgttggtt gaattgcctg tatcatcagg ctgagtcaat aggttttctc 108240

cgtcattttc atccatattg agtccaacga acacaaacga gtaagtgttc ctctatttaa 108300

agtattgatt ttagaaaaag gcaagcctcg ctgccctgat tcggcggcaa acacgggttg 108360

aacacgcgga agtcgctcgc ggccgtgaag atctcgtccg cgcacgcctc cacgctcgcg 108420

aagcgcccag gcgagacgcc gtcgtgcgag cggaacccga actccgaggc cgccaccgcc 108480

gcgcccttga agagcacgca gcgccacttc ttgcgcacgt cgaaggcctc gtcgttgggg 108540

tcgaacacgc gccggtccac gcgcgggccg cccgccgtgc gcgcgaactc cagcgccgcg 108600

ttcgccgcgt tgaactcgcg gatgttgtcg tagttctcgt agacggccca gagctgcagc 108660

gccacgaaca tggcggccgc cgccgcgagg gccacgcaga gcgcggacac cgcgtccatc 108720

ttttatgtgc agaattattc gttggcgcgg agctcgcgca gctccgcggc gcgcagccgc 108780

gcgaaggctg ctttgagcgc gcgcagcagc tcctcggtgt ccgcgcgcag catgtcgaag 108840

cggtggtagc tgtccaggcg cgcgcggcag ccgaagaagc gtgcgacgca cgcggtgacg 108900

atgtcgttca cgtagagcac gcccgaggcc gtgcagtaca cggagcgcgg ctcgcgcggg 108960

tccggcggca cgtccacggc gaccgcgtgc gcggccacgt cctcgagcac cttgcgctcg 109020

agcacggcga ggaagtcgcg cagctggcgg cggttgtcca gccaggcgta ggtggtcgcg 109080

aagagcgtga gccgcccgcg cggcgcgatc gcggtgtagg gcgcgtaccc gcggaactcc 109140

cgggggtgca cgaccttgac gttctcgtgc tcgcgacgga aggcctcggt gtcgagcagc 109200

gccgcgagcg cgtccacgag cttgtcggag acctccacgc ccgcgccgaa ggcgatgagc 109260

tcgatcttct gctcgctctt ggggcggaag tcgtggaagg tgtgcagcag catctcgcgg 109320

agctgcggcg gcttctcgac ggcctcgagc gcgtcgccgc ggacgaggaa gtagtcgagg 109380

tcgtgcagcg agacgtgctg cccggcggcg ctctgcacaa acttgaggaa gacgcagagg 109440

cccgcgcggc gctcgagcac gtcctcgacg tgcgcgtgga atacgtgccg cgagggcatg 109500

gcctcgatcg cggagagcca ctcctcgttg acgcaggtgg tggtgttctc cagcaccacg 109560

ccctgcgtga gcgcgggcca ctgcaggtgg aaggcgaact cgtgcttgat gagcgaggcc 109620

acggccgggt ccaggtccac ggccagcgcg gcctcgccga cgaggggagc gtccgccatc 109680

acgcggagga cgcctggccc atctcctttt tcgccttttt attcaggatc attattcttt 109740

cgttgacgag gtccatgagc atcttgatgg cggcggccgc ggccgccgcg tcgccgccgc 109800

acatctgcgc gatgcgcgtg agcatgtgca gcagcgcggc ctcgtttagg tcctcctcca 109860

tttagaggcc gtgagggcgc gcgtcgtcgc gacgagggga cgcctcccgc ggcagcgtgg 109920

tgcgcacggc gaaggcgagc agcgcgccgg cgcactgcgt gagcacacac tccgcgagcg 109980

cgacgaggag ctcggagggc gcgagcacca tttagaggcg cgcgcgggtt taattgccgc 110040

cgtcagagtc ggcatcatcg cccttgtcgc cgccgtcctt gcagtcgccc ttggtctcgg 110100

cgtcgacgat gtcggcgagc cgcgtcttca tgtgcgagaa ctgcgcgagc aggatgccgg 110160

ggtcgagaca gcgcttgacg acgctctcgt cggcgaagtc gtagcagatg cgctcctggt 110220

tctggcagaa caccgagtct tcgatgatca acaccctcct ggtcccggcc gaccgcatga 110280

tggccatggc ccggatgagc ctcttcttcg atccgcgtat ggacatggac cggagcacgt 110340

tctccacgtc ggagtcggag acgttgcagc agcagaggtg cgtgatgctg gcgcgcccgt 110400

tgacggggat gtgcttgtag gtctggcaga gcagcaccag cgacacgttg atgtgccgcc 110460

cgtagttcat gaggcccaag agtgtggcgg accgcgtctg cgtgtcgccc atatcgtcga 110520

gaatgatgag gaacttctgc ttcttcgtct gcgcgtgccg ctcgatcttg cgcttggcga 110580

ccgagaggtt gtactcgagc tcctcgtgcg tggtgacctt gtggatgtgg tccggccaca 110640

cgaagccgtc gtaggcggcg ttgtagacgg gcgtgaagag caggatgtgc ttgaagcggc 110700

gcacgagcgt gcggaagagc gagagcaggt aggcggtctt gccggagccg gagccgccga 110760

cgagcgccat cctgaagggc gcctcgatga gactctcccg cttgaagcgc acctcctgca 110820

cgacatccat cgtatattta ctgtcactaa attaccggct ccgagaaata tagaaattag 110880

agcctcctag agcacaccga ggcgcatcgg caagatggca cataacacgt tcgaaaacga 110940

tagcgagtcg gcagctaaca accagtacgt ggcgtcagtc aagcgccaga aaatgattcg 111000

gcgatacatt aagatgttct tccggttcgt tacggcgata gctatcattg tcctggctat 111060

tctagttgtg attctgtcgc tatctctaga cgaatgtctg cacagagaac accctcatga 111120

ctattcacat gtacaaaatt caacatgcga cggcattact ttaggtggtg aaaagtgtct 111180

cagacttaat ttgccagcaa cgtgggaaga tgctaataga caatgtggta atcttgggtt 111240

ttacctacca tctactggcc ttgaaaagaa atttccttgg cttgtgacct atctcgacgg 111300

aacttgggga aacactcaga actccgtatt tggaccaacc ggtgacttgc agaatgtcat 111360

aggaccgaaa gaatacaaat atttttgtgt gtccgattag atgattataa tctaataaat 111420

gggttgctgt aaggtcccta accgccagtc tataaggact ttgaaaaagg cgtcctgtcc 111480

ggtcgccagt ctcgtcacca ttctctccct agtcaccagc ctcggtgcga tagtcagata 111540

caccaatttt tttctaaaag aagcatgtga cgaaggatgg atgcccataa aaaacatatg 111600

cattttaaac acgcactttg aagccaccaa tgacgatgcc cacaggatat gtgaaaacct 111660

agacggaaag ccgccggcca tccctaaccc tactctgtta aagggtgtca cagttctcac 111720

cggcgaaaag aaattttgga tgacccatca cgaagactat actactgtgt ttgagcatat 111780

agacgatagg acgactccta aaaacacaga ctatgacagt aaaaaacaca cttgtttgat 111840

gagcgaggac ggattgatac accataactg catgatgaac gtgactgtgg tatgcatgaa 111900

ggagatgcac ggataactga aaatatactg tttgaacgca aagacgccat gtcgcgactt 111960

caaatactga cctcatttgg acaaatcttc gcacccgacg aagctcggct gcgcgagatc 112020

gcgcgtgatt tgggaatatg caccataaaa cgcgcattcg gcgacatgct gtacggcttt 112080

atagacttcg acccggtgcc cctgacccaa gtaaacatgc tcatgtccaa ctgctacttc 112140

gcggtcaacg gcaacctgct tccgtgcacg gaggacttcc ggctcagact cccggcaacg 112200

gagatctctg cggcctacct gacgagaacg ggacggacga tcctgtgcgg caaagacttc 112260

aacatagtag cgccgtcggg gttcaagccg tccatgcggc tgcgcgacct cagtcacgtg 112320

tctgcgcttg tagagatcct ggaagtctac gacgagtccg ggagtaccaa attcgtgctc 112380

ggccccagcg cgcagttcat gctgcggctg atggagaagg agaacgtctg tctgttcggc 112440

agcgggtggt gcatagtgga cctgcgcaag ctggacgtac ccatataatc agcatccttg 112500

tttttatcct gtctttttat cagtttttta gctagttaaa acataaatag taaagctaaa 112560

aagaggagtt ctggagtctt gcaacaacca ggatgaaggc ggtgttgttg ctggcgttac 112620

tgggagcgtt caccaacgca gcgcctttgt tagaaagcca gcgttctaac agtgaggaaa 112680

aagcaaattt ctgctcgacg cataatgatg aagtgtacgc caggttcagg cttcagatgc 112740

gcgtgggtgt acgacacagt ccgctctaca ctcccagcaa catgtgcatg ctggacatag 112800

aagactccgt tgaggacata gaagagtcca cagaaaaaga atacgcgtct acggccacgg 112860

gtgaggcggc cggagtgaac gtgtcagtgg cactagtggg agaaggcgtg agcataccgt 112920

ttagttacat aggccttgga ttcaacccgt cgcttgaaga tagctacctg tacgtcaacg 112980

tctcgtcacg agctccttgg gttaaacaga cttcggacct atccgcgaac ggcggctggg 113040

gtatcaaaca ggttctagaa aaagagttac tggccatcca aatagggtgc gacaaccaaa 113100

aatttcccga agaacccaca actacacccc cctcacctgt cactacaacg ctttcctcaa 113160

caactccaga tctgaatgaa gaaaacacag aaaatacgcc gacgaccacc ggcgccagtg 113220

tagacagaaa gcgcaatcca gctgacattg acttctcgct gctcgtggac ccccgatgcg 113280

tgacctctgt agacctgcac gtcgagctca gggacgcgtg catagactac aaacaagagt 113340

cgccgttgtc gctgaagggg aaatatggag acggcgaact agtaaaaaag gagattaaag 113400

acgtgggaaa gaatcacaat atgtgcagtc ttaacctcaa ccctggcaat tgagctgttt 113460

ttattcggca atataatagg tgattattga acattaaaca aaacttatcc cacaacgccg 113520

caacaatgga agtgctggtg atcatctcta ttatcgtcgc cgtaatatgc ttgaccggag 113580

cggtaatgta cctccttatt gaactcggct tagccgccga gcgcgctaac aaacgcgcgc 113640

gcgtgaagaa aaatatgcgc aaattagcca ctcaattggg aaatggatct gtcgactccg 113700

gcataggcat aggcccgtgc ataatgtcgc gcaccatgga ctctggaccc agtcgctggg 113760

acagcgacag tgaggatgac ggggacagcc tgtccacgac gtccaccagc ggagggggga 113820

ctctcacccg agtgtgggtt gggagcgccg ggcctatgta cgaaaacttc tgcgggaacg 113880

gcacccgcca ctcccccacc aacgaccctg gctaccactc gcgggagact ctctgcagcg 113940

gacctccccg tcaggcgccg gcgctaccgc ccaccccgaa gcccgacgag gtaacggtgg 114000

acgtggggcc cggtcccgac gaccaacacg gtccgtacga ggaacctgat cccattcccc 114060

cgcaggaacc cgagccgccg gtgcagattg aggtaaccat caacggcccc ggtggagaag 114120

gcgaggcgga aggagaattt ttctacgacg agtagccgcc aaaactgaat aactatcggg 114180

cttcgtaaac gcgcagacat gccgctgttc cggaagctca tggtttcgcg ctccctggtc 114240

aaggaatgtc tgactctgga cttccggcag ggcgagcgtc tccctacgcg atgcttcctc 114300

ccggtgcccg cggggacgac attccacaga gtctgcgaca cctcgccgct gacggacgaa 114360

gtctcccggc acgtgcagga gcccgtcatg ggcaccggac gggtccagta ctactacttc 114420

gagagcggcc agggcatgat cggcgacaac gcgggcatgg cgcgcatgct cgtgtgcacg 114480

cggtcggcgt acaacggcgg cgacgtcgtc gtgcggtcca cgcggagcag agcagacaag 114540

accgtggtcg cgccctgcca gggcatggcg ctgctgctga gccccttctg cgccttcgac 114600

atcacgccgg tcgagagcgg ctccgcgata ttcgcggagg tcatcgtcac cgcgcccagc 114660

atggactacg tcgaggcggt caccggcacg ggcgaggcgg ccgtgcggat attcaactcg 114720

caccacccgc tctggccgcg acacggctcg aacgtctgct tcgcgctgcg gttgctgcga 114780

gacgtgcgca cggcgagcgg cgtggtcgag cagatgttca tggacggcgg ctggcacacc 114840

gtgctgagga cgtcctgcgg caacaaggtc tgcgtgcccg ccgacctcgt gggccagacg 114900

aacctcgagg aggtgccctt ctgcgacgtg acgcccgaga tcatgcgccg cgcgctggcg 114960

atcgacccgc cgtacgaggc cgtggcgcac ccgcaccgct gcgtgtacgg cgccatggac 115020

gtccggtgcg cgaacgagta cctcgtgtac tgcaccttca agacggagcc gacacggcgc 115080

agcacgtcct cgccgggccc ggacagcccg ctgtcgcccg cgactccgtc gacctcgcgg 115140

gccgcggccg cgcgcgtccc cacgacgccg caggaagtgg cctcgccgac cacgaggctc 115200

ctggagacct gtctgcgcga cgccctcgac ggactctgac ccgaaggacc caccgtccac 115260

tcacattcca ctgccagaca actcaagctt tttctgcatc tacctcgcta ataattgaat 115320

tgttatagga caaacaggcg cactcgagca caatggcgtg ttttatcgaa ttgttagact 115380

ccatcttcaa ccgacgccac cgtaatttcg ggccggagga catgtacagg ccctctgacg 115440

ccccgccccc caaatcgcac acgcctcgca ctccccgcac cccgcggacc cagtgtcccg 115500

gacacccgcg gcgacaaagc tcctctccca tctacggcgc ttatgtggac tccctgccga 115560

ggaacagaaa gcggttccag aatcaacaca gttgtcccgg agattacgag cggtgtcaac 115620

tctcggacac catcagcctg gatgcgacgc tactcacggt tacagtgacg tccatctcca 115680

gcatatccag ctctagtagc tcagactcta tctctctggg gcagtgcaga ctgtccatgg 115740

tgtccgcaac atcaacctcc acctccacaa ccttctcctc ctcggaatga gcgccacact 115800

tatttttgta taatagtttg tattgaacct tagagacatc cacaaatagt taggaagcat 115860

gagtagttca agtagcgaga ccacccctaa gcccaagccc atccctgctc ctcccatgac 115920

tcaggaggag tttaacaaag aagtgaagaa acgaaaagaa cagaaaaagg aaaaatctag 115980

aaccgttgaa cgtgagtcag aaaccgtaac tgtatcttcc gacggatcag agataaaaaa 116040

gacttacgag cgcgagtctg agagaacaac cgaaacagaa aagaacaaca cgtcaaccga 116100

tgataataag cagaacaccc ctgtagagaa accagaggaa accaagcctg cttctactcc 116160

tgaaggtgtg aagccagccg agactcctgc cccgactact gacccccaac ctactacaca 116220

accacccgca gaatcaaacc ctggaagtca acccgcacct gcttcagaac caacccccgc 116280

acctgagcct gcaccggaac ccactcagcc tgcatcagta actcaacccg ctccaacacc 116340

agagccaagt ccagccccta agcctactcc ggcttctgaa ccaaccccag catctgagcc 116400

tacttctgct ccagaaccta caccatccgc agaaccaact cctcaaccaa ctgtagaaac 116460

accaccatct gctccagcac caactcccga ggcccaacca cccgccaaca gcaatcccac 116520

tactgaaact accactggta ccagcacctc ctaagtgagt acgtaagcat ttcggagtaa 116580

cgtcgtagca agcgctagtc cgccgcgagc ggttctcgca agttttttcg ggtaaaaagc 116640

gtacaccgtc gccttgtcgc ggcggtgtac gcttttttca cgcccttttt gcaaaattta 116700

aattgtaccc gcgccggctc taggaaagat ggcgtgcctc agagtgttcc tggcggtgct 116760

cgcgctgtgc gggagcgtgc actcggcgca atggatcggc gagcgcgact tctgcacggc 116820

ccacgcgcag gacgtcttcg cgcggctgca ggtgtggatg cgcattgacc ggaacgtgac 116880

cgccgcggac aacagctcgg cctgcgcgct ggcgatagag acgccgccga gcaacttcga 116940

cgcggacgtc tacgtcgccg cggccggcat aaacgtcagc gtgtccgcga tcaactgcgg 117000

cttcttcaac atgcgccagg tagagacgac gtacaacacg gcacgccggc agatgtacgt 117060

gtacatggac tcctgggacc cttgggtgat cgacgacccc cagccgctct tcagccagga 117120

gtacgaaaac gaaacgcttc cgtacctgct ggaggttctg gagctagcga ggctgtacat 117180

tcgcgtgggc tgcacggtgc ccggagagca gcccttcgag gtgatcccgg ggatcgacta 117240

cccgcacacc ggcatggagt ttctccagca cgttctacgg ccgaaccgcc ggttcgctcc 117300

ggcgaagctg cacatggacc tcgaggtgga ccaccggtgc gtgagcgccg tccacgtgaa 117360

ggcgttcctg caggacgcct gtagcgcccg caaggcgcgg acgccactct acttcgcggg 117420

gcatggctgc aaccatccag atcgccggcc aaaaaaccca gtaccgcgcc ctcagcacgt 117480

atcgtcgccg atctccagga agtgcagcat gcagacggcg cgctaagggc gctcaccgcg 117540

ctgacggcgg ccgtggtgtg cgcgatcgcc atcgcgctcg agcgcggggc ggaggccgac 117600

gccgtggacc ttatccttat aaaattttca atgatatgct agtttttatg cgaccttcct 117660

tagaaaattc ggaattcaaa aatgaaataa aacggcgttt agcacgcata ttattaatac 117720

cgaccaccat ggcaggcgtc cgcagctgcc agaagaaagt cccttctact gcgggctcca 117780

tgtcatttca acggggcaac cggagcatcc agccggcgat gtccgaggcg ttgcagaatg 117840

atttcagcta caacccgcga ccgcctccgc cgagcgcaga agagattgac ttcttctgcg 117900

tggacatgcg caaagtactg atggaaattg aggccaagcc cagcagctcc aagtaccccg 117960

atttcatcca cccggttgac agcagcccgc cgtgcacgcc ggcgcgcaag cgcaacggct 118020

tcggccgcaa ggcactgaac aaaacccagg tgccgcagca ggccaagcgt gacggctact 118080

cccgctaatg cagtccacac acttcacaca ctacatcagc actcaagctt ataatcacta 118140

cacaatgaat cagcccacca cgtgcgaagc acacacataa aatcacccac ctgtcctgat 118200

cgttcccaat actcccaatc accgtgcttt acacgcacgt taatcaccct ttccttcctt 118260

catgcgttcc tgatcgttcc tcctccttaa tcacacacac accccgtaat tttgtacttt 118320

tgtactttaa tttgtacact ttacacactg actttgtact gcctttgtac tttatttttg 118380

tactgaaatt ggacgatact tatctttgta ttcacatcca agttttgcaa attccacagc 118440

cggtagcgaa aagtgaaatc gtaccgtttt aggcttcgat ccccctcccg cgcgaagact 118500

cgccagcatg gactctcgta ggctcgctct tgccgtcgcc ttcggaggcg tcctcgccag 118560

catgacacag cgccgccgcc tggcttctct catcgccagc atcggccaac ggctgatggg 118620

cggcgacggc atgcgtcgcg tcgccgttcg gttgatcgac cagctcatgg ccggaccccc 118680

ggacatcaac gacgaggcct tccagcgcga gatccgcgtg ggcgagctct tccaggcgct 118740

ccaccgcgtg gtcgagcagg cacgccgaga gaagtacttc gaggtctgcg gcgccggcaa 118800

cgacgccgac gcgcccgtcg tcgagatgga caccgcggcc gcacccccgc agccccagcc 118860

cgcgcccctc gtggtcacgc cgcagaacgc gttcatgttc gtgccgcaag gcagccacgt 118920

gcacgtggac gagagcgtgg acccgttctt cggcatgagc ccctccatct tcgggcgcga 118980

cctcccccct cagccgcccg aggagctgct gagcgactac gacccgctca tgagccaggc 119040

cggcgagccg ccgagcccgc ggtcgccctg cgaggccgac ctctggtgct tcgagacgct 119100

cggcgacagc gacagcgatt gagaccgcac cacaccctac ctcacccacc ccacactcca 119160

cctcacctca ccctaacacc cgacacccaa cacttcaacc ggacaatgaa ggagtcccac 119220

atttcactga aggacgcgga tgaagccgca ctcccccaca tgaaggattg gcaacggtca 119280

aacatttcac ctgcaatgaa ggacgatgcg cggtcgcatt ggcctgcgac cgacatcgca 119340

cacatgaagg acacaattgg tttgttaatc cggacaatga aggacaaatt gtttttgtta 119400

atcaggacaa ttagaacaca atcaaatttt tgtacgatca taaaatcgat atttgatgca 119460

catatattag taagtatatt agactaaatt ctccggggag gcaagcagtt ggatacggcg 119520

gggcggggca cgacgtgcac ggagagttcg ggcgggtccc ccttcccccc acccccacgg 119580

caccacgatg cgcctaatct tagcgctcgt ggcctgcttg ttggcggcgc cgatgccgtt 119640

atcgggtcgt tcgacaagca ctccaaacac gtccccctcc gcactcggct cgacgagttc 119700

ggaaccaagc tcggaagacg ctgtggcttc gagcacaacg acaagcacac tcacaagcac 119760

tacaagcaca ctcactatgt ccacaagtgt ggacaccact actacctcgg gcgctacaac 119820

gtccgcaaac agcactcctg cagcgagtgt gagctcctcc acacccgcaa ctaccgaggc 119880

atcgacggca ccaacgacgc cgtcgacgcc gacgacagtg aaggtaacga agggcaagga 119940

agacacgaag gcgtctgcct acctcgtttt actaatcacg ttcatggtca tgaccacgct 120000

cgtgatggtc gtggtcgtgg tcgtggtcgt gtacaaacag ggactctgta actgctgctg 120060

taagtttccc ctgctgcaaa gagctcaagg actacctcga cgaggaggag agcgccgggc 120120

tgtacgacgc cttgacgtgg agccactcag actccggcct ccggctcgtc gtgcgcgcgg 120180

accccagatg atgaggatcg gataagatcg gcgtgttttt cccgcccgtc gcgaacatta 120240

tgcctctaaa tgccgagaat taactgaaat tcaaacacgc tttgggactc aactccgtga 120300

cccacactca accatggctg gcttcctagg cgcattcaga ggcgtgtgct ccgacttatg 120360

gcagtcgctc cgtggacacg gacaccactc ttccagctgc ccgcgacgac gcgccaacag 120420

catggacgac cgcgaccggc gccgacaccg ccaccgcgag atccccaaca gctcggcgtc 120480

gctgaacagc gacccgatgc cgcaacgcag tgcgggtgcg cgccggcact acgactgccg 120540

cccctcggaa aagagcagac actcctccga caagcaccac tcggcggacc gacaccactc 120600

ggcggaccga caccaatcgg cggacaggga cagacaccgt cgcagtcgca agaactacga 120660

ctcgcacccg tcgcgcagga accgcaacta cgagcgggcg gactaccaga gacacccctc 120720

acaaacccac ccagacgccc ccgcgcagac ctcgacgctc aaggtgacct ccctaagcac 120780

cagctgcagc accctgtccc aacatcacta cgagaccccc gaccacatct acgacatccc 120840

ggaagacagt cgcggggcgt cggctccccc tcgcgcggac ctcgcgctcc ccccgctcgc 120900

catgcccaaa tccaagccgc gtcgcacgcg cccggcgtcc atgaacgact gcctgatgaa 120960

gcactgcggc gccggcagac ccaacctcca agacgacata tgcacactat gtactgatat 121020

agagacacag ctgagcgcac tagagaagtc tctggagtca gagctcaact tctatcgtcg 121080

ctacatacaa gacactaaga cattgctcgc cacgcgagca gcaaacatcg gcagcaaagc 121140

tctgatctac accgacgact acaacggcag tggcgacgtc ggcgaaaagg agcactgctc 121200

ggaggagtgc tgcaaagtgg aggaagttct gtgagaaagt gcgtttttct gtaatgtgaa 121260

ataagatagc cttatgtgtg cacagacatg gcgaacaggc tcgtgttttt cgaccccgag 121320

accctagccg aggccgacgg catccccggc tatggggtgt tcgagcccgg caagaagaaa 121380

tgcatcttca caaagatccg caccagcgtc gcactcgcgt gccggtacgc cgtctcggac 121440

ggcggcctca tcgacgagtt cgtcatggct acatacggga ccagacgcgc gtgccggctc 121500

gtccggcacc tgacgatagg cgcggagggc gtgatgaccc ggcccgccag caactgcgcg 121560

ccgcacatgg tgctcatctg cctcagaggc gtggccgccg tgtccagcga ggacatgggc 121620

ttcggtcgct gcatcatgga gcgcggcacc atgttcatgg tcaagtccgc gcacagcgcc 121680

gtcgtctgcg gcaaccccgc ctgcgagctg ctcgtcctct tctacgacta cttcaccccc 121740

atcccccggc cgctctccgg agacgaggtg ctgttcaccc gcgacctcgc gcacgtggac 121800

tacgcccccg agtcggcggt cgtcttcaag atggattaca acctcgagac cgacgtggcc 121860

acgctgtttg tcggggggta catattccgc gccaagggcc tgatgatgga gacgcgcgaa 121920

caagtgggcg acgagtgcga ctgctgccgc cacagctcgc cggtgctcgt catggatcgc 121980

gagaagatga tgtcgtcgct gcgcatgatc cccagcatcg tgcccggcca gcgggagatt 122040

tggcttcgcg agcgcggctg ggccgtcctc gagacggacg cccgcggaca ctgcgagccc 122100

ggcgtcctga ggctggcgct cgccggcctg cggctgttcg caggatgcct gcgctccgtc 122160

gtggggcggc gcgagctgtc gctgttctgc tacggcgtcg ctcccaagtt cggcggggag 122220

ttcgaggacg cgccgcgccc catggagatc gacggttagt tgtttttatc cctgtacata 122280

cgccgcaaac tgaaacttta gggcaccgcg taatagtgca cgaacgccca gtggaccgct 122340

tccgcagcca tggaaaacaa cgacggcaac gaacgcaaca acgaacaccc gcacgttcga 122400

gaattcaagg aggcgtccct gtacgggttt ctggtggcgg ccgcggacgt gaccgtcgaa 122460

gacgtgcacc ggtaccttca gttcggcgcg gacgtgaact acaggggcgc gtacctgtgc 122520

acgccgctgc acgcgtacct gcagtccggc tgcgaaaagc gcctagacgt cgtggacgcg 122580

ctgctggacg ccggcgcaga catcaacgcc aaggagatct gcggtctcac gcccgtgcac 122640

ctgtacgcga gctacgcgga cgtggacgta gagttcatgc gcgggctcat cgagcgcggc 122700

gcgagcgtgt gcggcgagag ctcggtcacg ggctgcctgt actcgtacct gtacacacac 122760

agcgtggacg gcggcgcgcg cctggacgtg gtcgagctgc tcgtgcaggc gggcgcggac 122820

gtgaacgtcc gcggcgaggc gcgcaagacg ccgctgcacg tgcactgcgc gggcttcgag 122880

gtggattcgg acatcgtgga tctgctgctg cgcgcgggcg cggaccccga ggcgctcgac 122940

gaacacgggc tcacgcccgc ggacgtgctc gtgaagtccg tgggcgccaa cgtggagacg 123000

ctgcggctct tcctcgacgc gggcgtgagc gtggccacgt cgcgcgacgc gcgcggacgc 123060

acgccgctgc accaccacgc agactccttc cgggcgagtg cgggcatcgt gcgcgaactg 123120

ctcgccgccg gctgcgacgc ggcggccgcc gacgacctcg gaaacacgcc cctgcacagc 123180

ctcgccacct tctgctcgtg ccggcgctcg gtgctcgacc agctcatcgc cggcggcgcg 123240

gacatcaacg cccgcaacca ctacggccac acctgtctgt actacgcgtc catctacaac 123300

ccctccgtct gctcgcggct catcgccgcg ggtgcggacg tgaccgcgcg cacgccggac 123360

ggacgcacgc cgctctcggg catgatcatg cgcaagcaca cgcgcgccgt gcgcgccgcc 123420

ctggcgacgc ggcctcccgc ggacgccgtc gccgcgtcgc tggacgtcgc agtacagccc 123480

gagcccacgg acgccactcg cgcgtgcgtg cggtacgtgg tgctctgcgg cggcacgctc 123540

tcggcgcgcg tgcggtcgcg acacgcggac ttcgtgcgag agtgcgaaag cgaggtggtc 123600

gtgctcagaa ccaccgtggt ggggctgccc ggcacctcgc tgctggacat cgtgcgtgcg 123660

gcgcagccgc cgccggtact gctctccccg cgcgtgcacc acgtgctgca gaagctgtgc 123720

gtgtacgcgg agttggtgga cgcgcggctg cgcgagatgc ggcacaagac caacctcgtg 123780

gacgcggtgt cgcggctcgt gtgtccgtgc gcgctgccgc cggaggtggt gcgcggcatc 123840

ctcgtgcacg tgccgataga cagcctgcgg cacacgttga ccctcggcgt ggcgcaggcc 123900

tcgcgtttcc ttccctcgca taaatgaaat attatttttt gtggtagacc ggatctcccc 123960

gatggacccc gccggacaac gactgcgcgc gccagggccg tggcgcctga acccgccgac 124020

cgcggccgcg ctggaaagcg cgctgctgcg gcccgcggcg tcggcgggcg ccgaccgctg 124080

cgcgaacgcg cacgtggacc gccgcaacat gggcgtcggc gagggccgcg aggtgcccgc 124140

ggacgtcgag gggctcatga ccgagatcca cctgcggtac ggaatgacgc gcgtccaccg 124200

gaacgttcac ttcgtgcagt tctggcacgg cgagcacgtg cgccggcgcc ccgcgcgaca 124260

cgtgttcacg gtctggatct gcctcagcgg cgaggtgcgc atctacgcag agtgctgcca 124320

ggcggggcac ggcttcgtgc tctgccgaca gatggcggca gggtacatgt tcgtgaccga 124380

acccacggac tcggtcacgg tctcggtgcc gcaccgactg cgcaactcgc ggtcgccggt 124440

gtggctggcg gcggtcttcg ccacgcggca cttcgagccg ctgccgccgc ccatgtacgc 124500

cgtgcccggg cacgtggtgc tcgcgcgcag cgcctccatg ctctgcgact gctggccgtc 124560

ggacccgcgg cgccgcaacg tgatcttcta catgcggctg tcgggcgcga tggtgcgcgt 124620

ggtcgtgccg ggcgcggagc tcgagatcga gtgcacctcg gggttccggc cggaccactt 124680

ctccatcaac gacgagtgcg tgtgctgcga gcgtccgcac gtcgcgcgaa ccgcggtgtg 124740

gacgctggcg gagatttgcc gcggcgccac ggtggtgctc gcgccgccac tgccccgcga 124800

ccgcgccgcg gggctgctcg cggagatccg cctggcctcg ctgcgatggg tgcgcgtgcg 124860

tgcggtccgc agcggcagag aaagcgtggg cccgttcccc tcggtggtgt gggcggcggt 124920

cttctccgcc gttcggctct tcctggacgg aaccgtgcct gccttcccgg cgtgtgtgga 124980

gaatggacgc gcggcgtacg gcatggtgta cgtgcccccg gaggagccgc ggatggacgg 125040

gctctgtgtg ttcccgacgc ccgccgagcc ggcggcgctc ttcgtccgcg gagaccaggt 125100

gcttgaggcc ggcgcggccg ccgccataat cgcggccgct gagaagcgcg tccaggccgc 125160

caatgggtct cctgctgccg cggaggagga cataggtgcg gcggccgatg ccgccgcaga 125220

gagcgtggag caggaccagc gcgtcgagtt cgaccttggg cctgggcctg accccagcca 125280

agaagcgccc gcggacgcgc agcgtgccga ttcggacgac gacaccggct ccgagaccga 125340

gaccggcgac gagagtgtgg gcggcgagga tgacagcgac tcctcctcct cttactcggt 125400

gatgtcggac gacgaaaacg acagcggcga cgagggctgg ggcgactcta gcgactccgg 125460

catcgaggac gacgacggcg gtgtcgccag gccgccgagg aagaagagga ggaagagcgc 125520

gacgtcctcg gcgcagcggc ccagatgctc ggagactgac cggtggtgaa aacataaaaa 125580

taaactgttc aacacttgta ctccgggcac caacactact atccataccc accctccctc 125640

cacacactac aatggcaaac agagaagaga ttgacgcctc cgccgtcatg gctgcctacc 125700

tcgcgagaga gtacgcggcg gctgtagaag aacagctgac gccgcgcgag cgcgatgcgc 125760

tcgaagccct tcgcgtttcc ggcgaggagg tccggtcgcc gctgctgcaa gaactctcga 125820

acgcgggcga gcaccgcgcc aaccccgaaa actcgcacat ccccgccgcc ctcgtctccg 125880

cgcttctcga agcccccacc tcccccggcc gcatggtcac tgcgattgag ctctgcgcgc 125940

agatgggccg ggtatggacg cgcggccgcc agctcgtcga attcatgcgg gtcgtgtacg 126000

tgctcctaga ccgtctgccg cccacggccg acgaggacct cagcacctgg ctgcaggccg 126060

tcgcgcgcgt gcacggcacg cggcgccgcc tgcaccgcgt tctcggcgtc ggggccgtca 126120

tggcaggcgt cggtatgctg ctgctcggcg tgcgcgtgtt gcggcgcaca taacttttta 126180

tctcggctca aactgaaata cgacattgga ctacgaaacc tatgattttg ctcacggccg 126240

cgcgagatag gataataaat aacctttgag caactaacat ggccgatgag agagaggccg 126300

acggcgcgct gttccggtac ctggagagcg aggaccgtcc ggacgtggag cacatgcgcc 126360

ggctgctgga cgagggtgcg gacgtgaact acgcgggccc gcgcgggtac gcgccgctgc 126420

acatgctcat gcgcggcaac ccgctagacc ccgacgcggt gcgactgctg ctcgccgcgg 126480

gcgcggacgt gaacgcgaca tcgctctgcg ggttcacgcc gctgcactcc tacatgtgct 126540

tcgggaccgt gacgccagac acgctgcgtg cgctcatgcg ccacggcgcg agcgtcagcg 126600

acctcgagcg caacatcaac gcgctgatcg agtacttcaa ccgcgacggc tgcatgggcg 126660

gcgcggaggc gaccgtgatc gcactgctgg tggagcacgg cgcgcacgtg aacgccaaag 126720

acgaccttgg acgaacgccg ctgcacatct acctgtccgg cttcttcgtg tcggcaccgg 126780

tggcgctcgc gctgatcgcg ctcggcgcga acccgaacgc cacggacgcg tacgggcgca 126840

cgccactgca cgccttcctg cgctcccgcg acgtggaccc cgctgtgctg aagacgctca 126900

tcgccgcggg cgcagacccg ctcgcgcgcg acatcatccg gcgcacggcg ctgcactacc 126960

actgcgagtc cttcaagacg cgcgctagtg ttatagagac gctggtggcc gccggctgcg 127020

accccgcgag cacagacctg ctcgacaaca cggcgctgca cagcatggcc atgggcagct 127080

cctgccgcgc ctcgctgatc cgcccgctgc tggccgcggg cgtgtccgtg aacgcgcgca 127140

acgcgcggct gcagacgccg ctgcacctcg cggccgtgtt caacccgccg gcctgcgcgc 127200

ggctgctggc cgcgggcgcg gaccccgcgc tcgcggacct ggacgagaca acgccgctgc 127260

tgagcatggt gcggcacaac tgcgcacgcg cgctgcgcac ggcgctgccc ttggcgccgg 127320

acgcgctggt ggccggcgcg gtgaaccgcg tgaacgcgcg cacgccgagc gcggccacgc 127380

gagagtgcgt gatggcgctg gcgctgcgcg gcgcgctgga cctgctgagc gcggagagcg 127440

ttgccaccca cgcggccgcg atccgcgcct gcgaggcgga ggtcgcgctg ctgcggagca 127500

cgcgcctggg cgcgccgccg acgacgctct tcgcgctgct gacaggacga ccgaacacgc 127560

tggtttccgc gaaggcggcg cgacgcgcga tggcggacgt gtgtgtctac cgcgcggcgc 127620

tggccgcgcg cgtggagcgc gtgcgccgca agtcctcgct ggtcgagcgc ctcaccgcca 127680

tggtgtgtcc gtgcgctctg ccgccagagc tagtgacgcg catcctcgcg ctcctgaccg 127740

tggaggaact cgcttgcgca atgcgcaaat aataatgaac tataactagg cttattagag 127800

gcactatttg tgcagagtcg ttagttatag ttagtgtact taccattgga atgtcgaaga 127860

acaaaattct ggtgtgtttg gtaattattc ttacttatac attatacaca gatgcgtatt 127920

gtgttgagta tgaggaaagt gaggaagata aacaacagtg cggtagtagt agtaattttc 127980

ctgcgagttt accgcacatg cttagagaac tcagggcagc gttcggaaag gtaaaaactt 128040

tcttccagat gaaagaccaa ctgaacagta tgctactcac acagtcgctc ctcgacgact 128100

tcaaaggcta cctcgggtgt caggcacttt ctgagatgat acagttttac ttggaagagg 128160

tgatgccgca ggcggaaaat cacgggccgg acatcaaaga gcacgttaac tcgctgggag 128220

aaaaactcaa aacgctgcgt cttcgactgc gtcgctgcca ccgcttcctg ccgtgtgaga 128280

acaagagtaa ggccgtggag caagtcaaac gtgtgttcaa catgctgcag gaacgaggtg 128340

tttacaaggc catgagcgag ttcgacatat tcatcaacta catagaatca tacatgacta 128400

ctaaaatgta aaaatgtata caacttttag ttatcgttcg gattctcgta tcgttctgca 128460

tactatgtat ataaaatgta tattaacata gttacagtta cagttacagc tatattttta 128520

tgctcacaag atgctatata attgaaagga aattgttcac tctctgtcag ggcgccatgg 128580

actttctagg cgccgcgctt cacgactacg ttgccgatgc gcccaaggtc tgcgccgagg 128640

aggtgcggcg gctgctggcc gcaggcgcct ctgtggagta cgcgggcgag ttcgggaaga 128700

ccgcgctgca ccagtacatg ggccgttccg gcgcggaccc cgccgtcgtg cgcgcgctgc 128760

tggacgccgg cgcgcgcgtg gacctcccgg agacctgctg cggctgcacg cccgtgcacc 128820

tctgcctcat ggccgccaat atcgacgtgg aggttctccg catgctcgtc cacgagggcc 128880

gcgtcgagga ctgcggccgc gccgagctcg cctccgtggt gctcaaggag ttcgtggtga 128940

accgcgcctt cgacgagaac gtcagcgagc gagtgatgcg cgttcttgtg gccgcgggcg 129000

cggacgtgaa cgccgccagc gtggtcgacc gcacgccgct gcacgtctgc ctcacgggca 129060

tgtccacgca cccgggcacc atcgccgcgc tgctgcgctt cggcgcggac gtgaacgccg 129120

tggacctctg cggcatgtcg ccgctggcgg tgctcgtgcg ctcgcgcgcg gcgaccgcag 129180

agctggtgcg catgctgctc gacgcgggcg cagacgcaca cgctgtcgac agtcgcctgg 129240

actcgctgct gcaccagcac tttcagtccg cgcgcccgcg gccggaggtg gtgcgcgagc 129300

tcatccgcca cggctgctcg ccgcgggcgc ggaaccgaat cggcaacacg ccgctgcacg 129360

aggccgcaaa acactcctcc tgcaaacact cgctggtggg gccgctgctg gctgccggcg 129420

cgagcgtgga cgcgcgaaat aacacgggca ggacgccgct ccacttggcg gtggcgtcca 129480

acccgcgcgc gtgccgccgg ctgatcgcgc ttggggcgga cgtggtcgcg cgcagttacg 129540

cgggcgtcac gccgctggcg cagctgatcg cggacaataa ctccgcgctg gtgaccgcgg 129600

cgctgaacac gcagcccgag ccgcgggccg tggcagagtc gctgcgagcg accacgcccg 129660

tcggcgagac agcgtgctcg cggctctgtg tggcgtacgt ggtggcgcgc gcgccgagcg 129720

aggtcctcgg cgagcccgag cgcgccctgc acgcggcctt cgtggcggag tgcttagcgg 129780

aggtagcggc catacacgcc gtgcgctgcg gcacacctcc ggtctcgctg ctggagatcc 129840

tggtggccgc gcgcccgccg cggagcctgc tctcgcgccg cgcgtggcgg ctggccagcc 129900

ggacgacagt ttaccgcgcg ccgctccgtg cacgcatcgc ggccatgcgc catcgctcgc 129960

gactggtgga gcgcgcgctg cgcacgctgc gcggctgcgt gctcccgcgc gaggtgctgg 130020

agcgcgtgct gcggtgtctg tccacacagg acctgcgggc ctccggactg gccgagtagc 130080

tttttctgag ataagtgaat aaacatggtg ggattcgatc ggcgccgcca acgccacgcc 130140

atggacgccg ccgagatgga ggatctcgac atcaacgcgg agtcggcgct gtacgactac 130200

ttcatcctga acgcggacag agcccgcgtg ggcgaggtgg ttatgcttct cgcacagggc 130260

gcggaaataa actacgcgga cagcttcgac aagacgccgc tgcacctgta cttgcacacg 130320

cgacacccgc gctcggacgt gattctggcg ctgatggagg cgggcgcggt cgtggacacg 130380

ccggagcgct gctgcggcgc gaccgcggcg cacctgtaca tcctcaacgc ggccaaggtc 130440

gacctgtcgg tgctggaggc catgctgacc tggggcgtgc gccagaacga ccagcactcg 130500

gagcgggtgc tctcgagctt gttgcgcgag tacgtggtga cccgcgccta ctcggatcag 130560

accgagccga tcatggactt gctcatcggc atgggcgccg acgtggacat gccggtcggc 130620

gtgagtcgca cggggctgca cgcctgcctt acgggcctga acgcgaaccc gtgcatgatt 130680

cgcgcgctgc ttcggcgcgg cgccagcgtg accgcaaaag acacctacga gatgacgccg 130740

ctggcggtgc tgctgaagtc cgcgagcgcg acgccggagc tcgtgcgcat cctcgtggag 130800

gcaggctccg acgtgagcgc caccgacttc cgcctcaacg gcatgctgca ccagcacgcg 130860

cagtccacgc gcccgcgcgc gagcgtcatg cgcgagctca tccggctggg gtgcagccca 130920

gcggccaaaa acatgtttgg gaacacgccg atgcacatgc tggccatgga aagctcctgc 130980

cgccgctcac tgatcctccc gctgctggag gcagggcttt ccgtgaacga ggagaacccg 131040

cactacggca ccgtgcctct gcacgtggcc tcggggtacg acaacacgca gggctgcctc 131100

aagctcctcc gggatggagg agaccccacc gtcgtgtcgg ccgccggacg cacgccgctc 131160

tcgaacatgc tcgtcaaacg caaccacgtg gcggtcgccg gcgcgctgtc gacgcacccg 131220

agcgcggcag tagtcgtgca ggctctcgag caggctctcg agaacgtgct gaacgccggg 131280

cccagcgagg cctcgcggct cgccgtggcc tttgtggtgg cgcgcgccgg cgcatccgcg 131340

ctaccggagg ccgtgcgccg tctgcacgag ggctttgtcg ccgactgcga gcgcgaagtc 131400

gagctgcttt cccgcaccat gctcggcaca ccggccgtga gcgcgctggt cgtgctggtc 131460

agcaaggagg tctttggcac tgttatctcc tcgcgtgcgc tgcgcgtcgt gcgggaggtc 131520

cgcgtgtacg caaggccgct ccgcgaggcg ctcataaatc tgcgccacaa atgccgctta 131580

gtttccagcc ttaaaaggca ggtgggacct tgctcgctgc ccggcgaact ggtggagcgc 131640

gtgctcgcga ccgtgccact gaccgacttg cgccgctcgt gcggccgccg cgcgcccgag 131700

taactgcccg tcccgttgct acgcgactcg agactgcccg ctgtttttct ttccccgttt 131760

cttcttatta ggagttgttg cccgcctcca tgatcctcgc acgcgccggc gggcaacctc 131820

gcacgcccgc ggcggccgcg ggcgccgccg aggacggcga gcacagtgat cgccggaagc 131880

gcaagcgcaa gacgcccaac tgcgaagacg ccgacaactc cgacgacgag ctagcgcaga 131940

cgccgtgcga ccgcgagtgg ccggactgtc gcgcgagctc gatcacgagc tccgactcgg 132000

tctctctcgg cgacgagatc tacctgcgat acgtggcctc gcaggtggac ttcgcgcaga 132060

cctgggcccc gccagtgcgg ctgctgcgct ccttcgggaa cttctcgaag gaaacgctca 132120

accgcatgtc gcggcgcggg tacgtgaacc gctcctactt ccagatggcg cacgcgcgct 132180

tctcgcccac caacgacgac atgtaccaca tggccacggg cgggtacggc atcgtgttcc 132240

gcttcgaccg ctacgtggtt aagtacgtct tcgagcaccg caacggcatg tccgagatgg 132300

acgcctctac ggagtacacg gtgccgcggt tcctgcgcaa taacctcaag ggcgacgagc 132360

gcgagttcgt ggtctgcgcg ctgcccatgg ggctgaacta ccggctgggc ttcctgcact 132420

cgctgtaccg gcgcgtgctg cacacgctgc tgctgctcat gcgcgtggag gaaggccagc 132480

ggccctcggt ggagatgtcc aagaagccgc tgctgcgctg gttcgaggcg cgcaaggaca 132540

gcgagtcctt cgtgcgcctg atctcgtact tctacccctc ggccgtgcag agcaacgtga 132600

acctgatcaa caacttccac cacctggtgc acttcttcga gcacgagaag cgcgcgcggt 132660

acgtgttcga ccgcggggcc gtgatcgtgt tccctctggc gcgcgggtcc gcggactcga 132720

tctcgccgga ggcggcggcg gcgctgggct tcgcgccgca ctcggagttc ctcaagttcg 132780

tgttcctgca gatcgcgctg ctgtacctga agatctacga gctcccgggc tgcacgaact 132840

tcctgcacgt ggacctgaag cccgacaacg tgctcatctt cgacagtgcg cgcgcgctca 132900

gcgtgaccgc ggccggcgcg actttccgct tcgaggagcc cgtgcgcgcg gcgctgaacg 132960

acttcgactt cgcgcgcgtg gccaccatcg agaaccgcaa gatcgcgggc agcgtccgcg 133020

tgccgcagaa ctggtactac gacttccact tcttcgcgca cacgctgctg cgcgcgtacc 133080

cgcacatcgc cgcggaggac ccgggcttcc acgcgctgct ctcggagctc acggtctcgt 133140

gctcgcgcgg gacctgcgac cgcttccggc tgcgcgtgtc ctcgccgcac cccatcgagc 133200

acctcgcgcg gctggtgcgc cgcgacgtct tctcccgctg gataaatgcc gccgcggacg 133260

cccccgacgc cgccgcactc tcctgagccc acgcccgcgg cgccgggctc gctgtacgac 133320

gtcttcctcg cgcgcttcct gcgccggctg gccgcgcgcg cggcgccggc ctcggccgcc 133380

tgcgccgtgc gcgtgggtgc ggtgcgcggc cgcctgcgga actgcgagct agtggtgctg 133440

aaccgctgcc acgcggacgc ggccggcgcg ctcgcgctag cctccgcggc gctcgccgag 133500

acgctggcgg agctcccgcg cgcggacaag ctcgccgtcg cgctcgagct gggcgtggac 133560

cccgagcacc cggagctgac gccggacccc gcctgcgcag gcgagagcgc actcgcacag 133620

aacatcgaca tccagacgct ggacctgggc gactgcggag accccaaagg ccgccgactg 133680

cgcgtggcgc tggtgaacag cggccacgcg gccgcgaact gcgcgctcgc gcgcgtggcg 133740

accgcgctga cgcgccgcgt gcccgcgagc cggcacggcc tcgcggaggg cggcacgccg 133800

ccgtggacgc tgctgctggc ggtggccgcg gtgacggtgc tcggcgtggt ggcggtttca 133860

ctgctgcggc gcgcgctgcg ggtacgctac cgcttcgcgc ggccggccgc gctgcgcgcg 133920

tagccgcgca aaatgtaaat tataacgccc aacttttaag ggtgaggcgc catgaagttg 133980

ctcgtcggca tactagtagc cgtgtgcttg caccagtatc tgctgaacgc ggacagcaac 134040

acgaaaggat ggtccgaagt gctgaaaggc agcgagtgca agcctaggcc gattgttgtt 134100

cctgtaagcg agacgcaccc agagctgact tctcagcggt tcaacccgcc gtgtgtcacg 134160

ttgatgcgat gcggcgggtg ctgcaacgac gagagcttgg aatgcgtccc cacggaagaa 134220

gtaaacgtga cgatggaact cctgggggcg tcgggctccg gtagtaacgg gatgcaacgt 134280

ctgagcttcg tagagcataa gaaatgcgat tgtagaccac gattcacaac cacgccaccg 134340

acgaccacaa ggccgcccag aagacgccgc tagaactttt tatggaccgc agatccaaac 134400

gatgatgcga tcaggtcatg cggaagaagg cgccacggag caaagtgaaa aaggaccgcc 134460

tagcagtcga gaccctcccg ccgcagccgc ggacacccca cacccgcctt ccacccgcca 134520

gacgccaaca ccgcagccaa caagcatgca cccctcgccg cgcaggctgc tcggcgcgct 134580

cgcgctggtg gcgctgggct tcctcctcgg cgggctcttc cgccccgcgg cgccgccgct 134640

gccggccgcc ctcgtggagg cgggccccgt ccgcgcgaac ggctccgcct cggtgacctg 134700

cctgaccgtc ggcggcgacg ggcggcacat ggcggtggtc gcgcacggcg gcgggacgct 134760

ctcgccggtg tacccgctcg ccgccggcat gcacgcgacc ttcgcctcgc tgcgcaaggg 134820

cgcgctgctg ctgaacgtcg cgaccgtgca catctacgac gtgcgcgcgc tcggccggga 134880

gttcgagctg acctgcgtcg cggtggcggg cggctacaac gcggcctggg cggccgcgcg 134940

gcccgcggcc gagtggcgcc gccagctggc gcggatgcac cgctcggagc tgtgaccctc 135000

tccctcccgg tctcccatcc gtttttgtaa tcggccttag tagattagac cagcatcccg 135060

cgcccttgtc cgagaacaag taacagtaac cgttacctca ctcgccactc ctcggaataa 135120

tagaacgaga gaacgagaga acgagttaac cgttgctcac tcgctcactc ggtgtgagag 135180

aacaagtaac gttgctcact cgctcactcg gtgtgagaga acaagagaac gagtagctgt 135240

tgctaactca atcacccctc ggagtaagag aacaagagca gtcaactacc cactcagtct 135300

tggatgagag gcagaggacg agttgacgag ttgaacagtt aatcctcact cactcagagc 135360

gagagagcga gagagtggag gacgagttaa caagtcaatc ctcactcaga gtgagagagt 135420

gagagagtgg aggacgagtt aatggttaac agttatcacc actcagagtg agagcggagg 135480

acgagtcaac cactcgctcg cccactccga gttagagagg gaaccagtgc gagttaacgc 135540

gcacacgagc gagagaacgt aaactcgctc gcgcgctcgc tcggctaacc gtcggcctct 135600

cccaaaactc ttcgtaaaac tttcccgatg acagttcacc ctccaaaact ttgtaaaact 135660

aaactgttcg gaggtcggtc tgctgcctct ctaactctcc gtaaaacgtt tgtaaactgt 135720

cggaggtcgg tgacccgctc aaccgtccgc gaaaactttt cgcaggcagt gtctgcctct 135780

ctcggactct ccgcaaacac tttcgcggaa cctcggaggg tggtcgacct ctctccaaac 135840

tcttgcaaaa ctttttcgcg gaaccgttgg aggccagtcc tccctccaaa ctctttgtaa 135900

aatcttttcg aggccagtcc tcctctccaa aacgttccgc aaaatctttg ggaggtcggc 135960

ctctcctctc cagaacgttc cgtaaaactc ttggaggccg cccgcggcac gcgaggcgga 136020

ggatccgagg tgtcgacctc cctcaaaaac tttgtaaaaa ctttttataa aactttccgc 136080

ggaacctcgg agagtaggtc gacctctctc aaaactctta taaaactttt ccgcggaacc 136140

gttggaaggt aggtcgacct ctctcaaaac tcttataaaa cttttccgcg gaaccgttgg 136200

aaggtaggtc gacctctctc aaaactctta taaaactttt ccgcggaacc gttggaggca 136260

ggtcggcctc tcaaactctt tgcgagaact cttcgcgaga actcttcgat aactttagga 136320

ggtcaggtcg acctcccaaa acttttgcga gaactctctg taaaacttta ggaggtcggt 136380

acctccctca aaacttttta taaaactttt cgcggaacct ccggagacgg gccgccgccg 136440

cgaccgcggg agcggagagg ccgacctccc gagacgttcc gcgttaccgt cggggtaggc 136500

gtcctctcga gaacgccaaa agacttcgtg caaaaacttt tcggaggggc gcggagggcg 136560

ggtcagctcc cgcgaactcc cgcagaacct tttcgcgcga ccgcgaaggc cggccgcctc 136620

tcccacactc tcaagagctt ttcggaggag aggaagggca ggtcgccccc acctccgacg 136680

ctttgtaaaa acgtttacgc ggaacctcgg aggcaggtcg cctccctcga aaactcctcg 136740

cgaaaccttt aaaaactttt tgcgaaaact tttcggagga tgtcggaggg cgggcggctc 136800

ttccaaacct ccgcagaacc ttttcgcgca accgttggaa gacaggtcgg cctctctcga 136860

aaacttttaa aactttgtaa acgcgttggc gggaccgtcg cgggagagcg gccgcccgcg 136920

gcacgcgaga ggaggaaccg ttggaaggca gtcggcctct cccgaaaact ttttataaaa 136980

acttttccgc ggaaccgttg gaggcaggtc ggcctctctc agagtctaaa aactttttgc 137040

gggactcgga cggcgcggtc acccgaccac ctgactcctg tctcacccgt actacttgga 137100

cttctgtttc cctgactccc gactccctga cctcccgact ccctgactcc cgactccctg 137160

actcccgact ccctgactcc cgactccctg actcccgact ccctgactcc cgactccctg 137220

actcccgact ccctgactcc cgactccctg actcccgact ccctgactcc cgactccctg 137280

actcccgact ccctgactcc cgactccctg actcccgact ccctgactcc cgactccctg 137340

actcccgact ccctgactcc ctgactccag agcgaggtct cgcggctgcg gggtgccgcc 137400

tccgcggagt cgcgttcccg cggacgcccg tcctcgaaag cattcagcag ttccagcctc 137460

tgccgtagct cctcccgcag gaactcctgg tccgcgttcg tcgcggcacc gcggctcagc 137520

cgccgcggga gccggccgcc gcccgcgaag ccgcggatcc 137560